# EUROPEAN PATENT APPLICATION

(11) **EP 2 338 990 A2**
(43) Date of publication of application: **29.06.2011**
(21) Application number: 10012326.4
(22) Date of filing: 09.06.2006
(51) Int. Cl.: C12N 15/10, C07H 21/00, C40B 40/06, C40B 50/06, C07B 61/00

(54) **Methods for synthesis of encoded libraries**

(30) Priority: 09.06.2005 US 689466 P; 28.10.2005 US 731041 P
(62) Divisional of application: 06784718.6
(71) Applicant: Praecis Pharmaceuticals Inc., Waltham, MA 02451-1420 (US)
(72) Inventor: Morgan, Barry, Franklin, MA 02038 (US); Hale, Stephen, Belmont, MA 02478 (US); Arico-Muendel, Christopher C., West Roxbury, MA 02132 (US); Clark, Matthew, Cambridge, MA 02139 (US); Wagner, Richard, Cambridge, MA 02138 (US); Kavarana, Malcolm J., Fairfax, VA 22033-4644 (US); Creaser, Steffen, Phillip, Cambridge, MA 02139 (US); Franklin, George J., Lynn, MA 01902 (US); Centrella, Paolo A., Acton, MA 01720 (US); Israel, David I., Concord, MA 01742 (US); Gefter, Malcolm L., Lincoln, MA 01773 (US); Benjamin, Dennis, Redmond, WA 98052 (US); Hansen, Nils Jakob Vest, San Francisco, CA 94114 (US); Acharya, Raksha A., Bedford, MA 01730 (US)
(74) Representative: Lock, Graham James

(57) **Abstract**

The present invention provides a method of synthesizing libraries of molecules which include an encoding oligonucleotides tag.

SEQUENCE LISTING

<110> Praecis Pharmaceuticals, Inc.

<120> METHODS FOR SYNTHESIS OF ENCODED
  LIBRARIES

<130> PPI-156CPPC

<150> 11/015458
<151> 2004-12-17

<150> 60/530854
<151> 2003-12-17

<150> 60/540681
<151> 2004-01-30

<150> 60/553715
<151> 2004-03-15

<150> 60/588672
<151> 2004-07-16

<150> 60/689466
<151> 2005-06-09

<150> 60/731041
<151> 2005-10-28

<160> 890

<170> FastSEQ for Windows Version 4.0

<210> 1
<211> 9
<212> DNA
<213> Artificial Sequence

<220>
<223> synthetic construct

<400> 1
gcaacgaag                                                                9

<210> 2
<211> 9
<212> DNA
<213> Artificial Sequence

<220>
<223> synthetic construct

<400> 2
tcgttgcca                                                                9

<210> 3
<211> 9
<212> DNA
<213> Artificial Sequence

<220>
<223> synthetic construct

<400> 3

gcgtacaag                                                                9

<210> 4
<211> 9
<212> DNA
<213> Artificial Sequence

<220>
<223> synthetic construct

<400> 4
tgtacgcca                                                                9

<210> 5
<211> 9
<212> DNA
<213> Artificial Sequence

<220>
<223> synthetic construct

<400> 5
gctctgtag                                                                9

<210> 6
<211> 9
<212> DNA
<213> Artificial Sequence

<220>
<223> synthetic construct

<400> 6
acagagcca                                                                9

<210> 7
<211> 9
<212> DNA
<213> Artificial Sequence

<220>
<223> synthetic construct

<400> 7
gtgccatag                                                                9

<210> 8
<211> 9
<212> DNA
<213> Artificial Sequence

<220>
<223> synthetic construct

<400> 8
atggcacca                                                                9

<210> 9
<211> 9
<212> DNA
<213> Artificial Sequence

<220>
<223> synthetic construct

<400> 9
gttgaccag                                                                9

<210> 10
<211> 9
<212> DNA
<213> Artificial Sequence

<220>
<223> synthetic construct

<400> 10
ggtcaacca                                                                9

<210> 11
<211> 9
<212> DNA
<213> Artificial Sequence

<220>
<223> synthetic construct

<400> 11
cgacttgac                                                                9

<210> 12
<211> 9
<212> DNA
<213> Artificial Sequence

<220>
<223> synthetic construct

<400> 12
acgctgaac                                                                9

<210> 13
<211> 9
<212> DNA
<213> Artificial Sequence

<220>
<223> synthetic construct

<400> 13
cgtagtcag                                                                9

<210> 14
<211> 9
<212> DNA
<213> Artificial Sequence

<220>
<223> synthetic construct

<400> 14
gactacgca                                                                9

<210> 15
<211> 9
<212> DNA
<213> Artificial Sequence

<220>
<223> synthetic construct

<400> 15
ccagcatag                                                                9

<210> 16
<211> 9
<212> DNA
<213> Artificial Sequence

<220>
<223> synthetic construct

<400> 16
atgctggca                                                                9

<210> 17
<211> 9
<212> DNA
<213> Artificial Sequence

<220>
<223> synthetic construct

<400> 17
cctacagag                                                                9

<210> 18
<211> 9
<212> DNA
<213> Artificial Sequence

<220>
<223> synthetic construct

<400> 18
ctgtaggca                                                                9

<210> 19
<211> 9
<212> DNA
<213> Artificial Sequence

<220>
<223> synthetic construct

<400> 19
ctgaacgag                                                                9

<210> 20
<211> 9
<212> DNA
<213> Artificial Sequence

<220>
<223> synthetic construct

<400> 20
acgacttgc                                                                9

<210> 21
<211> 9
<212> DNA
<213> Artificial Sequence

<220>
<223> synthetic construct

<400> 21
ctccagtag 9

<210> 22
<211> 9
<212> DNA
<213> Artificial Sequence

<220>
<223> synthetic construct

<400> 22
actggagca 9

<210> 23
<211> 9
<212> DNA
<213> Artificial Sequence

<220>
<223> synthetic construct

<400> 23
taggtccag 9

<210> 24
<211> 9
<212> DNA
<213> Artificial Sequence

<220>
<223> synthetic construct

<400> 24
ggacctaca 9

<210> 25
<211> 9
<212> DNA
<213> Artificial Sequence

<220>
<223> synthetic construct

<400> 25
gcgtgttgt 9

<210> 26
<211> 9
<212> DNA
<213> Artificial Sequence

<220>
<223> synthetic construct

<400> 26
aacacgcct 9

<210> 27
<211> 9
<212> DNA
<213> Artificial Sequence

<220>
<223> synthetic construct

<400> 27
gcttggagt                                                                9

<210> 28
<211> 9
<212> DNA
<213> Artificial Sequence

<220>
<223> synthetic construct

<400> 28
tccaagcct                                                                9

<210> 29
<211> 9
<212> DNA
<213> Artificial Sequence

<220>
<223> synthetic construct

<400> 29
gtcaagcgt                                                                9

<210> 30
<211> 9
<212> DNA
<213> Artificial Sequence

<220>
<223> synthetic construct

<400> 30
gcttgacct                                                                9

<210> 31
<211> 9
<212> DNA
<213> Artificial Sequence

<220>
<223> synthetic construct

<400> 31
caagagcgt                                                                9

<210> 32
<211> 9
<212> DNA
<213> Artificial Sequence

<220>
<223> synthetic construct

<400> 32
gctcttgct                                                                9

<210> 33
<211> 9
<212> DNA
<213> Artificial Sequence

<220>
<223> synthetic construct

<400> 33
cagttcggt                                                                9

<210> 34
<211> 9
<212> DNA
<213> Artificial Sequence

<220>
<223> synthetic construct

<400> 34
cgaactgct                                                                9

<210> 35
<211> 9
<212> DNA
<213> Artificial Sequence

<220>
<223> synthetic construct

<400> 35
cgaaggagt                                                                9

<210> 36
<211> 9
<212> DNA
<213> Artificial Sequence

<220>
<223> synthetic construct

<400> 36
tccttcgct                                                                9

<210> 37
<211> 9
<212> DNA
<213> Artificial Sequence

<220>
<223> synthetic construct

<400> 37
cggtgttgt                                                                9

<210> 38
<211> 9
<212> DNA
<213> Artificial Sequence

<220>

<223> synthetic construct

<400> 38
aacaccgct                                                                9

<210> 39
<211> 9
<212> DNA
<213> Artificial Sequence

<220>

<223> synthetic construct

<400> 39
cgttgctgt                                                                9

<210> 40
<211> 9
<212> DNA
<213> Artificial Sequence

<220>
<223> synthetic construct

<400> 40
agcaacgct                                                                9

<210> 41
<211> 9
<212> DNA
<213> Artificial Sequence

<220>
<223> synthetic construct

<400> 41
ccgatctgt                                                                9

<210> 42
<211> 9
<212> DNA
<213> Artificial Sequence

<220>
<223> synthetic construct

<400> 42
agatcggct                                                                9

<210> 43
<211> 9
<212> DNA
<213> Artificial Sequence

<220>
<223> synthetic construct

<400> 43
ccttctcgt                                                                9

<210> 44
<211> 9
<212> DNA
<213> Artificial Sequence

<220>
<223> synthetic construct

<400> 44
gagaaggct                                                                9

<210> 45
<211> 9
<212> DNA
<213> Artificial Sequence

<220>

<223> synthetic construct

<400> 45
tgagtccgt                                                                9

<210> 46
<211> 9
<212> DNA
<213> Artificial Sequence

<220>

<223> synthètic construct

<400> 46
ggactcact                                                                9

<210> 47
<211> 9
<212> DNA
<213> Artificial Sequence

<220>
<223> synthetic construct

<400> 47
tgctacggt                                                                9

<210> 48
<211> 9
<212> DNA
<213> Artificial Sequence

<220>
<223> synthetic construct

<400> 48
cgttagact                                                                9

<210> 49
<211> 9
<212> DNA
<213> Artificial Sequence

<220>
<223> synthetic construct

<400> 49
gtgcgttga                                                                9

<210> 50
<211> 9
<212> DNA
<213> Artificial Sequence

<220>
<223> synthetic construct

<400> 50
aacgcacac                                                                9

<210> 51
<211> 9
<212> DNA
<213> Artificial Sequence

<220>
<223> synthetic construct

<400> 51
gttggcaga                                                                9

<210> 52
<211> 9
<212> DNA
<213> Artificial Sequence

<220>
<223> synthetic construct

<400> 52
tgccaacac                                                                9

<210> 53
<211> 9
<212> DNA
<213> Artificial Sequence

<220>
<223> synthetic construct

<400> 53
cctgtagga                                                                9

<210> 54
<211> 9
<212> DNA
<213> Artificial Sequence

<220>
<223> synthetic construct

<400> 54
ctacaggac                                                                9

<210> 55

<211> 9
<212> DNA
<213> Artificial Sequence

<220>
<223> synthetic construct

<400> 55
ctgcgtaga                                                                9

<210> 56
<211> 9
<212> DNA
<213> Artificial Sequence

<220>
<223> synthetic construct

<400> 56
tacgcagac                                                                9

<210> 57
<211> 9
<212> DNA
<213> Artificial Sequence

<220>
<223> synthetic construct

<400> 57
cttacgcga                                                              9

<210> 58
<211> 9
<212> DNA
<213> Artificial Sequence

<220>
<223> synthetic construct

<400> 58
gcgtaagac                                                              9

<210> 59
<211> 9
<212> DNA
<213> Artificial Sequence

<220>
<223> synthetic construct

<400> 59
tggtcacga                                                              9

<210> 60
<211> 9
<212> DNA
<213> Artificial Sequence

<220>
<223> synthetic construct

<400> 60
gtgaccaac                                                              9

<210> 61
<211> 9
<212> DNA
<213> Artificial Sequence

<220>
<223> synthetic construct

<400> 61
tcagagcga                                                              9

<210> 62
<211> 9
<212> DNA
<213> Artificial Sequence

<220>
<223> synthetic construct

<400> 62
gctctgaac                                                              9

<210> 63
<211> 9
<212> DNA
<213> Artificial Sequence

<220>
<223> synthetic construct

<400> 63
ttgctcgga                                                              9

<210> 64
<211> 9
<212> DNA
<213> Artificial Sequence

<220>
<223> synthetic construct

<400> 64
cgagcaaac                                                              9

<210> 65
<211> 9
<212> DNA
<213> Artificial Sequence

<220>
<223> synthetic construct

<400> 65
gcagttgga                                                              9

<210> 66
<211> 9
<212> DNA
<213> Artificial Sequence

<220>
<223> synthetic construct

<400> 66
caactgcac                                                              9

<210> 67
<211> 9
<212> DNA
<213> Artificial Sequence

<220>
<223> synthetic construct

<400> 67
gcctgaaga                                                              9

<210> 68
<211> 9
<212> DNA
<213> Artificial Sequence

<220>
<223> synthetic construct

<400> 68
ttcaggcac                                                              9

<210> 69
<211> 9
<212> DNA
<213> Artificial Sequence

<220>
<223> synthetic construct

<400> 69
gtagccaga 9

<210> 70
<211> 9
<212> DNA
<213> Artificial Sequence

<220>
<223> synthetic construct

<400> 70
tggctacac 9

<210> 71
<211> 9
<212> DNA
<213> Artificial Sequence

<220>
<223> synthetic construct

<400> 71
gtcgcttga 9

<210> 72
<211> 9
<212> DNA
<213> Artificial Sequence

<220>
<223> synthetic construct

<400> 72
aagcgacac 9

<210> 73
<211> 9
<212> DNA
<213> Artificial Sequence

<220>
<223> synthetic construct

<400> 73
gcctaagtt 9

<210> 74
<211> 9
<212> DNA
<213> Artificial Sequence

<220>
<223> synthetic construct

<400> 74
cttaggctc 9

<210> 75
<211> 9
<212> DNA
<213> Artificial Sequence

<220>
<223> synthetic construct

<400> 75
gtagtgctt                                                                9

<210> 76
<211> 9
<212> DNA
<213> Artificial Sequence

<220>
<223> synthetic construct

<400> 76
gcactactc                                                                9

<210> 77
<211> 9
<212> DNA
<213> Artificial Sequence

<220>
<223> synthetic construct

<400> 77
gtcgaagtt                                                                9

<210> 78
<211> 9
<212> DNA
<213> Artificial Sequence

<220>
<223> synthetic construct

<400> 78
cttcgactc                                                                9

<210> 79
<211> 9
<212> DNA
<213> Artificial Sequence

<220>
<223> synthetic construct

<400> 79
gtttcggtt                                                                9

<210> 80
<211> 9
<212> DNA
<213> Artificial Sequence

<220>
<223> synthetic construct

<400> 80
ccgaaactc                                                                9

<210> 81
<211> 9
<212> DNA
<213> Artificial Sequence

<220>
<223> synthetic construct

<400> 81
cagcgtttt                                                              9

<210> 82
<211> 9
<212> DNA
<213> Artificial Sequence

<220>
<223> synthetic construct

<400> 82
aacgctgtc                                                              9

<210> 83
<211> 9
<212> DNA
<213> Artificial Sequence

<220>
<223> synthetic construct

<400> 83
catacgctt                                                              9

<210> 84
<211> 9
<212> DNA
<213> Artificial Sequence

<220>
<223> synthetic construct

<400> 84
gcgtatgtc                                                              9

<210> 85
<211> 9
<212> DNA
<213> Artificial Sequence

<220>
<223> synthetic construct

<400> 85
cgatctgtt                                                              9

<210> 86
<211> 9
<212> DNA
<213> Artificial Sequence

<220>
<223> synthetic construct

<400> 86
cagatcgtc                                                              9

<210> 87
<211> 9
<212> DNA
<213> Artificial Sequence

<220>
<223> synthetic construct

<400> 87
cgctttgtt                                                                9

<210> 88
<211> 9
<212> DNA
<213> Artificial Sequence

<220>
<223> synthetic construct

<400> 88
caaagcgtc                                                                9

<210> 89
<211> 9
<212> DNA
<213> Artificial Sequence

<220>
<223> synthetic construct

<400> 89
ccacagttt                                                                9

<210> 90
<211> 9
<212> DNA
<213> Artificial Sequence

<220>
<223> synthetic construct

<400> 90
actgtggtc                                                                9

<210> 91
<211> 9
<212> DNA
<213> Artificial Sequence

<220>
<223> synthetic construct

<400> 91
cctgaagtt                                                                9

<210> 92
<211> 9
<212> DNA
<213> Artificial Sequence

<220>
<223> synthetic construct

<400> 92
cttcaggtc                                                                9

<210> 93
<211> 9

<212> DNA
<213> Artificial Sequence

<220>
<223> synthetic construct

<400> 93
ctgacgatt 9

<210> 94
<211> 9

<212> DNA
<213> Artificial Sequence

<220>
<223> synthetic construct

<400> 94
tcgtcagtc 9

<210> 95
<211> 9
<212> DNA
<213> Artificial Sequence

<220>
<223> synthetic construct

<400> 95
ctccacttt 9

<210> 96
<211> 9
<212> DNA
<213> Artificial Sequence

<220>
<223> synthetic construct

<400> 96
agtggagtc 9

<210> 97
<211> 9
<212> DNA
<213> Artificial Sequence

<220>
<223> synthetic construct

<400> 97
accagagcc 9

<210> 98
<211> 9
<212> DNA
<213> Artificial Sequence

<220>
<223> synthetic construct

<400> 98
ctctggtaa 9

<210> 99
<211> 9
<212> DNA
<213> Artificial Sequence

<220>
<223> synthetic construct

<400> 99
atccgcacc                                                                9

<210> 100
<211> 9
<212> DNA
<213> Artificial Sequence

<220>
<223> synthetic construct

<400> 100
tgcggataa                                                                9

<210> 101
<211> 9
<212> DNA
<213> Artificial Sequence


<220>
<223> synthetic construct

<400> 101
gacgacacc                                                                9

<210> 102
<211> 9
<212> DNA
<213> Artificial Sequence

<220>
<223> synthetic construct

<400> 102
tgtcgtcaa                                                                9

<210> 103
<211> 9
<212> DNA
<213> Artificial Sequence

<220>
<223> synthetic construct

<400> 103
ggatggacc                                                                9

<210> 104
<211> 9
<212> DNA
<213> Artificial Sequence

<220>
<223> synthetic construct

<400> 104
tccatccaa                                                                9

<210> 105
<211> 9
<212> DNA
<213> Artificial Sequence

<220>
<223> synthetic construct

<400> 105
gcagaagcc                                                                9

<210> 106
<211> 9
<212> DNA
<213> Artificial Sequence

<220>
<223> synthetic construct

<400> 106
cttctgcaa                                                                9

<210> 107
<211> 9
<212> DNA
<213> Artificial Sequence

<220>
<223> synthetic construct

<400> 107
gccatgtcc                                                                9

<210> 108
<211> 9
<212> DNA
<213> Artificial Sequence

<220>
<223> synthetic construct

<400> 108
acatggcaa                                                                9

<210> 109
<211> 9
<212> DNA
<213> Artificial Sequence

<220>
<223> synthetic construct

<400> 109
gtctgctcc                                                                9

<210> 110
<211> 9
<212> DNA
<213> Artificial Sequence

<220>
<223> synthetic construct

<400> 110
agcagacaa                                                                9

<210> 111
<211> 9
<212> DNA
<213> Artificial Sequence

<220>
<223> synthetic construct

<400> 111
cgacagacc                                                                9

<210> 112
<211> 9
<212> DNA
<213> Artificial Sequence

<220>
<223> synthetic construct

<400> 112
tctgtcgaa                                                                9

<210> 113
<211> 9
<212> DNA
<213> Artificial Sequence

<220>
<223> synthetic construct

<400> 113
cgctactcc                                                                9

<210> 114
<211> 9
<212> DNA
<213> Artificial Sequence

<220>
<223> synthetic construct

<400> 114
agtagcgaa                                                                9

<210> 115
<211> 9
<212> DNA
<213> Artificial Sequence

<220>
<223> synthetic construct

<400> 115
ccacagacc                                                                9

<210> 116
<211> 9
<212> DNA
<213> Artificial Sequence

<220>
<223> synthetic construct

<400> 116

tctgtggaa                                                              9

<210> 117
<211> 9
<212> DNA
<213> Artificial Sequence

<220>
<223> synthetic construct

<400> 117
cctctctcc                                                              9

<210> 118
<211> 9
<212> DNA
<213> Artificial Sequence

<220>
<223> synthetic construct

<400> 118
agagaggaa                                                              9

<210> 119
<211> 9
<212> DNA
<213> Artificial Sequence

<220>
<223> synthetic construct

<400> 119
ctcgtagcc                                                              9

<210> 120
<211> 9
<212> DNA
<213> Artificial Sequence

<220>
<223> synthetic construct

<400> 120
ctacgagaa                                                              9

<210> 121
<211> 20
<212> DNA
<213> Artificial Sequence

<220>
<223> synthetic construct

<400> 121
aaatcgatgt ggtcactcag                                                 20

<210> 122
<211> 20
<212> DNA
<213> Artificial Sequence

<220>
<223> synthetic construct

<400> 122

gagtgaccac atcgatttgg                                                    20

<210> 123
<211> 20
<212> DNA
<213> Artificial Sequence

<220>
<223> synthetic construct

<400> 123
aaatcgatgt ggactaggag                                                    20

<210> 124
<211> 20
<212> DNA
<213> Artificial Sequence

<220>
<223> synthetic construct

<400> 124
cctagtccac atcgatttgg                                                    20

<210> 125
<211> 20
<212> DNA
<213> Artificial Sequence

<220>
<223> synthetic construct

<400> 125
aaatcgatgt gccgtatgag                                                    20

<210> 126
<211> 20
<212> DNA
<213> Artificial Sequence

<220>
<223> synthetic construct

<400> 126
catacggcac atcgatttgg                                                    20


<210> 127
<211> 20
<212> DNA
<213> Artificial Sequence

<220>
<223> synthetic construct

<400> 127
aaatcgatgt gctgaaggag                                                    20

<210> 128
<211> 20
<212> DNA
<213> Artificial Sequence

<220>
<223> synthetic construct

<400> 128
ccttcagcac atcgatttgg                                                    20

<210> 129
<211> 20
<212> DNA
<213> Artificial Sequence

<220>
<223> synthetic construct

<400> 129
aaatcgatgt ggactagcag                                                    20

<210> 130
<211> 20
<212> DNA
<213> Artificial Sequence

<220>
<223> synthetic construct

<400> 130
gctagtccac atcgatttgg                                                    20

<210> 131
<211> 20
<212> DNA
<213> Artificial Sequence

<220>
<223> synthetic construct

<400> 131
aaatcgatgt gcgctaagag                                                    20

<210> 132
<211> 20
<212> DNA
<213> Artificial Sequence

<220>
<223> synthetic construct

<400> 132
cttagcgcac atcgatttgg                                                    20

<210> 133
<211> 20
<212> DNA
<213> Artificial Sequence

<220>
<223> synthetic construct

<400> 133
aaatcgatgt gagccgagag                                                    20

<210> 134
<211> 20
<212> DNA
<213> Artificial Sequence

<220>
<223> synthetic construct

<400> 134
ctcggctcac atcgatttgg                                                    20

<210> 135
<211> 20
<212> DNA
<213> Artificial Sequence

<220>
<223> synthetic construct

<400> 135
aaatcgatgt gccgtatcag                                                    20

<210> 136
<211> 20
<212> DNA
<213> Artificial Sequence

<220>
<223> synthetic construct

<400> 136
gatacggcac atcgatttgg                                                    20

<210> 137
<211> 20
<212> DNA
<213> Artificial Sequence

<220>
<223> synthetic construct

<400> 137
aaatcgatgt gctgaagcag                                                    20

<210> 138
<211> 20
<212> DNA
<213> Artificial Sequence

<220>
<223> synthetic construct

<400> 138
gcttcagcac atcgatttgg                                                    20

<210> 139
<211> 20
<212> DNA
<213> Artificial Sequence

<220>
<223> synthetic construct

<400> 139
aaatcgatgt gtgcgagtag                                                    20

<210> 140
<211> 20
<212> DNA
<213> Artificial Sequence

<220>
<223> synthetic construct

<400> 140
actcgcacac atcgatttgg                                                    20

<210> 141
<211> 20
<212> DNA
<213> Artificial Sequence

<220>
<223> synthetic construct

<400> 141
aaatcgatgt gtttggcgag                                                    20

<210> 142
<211> 20
<212> DNA
<213> Artificial Sequence

<220>
<223> synthetic construct

<400> 142
cgccaaacac atcgatttgg                                                    20

<210> 143
<211> 20
<212> DNA
<213> Artificial Sequence

<220>
<223> synthetic construct

<400> 143
aaatcgatgt gcgctaacag                                                    20

<210> 144
<211> 20
<212> DNA
<213> Artificial Sequence

<220>
<223> synthetic construct

<400> 144
gttagcgcac atcgatttgg                                                    20

<210> 145
<211> 20
<212> DNA
<213> Artificial Sequence

<220>
<223> synthetic construct

<400> 145
aaatcgatgt gagccgacag                                                    20

<210> 146
<211> 20
<212> DNA
<213> Artificial Sequence

<220>
<223> synthetic construct

<400> 146
gtcggctcac atcgatttgg                                                    20

<210> 147
<211> 20
<212> DNA
<213> Artificial Sequence

<220>
<223> synthetic construct

<400> 147
aaatcgatgt gagccgaaag                                                    20

<210> 148
<211> 20
<212> DNA
<213> Artificial Sequence

<220>
<223> synthetic construct

<400> 148
ttcggctcac atcgatttgg                                                    20

<210> 149
<211> 20
<212> DNA
<213> Artificial Sequence

<220>
<223> synthetic construct

<400> 149
aaatcgatgt gtcggtagag                                                    20

<210> 150
<211> 20
<212> DNA
<213> Artificial Sequence

<220>
<223> synthetic construct

<400> 150
ctaccgacac atcgatttgg                                                    20

<210> 151
<211> 20
<212> DNA
<213> Artificial Sequence

<220>
<223> synthetic construct

<400> 151
aaatcgatgt ggttgccgag                                                    20

<210> 152
<211> 20
<212> DNA
<213> Artificial Sequence

<220>
<223> synthetic construct

<400> 152
cggcaaccac atcgatttgg                                                    20

<210> 153
<211> 20
<212> DNA
<213> Artificial Sequence

<220>
<223> synthetic construct

<400> 153
aaatcgatgt gagtgcgtag                                                    20

<210> 154
<211> 20
<212> DNA
<213> Artificial Sequence

<220>
<223> synthetic construct

<400> 154
acgcactcac atcgatttgg                                                    20

<210> 155
<211> 20
<212> DNA
<213> Artificial Sequence

<220>
<223> synthetic construct

<400> 155
aaatcgatgt ggttgccaag                                                    20

<210> 156
<211> 20
<212> DNA
<213> Artificial Sequence

<220>
<223> synthetic construct

<400> 156
tggcaaccac atcgatttgg                                                    20

<210> 157
<211> 20
<212> DNA
<213> Artificial Sequence

<220>
<223> synthetic construct

<400> 157
aaatcgatgt gtgcgaggag                                                    20

<210> 158
<211> 20

<212> DNA
<213> Artificial Sequence

<220>
<223> synthetic construct

<400> 158
cctcgcacac atcgatttgg                                                    20

<210> 159
<211> 20
<212> DNA
<213> Artificial Sequence

<220>
<223> synthetic construct


<400> 159
aaatcgatgt ggaacacgag                                                    20

<210> 160
<211> 20
<212> DNA
<213> Artificial Sequence

<220>
<223> synthetic construct

<400> 160
cgtgttccac atcgatttgg                                                    20

<210> 161
<211> 20
<212> DNA
<213> Artificial Sequence

<220>
<223> synthetic construct

<400> 161
aaatcgatgt gcttgtcgag                                                    20

<210> 162
<211> 20
<212> DNA
<213> Artificial Sequence

<220>
<223> synthetic construct

<400> 162
cgacaagcac atcgatttgg                                                    20

<210> 163
<211> 20
<212> DNA
<213> Artificial Sequence

<220>
<223> synthetic construct

<400> 163
aaatcgatgt gttccggtag                                                    20

<210> 164
<211> 20
<212> DNA
<213> Artificial Sequence

<220>

<223> synthetic construct

<400> 164
accggaacac atcgatttgg                                                    20

<210> 165
<211> 20
<212> DNA
<213> Artificial Sequence

<220>
<223> synthetic construct

<400> 165
aaatcgatgt gtgcgagcag                                                    20

<210> 166
<211> 20
<212> DNA
<213> Artificial Sequence

<220>
<223> synthetic construct

<400> 166

gctcgcacac atcgatttgg                                                    20

<210> 167
<211> 20
<212> DNA
<213> Artificial Sequence

<220>
<223> synthetic construct

<400> 167
aaatcgatgt ggtcaggtac                                                    20

<210> 168
<211> 20
<212> DNA
<213> Artificial Sequence

<220>
<223> synthetic construct

<400> 168
acctgaccac atcgatttgg                                                    20

<210> 169
<211> 20
<212> DNA
<213> Artificial Sequence

<220>
<223> synthetic construct

<400> 169
aaatcgatgt ggcctgttag                                                    20

<210> 170
<211> 20
<212> DNA
<213> Artificial Sequence

<220>
<223> synthetic construct

<400> 170
aacaggccac atcgatttgg                                                    20

<210> 171
<211> 20
<212> DNA
<213> Artificial Sequence

<220>
<223> synthetic construct

<400> 171
aaatcgatgt ggaacaccag                                                    20

<210> 172
<211> 20
<212> DNA
<213> Artificial Sequence

<220>
<223> synthetic construct

<400> 172
ggtgttccac atcgatttgg                                                    20

<210> 173
<211> 20
<212> DNA
<213> Artificial Sequence

<220>
<223> synthetic construct

<400> 173
aaatcgatgt gcttgtccag                                                    20

<210> 174
<211> 20
<212> DNA
<213> Artificial Sequence

<220>
<223> synthetic construct

<400> 174
ggacaagcac atcgatttgg                                                    20

<210> 175
<211> 20
<212> DNA
<213> Artificial Sequence

<220>
<223> synthetic construct

<400> 175
aaatcgatgt gtgcgagaag                                                    20

<210> 176
<211> 20
<212> DNA
<213> Artificial Sequence

<220>
<223> synthetic construct

<400> 176
tctcgcacac atcgatttgg                                                    20

<210> 177
<211> 20
<212> DNA
<213> Artificial Sequence

<220>
<223> synthetic construct

<400> 177
aaatcgatgt gagtgcggag                                                    20

<210> 178
<211> 20
<212> DNA
<213> Artificial Sequence

<220>
<223> synthetic construct

<400> 178
ccgcactcac atcgatttgg                                                    20

<210> 179
<211> 20
<212> DNA
<213> Artificial Sequence

<220>
<223> synthetic construct

<400> 179
aaatcgatgt gttgtccgag                                                    20

<210> 180
<211> 20
<212> DNA
<213> Artificial Sequence

<220>
<223> synthetic construct

<400> 180
cggacaacac atcgatttgg                                                    20

<210> 181
<211> 20
<212> DNA
<213> Artificial Sequence

<220>
<223> synthetic construct

<400> 181
aaatcgatgt gtggaacgag                                                    20

<210> 182
<211> 20
<212> DNA
<213> Artificial Sequence

<220>
<223> synthetic construct

<400> 182
cgttccacac atcgatttgg                                                    20

<210> 183
<211> 20
<212> DNA
<213> Artificial Sequence

<220>
<223> synthetic construct

<400> 183
aaatcgatgt gagtgcgaag                                                    20

<210> 184
<211> 20
<212> DNA
<213> Artificial Sequence

<220>
<223> synthetic construct

<400> 184
tcgcactcac atcgatttgg                                                    20


<210> 185
<211> 20
<212> DNA
<213> Artificial Sequence

<220>
<223> synthetic construct

<400> 185
aaatcgatgt gtggaaccag                                                    20

<210> 186
<211> 20
<212> DNA
<213> Artificial Sequence

<220>
<223> synthetic construct

<400> 186
ggttccacac atcgatttgg                                                    20

<210> 187
<211> 20
<212> DNA
<213> Artificial Sequence

<220>
<223> synthetic construct

<400> 187
aaatcgatgt gttaggcgag                                                    20

<210> 188
<211> 20
<212> DNA
<213> Artificial Sequence

<220>
<223> synthetic construct

<400> 188
cgcctaacac atcgatttgg                                                    20

<210> 189
<211> 20
<212> DNA
<213> Artificial Sequence

<220>
<223> synthetic construct

<400> 189
aaatcgatgt ggcctgtgag                                                    20

<210> 190
<211> 20
<212> DNA
<213> Artificial Sequence

<220>
<223> synthetic construct

<400> 190
cacaggccac atcgatttgg                                                    20

<210> 191
<211> 20
<212> DNA
<213> Artificial Sequence

<220>
<223> synthetic construct

<400> 191
aaatcgatgt gctcctgtag                                                    20

<210> 192

<211> 20
<212> DNA
<213> Artificial Sequence

<220>
<223> synthetic construct

<400> 192
acaggagcac atcgatttgg                                                    20

<210> 193
<211> 20
<212> DNA
<213> Artificial Sequence

<220>
<223> synthetic construct

<400> 193
aaatcgatgt ggtcaggcag                                                    20

<210> 194
<211> 20
<212> DNA

<213> Artificial Sequence

<220>
<223> synthetic construct

<400> 194
gcctgaccac atcgatttgg                                                    20

<210> 195
<211> 20
<212> DNA
<213> Artificial Sequence

<220>
<223> synthetic construct

<400> 195
aaatcgatgt ggtcaggaag                                                    20

<210> 196
<211> 20
<212> DNA
<213> Artificial Sequence

<220>
<223> synthetic construct

<400> 196
tcctgaccac atcgatttgg                                                    20

<210> 197
<211> 20
<212> DNA
<213> Artificial Sequence

<220>
<223> synthetic construct

<400> 197
aaatcgatgt ggtagccgag                                                    20

<210> 198
<211> 20
<212> DNA
<213> Artificial Sequence

<220>
<223> synthetic construct

<400> 198
cggctaccac atcgatttgg                                                    20

<210> 199
<211> 20
<212> DNA
<213> Artificial Sequence

<220>
<223> synthetic construct

<400> 199
aaatcgatgt ggcctgtaag                                                    20

<210> 200
<211> 20
<212> DNA

<213> Artificial Sequence

<220>
<223> synthetic construct

<400> 200
tacaggccac atcgatttgg                                                    20

<210> 201
<211> 20
<212> DNA
<213> Artificial Sequence

<220>
<223> synthetic construct

<400> 201
aaatcgatgt gctttcggag                                                    20

<210> 202
<211> 20
<212> DNA
<213> Artificial Sequence

<220>
<223> synthetic construct

<400> 202
ccgaaagcac atcgatttgg                                                    20

<210> 203
<211> 20
<212> DNA
<213> Artificial Sequence

<220>
<223> synthetic construct

<400> 203
aaatcgatgt gcgtaaggag                                                    20

<210> 204
<211> 20
<212> DNA
<213> Artificial Sequence

<220>
<223> synthetic construct

<400> 204
ccttacgcac atcgatttgg                                                    20

<210> 205
<211> 20
<212> DNA
<213> Artificial Sequence

<220>
<223> synthetic construct

<400> 205
aaatcgatgt gagagcgtag                                                    20

<210> 206
<211> 20
<212> DNA

<213> Artificial Sequence

<220>
<223> synthetic construct

<400> 206
acgctctcac atcgatttgg                                                    20

<210> 207
<211> 20
<212> DNA
<213> Artificial Sequence

<220>
<223> synthetic construct

<400> 207
aaatcgatgt ggacggcaag                                                    20

<210> 208
<211> 20
<212> DNA
<213> Artificial Sequence

<220>
<223> synthetic construct

<400> 208
tgccgtccac atcgatttgg                                                    20

<210> 209
<211> 20
<212> DNA
<213> Artificial Sequence

<220>
<223> synthetic construct

<400> 209
aaatcgatgt gctttcgcag                                                    20

<210> 210
<211> 20
<212> DNA
<213> Artificial Sequence

<220>
<223> synthetic construct

<400> 210
gcgaaagcac atcgatttgg                                                    20

<210> 211
<211> 20
<212> DNA
<213> Artificial Sequence

<220>
<223> synthetic construct

<400> 211
aaatcgatgt gcgtaagcag                                                    20

<210> 212
<211> 20
<212> DNA

<213> Artificial Sequence

<220>
<223> synthetic construct

<400> 212
gcttacgcac atcgatttgg                                                    20

<210> 213
<211> 20
<212> DNA
<213> Artificial Sequence

<220>
<223> synthetic construct

<400> 213
aaatcgatgt ggctatggag                                                    20

<210> 214
<211> 20
<212> DNA
<213> Artificial Sequence

<220>
<223> synthetic construct

<400> 214
ccatagccac atcgatttgg                                                    20

<210> 215
<211> 20
<212> DNA
<213> Artificial Sequence

<220>
<223> synthetic construct

<400> 215
aaatcgatgt gactctggag                                                    20

<210> 216
<211> 20
<212> DNA
<213> Artificial Sequence

<220>
<223> synthetic construct

<400> 216
ccagagtcac atcgatttgg                                                    20

<210> 217
<211> 19
<212> DNA
<213> Artificial Sequence

<220>
<223> synthetic construct

<400> 217
aaatcgatgt gctggaaag                                                     19

<210> 218
<211> 19
<212> DNA

<213> Artificial Sequence

<220>
<223> synthetic construct

<400> 218
ttccagcaca tcgatttgg                                                    19

<210> 219
<211> 20
<212> DNA
<213> Artificial Sequence

<220>
<223> synthetic construct

<400> 219
aaatcgatgt gccgaagtag                                                   20

<210> 220
<211> 20
<212> DNA
<213> Artificial Sequence

<220>
<223> synthetic construct

<400> 220
acttcggcac atcgatttgg                                                   20

<210> 221
<211> 20
<212> DNA
<213> Artificial Sequence

<220>
<223> synthetic construct

<400> 221
aaatcgatgt gctcctgaag                                                   20

<210> 222
<211> 20
<212> DNA
<213> Artificial Sequence

<220>
<223> synthetic construct

<400> 222
tcaggagcac atcgatttgg                                                   20

<210> 223
<211> 20
<212> DNA

<213> Artificial Sequence

<220>
<223> synthetic construct

<400> 223
aaatcgatgt gtccagtcag                                                   20

<210> 224
<211> 20

<212> DNA
<213> Artificial Sequence

<220>
<223> synthetic construct

<400> 224
gactggacac atcgatttgg                                                    20

<210> 225
<211> 20
<212> DNA
<213> Artificial Sequence

<220>
<223> synthetic construct

<400> 225
aaatcgatgt gagagcggag                                                    20

<210> 226
<211> 20
<212> DNA
<213> Artificial Sequence

<220>
<223> synthetic construct

<400> 226
ccgctctcac atcgatttgg                                                    20

<210> 227
<211> 20
<212> DNA
<213> Artificial Sequence

<220>
<223> synthetic construct

<400> 227
aaatcgatgt gagagcgaag                                                    20

<210> 228
<211> 20
<212> DNA
<213> Artificial Sequence

<220>
<223> synthetic construct

<400> 228
tcgctctcac atcgatttgg                                                    20

<210> 229
<211> 20
<212> DNA
<213> Artificial Sequence

<220>
<223> synthetic construct

<400> 229
aaatcgatgt gccgaaggag                                                    20

<210> 230
<211> 20

<212> DNA
<213> Artificial Sequence

<220>
<223> synthetic construct

<400> 230
ccttcggcac atcgatttgg                                                    20

<210> 231
<211> 20
<212> DNA
<213> Artificial Sequence

<220>
<223> synthetic construct

<400> 231
aaatcgatgt gccgaagcag                                                    20

<210> 232
<211> 20
<212> DNA
<213> Artificial Sequence

<220>
<223> synthetic construct

<400> 232
gcttcggcac atcgatttgg                                                    20

<210> 233
<211> 20
<212> DNA
<213> Artificial Sequence

<220>
<223> synthetic construct

<400> 233
aaatcgatgt gtgttccgag                                                    20

<210> 234
<211> 20
<212> DNA
<213> Artificial Sequence

<220>
<223> synthetic construct

<400> 234
cggaacacac atcgatttgg                                                    20

<210> 235
<211> 20
<212> DNA
<213> Artificial Sequence

<220>
<223> synthetic construct

<400> 235
aaatcgatgt gtctggcgag                                                    20

<210> 236
<211> 20

<212> DNA
<213> Artificial Sequence

<220>
<223> synthetic construct

<400> 236
cgccagacac atcgatttgg                                                    20

<210> 237
<211> 20
<212> DNA
<213> Artificial Sequence

<220>
<223> synthetic construct

<400> 237
aaatcgatgt gctatcggag                                                    20

<210> 238
<211> 20
<212> DNA
<213> Artificial Sequence

<220>
<223> synthetic construct

<400> 238
ccgatagcac atcgatttgg                                                    20

<210> 239
<211> 20
<212> DNA
<213> Artificial Sequence

<220>
<223> synthetic construct

<400> 239
aaatcgatgt gcgaaaggag                                                    20

<210> 240
<211> 20
<212> DNA
<213> Artificial Sequence

<220>
<223> synthetic construct

<400> 240
cctttcgcac atcgatttgg                                                    20

<210> 241
<211> 20
<212> DNA
<213> Artificial Sequence

<220>
<223> synthetic construct

<400> 241
aaatcgatgt gccgaagaag                                                    20

<210> 242
<211> 20

<212> DNA
<213> Artificial Sequence

<220>
<223> synthetic construct

<400> 242
tcttcggcac atcgatttgg                                                    20

<210> 243
<211> 20
<212> DNA
<213> Artificial Sequence

<220>
<223> synthetic construct

<400> 243
aaatcgatgt ggttgcagag                                                    20

<210> 244
<211> 20
<212> DNA
<213> Artificial Sequence

<220>
<223> synthetic construct

<400> 244
ctgcaaccac atcgatttgg                                                    20

<210> 245
<211> 20
<212> DNA
<213> Artificial Sequence

<220>
<223> synthetic construct

<400> 245
aaatcgatgt ggatggtgag                                                    20

<210> 246
<211> 20
<212> DNA
<213> Artificial Sequence

<220>
<223> synthetic construct

<400> 246
caccatccac atcgatttgg                                                    20

<210> 247
<211> 20
<212> DNA
<213> Artificial Sequence

<220>
<223> synthetic construct

<400> 247
aaatcgatgt gctatcgcag                                                    20

<210> 248
<211> 20

<212> DNA
<213> Artificial Sequence

<220>
<223> synthetic construct

<400> 248
gcgatagcac atcgatttgg                                                    20

<210> 249
<211> 20
<212> DNA
<213> Artificial Sequence

<220>
<223> synthetic construct

<400> 249
aaatcgatgt gcgaaagcag                                                    20

<210> 250
<211> 20
<212> DNA
<213> Artificial Sequence

<220>
<223> synthetic construct

<400> 250
gctttcgcac atcgatttgg                                                    20

<210> 251
<211> 20
<212> DNA
<213> Artificial Sequence

<220>
<223> synthetic construct

<400> 251
aaatcgatgt gacactggag                                                    20

<210> 252
<211> 20
<212> DNA
<213> Artificial Sequence

<220>
<223> synthetic construct

<400> 252
ccagtgtcac atcgatttgg                                                    20

<210> 253
<211> 20
<212> DNA
<213> Artificial Sequence

<220>
<223> synthetic construct

<400> 253
aaatcgatgt gtctggcaag                                                    20

<210> 254
<211> 20

<212> DNA
<213> Artificial Sequence

<220>
<223> synthetic construct

<400> 254
tgccagacac atcgatttgg                                                    20

<210> 255
<211> 20
<212> DNA
<213> Artificial Sequence

<220>
<223> synthetic construct

<400> 255
aaatcgatgt ggatggtcag                                                    20

<210> 256
<211> 20
<212> DNA
<213> Artificial Sequence

<220>
<223> synthetic construct

<400> 256
gaccatccac atcgatttgg                                                    20

<210> 257
<211> 20
<212> DNA
<213> Artificial Sequence

<220>
<223> synthetic construct

<400> 257
aaatcgatgt ggttgcacag                                                    20

<210> 258
<211> 20
<212> DNA
<213> Artificial Sequence

<220>
<223> synthetic construct

<400> 258
gtgcaaccac atcgatttgg                                                    20

<210> 259
<211> 20
<212> DNA
<213> Artificial Sequence

<220>
<223> synthetic construct

<400> 259
aaatcgatgt gggcatcgag                                                    20

<210> 260
<211> 20

<212> DNA
<213> Artificial Sequence

<220>
<223> synthetic construct

<400> 260
cgatgcccca tccgatttgg                                                    20

<210> 261
<211> 20
<212> DNA
<213> Artificial Sequence

<220>
<223> synthetic construct

<400> 261
aaatcgatgt gtgcctccag                                                    20

<210> 262
<211> 20
<212> DNA
<213> Artificial Sequence

<220>
<223> synthetic construct

<400> 262
ggaggcacac atcgatttgg                                                    20

<210> 263
<211> 20
<212> DNA
<213> Artificial Sequence

<220>
<223> synthetic construct

<400> 263
aaatcgatgt gtgcctcaag                                                    20

<210> 264
<211> 20
<212> DNA
<213> Artificial Sequence

<220>
<223> synthetic construct

<400> 264
tgaggcacac atcgatttgg                                                    20

<210> 265
<211> 20
<212> DNA
<213> Artificial Sequence

<220>
<223> synthetic construct

<400> 265
aaatcgatgt gggcatccag                                                    20

<210> 266
<211> 20

<212> DNA
<213> Artificial Sequence

<220>
<223> synthetic construct

<400> 266
ggatgcccac atcgatttgg                                                    20

<210> 267
<211> 20
<212> DNA
<213> Artificial Sequence

<220>
<223> synthetic construct

<400> 267
aaatcgatgt gggcatcaag                                                    20

<210> 268
<211> 20
<212> DNA
<213> Artificial Sequence

<220>
<223> synthetic construct

<400> 268
tgatgcccac atcgatttgg                                                    20

<210> 269
<211> 20
<212> DNA
<213> Artificial Sequence

<220>
<223> synthetic construct

<400> 269
aaatcgatgt gcctgtcgag                                                    20

<210> 270
<211> 20
<212> DNA
<213> Artificial Sequence

<220>
<223> synthetic construct

<400> 270
cgacaggcac atcgatttgg                                                    20

<210> 271
<211> 20
<212> DNA
<213> Artificial Sequence

<220>
<223> synthetic construct

<400> 271
aaatcgatgt ggacggatag                                                    20

<210> 272
<211> 20

<212> DNA
<213> Artificial Sequence

<220>
<223> synthetic construct

<400> 272
atccgtccac atcgatttgg                                                    20

<210> 273
<211> 20
<212> DNA
<213> Artificial Sequence

<220>
<223> synthetic construct

<400> 273
aaatcgatgt gcctgtccag                                                    20

<210> 274
<211> 20
<212> DNA
<213> Artificial Sequence

<220>
<223> synthetic construct

<400> 274
ggacaggcac atcgatttgg                                                    20

<210> 275
<211> 20
<212> DNA
<213> Artificial Sequence

<220>
<223> synthetic construct

<400> 275
aaatcgatgt gaagcacgag                                                    20

<210> 276
<211> 20
<212> DNA
<213> Artificial Sequence

<220>
<223> synthetic construct

<400> 276
cgtgcttcac atcgatttgg                                                    20

<210> 277
<211> 20
<212> DNA
<213> Artificial Sequence

<220>
<223> synthetic construct

<400> 277
aaatcgatgt gcctgtcaag                                                    20

<210> 278
<211> 20

<212> DNA
<213> Artificial Sequence

<220>
<223> synthetic construct

<400> 278
tgacaggcac atcgatttgg                                                    20

<210> 279
<211> 20
<212> DNA
<213> Artificial Sequence

<220>
<223> synthetic construct

<400> 279
aaatcgatgt gaagcaccag                                                    20

<210> 280
<211> 20
<212> DNA
<213> Artificial Sequence

<220>
<223> synthetic construct

<400> 280
ggtgcttcac atcgatttgg                                                    20

<210> 281
<211> 20
<212> DNA
<213> Artificial Sequence

<220>
<223> synthetic construct

<400> 281
aaatcgatgt gccttcgtag                                                    20

<210> 282
<211> 20
<212> DNA
<213> Artificial Sequence

<220>
<223> synthetic construct

<400> 282
acgaaggcac atcgatttgg                                                    20

<210> 283
<211> 20
<212> DNA
<213> Artificial Sequence

<220>
<223> synthetic construct

<400> 283
aaatcgatgt gtcgtccgag                                                    20

<210> 284

<211> 20
<212> DNA
<213> Artificial Sequence

<220>
<223> synthetic construct

<400> 284
cggacgacac atcgatttgg                                                    20

<210> 285
<211> 20
<212> DNA
<213> Artificial Sequence

<220>
<223> synthetic construct

<400> 285
aaatcgatgt ggagtctgag                                                    20

<210> 286
<211> 20
<212> DNA
<213> Artificial Sequence

<220>
<223> synthetic construct

<400> 286
cagactccac atcgatttgg                                                    20

<210> 287
<211> 20
<212> DNA
<213> Artificial Sequence

<220>
<223> synthetic construct

<400> 287
aaatcgatgt gtgatccgag                                                    20

<210> 288
<211> 20
<212> DNA
<213> Artificial Sequence

<220>
<223> synthetic construct

<400> 288
cggatcacac atcgatttgg                                                    20

<210> 289
<211> 20
<212> DNA
<213> Artificial Sequence

<220>
<223> synthetic construct

<400> 289
aaatcgatgt gtcaggcgag                                                    20

<210> 290

<211> 20
<212> DNA
<213> Artificial Sequence

<220>
<223> synthetic construct

<400> 290
cgcctgacac atcgatttgg                                                    20

<210> 291
<211> 20
<212> DNA
<213> Artificial Sequence

<220>
<223> synthetic construct

<400> 291
aaatcgatgt gtcgtccaag                                                    20

<210> 292
<211> 20
<212> DNA
<213> Artificial Sequence

<220>
<223> synthetic construct

<400> 292
tggacgacac atcgatttgg                                                    20

<210> 293
<211> 20
<212> DNA
<213> Artificial Sequence

<220>
<223> synthetic construct

<400> 293
aaatcgatgt ggacggagag                                                    20

<210> 294
<211> 20
<212> DNA
<213> Artificial Sequence

<220>
<223> synthetic construct

<400> 294
ctccgtccac atcgatttgg                                                    20

<210> 295
<211> 20
<212> DNA
<213> Artificial Sequence

<220>
<223> synthetic construct

<400> 295
aaatcgatgt ggtagcagag                                                    20

<210> 296

<211> 20
<212> DNA
<213> Artificial Sequence

<220>
<223> synthetic construct

<400> 296
ctgctaccac atcgatttgg                                                    20

<210> 297
<211> 20
<212> DNA
<213> Artificial Sequence

<220>
<223> synthetic construct

<400> 297
aaatcgatgt ggctgtgtag                                                    20

<210> 298
<211> 20
<212> DNA
<213> Artificial Sequence

<220>
<223> synthetic construct

<400> 298
acacagccac atcgatttgg                                                    20

<210> 299
<211> 20
<212> DNA
<213> Artificial Sequence

<220>
<223> synthetic construct

<400> 299
aaatcgatgt ggacggacag                                                    20

<210> 300
<211> 20
<212> DNA
<213> Artificial Sequence

<220>
<223> synthetic construct

<400> 300
gtccgtccac atcgatttgg                                                    20

<210> 301
<211> 20
<212> DNA
<213> Artificial Sequence

<220>
<223> synthetic construct

<400> 301
aaatcgatgt gtcaggcaag                                                    20

<210> 302

<211> 20
<212> DNA
<213> Artificial Sequence

<220>

<223> synthetic construct

<400> 302
tgcctgacac atcgatttgg                                                    20

<210> 303
<211> 20
<212> DNA
<213> Artificial Sequence

<220>
<223> synthetic construct

<400> 303
aaatcgatgt ggctcgaaag                                                    20

<210> 304
<211> 20
<212> DNA
<213> Artificial Sequence

<220>
<223> synthetic construct

<400> 304
ttcgagccac atcgatttgg                                                    20

<210> 305
<211> 20
<212> DNA
<213> Artificial Sequence

<220>
<223> synthetic construct

<400> 305
aaatcgatgt gccttcggag                                                    20

<210> 306
<211> 20
<212> DNA
<213> Artificial Sequence

<220>
<223> synthetic construct

<400> 306
ccgaaggcac atcgatttgg                                                    20

<210> 307
<211> 20
<212> DNA
<213> Artificial Sequence

<220>
<223> synthetic construct

<400> 307
aaatcgatgt ggtagcacag                                                    20

<210> 308
<211> 20
<212> DNA
<213> Artificial Sequence

<220>
<223> synthetic construct

<400> 308
gtgctaccac atcgatttgg                                                    20

<210> 309
<211> 20
<212> DNA
<213> Artificial Sequence

<220>
<223> synthetic construct

<400> 309
aaatcgatgt ggaaggtcag                                                    20

<210> 310
<211> 20
<212> DNA
<213> Artificial Sequence

<220>
<223> synthetic construct

<400> 310
gaccttccac atcgatttgg                                                    20

<210> 311
<211> 20
<212> DNA
<213> Artificial Sequence

<220>
<223> synthetic construct

<400> 311
aaatcgatgt ggtgctgtag                                                    20

<210> 312
<211> 20
<212> DNA
<213> Artificial Sequence

<220>
<223> synthetic construct

<400> 312
acagcaccac atcgatttgg                                                    20

<210> 313
<211> 9
<212> DNA
<213> Artificial Sequence

<220>
<223> synthetic construct

<400> 313
gttgcctgt                                                                 9

<210> 314
<211> 9
<212> DNA
<213> Artificial Sequence

<220>
<223> synthetic construct

<400> 314
aggcaacct                                                                9

<210> 315
<211> 9
<212> DNA
<213> Artificial Sequence

<220>
<223> synthetic construct

<400> 315
caggacggt                                                                9

<210> 316
<211> 9
<212> DNA
<213> Artificial Sequence

<220>
<223> synthetic construct

<400> 316
cgtcctgct                                                                9

<210> 317
<211> 9
<212> DNA
<213> Artificial Sequence

<220>
<223> synthetic construct

<400> 317
agacgtggt                                                                9

<210> 318
<211> 9
<212> DNA
<213> Artificial Sequence

<220>
<223> synthetic construct

<400> 318
cacgtctct                                                                9

<210> 319
<211> 9
<212> DNA
<213> Artificial Sequence

<220>
<223> synthetic construct

<400> 319
caggaccgt                                                                9

<210> 320
<211> 9
<212> DNA
<213> Artificial Sequence

<220>
<223> synthetic construct

<400> 320
ggtcctgct                                                                9

<210> 321
<211> 9
<212> DNA
<213> Artificial Sequence

<220>
<223> synthetic construct

<400> 321
caggacagt                                                                9

<210> 322
<211> 9
<212> DNA
<213> Artificial Sequence

<220>
<223> synthetic construct

<400> 322
tgtcctgct                                                                9

<210> 323
<211> 9
<212> DNA
<213> Artificial Sequence

<220>
<223> synthetic construct

<400> 323
cactctggt                                                                9

<210> 324
<211> 9
<212> DNA
<213> Artificial Sequence

<220>
<223> synthetic construct

<400> 324
cagagtgct                                                                9

<210> 325
<211> 9
<212> DNA
<213> Artificial Sequence

<220>
<223> synthetic construct

<400> 325
gacggctgt                                                                9

<210> 326
<211> 9
<212> DNA
<213> Artificial Sequence

<220>
<223> synthetic construct

<400> 326
agccgtcct                                                                9

<210> 327
<211> 9
<212> DNA
<213> Artificial Sequence

<220>
<223> synthetic construct

<400> 327
cactctcgt                                                                9

<210> 328
<211> 9
<212> DNA
<213> Artificial Sequence

<220>
<223> synthetic construct

<400> 328
gagagtgct                                                                9

<210> 329
<211> 9
<212> DNA
<213> Artificial Sequence

<220>
<223> synthetic construct

<400> 329
gtagcctgt                                                                9

<210> 330
<211> 9
<212> DNA
<213> Artificial Sequence

<220>
<223> synthetic construct

<400> 330
aggctacct                                                                9

<210> 331
<211> 9
<212> DNA
<213> Artificial Sequence

<220>
<223> synthetic construct

<400> 331
gccacttgt                                                                9

<210> 332
<211> 9
<212> DNA
<213> Artificial Sequence

<220>
<223> synthetic construct

<400> 332
aagtggcct                                                              9

<210> 333
<211> 9
<212> DNA
<213> Artificial Sequence

<220>
<223> synthetic construct

<400> 333
catcgctgt                                                              9

<210> 334
<211> 9
<212> DNA
<213> Artificial Sequence

<220>
<223> synthetic construct

<400> 334
agcgatgct                                                              9

<210> 335
<211> 9
<212> DNA
<213> Artificial Sequence

<220>
<223> synthetic construct

<400> 335
cactggtgt                                                              9

<210> 336
<211> 9
<212> DNA
<213> Artificial Sequence

<220>
<223> synthetic construct

<400> 336
accagtgct                                                              9

<210> 337
<211> 9
<212> DNA
<213> Artificial Sequence

<220>
<223> synthetic construct

<400> 337
gccactggt                                                              9

<210> 338
<211> 9
<212> DNA
<213> Artificial Sequence

<220>
<223> synthetic construct

<400> 338
cagtggcct                                                              9

<210> 339
<211> 9
<212> DNA
<213> Artificial Sequence

<220>
<223> synthetic construct

<400> 339
tctggctgt                                                              9

<210> 340
<211> 9
<212> DNA
<213> Artificial Sequence

<220>
<223> synthetic construct

<400> 340
agccagact                                                              9

<210> 341
<211> 9
<212> DNA
<213> Artificial Sequence

<220>
<223> synthetic construct

<400> 341
gccactcgt                                                              9

<210> 342
<211> 9
<212> DNA
<213> Artificial Sequence

<220>
<223> synthetic construct

<400> 342
gagtggcct                                                              9

<210> 343
<211> 9
<212> DNA
<213> Artificial Sequence

<220>
<223> synthetic construct

<400> 343
tgcctctgt                                                              9

<210> 344
<211> 9
<212> DNA
<213> Artificial Sequence

<220>
<223> synthetic construct

<400> 344
agaggcact                                                                9

<210> 345
<211> 9
<212> DNA
<213> Artificial Sequence

<220>
<223> synthetic construct

<400> 345
catcgcagt                                                                9

<210> 346
<211> 9
<212> DNA
<213> Artificial Sequence

<220>
<223> synthetic construct

<400> 346
tgcgatgct                                                                9

<210> 347
<211> 9
<212> DNA
<213> Artificial Sequence

<220>
<223> synthetic construct

<400> 347
caggaaggt                                                                9

<210> 348
<211> 9
<212> DNA
<213> Artificial Sequence

<220>
<223> synthetic construct

<400> 348
cttcctgct                                                                9

<210> 349
<211> 9
<212> DNA
<213> Artificial Sequence

<220>
<223> synthetic construct

<400> 349
ggcatctgt                                                                9

<210> 350
<211> 9
<212> DNA
<213> Artificial Sequence

<220>
<223> synthetic construct

<400> 350
agatgccct                                                                9

<210> 351
<211> 9
<212> DNA
<213> Artificial Sequence

<220>
<223> synthetic construct

<400> 351
cggtgctgt                                                                9

<210> 352
<211> 9
<212> DNA
<213> Artificial Sequence

<220>
<223> synthetic construct

<400> 352
agcaccgct                                                                9

<210> 353
<211> 9
<212> DNA
<213> Artificial Sequence

<220>
<223> synthetic construct

<400> 353
cactggcgt                                                                9

<210> 354
<211> 9
<212> DNA
<213> Artificial Sequence

<220>
<223> synthetic construct

<400> 354
gccagtgct                                                                9

<210> 355
<211> 9
<212> DNA
<213> Artificial Sequence

<220>
<223> synthetic construct

<400> 355
tctcctcgt                                                                9

<210> 356
<211> 9
<212> DNA
<213> Artificial Sequence

<220>
<223> synthetic construct

<400> 356
gaggagact                                                                9

<210> 357
<211> 9
<212> DNA
<213> Artificial Sequence

<220>
<223> synthetic construct

<400> 357
cctgtctgt                                                                9

<210> 358
<211> 9
<212> DNA
<213> Artificial Sequence

<220>
<223> synthetic construct

<400> 358
agacaggct                                                                9

<210> 359
<211> 9
<212> DNA
<213> Artificial Sequence

<220>
<223> synthetic construct

<400> 359
caacgctgt                                                                9

<210> 360
<211> 9
<212> DNA
<213> Artificial Sequence

<220>
<223> synthetic construct

<400> 360
agcgttgct                                                                9

<210> 361
<211> 9
<212> DNA
<213> Artificial Sequence

<220>
<223> synthetic construct

<400> 361
tgcctcggt                                                                9

<210> 362
<211> 9
<212> DNA
<213> Artificial Sequence

<220>
<223> synthetic construct

<400> 362
cgaggcact                                                                9

<210> 363
<211> 9
<212> DNA
<213> Artificial Sequence

<220>
<223> synthetic construct

<400> 363
acactgcgt                                                                9

<210> 364
<211> 9
<212> DNA
<213> Artificial Sequence

<220>
<223> synthetic construct

<400> 364
gcagtgtct                                                                9

<210> 365
<211> 9
<212> DNA
<213> Artificial Sequence

<220>
<223> synthetic construct

<400> 365
tcgtcctgt                                                                9

<210> 366
<211> 9
<212> DNA
<213> Artificial Sequence

<220>
<223> synthetic construct

<400> 366
aggacgact                                                                9

<210> 367
<211> 9
<212> DNA
<213> Artificial Sequence

<220>
<223> synthetic construct

<400> 367
gctgccagt                                                                9

<210> 368
<211> 9
<212> DNA
<213> Artificial Sequence

<220>
<223> synthetic construct

<400> 368
tggcagcct                                                              9

<210> 369
<211> 9
<212> DNA
<213> Artificial Sequence

<220>
<223> synthetic construct

<400> 369
tcaggctgt                                                              9

<210> 370
<211> 9
<212> DNA
<213> Artificial Sequence

<220>
<223> synthetic construct

<400> 370
agcctgact                                                              9

<210> 371
<211> 9
<212> DNA
<213> Artificial Sequence

<220>
<223> synthetic construct

<400> 371
gccaggtgt                                                              9

<210> 372
<211> 9
<212> DNA
<213> Artificial Sequence

<220>
<223> synthetic construct

<400> 372
acctggcct                                                              9

<210> 373
<211> 9
<212> DNA
<213> Artificial Sequence

<220>
<223> synthetic construct

<400> 373
cggacctgt                                                              9

<210> 374
<211> 9
<212> DNA
<213> Artificial Sequence

<220>
<223> synthetic construct

<400> 374
aggtccgct                                                                9

<210> 375
<211> 9
<212> DNA
<213> Artificial Sequence

<220>
<223> synthetic construct

<400> 375
caacgcagt                                                                9

<210> 376
<211> 9
<212> DNA
<213> Artificial Sequence

<220>
<223> synthetic construct

<400> 376
tgcgttgct                                                                9

<210> 377
<211> 9
<212> DNA
<213> Artificial Sequence

<220>
<223> synthetic construct

<400> 377
cacacgagt                                                                9

<210> 378

<211> 9
<212> DNA
<213> Artificial Sequence

<220>
<223> synthetic construct

<400> 378
tcgtgtgct                                                                9

<210> 379
<211> 9
<212> DNA
<213> Artificial Sequence

<220>
<223> synthetic construct

<400> 379
atggcctgt                                                                9

<210> 380
<211> 9
<212> DNA
<213> Artificial Sequence

<220>
<223> synthetic construct

<400> 380
aggccatct                                                                9

<210> 381
<211> 9
<212> DNA
<213> Artificial Sequence

<220>
<223> synthetic construct

<400> 381
ccagtctgt                                                                9

<210> 382
<211> 9
<212> DNA
<213> Artificial Sequence

<220>
<223> synthetic construct

<400> 382
agactggct                                                                9

<210> 383
<211> 9
<212> DNA
<213> Artificial Sequence

<220>
<223> synthetic construct

<400> 383
gccaggagt                                                                9

<210> 384
<211> 9
<212> DNA
<213> Artificial Sequence

<220>
<223> synthetic construct

<400> 384
tcctggcct                                                                9

<210> 385
<211> 9
<212> DNA
<213> Artificial Sequence

<220>
<223> synthetic construct

<400> 385
cggaccagt                                                                9

<210> 386
<211> 9
<212> DNA
<213> Artificial Sequence

<220>
<223> synthetic construct

<400> 386
tggtccgct                                                              9

<210> 387
<211> 9
<212> DNA
<213> Artificial Sequence

<220>
<223> synthetic construct

<400> 387
ccttcgcgt                                                              9

<210> 388
<211> 9
<212> DNA
<213> Artificial Sequence

<220>
<223> synthetic construct

<400> 388
gcgaaggct                                                              9

<210> 389
<211> 9
<212> DNA
<213> Artificial Sequence

<220>
<223> synthetic construct

<400> 389
gcagccagt                                                              9

<210> 390
<211> 9
<212> DNA
<213> Artificial Sequence

<220>
<223> synthetic construct

<400> 390
tggctgcct                                                              9

<210> 391
<211> 9
<212> DNA
<213> Artificial Sequence

<220>
<223> synthetic construct

<400> 391
ccagtcggt                                                              9

<210> 392
<211> 9
<212> DNA
<213> Artificial Sequence

<220>
<223> synthetic construct

<400> 392
cgactggct                                                                9

<210> 393
<211> 9
<212> DNA
<213> Artificial Sequence

<220>
<223> synthetic construct

<400> 393
actgagcgt                                                                9

<210> 394
<211> 9
<212> DNA
<213> Artificial Sequence

<220>
<223> synthetic construct

<400> 394
gctcagtct                                                                9

<210> 395
<211> 9
<212> DNA
<213> Artificial Sequence

<220>
<223> synthetic construct

<400> 395
ccagtccgt                                                                9

<210> 396
<211> 9
<212> DNA
<213> Artificial Sequence

<220>
<223> synthetic construct

<400> 396
ggactggct                                                                9

<210> 397
<211> 9
<212> DNA
<213> Artificial Sequence

<220>
<223> synthetic construct

<400> 397
ccagtcagt                                                                9

<210> 398
<211> 9
<212> DNA
<213> Artificial Sequence

<220>
<223> synthetic construct

<400> 398
tgactggct                                                                9

<210> 399
<211> 9
<212> DNA
<213> Artificial Sequence

<220>
<223> synthetic construct

<400> 399
catcgaggt                                                                9

<210> 400
<211> 9
<212> DNA
<213> Artificial Sequence

<220>
<223> synthetic construct

<400> 400
ctcgatgct                                                                9

<210> 401
<211> 9
<212> DNA
<213> Artificial Sequence

<220>
<223> synthetic construct

<400> 401
ccatcgtgt                                                                9

<210> 402
<211> 9
<212> DNA
<213> Artificial Sequence

<220>
<223> synthetic construct

<400> 402
acgatggct                                                                9

<210> 403
<211> 9
<212> DNA
<213> Artificial Sequence

<220>
<223> synthetic construct

<400> 403
gtgctgcgt                                                                9

<210> 404
<211> 9
<212> DNA
<213> Artificial Sequence

<220>
<223> synthetic construct

<400> 404
gcagcacct                                                                9

<210> 405
<211> 9
<212> DNA
<213> Artificial Sequence

<220>
<223> synthetic construct

<400> 405
gactacggt                                                                9

<210> 406
<211> 9
<212> DNA
<213> Artificial Sequence

<220>
<223> synthetic construct

<400> 406
cgtagtcct                                                                9

<210> 407
<211> 9
<212> DNA
<213> Artificial Sequence

<220>
<223> synthetic construct

<400> 407
gtgctgagt                                                                9

<210> 408
<211> 9
<212> DNA
<213> Artificial Sequence

<220>
<223> synthetic construct

<400> 408
tcagcacct                                                                9

<210> 409
<211> 9
<212> DNA
<213> Artificial Sequence

<220>
<223> synthetic construct

<400> 409
gctgcatgt                                                                9

<210> 410
<211> 9
<212> DNA
<213> Artificial Sequence

<220>
<223> synthetic construct

<400> 410
atgcagcct                                                                9

<210> 411
<211> 9
<212> DNA
<213> Artificial Sequence

<220>
<223> synthetic construct

<400> 411
gagtggtgt                                                                9

<210> 412
<211> 9
<212> DNA
<213> Artificial Sequence

<220>
<223> synthetic construct

<400> 412
accactcct                                                                9

<210> 413
<211> 9
<212> DNA
<213> Artificial Sequence

<220>
<223> synthetic construct

<400> 413
gactaccgt                                                                9

<210> 414
<211> 9
<212> DNA
<213> Artificial Sequence

<220>
<223> synthetic construct

<400> 414
ggtagtcct                                                                9

<210> 415
<211> 9
<212> DNA
<213> Artificial Sequence

<220>
<223> synthetic construct

<400> 415
cggtgatgt                                                                9

<210> 416
<211> 9
<212> DNA
<213> Artificial Sequence

<220>
<223> synthetic construct

<400> 416
atcaccgct                                                                9

<210> 417
<211> 9
<212> DNA
<213> Artificial Sequence

<220>
<223> synthetic construct

<400> 417
tgcgactgt                                                                9

<210> 418
<211> 9
<212> DNA
<213> Artificial Sequence

<220>
<223> synthetic construct

<400> 418
agtcgcact                                                                9

<210> 419
<211> 9
<212> DNA
<213> Artificial Sequence

<220>
<223> synthetic construct

<400> 419
tctggaggt                                                                9

<210> 420
<211> 9
<212> DNA
<213> Artificial Sequence

<220>
<223> synthetic construct

<400> 420
ctccagact                                                                9

<210> 421
<211> 9
<212> DNA
<213> Artificial Sequence

<220>
<223> synthetic construct

<400> 421
agcactggt                                                                9

<210> 422
<211> 9
<212> DNA
<213> Artificial Sequence

<220>
<223> synthetic construct

<400> 422
cagtgctct                                                                9

<210> 423
<211> 9
<212> DNA
<213> Artificial Sequence

<220>
<223> synthetic construct

<400> 423
tcgcttggt                                                                9

<210> 424
<211> 9
<212> DNA
<213> Artificial Sequence

<220>
<223> synthetic construct

<400> 424
caagcgact                                                                9

<210> 425
<211> 9
<212> DNA
<213> Artificial Sequence

<220>
<223> synthetic construct

<400> 425
agcactcgt                                                                9

<210> 426
<211> 9
<212> DNA
<213> Artificial Sequence

<220>
<223> synthetic construct

<400> 426
gagtgctct                                                                9

<210> 427
<211> 9
<212> DNA
<213> Artificial Sequence

<220>
<223> synthetic construct

<400> 427
gcgattggt                                                                9

<210> 428
<211> 9
<212> DNA
<213> Artificial Sequence

<220>
<223> synthetic construct

<400> 428
caatcgcct                                                                9

<210> 429
<211> 9
<212> DNA
<213> Artificial Sequence

<220>
<223> synthetic construct

<400> 429
ccatcgcgt                                                                9

<210> 430
<211> 9
<212> DNA
<213> Artificial Sequence

<220>
<223> synthetic construct

<400> 430
gcgatggct                                                                9

<210> 431
<211> 9
<212> DNA
<213> Artificial Sequence

<220>
<223> synthetic construct

<400> 431
tcgcttcgt                                                                9

<210> 432
<211> 9
<212> DNA
<213> Artificial Sequence

<220>
<223> synthetic construct

<400> 432
gaagcgact                                                                9

<210> 433
<211> 9
<212> DNA
<213> Artificial Sequence

<220>
<223> synthetic construct

<400> 433
agtgcctgt                                                                9

<210> 434
<211> 9
<212> DNA
<213> Artificial Sequence

<220>
<223> synthetic construct

<400> 434
aggcactct                                                                9

<210> 435
<211> 9
<212> DNA
<213> Artificial Sequence

<220>
<223> synthetic construct

<400> 435
ggcataggt                                                                9

<210> 436
<211> 9
<212> DNA
<213> Artificial Sequence

<220>
<223> synthetic construct

<400> 436
ctatgccct                                                                9

<210> 437
<211> 9
<212> DNA
<213> Artificial Sequence

<220>
<223> synthetic construct

<400> 437
gcgattcgt                                                                9

<210> 438
<211> 9
<212> DNA
<213> Artificial Sequence

<220>
<223> synthetic construct

<400> 438
gaatcgcct                                                                9

<210> 439
<211> 9
<212> DNA
<213> Artificial Sequence

<220>
<223> synthetic construct

<400> 439
tgcgacggt                                                                9

<210> 440
<211> 9
<212> DNA
<213> Artificial Sequence

<220>
<223> synthetic construct

<400> 440
cgtcgcact                                                                9

<210> 441
<211> 9
<212> DNA
<213> Artificial Sequence

<220>
<223> synthetic construct

<400> 441
gagtggcgt                                                                9

<210> 442
<211> 9
<212> DNA
<213> Artificial Sequence

<220>
<223> synthetic construct

<400> 442
gccactcct                                                                9

<210> 443
<211> 9
<212> DNA
<213> Artificial Sequence

<220>
<223> synthetic construct

<400> 443
cggtgaggt                                                                9

<210> 444
<211> 9
<212> DNA
<213> Artificial Sequence

<220>
<223> synthetic construct

<400> 444
ctcaccgct                                                                9

<210> 445
<211> 9
<212> DNA
<213> Artificial Sequence

<220>
<223> synthetic construct

<400> 445

gctgcaagt 9

<210> 446
<211> 9
<212> DNA
<213> Artificial Sequence

<220>
<223> synthetic construct

<400> 446
ttgcagcct 9

<210> 447
<211> 9
<212> DNA
<213> Artificial Sequence

<220>
<223> synthetic construct

<400> 447
ttccgctgt 9

<210> 448
<211> 9
<212> DNA
<213> Artificial Sequence

<220>
<223> synthetic construct

<400> 448
agcggaact 9

<210> 449
<211> 9
<212> DNA
<213> Artificial Sequence

<220>
<223> synthetic construct

<400> 449
gagtggagt 9

<210> 450
<211> 9
<212> DNA
<213> Artificial Sequence

<220>
<223> synthetic construct

<400> 450
tccactcct 9

<210> 451
<211> 9
<212> DNA
<213> Artificial Sequence

<220>
<223> synthetic construct

<400> 451
acagagcgt                                                                9

<210> 452
<211> 9
<212> DNA
<213> Artificial Sequence

<220>
<223> synthetic construct

<400> 452
gctctgtct                                                                9

<210> 453
<211> 9
<212> DNA
<213> Artificial Sequence

<220>
<223> synthetic construct

<400> 453
tgcgaccgt                                                                9

<210> 454
<211> 9
<212> DNA
<213> Artificial Sequence

<220>
<223> synthetic construct

<400> 454
ggtcgcact                                                                9

<210> 455
<211> 9
<212> DNA
<213> Artificial Sequence

<220>
<223> synthetic construct

<400> 455
cctgtaggt                                                                9

<210> 456
<211> 9
<212> DNA
<213> Artificial Sequence

<220>
<223> synthetic construct

<400> 456
ctacaggct                                                                9

<210> 457
<211> 9
<212> DNA
<213> Artificial Sequence

<220>
<223> synthetic construct

<400> 457
tagccgtgt                                                                9

<210> 458
<211> 9
<212> DNA
<213> Artificial Sequence

<220>
<223> synthetic construct

<400> 458
acggctact                                                                9

<210> 459
<211> 9
<212> DNA
<213> Artificial Sequence

<220>
<223> synthetic construct

<400> 459
tgcgacagt                                                                9

<210> 460
<211> 9
<212> DNA
<213> Artificial Sequence

<220>
<223> synthetic construct

<400> 460
tgtcgcact                                                                9

<210> 461
<211> 9
<212> DNA
<213> Artificial Sequence

<220>
<223> synthetic construct

<400> 461
ggtctgtgt                                                                9

<210> 462
<211> 9
<212> DNA
<213> Artificial Sequence

<220>
<223> synthetic construct

<400> 462
acagaccct                                                                9

<210> 463
<211> 9
<212> DNA
<213> Artificial Sequence

<220>
<223> synthetic construct

<400> 463
cggtgaagt                                                                9

<210> 464
<211> 9
<212> DNA
<213> Artificial Sequence

<220>
<223> synthetic construct

<400> 464
ttcaccgct                                                                9

<210> 465
<211> 9
<212> DNA
<213> Artificial Sequence

<220>
<223> synthetic construct

<400> 465
caacgaggt                                                                9

<210> 466
<211> 9
<212> DNA
<213> Artificial Sequence

<220>
<223> synthetic construct

<400> 466
ctcgttgct                                                                9

<210> 467
<211> 9
<212> DNA
<213> Artificial Sequence

<220>
<223> synthetic construct

<400> 467
gcagcatgt                                                                9

<210> 468
<211> 9
<212> DNA
<213> Artificial Sequence

<220>
<223> synthetic construct

<400> 468
atgctgcct                                                                9

<210> 469
<211> 9
<212> DNA
<213> Artificial Sequence

<220>
<223> synthetic construct

<400> 469
tcgtcaggt                                                                9

<210> 470
<211> 9
<212> DNA
<213> Artificial Sequence

<220>
<223> synthetic construct

<400> 470
ctgacgact                                                                9

<210> 471
<211> 9
<212> DNA
<213> Artificial Sequence

<220>
<223> synthetic construct

<400> 471
agtgccagt                                                                9

<210> 472
<211> 9
<212> DNA
<213> Artificial Sequence

<220>
<223> synthetic construct

<400> 472
tggcactct                                                                9

<210> 473
<211> 9
<212> DNA
<213> Artificial Sequence

<220>
<223> synthetic construct

<400> 473
tagaggcgt                                                                9

<210> 474
<211> 9
<212> DNA
<213> Artificial Sequence

<220>
<223> synthetic construct

<400> 474
gcctctact                                                                9

<210> 475
<211> 9
<212> DNA
<213> Artificial Sequence

<220>
<223> synthetic construct

<400> 475
gtcagcggt                                                                9

<210> 476
<211> 9
<212> DNA
<213> Artificial Sequence

<220>
<223> synthetic construct

<400> 476
cgctgacct                                                                9

<210> 477
<211> 9
<212> DNA
<213> Artificial Sequence

<220>
<223> synthetic construct

<400> 477
tcaggaggt                                                                9

<210> 478
<211> 9
<212> DNA

<213> Artificial Sequence

<220>
<223> synthetic construct

<400> 478
ctcctgact                                                                9

<210> 479
<211> 9
<212> DNA
<213> Artificial Sequence

<220>
<223> synthetic construct

<400> 479

agcaggtgt                                                                9

<210> 480
<211> 9
<212> DNA
<213> Artificial Sequence

<220>
<223> synthetic construct

<400> 480
acctgctct                                                                9

<210> 481
<211> 9
<212> DNA
<213> Artificial Sequence

<220>

<223> synthetic construct

<400> 481
ttccgcagt                                                                9

<210> 482
<211> 9
<212> DNA
<213> Artificial Sequence

<220>
<223> synthetic construct

<400> 482
tgcggaact                                                                9

<210> 483
<211> 9
<212> DNA
<213> Artificial Sequence

<220>
<223> synthetic construct

<400> 483
gtcagccgt                                                                9

<210> 484
<211> 9
<212> DNA
<213> Artificial Sequence

<220>
<223> synthetic construct

<400> 484
ggctgacct                                                                9

<210> 485
<211> 9
<212> DNA
<213> Artificial Sequence

<220>
<223> synthetic construct

<400> 485
ggtctgcgt                                                                9

<210> 486
<211> 9
<212> DNA
<213> Artificial Sequence

<220>
<223> synthetic construct

<400> 486
gcagaccct                                                                9

<210> 487
<211> 9
<212> DNA
<213> Artificial Sequence

<220>
<223> synthetic construct

<400> 487
tagccgagt                                                                9

<210> 488
<211> 9
<212> DNA
<213> Artificial Sequence

<220>
<223> synthetic construct

<400> 488
tcggctact                                                                9

<210> 489
<211> 9
<212> DNA
<213> Artificial Sequence

<220>
<223> synthetic construct

<400> 489
gtcagcagt                                                                9

<210> 490
<211> 9
<212> DNA
<213> Artificial Sequence

<220>
<223> synthetic construct

<400> 490
tgctgacct                                                                9

<210> 491
<211> 9
<212> DNA
<213> Artificial Sequence

<220>
<223> synthetic construct

<400> 491
ggtctgagt                                                                9

<210> 492
<211> 9
<212> DNA
<213> Artificial Sequence

<220>
<223> synthetic construct

<400> 492
tcagaccct                                                                9

<210> 493
<211> 9
<212> DNA
<213> Artificial Sequence

<220>
<223> synthetic construct

<400> 493
cggacaggt                                                                9

<210> 494
<211> 9
<212> DNA
<213> Artificial Sequence

<220>
<223> synthetic construct

<400> 494
ctgtccgct                                                                9

<210> 495
<211> 9
<212> DNA
<213> Artificial Sequence


<220>
<223> synthetic construct

<400> 495
ttagccggt                                                                9

<210> 496
<211> 9
<212> DNA
<213> Artificial Sequence

<220>
<223> synthetic construct

<400> 496
cggctaact                                                                9

<210> 497
<211> 9
<212> DNA
<213> Artificial Sequence

<220>
<223> synthetic construct

<400> 497
gagacgagt                                                                9

<210> 498
<211> 9
<212> DNA
<213> Artificial Sequence

<220>
<223> synthetic construct

<400> 498
tcgtctcct                                                                9

<210> 499
<211> 9
<212> DNA
<213> Artificial Sequence

<220>
<223> synthetic construct

<400> 499
cgtaaccgt                                                                9

<210> 500
<211> 9
<212> DNA
<213> Artificial Sequence

<220>
<223> synthetic construct

<400> 500
ggttacgct                                                                9

<210> 501
<211> 9
<212> DNA
<213> Artificial Sequence

<220>
<223> synthetic construct

<400> 501
ttggcgtgt                                                                9

<210> 502
<211> 9
<212> DNA
<213> Artificial Sequence

<220>
<223> synthetic construct

<400> 502
acgccaact                                                                9

<210> 503
<211> 9
<212> DNA
<213> Artificial Sequence

<220>
<223> synthetic construct

<400> 503
atggcaggt                                                                9

<210> 504
<211> 9
<212> DNA
<213> Artificial Sequence

<220>
<223> synthetic construct

<400> 504
ctgccatct                                                                9

<210> 505
<211> 9
<212> DNA
<213> Artificial Sequence

<220>
<223> synthetic construct

<400> 505
cagctacga                                                                9

<210> 506
<211> 9
<212> DNA
<213> Artificial Sequence

<220>
<223> synthetic construct

<400> 506
gtagctgac                                                                9

<210> 507
<211> 9
<212> DNA
<213> Artificial Sequence

<220>
<223> synthetic construct

<400> 507
ctcctgcga                                                                9

<210> 508
<211> 9
<212> DNA
<213> Artificial Sequence

<220>
<223> synthetic construct

<400> 508
gcaggagac                                                                9

<210> 509
<211> 9
<212> DNA
<213> Artificial Sequence

<220>
<223> synthetic construct

<400> 509
gctgcctga                                                                9

<210> 510
<211> 9
<212> DNA
<213> Artificial Sequence

<220>
<223> synthetic construct

<400> 510
aggcagcac                                                                9

<210> 511
<211> 9
<212> DNA
<213> Artificial Sequence

<220>
<223> synthetic construct

<400> 511
caggaacga                                                                9

<210> 512
<211> 9
<212> DNA
<213> Artificial Sequence

<220>
<223> synthetic construct

<400> 512
gttcctgac                                                                9

<210> 513
<211> 9
<212> DNA
<213> Artificial Sequence

<220>
<223> synthetic construct

<400> 513
cacacgcga                                                                9

<210> 514
<211> 9
<212> DNA
<213> Artificial Sequence

<220>
<223> synthetic construct

<400> 514
gcgtgtgac                                                                9

<210> 515
<211> 9
<212> DNA
<213> Artificial Sequence

<220>
<223> synthetic construct

<400> 515
gcagcctga                                                                9

<210> 516
<211> 9
<212> DNA
<213> Artificial Sequence

<220>
<223> synthetic construct

<400> 516
aggctgcac                                                                9

<210> 517
<211> 9
<212> DNA
<213> Artificial Sequence

<220>
<223> synthetic construct

<400> 517
ctgaacgga                                                                9

<210> 518
<211> 9
<212> DNA
<213> Artificial Sequence

<220>
<223> synthetic construct

<400> 518
cgttcagac                                                                9

<210> 519
<211> 9
<212> DNA
<213> Artificial Sequence

<220>
<223> synthetic construct

<400> 519
ctgaaccga                                                                9

<210> 520
<211> 9
<212> DNA
<213> Artificial Sequence

<220>
<223> synthetic construct

<400> 520
ggttcagac                                                                9

<210> 521
<211> 9
<212> DNA
<213> Artificial Sequence

<220>
<223> synthetic construct

<400> 521
tctggacga                                                                9

<210> 522
<211> 9
<212> DNA
<213> Artificial Sequence

<220>
<223> synthetic construct

<400> 522
gtccagaac                                                                9

<210> 523
<211> 9
<212> DNA
<213> Artificial Sequence

<220>
<223> synthetic construct

<400> 523
tgcctacga                                                              9

<210> 524
<211> 9
<212> DNA
<213> Artificial Sequence

<220>
<223> synthetic construct

<400> 524
gtaggcaac                                                              9

<210> 525
<211> 9
<212> DNA
<213> Artificial Sequence

<220>
<223> synthetic construct

<400> 525
ggcatacga                                                              9

<210> 526
<211> 9
<212> DNA
<213> Artificial Sequence

<220>
<223> synthetic construct

<400> 526
gtatgccac                                                              9

<210> 527
<211> 9
<212> DNA
<213> Artificial Sequence

<220>
<223> synthetic construct

<400> 527
cggtgacga                                                              9

<210> 528
<211> 9
<212> DNA
<213> Artificial Sequence

<220>
<223> synthetic construct

<400> 528
gtcaccgac                                                              9

<210> 529
<211> 9
<212> DNA
<213> Artificial Sequence

<220>
<223> synthetic construct

<400> 529
caacgacga                                                              9

<210> 530
<211> 9
<212> DNA
<213> Artificial Sequence

<220>
<223> synthetic construct

<400> 530
gtcgttgac                                                              9

<210> 531
<211> 9
<212> DNA
<213> Artificial Sequence

<220>
<223> synthetic construct

<400> 531
ctcctctga                                                              9

<210> 532
<211> 9
<212> DNA
<213> Artificial Sequence

<220>
<223> synthetic construct

<400> 532
agaggagac                                                              9

<210> 533
<211> 9
<212> DNA
<213> Artificial Sequence

<220>
<223> synthetic construct

<400> 533
tcaggacga                                                              9

<210> 534
<211> 9
<212> DNA
<213> Artificial Sequence

<220>
<223> synthetic construct

<400> 534
gtcctgaac                                                              9

<210> 535
<211> 9
<212> DNA
<213> Artificial Sequence

<220>

<223> synthetic construct

<400> 535
aaaggcgga                                                                9

<210> 536
<211> 9
<212> DNA
<213> Artificial Sequence

<220>
<223> synthetic construct

<400> 536
cgcctttac                                                                9

<210> 537
<211> 9
<212> DNA
<213> Artificial Sequence

<220>
<223> synthetic construct

<400> 537
ctcctcgga                                                                9

<210> 538
<211> 9
<212> DNA
<213> Artificial Sequence

<220>
<223> synthetic construct

<400> 538
cgaggagac                                                                9

<210> 539
<211> 9
<212> DNA
<213> Artificial Sequence

<220>
<223> synthetic construct

<400> 539
cagatgcga                                                                9

<210> 540
<211> 9
<212> DNA
<213> Artificial Sequence

<220>
<223> synthetic construct

<400> 540
gcatctgac                                                                9

<210> 541
<211> 9
<212> DNA

<213> Artificial Sequence

<220>
<223> synthetic construct

<400> 541
gcagcaaga                                                                9

<210> 542
<211> 9
<212> DNA
<213> Artificial Sequence

<220>
<223> synthetic construct


<400> 542
ttgctgcac                                                                9

<210> 543
<211> 9
<212> DNA
<213> Artificial Sequence

<220>
<223> synthetic construct

<400> 543
gtggagtga                                                                9

<210> 544
<211> 9
<212> DNA
<213> Artificial Sequence

<220>
<223> synthetic construct

<400> 544
actccacac                                                                9

<210> 545
<211> 9
<212> DNA
<213> Artificial Sequence

<220>
<223> synthetic construct

<400> 545
ccagtagga                                                                9

<210> 546
<211> 9
<212> DNA
<213> Artificial Sequence

<220>
<223> synthetic construct

<400> 546
ctactggac                                                                9

<210> 547
<211> 9

<212> DNA
<213> Artificial Sequence

<220>
<223> synthetic construct

<400> 547
atggcacga                                                                9

<210> 548
<211> 9
<212> DNA
<213> Artificial Sequence

<220>
<223> synthetic construct

<400> 548
gtgccatac                                                                9

<210> 549
<211> 9
<212> DNA.
<213> Artificial Sequence

<220>
<223> synthetic construct

<400> 549
ggactgtga                                                                9

<210> 550
<211> 9
<212> DNA
<213> Artificial Sequence

<220>
<223> synthetic construct

<400> 550
acagtccac                                                                9

<210> 551
<211> 9

<212> DNA
<213> Artificial Sequence

<220>
<223> synthetic construct

<400> 551
ccgaactga                                                                9

<210> 552
<211> 9
<212> DNA
<213> Artificial Sequence

<220>
<223> synthetic construct


<400> 552
agttcggac                                                                9

<210> 553
<211> 9
<212> DNA
<213> Artificial Sequence

<220>
<223> synthetic construct

<400> 553
ctcctcaga                                                                9

<210> 554
<211> 9
<212> DNA
<213> Artificial Sequence

<220>
<223> synthetic construct

<400> 554
tgaggagac                                                                9

<210> 555
<211> 9
<212> DNA
<213> Artificial Sequence

<220>
<223> synthetic construct

<400> 555
cactgctga                                                                9

<210> 556
<211> 9
<212> DNA
<213> Artificial Sequence

<220>
<223> synthetic construct

<400> 556
agcagtgac                                                                9

<210> 557
<211> 9
<212> DNA
<213> Artificial Sequence

<220>
<223> synthetic construct

<400> 557
agcaggcga                                                                9

<210> 558
<211> 9
<212> DNA
<213> Artificial Sequence

<220>
<223> synthetic construct

<400> 558
gcctgctac                                                                9

<210> 559
<211> 9
<212> DNA
<213> Artificial Sequence

<220>
<223> synthetic construct

<400> 559
agcaggaga                                                                9

<210> 560
<211> 9
<212> DNA
<213> Artificial Sequence

<220>
<223> synthetic construct

<400> 560
tcctgctac                                                                9

<210> 561
<211> 9
<212> DNA
<213> Artificial Sequence

<220>
<223> synthetic construct

<400> 561
agagccaga                                                                9

<210> 562
<211> 9
<212> DNA
<213> Artificial Sequence

<220>
<223> synthetic construct

<400> 562
tggctctac                                                                9

<210> 563
<211> 9
<212> DNA
<213> Artificial Sequence

<220>
<223> synthetic construct

<400> 563
gtcgttgga                                                                9

<210> 564
<211> 9
<212> DNA
<213> Artificial Sequence

<220>
<223> synthetic construct

<400> 564
caacgacac                                                                9

<210> 565
<211> 9
<212> DNA
<213> Artificial Sequence

<220>
<223> synthetic construct

<400> 565
ccgaacgga                                                                9

<210> 566
<211> 9
<212> DNA
<213> Artificial Sequence

<220>
<223> synthetic construct

<400> 566
cgttcggac                                                                9

<210> 567
<211> 9
<212> DNA
<213> Artificial Sequence

<220>
<223> synthetic construct

<400> 567
cactgcgga                                                                9

<210> 568
<211> 9
<212> DNA
<213> Artificial Sequence

<220>
<223> synthetic construct

<400> 568
cgcagtgac                                                                9

<210> 569
<211> 9
<212> DNA
<213> Artificial Sequence

<220>
<223> synthetic construct

<400> 569
gtggagcga                                                                9

<210> 570
<211> 9
<212> DNA
<213> Artificial Sequence

<220>
<223> synthetic construct

<400> 570
gctccacac                                                                9

<210> 571
<211> 9
<212> DNA
<213> Artificial Sequence

<220>
<223> synthetic construct

<400> 571
gtggagaga                                                                9

<210> 572
<211> 9
<212> DNA
<213> Artificial Sequence

<220>
<223> synthetic construct

<400> 572
tctccacac                                                                9

<210> 573
<211> 9
<212> DNA
<213> Artificial Sequence

<220>
<223> synthetic construct

<400> 573
ggactgcga                                                                9

<210> 574
<211> 9
<212> DNA
<213> Artificial Sequence

<220>
<223> synthetic construct

<400> 574
gcagtccac                                                                9

<210> 575
<211> 9
<212> DNA
<213> Artificial Sequence

<220>
<223> synthetic construct

<400> 575
ccgaaccga                                                                9

<210> 576
<211> 9
<212> DNA
<213> Artificial Sequence

<220>
<223> synthetic construct

<400> 576
ggttcggac                                                                9

<210> 577
<211> 9
<212> DNA
<213> Artificial Sequence

<220>
<223> synthetic construct

<400> 577
cactgccga                                                                9

<210> 578
<211> 9
<212> DNA
<213> Artificial Sequence

<220>
<223> synthetic construct

<400> 578
ggcagtgac                                                                9

<210> 579
<211> 9
<212> DNA
<213> Artificial Sequence

<220>
<223> synthetic construct

<400> 579
cgaaacgga                                                                9

<210> 580
<211> 9
<212> DNA
<213> Artificial Sequence

<220>
<223> synthetic construct

<400> 580
cgtttcgac                                                                9

<210> 581
<211> 9
<212> DNA
<213> Artificial Sequence

<220>
<223> synthetic construct

<400> 581
ggactgaga                                                                9

<210> 582
<211> 9
<212> DNA
<213> Artificial Sequence

<220>
<223> synthetic construct

<400> 582
tcagtccac                                                                9

<210> 583
<211> 9
<212> DNA
<213> Artificial Sequence

<220>
<223> synthetic construct

<400> 583
ccgaacaga                                                                9

<210> 584
<211> 9
<212> DNA
<213> Artificial Sequence

<220>
<223> synthetic construct

<400> 584
tgttcggac                                                                9

<210> 585
<211> 9
<212> DNA
<213> Artificial Sequence

<220>
<223> synthetic construct

<400> 585
cgaaaccga                                                                9

<210> 586
<211> 9
<212> DNA
<213> Artificial Sequence

<220>
<223> synthetic construct

<400> 586
ggtttcgac                                                                9

<210> 587
<211> 9
<212> DNA
<213> Artificial Sequence

<220>
<223> synthetic construct

<400> 587
ctggcttga                                                                9

<210> 588
<211> 9
<212> DNA
<213> Artificial Sequence

<220>
<223> synthetic construct

<400> 588
aagccagac                                                                9

<210> 589
<211> 9
<212> DNA
<213> Artificial Sequence

<220>
<223> synthetic construct

<400> 589
cacacctga                                                                9

<210> 590
<211> 9
<212> DNA
<213> Artificial Sequence

<220>
<223> synthetic construct

<400> 590
aggtgtgac                                                                9

<210> 591
<211> 9
<212> DNA
<213> Artificial Sequence

<220>
<223> synthetic construct

<400> 591
aacgaccga                                                                9

<210> 592
<211> 9
<212> DNA
<213> Artificial Sequence

<220>
<223> synthetic construct

<400> 592
ggtcgttac                                                                9

<210> 593
<211> 9
<212> DNA
<213> Artificial Sequence

<220>
<223> synthetic construct

<400> 593
atccagcga                                                                9

<210> 594
<211> 9
<212> DNA
<213> Artificial Sequence

<220>
<223> synthetic construct

<400> 594
gctggatac                                                                9

<210> 595
<211> 9
<212> DNA
<213> Artificial Sequence

<220>
<223> synthetic construct

<400> 595
tgcgaagga                                                                9

<210> 596
<211> 9
<212> DNA
<213> Artificial Sequence

<220>
<223> synthetic construct

<400> 596
cttcgcaac                                                                9

<210> 597
<211> 9
<212> DNA
<213> Artificial Sequence

<220>
<223> synthetic construct

<400> 597
tgcgaacga                                                                9

<210> 598
<211> 9
<212> DNA
<213> Artificial Sequence

<220>
<223> synthetic construct

<400> 598
gttcgcaac                                                                9

<210> 599
<211> 9
<212> DNA
<213> Artificial Sequence

<220>
<223> synthetic construct

<400> 599
ctggctgga                                                                9

<210> 600
<211> 9
<212> DNA
<213> Artificial Sequence

<220>
<223> synthetic construct

<400> 600
cagccagac                                                                9

<210> 601
<211> 9
<212> DNA
<213> Artificial Sequence

<220>
<223> synthetic construct

<400> 601
cacaccgga                                                                9

<210> 602
<211> 9
<212> DNA
<213> Artificial Sequence

<220>
<223> synthetic construct

<400> 602
cggtgtgac                                                                9

<210> 603
<211> 9
<212> DNA
<213> Artificial Sequence

<220>
<223> synthetic construct

<400> 603
agtgcagga                                                                9

<210> 604
<211> 9
<212> DNA
<213> Artificial Sequence

<220>
<223> synthetic construct

<400> 604
ctgcactac                                                                9

<210> 605
<211> 9
<212> DNA
<213> Artificial Sequence

<220>
<223> synthetic construct

<400> 605
gaccgttga                                                                9

<210> 606
<211> 9
<212> DNA
<213> Artificial Sequence

<220>
<223> synthetic construct

<400> 606
aacggtcac                                                                9

<210> 607
<211> 9
<212> DNA
<213> Artificial Sequence

<220>
<223> synthetic construct

<400> 607
ggtgagtga                                                                9

<210> 608
<211> 9
<212> DNA
<213> Artificial Sequence

<220>
<223> synthetic construct

<400> 608
actcaccac                                                                9

<210> 609
<211> 9
<212> DNA
<213> Artificial Sequence

<220>
<223> synthetic construct

<400> 609
ccttcctga                                                                9

<210> 610
<211> 9
<212> DNA
<213> Artificial Sequence

<220>
<223> synthetic construct

<400> 610
aggaaggac                                                                9

<210> 611
<211> 9
<212> DNA
<213> Artificial Sequence

<220>
<223> synthetic construct

<400> 611
ctggctaga                                                                9

<210> 612
<211> 9
<212> DNA
<213> Artificial Sequence

<220>
<223> synthetic construct

<400> 612
tagccagac                                                                9

<210> 613
<211> 9
<212> DNA
<213> Artificial Sequence

<220>
<223> synthetic construct

<400> 613
cacaccaga                                                                9

<210> 614
<211> 9
<212> DNA
<213> Artificial Sequence

<220>
<223> synthetic construct

<400> 614
tggtgtgac                                                                9

<210> 615
<211> 9
<212> DNA
<213> Artificial Sequence

<220>
<223> synthetic construct

<400> 615
agcggtaga                                                                9

<210> 616
<211> 9
<212> DNA
<213> Artificial Sequence

<220>
<223> synthetic construct

<400> 616
taccgctac                                                                9

<210> 617
<211> 9
<212> DNA
<213> Artificial Sequence

<220>
<223> synthetic construct

<400> 617
gtcagagga                                                                9

<210> 618
<211> 9
<212> DNA
<213> Artificial Sequence

<220>
<223> synthetic construct

<400> 618
ctctgacac                                                                9

<210> 619
<211> 9
<212> DNA
<213> Artificial Sequence

<220>
<223> synthetic construct

<400> 619
ttccgacga                                                                9

<210> 620
<211> 9
<212> DNA
<213> Artificial Sequence

<220>
<223> synthetic construct

<400> 620
gtcggaaac                                                                9

<210> 621
<211> 9
<212> DNA
<213> Artificial Sequence

<220>
<223> synthetic construct

<400> 621
aggcgtaga                                                                9

<210> 622
<211> 9
<212> DNA
<213> Artificial Sequence

<220>
<223> synthetic construct

<400> 622
tacgcctac                                                                9

<210> 623
<211> 9
<212> DNA
<213> Artificial Sequence

<220>
<223> synthetic construct

<400> 623
ctcgactga                                                                9

<210> 624
<211> 9
<212> DNA
<213> Artificial Sequence

<220>
<223> synthetic construct

<400> 624
agtcgagac                                                                9

<210> 625
<211> 9
<212> DNA
<213> Artificial Sequence

<220>
<223> synthetic construct

<400> 625
tacgctgga                                                              9

<210> 626
<211> 9
<212> DNA
<213> Artificial Sequence

<220>
<223> synthetic construct

<400> 626
cagcgtaac                                                              9

<210> 627
<211> 9
<212> DNA
<213> Artificial Sequence

<220>
<223> synthetic construct

<400> 627
gttcggtga                                                              9

<210> 628
<211> 9
<212> DNA
<213> Artificial Sequence

<220>
<223> synthetic construct

<400> 628
accgaacac                                                              9

<210> 629
<211> 9
<212> DNA
<213> Artificial Sequence

<220>
<223> synthetic construct

<400> 629
gccagcaga                                                              9

<210> 630
<211> 9
<212> DNA
<213> Artificial Sequence

<220>
<223> synthetic construct

<400> 630
tgctggcac                                                              9

<210> 631
<211> 9
<212> DNA
<213> Artificial Sequence

<220>
<223> synthetic construct

<400> 631
gaccgtaga                                                              9

<210> 632
<211> 9
<212> DNA
<213> Artificial Sequence

<220>
<223> synthetic construct

<400> 632
tacggtcac                                                              9

<210> 633
<211> 9
<212> DNA
<213> Artificial Sequence

<220>
<223> synthetic construct

<400> 633
gtgctctga                                                              9

<210> 634

<211> 9
<212> DNA
<213> Artificial Sequence

<220>
<223> synthetic construct

<400> 634
agagcacac                                                              9

<210> 635
<211> 9
<212> DNA
<213> Artificial Sequence

<220>
<223> synthetic construct

<400> 635
ggtgagcga                                                              9

<210> 636
<211> 9
<212> DNA
<213> Artificial Sequence

<220>
<223> synthetic construct

<400> 636
gctcaccac                                                              9

<210> 637
<211> 9
<212> DNA
<213> Artificial Sequence

<220>
<223> synthetic construct

<400> 637
ggtgagaga                                                                9

<210> 638
<211> 9
<212> DNA
<213> Artificial Sequence

<220>
<223> synthetic construct

<400> 638
tctcaccac                                                                9

<210> 639
<211> 9
<212> DNA
<213> Artificial Sequence

<220>
<223> synthetic construct

<400> 639
ccttccaga                                                                9

<210> 640
<211> 9
<212> DNA
<213> Artificial Sequence

<220>
<223> synthetic construct

<400> 640
tggaaggac                                                                9

<210> 641
<211> 9
<212> DNA
<213> Artificial Sequence

<220>
<223> synthetic construct

<400> 641
ctcctacga                                                                9

<210> 642
<211> 9
<212> DNA
<213> Artificial Sequence

<220>
<223> synthetic construct

<400> 642
gtaggagac                                                                9

<210> 643
<211> 9
<212> DNA
<213> Artificial Sequence

<220>
<223> synthetic construct

<400> 643
ctcgacgga                                                                9

<210> 644
<211> 9
<212> DNA
<213> Artificial Sequence

<220>
<223> synthetic construct

<400> 644
cgtcgagac                                                                9

<210> 645
<211> 9
<212> DNA
<213> Artificial Sequence

<220>
<223> synthetic construct

<400> 645
gccgtttga                                                                9

<210> 646
<211> 9
<212> DNA
<213> Artificial Sequence

<220>
<223> synthetic construct

<400> 646
aaacggcac                                                                9

<210> 647
<211> 9
<212> DNA
<213> Artificial Sequence

<220>
<223> synthetic construct

<400> 647
gcggagtga                                                                9

<210> 648
<211> 9
<212> DNA
<213> Artificial Sequence

<220>
<223> synthetic construct

<400> 648
actccgcac                                                                9

<210> 649
<211> 9
<212> DNA
<213> Artificial Sequence

<220>
<223> synthetic construct

<400> 649
cgtgcttga                                                                9

<210> 650
<211> 9
<212> DNA
<213> Artificial Sequence

<220>
<223> synthetic construct

<400> 650
aagcacgac                                                                9

<210> 651
<211> 9
<212> DNA
<213> Artificial Sequence

<220>
<223> synthetic construct

<400> 651
ctcgaccga                                                                9

<210> 652
<211> 9
<212> DNA
<213> Artificial Sequence

<220>
<223> synthetic construct

<400> 652
ggtcgagac                                                                9

<210> 653
<211> 9
<212> DNA
<213> Artificial Sequence

<220>
<223> synthetic construct

<400> 653
agagcagga                                                                9

<210> 654
<211> 9
<212> DNA
<213> Artificial Sequence

<220>
<223> synthetic construct

<400> 654
ctgctctac                                                                9

<210> 655
<211> 9
<212> DNA
<213> Artificial Sequence

<220>
<223> synthetic construct

<400> 655
gtgctcgga                                                                9

<210> 656
<211> 9
<212> DNA
<213> Artificial Sequence

<220>
<223> synthetic construct

<400> 656
cgagcacac                                                                9

<210> 657
<211> 9
<212> DNA
<213> Artificial Sequence

<220>
<223> synthetic construct

<400> 657
ctcgacaga                                                                9

<210> 658
<211> 9
<212> DNA
<213> Artificial Sequence

<220>
<223> synthetic construct

<400> 658
tgtcgagac                                                                9

<210> 659
<211> 9
<212> DNA
<213> Artificial Sequence

<220>
<223> synthetic construct

<400> 659
ggagagtga                                                                9

<210> 660
<211> 9
<212> DNA
<213> Artificial Sequence

<220>
<223> synthetic construct

<400> 660
actctccac                                                                9

<210> 661
<211> 9
<212> DNA
<213> Artificial Sequence

<220>
<223> synthetic construct

<400> 661
aggctgtga                                                              9

<210> 662
<211> 9
<212> DNA
<213> Artificial Sequence

<220>
<223> synthetic construct

<400> 662
acagcctac                                                              9

<210> 663
<211> 9
<212> DNA
<213> Artificial Sequence

<220>
<223> synthetic construct

<400> 663
agagcacga                                                              9

<210> 664
<211> 9
<212> DNA
<213> Artificial Sequence

<220>
<223> synthetic construct

<400> 664
gtgctctac                                                              9

<210> 665
<211> 9
<212> DNA
<213> Artificial Sequence

<220>
<223> synthetic construct

<400> 665
ccatcctga                                                              9

<210> 666
<211> 9
<212> DNA
<213> Artificial Sequence

<220>
<223> synthetic construct

<400> 666
aggatggac                                                              9

<210> 667
<211> 9
<212> DNA
<213> Artificial Sequence

<220>
<223> synthetic construct

<400> 667
gttcggaga                                                                9

<210> 668
<211> 9
<212> DNA
<213> Artificial Sequence

<220>
<223> synthetic construct

<400> 668
tccgaacac                                                                9

<210> 669
<211> 9
<212> DNA
<213> Artificial Sequence

<220>
<223> synthetic construct

<400> 669
tggtagcga                                                                9

<210> 670
<211> 9
<212> DNA
<213> Artificial Sequence

<220>
<223> synthetic construct

<400> 670
gctaccaac                                                                9

<210> 671
<211> 9
<212> DNA
<213> Artificial Sequence

<220>
<223> synthetic construct

<400> 671
gtgctccga                                                                9

<210> 672
<211> 9
<212> DNA
<213> Artificial Sequence

<220>
<223> synthetic construct

<400> 672
ggagcacac                                                                9

<210> 673
<211> 9
<212> DNA
<213> Artificial Sequence

<220>
<223> synthetic construct

<400> 673
gtgctcaga                                                                9

<210> 674
<211> 9
<212> DNA
<213> Artificial Sequence

<220>
<223> synthetic construct

<400> 674
tgagcacac                                                                9

<210> 675
<211> 9
<212> DNA
<213> Artificial Sequence

<220>
<223> synthetic construct

<400> 675
gccgttgga                                                                9

<210> 676
<211> 9
<212> DNA
<213> Artificial Sequence

<220>
<223> synthetic construct

<400> 676
caacggcac                                                                9

<210> 677
<211> 9
<212> DNA
<213> Artificial Sequence

<220>
<223> synthetic construct

<400> 677
gagtgctga                                                                9

<210> 678
<211> 9
<212> DNA
<213> Artificial Sequence

<220>
<223> synthetic construct

<400> 678
agcactcac                                                                9

<210> 679
<211> 9
<212> DNA
<213> Artificial Sequence

<220>
<223> synthetic construct

<400> 679
gctccttga                                                              9

<210> 680
<211> 9
<212> DNA
<213> Artificial Sequence

<220>
<223> synthetic construct

<400> 680
aaggagcac                                                              9

<210> 681
<211> 9
<212> DNA
<213> Artificial Sequence

<220>
<223> synthetic construct

<400> 681
ccgaaagga                                                              9

<210> 682
<211> 9
<212> DNA
<213> Artificial Sequence

<220>
<223> synthetic construct

<400> 682
ctttcggac                                                              9

<210> 683
<211> 9
<212> DNA
<213> Artificial Sequence

<220>
<223> synthetic construct

<400> 683
cactgagga                                                              9

<210> 684
<211> 9
<212> DNA
<213> Artificial Sequence

<220>
<223> synthetic construct

<400> 684
ctcagtgac                                                              9

<210> 685
<211> 9
<212> DNA
<213> Artificial Sequence

<220>
<223> synthetic construct

<400> 685
cgtgctgga                                                                9

<210> 686
<211> 9
<212> DNA
<213> Artificial Sequence

<220>
<223> synthetic construct

<400> 686
cagcacgac                                                                9

<210> 687
<211> 9
<212> DNA
<213> Artificial Sequence

<220>
<223> synthetic construct

<400> 687
ccgaaacga                                                                9

<210> 688
<211> 9
<212> DNA
<213> Artificial Sequence

<220>
<223> synthetic construct

<400> 688
gtttcggac                                                                9

<210> 689
<211> 9
<212> DNA
<213> Artificial Sequence

<220>
<223> synthetic construct

<400> 689
gcggagaga                                                                9

<210> 690
<211> 9
<212> DNA
<213> Artificial Sequence

<220>
<223> synthetic construct

<400> 690
tctccgcac                                                                9

<210> 691
<211> 9
<212> DNA
<213> Artificial Sequence

<220>
<223> synthetic construct

<400> 691
gccgttaga                                                                9

<210> 692
<211> 9
<212> DNA
<213> Artificial Sequence

<220>
<223> synthetic construct

<400> 692
taacggcac                                                                9

<210> 693
<211> 9
<212> DNA
<213> Artificial Sequence

<220>
<223> synthetic construct

<400> 693
tctcgtgga                                                                9

<210> 694
<211> 9
<212> DNA
<213> Artificial Sequence

<220>
<223> synthetic construct

<400> 694
cacgagaac                                                                9

<210> 695
<211> 9
<212> DNA
<213> Artificial Sequence

<220>
<223> synthetic construct

<400> 695
cgtgctaga                                                                9

<210> 696
<211> 9
<212> DNA
<213> Artificial Sequence

<220>
<223> synthetic construct

<400> 696
tagcacgac                                                                9

<210> 697
<211> 9
<212> DNA
<213> Artificial Sequence

<220>
<223> synthetic construct

<400> 697
gcctgtctt                                                                9

<210> 698
<211> 9
<212> DNA
<213> Artificial Sequence

<220>
<223> synthetic construct

<400> 698
gacaggctc                                                                9

<210> 699
<211> 9
<212> DNA
<213> Artificial Sequence

<220>
<223> synthetic construct

<400> 699
ctcctggtt                                                                9

<210> 700
<211> 9
<212> DNA
<213> Artificial Sequence

<220>
<223> synthetic construct

<400> 700
ccaggagtc                                                                9

<210> 701
<211> 9
<212> DNA
<213> Artificial Sequence

<220>
<223> synthetic construct

<400> 701
actctgctt                                                                9

<210> 702
<211> 9
<212> DNA
<213> Artificial Sequence

<220>
<223> synthetic construct

<400> 702

gcagagttc                                                              9

<210> 703
<211> 9
<212> DNA
<213> Artificial Sequence

<220>
<223> synthetic construct

<400> 703
catcgcctt                                                              9

<210> 704
<211> 9
<212> DNA
<213> Artificial Sequence

<220>
<223> synthetic construct

<400> 704
ggcgatgtc                                                              9

<210> 705
<211> 9
<212> DNA
<213> Artificial Sequence

<220>
<223> synthetic construct

<400> 705
gccactatt                                                              9

<210> 706
<211> 9
<212> DNA
<213> Artificial Sequence

<220>
<223> synthetic construct

<400> 706
tagtggctc                                                              9

<210> 707
<211> 9
<212> DNA
<213> Artificial Sequence

<220>
<223> synthetic construct

<400> 707
cacacggtt                                                              9

<210> 708
<211> 9
<212> DNA
<213> Artificial Sequence

<220>
<223> synthetic construct

<400> 708
ccgtgtgtc                                                                9

<210> 709
<211> 9
<212> DNA
<213> Artificial Sequence

<220>
<223> synthetic construct

<400> 709
caacgcctt                                                                9

<210> 710
<211> 9
<212> DNA
<213> Artificial Sequence

<220>
<223> synthetic construct

<400> 710
ggcgttgtc                                                                9

<210> 711
<211> 9
<212> DNA
<213> Artificial Sequence

<220>
<223> synthetic construct

<400> 711
actgaggtt                                                                9

<210> 712
<211> 9
<212> DNA
<213> Artificial Sequence

<220>
<223> synthetic construct

<400> 712
cctcagttc                                                                9

<210> 713
<211> 9
<212> DNA
<213> Artificial Sequence

<220>
<223> synthetic construct

<400> 713
gtgctggtt                                                                9

<210> 714
<211> 9
<212> DNA
<213> Artificial Sequence

<220>
<223> synthetic construct

<400> 714
ccagcactc                                                                9

<210> 715
<211> 9
<212> DNA
<213> Artificial Sequence

<220>
<223> synthetic construct

<400> 715
catcgactt                                                                9

<210> 716
<211> 9
<212> DNA
<213> Artificial Sequence

<220>
<223> synthetic construct

<400> 716
gtcgatgtc                                                                9

<210> 717
<211> 9
<212> DNA
<213> Artificial Sequence

<220>
<223> synthetic construct

<400> 717
ccatcggtt                                                                9

<210> 718
<211> 9
<212> DNA
<213> Artificial Sequence

<220>
<223> synthetic construct

<400> 718
ccgatggtc                                                                9

<210> 719
<211> 9
<212> DNA
<213> Artificial Sequence

<220>
<223> synthetic construct

<400> 719
gctgcactt                                                                9

<210> 720
<211> 9
<212> DNA
<213> Artificial Sequence

<220>
<223> synthetic construct

<400> 720
gtgcagctc                                                                9

<210> 721
<211> 9
<212> DNA
<213> Artificial Sequence

<220>
<223> synthetic construct

<400> 721
acagaggtt                                                                9

<210> 722
<211> 9
<212> DNA
<213> Artificial Sequence

<220>
<223> synthetic construct

<400> 722
cctctgttc                                                                9

<210> 723
<211> 9
<212> DNA
<213> Artificial Sequence

<220>
<223> synthetic construct

<400> 723
agtgccgtt                                                                9

<210> 724
<211> 9
<212> DNA
<213> Artificial Sequence

<220>
<223> synthetic construct

<400> 724
cggcacttc                                                                9

<210> 725
<211> 9
<212> DNA
<213> Artificial Sequence

<220>
<223> synthetic construct

<400> 725
cggacattt                                                                9

<210> 726
<211> 9
<212> DNA
<213> Artificial Sequence

<220>
<223> synthetic construct

<400> 726
atgtccgtc                                                                9

<210> 727
<211> 9
<212> DNA
<213> Artificial Sequence

<220>
<223> synthetic construct

<400> 727
ggtctggtt                                                                9

<210> 728
<211> 9
<212> DNA
<213> Artificial Sequence

<220>
<223> synthetic construct

<400> 728
ccagacctc                                                                9

<210> 729
<211> 9
<212> DNA
<213> Artificial Sequence

<220>
<223> synthetic construct

<400> 729
gagacggtt                                                                9

<210> 730
<211> 9
<212> DNA
<213> Artificial Sequence

<220>
<223> synthetic construct

<400> 730
ccgtctctc                                                                9

<210> 731
<211> 9
<212> DNA
<213> Artificial Sequence

<220>
<223> synthetic construct

<400> 731
ctttccgtt                                                                9

<210> 732
<211> 9
<212> DNA
<213> Artificial Sequence

<220>
<223> synthetic construct

<400> 732
cggaaagtc                                                                9

<210> 733
<211> 9
<212> DNA
<213> Artificial Sequence

<220>
<223> synthetic construct

<400> 733
cagatggtt                                                                9

<210> 734
<211> 9
<212> DNA

<213> Artificial Sequence

<220>
<223> synthetic construct

<400> 734
ccatctgtc                                                                9

<210> 735
<211> 9
<212> DNA
<213> Artificial Sequence

<220>
<223> synthetic construct

<400> 735

cggacactt                                                                9

<210> 736
<211> 9
<212> DNA
<213> Artificial Sequence

<220>
<223> synthetic construct

<400> 736
gtgtccgtc                                                                9

<210> 737
<211> 9
<212> DNA
<213> Artificial Sequence

<220>
<223> synthetic construct

<400> 737
actctcgtt                                                                9

<210> 738
<211> 9
<212> DNA
<213> Artificial Sequence

<220>

<223> synthetic construct

<400> 738
cgagagttc                                                                9

<210> 739
<211> 9
<212> DNA
<213> Artificial Sequence

<220>
<223> synthetic construct

<400> 739
gcagcactt                                                                9

<210> 740
<211> 9
<212> DNA
<213> Artificial Sequence

<220>
<223> synthetic construct

<400> 740
gtgctgctc                                                                9

<210> 741
<211> 9
<212> DNA
<213> Artificial Sequence

<220>
<223> synthetic construct

<400> 741
actctcctt                                                                9

<210> 742
<211> 9
<212> DNA
<213> Artificial Sequence

<220>
<223> synthetic construct

<400> 742
ggagagttc                                                                9

<210> 743
<211> 9
<212> DNA
<213> Artificial Sequence

<220>
<223> synthetic construct

<400> 743
accttggtt                                                                9

<210> 744
<211> 9
<212> DNA
<213> Artificial Sequence

<220>
<223> synthetic construct

<400> 744
ccaaggttc                                                                9

<210> 745
<211> 9
<212> DNA
<213> Artificial Sequence

<220>
<223> synthetic construct

<400> 745
agagccgtt                                                                9

<210> 746
<211> 9
<212> DNA
<213> Artificial Sequence

<220>
<223> synthetic construct

<400> 746
cggctcttc                                                                9

<210> 747
<211> 9
<212> DNA
<213> Artificial Sequence

<220>
<223> synthetic construct

<400> 747
accttgctt                                                                9

<210> 748
<211> 9
<212> DNA
<213> Artificial Sequence

<220>
<223> synthetic construct

<400> 748
gcaaggttc                                                                9

<210> 749
<211> 9
<212> DNA
<213> Artificial Sequence

<220>
<223> synthetic construct

<400> 749
aagtccgtt                                                                9

<210> 750
<211> 9
<212> DNA
<213> Artificial Sequence

<220>
<223> synthetic construct

<400> 750
cggactttc                                                                9

<210> 751
<211> 9
<212> DNA
<213> Artificial Sequence


<220>
<223> synthetic construct

<400> 751
ggactggtt                                                                9

<210> 752
<211> 9
<212> DNA
<213> Artificial Sequence

<220>
<223> synthetic construct

<400> 752
ccagtcctc                                                                9

<210> 753
<211> 9
<212> DNA
<213> Artificial Sequence

<220>
<223> synthetic construct

<400> 753
gtcgttctt                                                                9

<210> 754
<211> 9
<212> DNA
<213> Artificial Sequence

<220>
<223> synthetic construct

<400> 754
gaacgactc                                                                9

<210> 755
<211> 9
<212> DNA
<213> Artificial Sequence

<220>
<223> synthetic construct

<400> 755
cagcatctt                                                                9

<210> 756
<211> 9
<212> DNA
<213> Artificial Sequence

<220>
<223> synthetic construct

<400> 756
gatgctgtc                                                                9

<210> 757
<211> 9
<212> DNA
<213> Artificial Sequence

<220>
<223> synthetic construct

<400> 757
ctatccgtt                                                                9

<210> 758
<211> 9
<212> DNA
<213> Artificial Sequence

<220>
<223> synthetic construct

<400> 758
cggatagtc                                                                9

<210> 759
<211> 9
<212> DNA
<213> Artificial Sequence

<220>
<223> synthetic construct

<400> 759
acactcgtt                                                                9

<210> 760
<211> 9
<212> DNA
<213> Artificial Sequence

<220>
<223> synthetic construct

<400> 760
cgagtgttc                                                                9

<210> 761
<211> 9
<212> DNA
<213> Artificial Sequence

<220>
<223> synthetic construct

<400> 761
atccaggtt                                                                9

<210> 762
<211> 9
<212> DNA
<213> Artificial Sequence

<220>
<223> synthetic construct

<400> 762
cctggattc                                                                9

<210> 763
<211> 9
<212> DNA
<213> Artificial Sequence

<220>
<223> synthetic construct

<400> 763
gttcctgtt                                                                9

<210> 764
<211> 9
<212> DNA
<213> Artificial Sequence

<220>
<223> synthetic construct

<400> 764
caggaactc                                                                9

<210> 765
<211> 9
<212> DNA
<213> Artificial Sequence

<220>
<223> synthetic construct

<400> 765
acactcctt                                                                9

<210> 766
<211> 9
<212> DNA
<213> Artificial Sequence

<220>
<223> synthetic construct

<400> 766
ggagtgttc                                                                9

<210> 767
<211> 9
<212> DNA
<213> Artificial Sequence

<220>
<223> synthetic construct

<400> 767
gttcctctt                                                                9

<210> 768
<211> 9
<212> DNA
<213> Artificial Sequence

<220>
<223> synthetic construct

<400> 768
gaggaactc                                                                9

<210> 769
<211> 9
<212> DNA
<213> Artificial Sequence

<220>
<223> synthetic construct

<400> 769
ctggctctt                                                                9

<210> 770
<211> 9
<212> DNA
<213> Artificial Sequence

<220>
<223> synthetic construct

<400> 770
gagccagtc                                                                9

<210> 771
<211> 9
<212> DNA
<213> Artificial Sequence

<220>
<223> synthetic construct

<400> 771
acggcattt                                                                9

<210> 772
<211> 9
<212> DNA
<213> Artificial Sequence

<220>
<223> synthetic construct

<400> 772
atgccgttc                                                                9

<210> 773
<211> 9
<212> DNA
<213> Artificial Sequence

<220>
<223> synthetic construct

<400> 773
ggtgaggtt                                                                9

<210> 774
<211> 9
<212> DNA
<213> Artificial Sequence

<220>
<223> synthetic construct

<400> 774
cctcacctc                                                                9

<210> 775
<211> 9
<212> DNA
<213> Artificial Sequence

<220>
<223> synthetic construct

<400> 775
ccttccgtt                                                                9

<210> 776
<211> 9
<212> DNA
<213> Artificial Sequence

<220>
<223> synthetic construct

<400> 776
cggaaggtc                                                                9

<210> 777
<211> 9
<212> DNA
<213> Artificial Sequence

<220>
<223> synthetic construct

<400> 777
tacgctctt                                                                9

<210> 778
<211> 9
<212> DNA
<213> Artificial Sequence

<220>
<223> synthetic construct

<400> 778
gagcgtatc                                                                9

<210> 779
<211> 9
<212> DNA
<213> Artificial Sequence

<220>
<223> synthetic construct

<400> 779
acggcagtt                                                                9

<210> 780
<211> 9
<212> DNA
<213> Artificial Sequence

<220>
<223> synthetic construct

<400> 780
ctgccgttc                                                                9

<210> 781
<211> 9
<212> DNA
<213> Artificial Sequence

<220>
<223> synthetic construct

<400> 781
actgacgtt                                                                9

<210> 782
<211> 9
<212> DNA
<213> Artificial Sequence

<220>
<223> synthetic construct

<400> 782
cgtcagttc                                                                9

<210> 783
<211> 9
<212> DNA
<213> Artificial Sequence

<220>
<223> synthetic construct

<400> 783
acggcactt                                                                9

<210> 784
<211> 9
<212> DNA
<213> Artificial Sequence

<220>
<223> synthetic construct

<400> 784
gtgccgttc                                                                9

<210> 785
<211> 9
<212> DNA
<213> Artificial Sequence

<220>
<223> synthetic construct

<400> 785
actgacctt                                                                9

<210> 786
<211> 9
<212> DNA
<213> Artificial Sequence

<220>
<223> synthetic construct

<400> 786
ggtcagttc 9

<210> 787
<211> 9
<212> DNA
<213> Artificial Sequence

<220>
<223> synthetic construct

<400> 787
tttgcggtt 9

<210> 788
<211> 9
<212> DNA
<213> Artificial Sequence

<220>
<223> synthetic construct

<400> 788
ccgcaaatc 9

<210> 789
<211> 9
<212> DNA
<213> Artificial Sequence

<220>
<223> synthetic construct

<400> 789
tggtaggtt 9

<210> 790
<211> 9
<212> DNA
<213> Artificial Sequence

<220>
<223> synthetic construct

<400> 790
cctaccatc 9

<210> 791
<211> 9
<212> DNA
<213> Artificial Sequence

<220>

<223> synthetic construct

<400> 791
gttcggctt 9

<210> 792
<211> 9
<212> DNA

<213> Artificial Sequence

<220>
<223> synthetic construct

<400> 792
gccgaactc                                                                9

<210> 793
<211> 9
<212> DNA
<213> Artificial Sequence

<220>
<223> synthetic construct

<400> 793
gccgttctt                                                                9

<210> 794
<211> 9
<212> DNA
<213> Artificial Sequence

<220>
<223> synthetic construct

<400> 794
gaacggctc                                                                9

<210> 795
<211> 9
<212> DNA
<213> Artificial Sequence

<220>
<223> synthetic construct

<400> 795
ggagaggtt                                                                9

<210> 796
<211> 9
<212> DNA
<213> Artificial Sequence

<220>
<223> synthetic construct

<400> 796
cctctcctc                                                                9

<210> 797
<211> 9
<212> DNA
<213> Artificial Sequence

<220>
<223> synthetic construct

<400> 797
cactgactt                                                                9

<210> 798
<211> 9
<212> DNA

<213> Artificial Sequence

<220>
<223> synthetic construct


<400> 798
gtcagtgtc                                                                9

<210> 799
<211> 9
<212> DNA
<213> Artificial Sequence

<220>
<223> synthetic construct

<400> 799
cgtgctctt                                                                9

<210> 800
<211> 9
<212> DNA
<213> Artificial Sequence

<220>
<223> synthetic construct

<400> 800
gagcacgtc                                                                9

<210> 801
<211> 9
<212> DNA
<213> Artificial Sequence

<220>
<223> synthetic construct

<400> 801
aatccgctt                                                                9

<210> 802
<211> 9
<212> DNA
<213> Artificial Sequence

<220>
<223> synthetic construct

<400> 802
gcggatttc                                                                9

<210> 803
<211> 9
<212> DNA
<213> Artificial Sequence

<220>
<223> synthetic construct

<400> 803
aggctggtt                                                                9

<210> 804
<211> 9

<212> DNA
<213> Artificial Sequence

<220>
<223> synthetic construct

<400> 804
ccagccttc                                                              9

<210> 805
<211> 9
<212> DNA
<213> Artificial Sequence

<220>
<223> synthetic construct

<400> 805
gctagtgtt                                                              9

<210> 806
<211> 9
<212> DNA
<213> Artificial Sequence

<220>
<223> synthetic construct

<400> 806
cactagctc                                                              9

<210> 807
<211> 9

<212> DNA
<213> Artificial Sequence

<220>
<223> synthetic construct

<400> 807
ggagagctt                                                              9

<210> 808
<211> 9
<212> DNA
<213> Artificial Sequence

<220>
<223> synthetic construct


<400> 808
gctctcctc                                                              9

<210> 809
<211> 9
<212> DNA
<213> Artificial Sequence

<220>
<223> synthetic construct

<400> 809
ggagagatt                                                              9

<210> 816
<211> 9
<212> DNA
<213> Artificial Sequence

<220>
<223> synthetic construct

<400> 816
tggatggtc                                                                9

<210> 817
<211> 9
<212> DNA
<213> Artificial Sequence

<220>
<223> synthetic construct

<400> 817
gctagtctt                                                                9

<210> 818
<211> 9
<212> DNA
<213> Artificial Sequence

<220>
<223> synthetic construct

<400> 818
gactagctc                                                                9

<210> 819
<211> 9
<212> DNA
<213> Artificial Sequence

<220>
<223> synthetic construct

<400> 819
aggctgatt                                                                9

<210> 820
<211> 9
<212> DNA
<213> Artificial Sequence

<220>
<223> synthetic construct

<400> 820
tcagccttc                                                                9

<210> 821
<211> 9
<212> DNA
<213> Artificial Sequence

<220>
<223> synthetic construct

<400> 821
acagacgtt                                                                9

<210> 810
<211> 9
<212> DNA
<213> Artificial Sequence

<220>
<223> synthetic construct

<400> 810
tctctcctc                                                                9

<210> 811
<211> 9
<212> DNA
<213> Artificial Sequence

<220>
<223> synthetic construct

<400> 811
aggctgctt                                                                9

<210> 812
<211> 9
<212> DNA
<213> Artificial Sequence

<220>
<223> synthetic construct

<400> 812
gcagccttc                                                                9

<210> 813
<211> 9
<212> DNA
<213> Artificial Sequence

<220>
<223> synthetic construct

<400> 813
gagtgcgtt                                                                9

<210> 814
<211> 9
<212> DNA
<213> Artificial Sequence

<220>
<223> synthetic construct

<400> 814
cgcactctc                                                                9

<210> 815
<211> 9
<212> DNA
<213> Artificial Sequence

<220>
<223> synthetic construct

<400> 815
ccatccatt                                                                9

<210> 822
<211> 9
<212> DNA
<213> Artificial Sequence

<220>
<223> synthetic construct

<400> 822
cgtctgttc                                                                9

<210> 823
<211> 9
<212> DNA
<213> Artificial Sequence

<220>
<223> synthetic construct

<400> 823
gagtgcctt                                                                9

<210> 824
<211> 9
<212> DNA
<213> Artificial Sequence

<220>
<223> synthetic construct

<400> 824
ggcactctc                                                                9

<210> 825
<211> 9
<212> DNA
<213> Artificial Sequence

<220>
<223> synthetic construct

<400> 825
acagacctt                                                                9

<210> 826
<211> 9
<212> DNA
<213> Artificial Sequence

<220>
<223> synthetic construct

<400> 826
ggtctgttc                                                                9

<210> 827
<211> 9
<212> DNA
<213> Artificial Sequence

<220>
<223> synthetic construct

<400> 827
cgagctttt                                                                9

<210> 828
<211> 9
<212> DNA
<213> Artificial Sequence

<220>
<223> synthetic construct

<400> 828
aagctcgtc                                                                9

<210> 829
<211> 9
<212> DNA
<213> Artificial Sequence

<220>
<223> synthetic construct

<400> 829
ttagcggtt                                                                9

<210> 830
<211> 9
<212> DNA
<213> Artificial Sequence

<220>
<223> synthetic construct

<400> 830
ccgctaatc                                                                9

<210> 831
<211> 9
<212> DNA
<213> Artificial Sequence

<220>
<223> synthetic construct

<400> 831
cctcttgtt                                                                9

<210> 832
<211> 9
<212> DNA
<213> Artificial Sequence

<220>
<223> synthetic construct

<400> 832
caagaggtc                                                                9

<210> 833
<211> 9
<212> DNA
<213> Artificial Sequence

<220>
<223> synthetic construct

<400> 833
ggtctcttt                                                                9

<210> 834
<211> 9
<212> DNA
<213> Artificial Sequence

<220>
<223> synthetic construct

<400> 834
agagacctc                                                                9

<210> 835
<211> 9
<212> DNA
<213> Artificial Sequence

<220>
<223> synthetic construct

<400> 835
gccagattt                                                                9

<210> 836
<211> 9
<212> DNA
<213> Artificial Sequence

<220>
<223> synthetic construct

<400> 836
atctggctc                                                                9

<210> 837
<211> 9
<212> DNA
<213> Artificial Sequence

<220>
<223> synthetic construct

<400> 837
gagaccttt                                                                9

<210> 838
<211> 9
<212> DNA
<213> Artificial Sequence

<220>
<223> synthetic construct

<400> 838
aggtctctc                                                                9

<210> 839
<211> 9
<212> DNA
<213> Artificial Sequence

<220>
<223> synthetic construct

<400> 839
cacacagtt                                                                9

<210> 840
<211> 9
<212> DNA
<213> Artificial Sequence

<220>
<223> synthetic construct

<400> 840
ctgtgtgtc                                                                9

<210> 841
<211> 9
<212> DNA
<213> Artificial Sequence

<220>
<223> synthetic construct

<400> 841
cctcttctt                                                                9

<210> 842
<211> 9
<212> DNA
<213> Artificial Sequence

<220>
<223> synthetic construct

<400> 842
gaagaggtc                                                                9

<210> 843
<211> 9
<212> DNA
<213> Artificial Sequence

<220>
<223> synthetic construct

<400> 843
tagagcgtt                                                                9

<210> 844
<211> 9
<212> DNA
<213> Artificial Sequence

<220>
<223> synthetic construct

<400> 844
cgctctatc                                                                9

<210> 845
<211> 9
<212> DNA
<213> Artificial Sequence

<220>
<223> synthetic construct

<400> 845
gcacctttt                                                                9

<210> 846
<211> 9
<212> DNA
<213> Artificial Sequence

<220>
<223> synthetic construct

<400> 846
aaggtgctc                                                                9

<210> 847
<211> 9
<212> DNA
<213> Artificial Sequence

<220>
<223> synthetic construct

<400> 847
ggcttgttt                                                                9

<210> 848
<211> 9
<212> DNA
<213> Artificial Sequence

<220>
<223> synthetic construct

<400> 848
acaagcctc                                                                9

<210> 849
<211> 9
<212> DNA
<213> Artificial Sequence

<220>
<223> synthetic construct

<400> 849
gacgcgatt                                                                9

<210> 850
<211> 9
<212> DNA
<213> Artificial Sequence

<220>
<223> synthetic construct

<400> 850
tcgcgtctc                                                                9

<210> 851
<211> 9
<212> DNA
<213> Artificial Sequence

<220>
<223> synthetic construct

<400> 851
cgagctgtt                                                                9

<210> 852
<211> 9
<212> DNA
<213> Artificial Sequence

<220>
<223> synthetic construct

<400> 852
cagctcgtc                                                                9

<210> 853
<211> 9
<212> DNA
<213> Artificial Sequence

<220>
<223> synthetic construct

<400> 853
tagagcctt                                                                9

<210> 854
<211> 9
<212> DNA
<213> Artificial Sequence

<220>
<223> synthetic construct

<400> 854
ggctctatc                                                                9

<210> 855
<211> 9
<212> DNA
<213> Artificial Sequence

<220>
<223> synthetic construct

<400> 855
catccgttt                                                                9

<210> 856
<211> 9
<212> DNA
<213> Artificial Sequence

<220>
<223> synthetic construct

<400> 856
acggatgtc                                                                9

<210> 857
<211> 9
<212> DNA
<213> Artificial Sequence

<220>
<223> synthetic construct

<400> 857
ggtctcgtt                                                                9

<210> 858
<211> 9
<212> DNA
<213> Artificial Sequence

<220>
<223> synthetic construct

<400> 858
cgagacctc                                                                9

<210> 859
<211> 9
<212> DNA
<213> Artificial Sequence

<220>
<223> synthetic construct

<400> 859
gccagagtt                                                                9

<210> 860
<211> 9
<212> DNA
<213> Artificial Sequence

<220>
<223> synthetic construct

<400> 860
ctctggctc                                                                9

<210> 861
<211> 9
<212> DNA
<213> Artificial Sequence

<220>
<223> synthetic construct

<400> 861
gagaccgtt                                                                9

<210> 862
<211> 9
<212> DNA
<213> Artificial Sequence

<220>
<223> synthetic construct

<400> 862
cggtctctc                                                                9

<210> 863
<211> 9
<212> DNA
<213> Artificial Sequence

<220>
<223> synthetic construct

<400> 863
cgagctatt                                                                9

<210> 864
<211> 9
<212> DNA
<213> Artificial Sequence

<220>
<223> synthetic construct

<400> 864
tagctcgtc                                                              9

<210> 865
<211> 9
<212> DNA
<213> Artificial Sequence

<220>
<223> synthetic construct

<400> 865
gcaagtgtt                                                              9

<210> 866
<211> 9
<212> DNA
<213> Artificial Sequence

<220>
<223> synthetic construct

<400> 866
cacttgctc                                                              9

<210> 867
<211> 9
<212> DNA
<213> Artificial Sequence

<220>
<223> synthetic construct

<400> 867
ggtctcctt                                                              9

<210> 868
<211> 9
<212> DNA
<213> Artificial Sequence

<220>
<223> synthetic construct

<400> 868
ggagacctc                                                              9

<210> 869
<211> 9
<212> DNA
<213> Artificial Sequence

<220>
<223> synthetic construct

<400> 869
gccagactt                                                              9

<210> 870
<211> 9
<212> DNA
<213> Artificial Sequence

<220>
<223> synthetic construct

<400> 870
gtctggctc                                                                9

<210> 871
<211> 9
<212> DNA
<213> Artificial Sequence

<220>
<223> synthetic construct

<400> 871
ggtctcatt                                                                9

<210> 872
<211> 9
<212> DNA
<213> Artificial Sequence

<220>
<223> synthetic construct

<400> 872
tgagacctc                                                                9

<210> 873
<211> 9
<212> DNA
<213> Artificial Sequence

<220>
<223> synthetic construct

<400> 873
gagaccatt                                                                9

<210> 874
<211> 9
<212> DNA
<213> Artificial Sequence

<220>
<223> synthetic construct

<400> 874
tggtctctc                                                                9

<210> 875
<211> 9
<212> DNA
<213> Artificial Sequence

<220>
<223> synthetic construct

<400> 875
ccttcagtt                                                                9

<210> 876
<211> 9
<212> DNA
<213> Artificial Sequence

<220>
<223> synthetic construct

<400> 876
ctgaaggtc                                                                9

<210> 877
<211> 9
<212> DNA
<213> Artificial Sequence

<220>
<223> synthetic construct

<400> 877
gcacctgtt                                                                9

<210> 878
<211> 9
<212> DNA
<213> Artificial Sequence

<220>
<223> synthetic construct

<400> 878
caggtgctc                                                                9

<210> 879
<211> 9
<212> DNA
<213> Artificial Sequence

<220>
<223> synthetic construct

<400> 879
aaaggcgtt                                                                9

<210> 880
<211> 9
<212> DNA
<213> Artificial Sequence

<220>
<223> synthetic construct

<400> 880
cgcctttc                                                                 9

<210> 881
<211> 9
<212> DNA
<213> Artificial Sequence

<220>
<223> synthetic construct

<400> 881
cagatcgtt                                                                9

<210> 882
<211> 9
<212> DNA
<213> Artificial Sequence

<220>
<223> synthetic construct

<400> 882
cgatctgtc                                                                9

<210> 883
<211> 9
<212> DNA
<213> Artificial Sequence

<220>
<223> synthetic construct

<400> 883
cataggctt                                                                9

<210> 884
<211> 9
<212> DNA
<213> Artificial Sequence

<220>
<223> synthetic construct

<400> 884
gcctatgtc                                                                9

<210> 885
<211> 9
<212> DNA
<213> Artificial Sequence

<220>
<223> synthetic construct

<400> 885
ccttcactt                                                                9

<210> 886
<211> 9
<212> DNA
<213> Artificial Sequence

<220>
<223> synthetic construct

<400> 886
gtgaaggtc                                                                9

<210> 887
<211> 9
<212> DNA
<213> Artificial Sequence

<220>
<223> synthetic construct

<400> 887
gcacctctt                                                                9

<210> 888
<211> 9
<212> DNA
<213> Artificial Sequence

<220>
<223> synthetic construct

<400> 888
gaggtgctc                                                              9

<210> 889
<211> 25
<212> DNA
<213> Artificial Sequence

<220>
<223> synthetic construct

<400> 889
cagaagacag acaagcttca cctgc                                           25

<210> 890
<211> 27
<212> DNA
<213> Artificial Sequence

<220>
<223> synthetic construct

<400> 890
gcaggtgaag cttgtctgtc ttctgaa                                         27

## Description

### Related Applications

This application is a continuation-in-part of U.S. Patent Application No. 11/015458 filed December 17, 2004, pending, which is related to U.S. Provisional Patent Application Serial No. 60/530854, filed on December 17, 2003; U.S. Provisional Patent Application Serial No. 60/540681, filed on January 30, 2004; U.S. Provisional Patent Application Serial No. 60/553,715 filed March 15, 2004; and U.S. Provisional Patent Application Serial No. 60/588,672 filed July 16, 2004. This application also claims priority to U.S. Provisional Patent Application Serial No. 60/689,466, filed June 9, 2005 and U.S. Provisional Patent Application Serial No. 60/731,041 filed October 28, 2005. The entire contents of each of the foregoing applications are incorporated herein by reference.

### Background of the invention

The search for more efficient methods of identifying compounds having useful biological activities has led to the development of methods for screening vast numbers of distinct compounds, present in collections referred to as combinatorial libraries. Such libraries can include 10⁵ or more distinct compounds. A variety of methods exist for producing combinatorial libraries, and combinatorial syntheses of peptides, peptidomimetics and small organic molecules have been reported.

The two major challenges in the use of combinatorial approaches in drug discovery are the synthesis of libraries of sufficient complexity and the identification of molecules which are active in the screens used. It is generally acknowledged that greater the degree of complexity of a library, i.e., the number of distinct structures present in the library, the greater the probability that the library contains molecules with the activity of interest. Therefore, the chemistry employed in library synthesis must be capable of producing vast numbers of compounds within a reasonable time frame. However, for a given formal or overall concentration, increasing the number of distinct members within the library lowers the concentration of any particular library member. This complicates the identification of active molecules from high complexity libraries.

One approach to overcoming these obstacles has been the development of encoded libraries, and particularly libraries in which each compound includes an amplifiable tag. Such libraries include DNA-encoded libraries, in which a DNA tag identifying a library member can be amplified using techniques of molecular biology, such as the polymerase chain reaction. However, the use of such methods for producing very large libraries is yet to be demonstrated, and it is clear that improved methods for producing such libraries are required for the realization of the potential of this approach to drug discovery.

### Summary of the invention

The present invention provides a method of synthesizing libraries of molecules which include an encoding oligonucleotide tag. The method utilizes a "split and pool" strategy in which a solution comprising an initiator, comprising a first building block linked to an encoding oligonucleotide, is divided ("split") into multiple fractions. In each fraction, the initiator is reacted with a second, unique, building block and a second, unique oligonucleotide which identifies the second building block. These reactions can be simultaneous or sequential and, if sequential, either reaction can precede the other. The dimeric molecules produced in each of the fractions are combined ("pooled") and then divided again into multiple fractions. Each of these fractions is then reacted with a third unique (fraction-specific) building block and a third unique oligonucleotide which encodes the building block. The number of unique molecules present in the product library is a function of (1) the number of different building blocks used at each step of the synthesis, and (2) the number of times the pooling and dividing process is repeated.

In one embodiment, the invention provides a method of synthesizing a molecule comprising or consisting of a functional moiety which is operatively linked to an encoding oligonucleotide. The method includes the steps of: (1) providing an initiator compound consisting of a functional moiety comprising n building blocks, where n is an integer of 1 or greater, wherein the functional moiety comprises at least one reactive group and wherein the functional moiety is operatively linked to an initial oligonucleotide; (2) reacting the initiator compound with a building block comprising at least one complementary reactive group, wherein the at least one complementary reactive group is complementary to the reactive group of step (1), under suitable conditions for reaction of the reactive group and the complementary reactive group to form a covalent bond; (3) reacting the initial oligonucleotide with an incoming oligonucleotide which identifies the building block of step (b) in the presence of an enzyme which catalyzes ligation of the initial oligonucleotide and the incoming oligonucleotide, under conditions suitable for ligation of the incoming oligonucleotide and the initial oligonucleotide, thereby producing a molecule which comprises or consists of a functional moiety comprising n+1 building blocks which is operatively linked to an encoding oligonucleotide. If the functional moiety of step (3) comprises a reactive group, steps 1-3 can repeated one or more times, thereby forming cycles 1 to i, where i is an integer of 2 or greater, with the product of step (3) of a cycle s, where s is an integer of i-1 or less, becoming the initiator compound of cycle s+1.

In one embodiment, the invention provides a method of synthesizing a library of compounds, wherein the compounds comprise a functional moiety comprising two or more building blocks which is operatively linked to an oligonucleotide which identifies the structure of the functional moiety. The method comprises the steps of (1) providing a solution comprising m initiator compounds, wherein m is an integer of 1 or greater, where the initiator compounds consist of a functional moiety comprising n building blocks, where n is an integer of 1 or greater, which is operatively linked to an initial oligonucleotide which identifies the n building blocks; (2) dividing the solution of step (1) into r fractions, wherein r is an integer of 2 or greater; (3) reacting the initiator compounds in each fraction with one of building blocks, thereby producing r fractions comprising compounds consisting of a functional moiety comprising n+1 building blocks operatively linked to the initial oligonucleotide; (4) reacting the initial oligonucleotide in each faction with one of a set of r distinct incoming oligonucleotides in the presence of an enzyme which catalyzes the ligation of the incoming oligonucleotide and the initial oligonucleotide, under conditions suitable for enzymatic ligation of the incoming oligonucleotide and the initial oligonucleotide, thereby producing r aliquots comprising molecules consisting of a functional moiety comprising n+1 building blocks operatively linked to an elongated oligonucleotide which encodes the n+1 building blocks. Optionally, the method can further include the step of (5) recombining the r fractions produced in step (4), thereby producing a solution comprising compounds consisting of a functional moiety comprising n+1 building blocks, which is operatively linked to an elongated oligonucleotide. Steps (1) to (5) can be conducted one or more times to yield cycles 1 to i, where i is an integer of 2 or greater. In cycle s+1, where s is an integer of i-1 or less, the solution comprising m initiator compounds of step (1) is the solution of step (5) of cycle s. Likewise, the initiator compounds of step (1) of cycle s+1 are the compounds of step (5) of cycle s.

In a preferred embodiment, the building blocks are coupled in each step using conventional chemical reactions. The building blocks can be coupled to produce linear or branched polymers or oligomers, such as peptides, peptidomimetics, and peptoids, or non-oligomeric molecules, such as molecules comprising a scaffold structure to which is attached one or more additional chemical moieties.. For example, if the building blocks are amino acid residues, the building blocks can be coupled using standard peptide synthesis strategies, such as solution-phase or solid phase synthesis using suitable protection/deprotection strategies as are known in the field. Preferably, the building blocks are coupled using solution phase chemistry. The encoding oligonucleotides are single stranded or double stranded oligonucleotides, preferably double-stranded oligonucleotides. The encoding oligonucleotides are preferably oligonucleotides of 4 to 12 bases or base pairs per building block; the encoding oligonucleotides can be coupled using standard solution phase or solid phase oligonucleotide synthetic methodology, but are preferably coupled using a solution phase enzymatic process. For example, the oligonucleotides can be coupled using a topoisomerase, a ligase, or a DNA polymerase, if the sequence of the encoding oligonucleotides includes an initiation sequence for ligation by one of these enzymes. Enzymatic coupling of the encoding oligonucleotides offers the advantages of (1) greater accuracy of addition compared to standard synthetic (non-enzymatic) coupling; and (2) the use of a simpler protection/deprotection strategy.

In another aspect, the invention provides compounds of Formula 1: 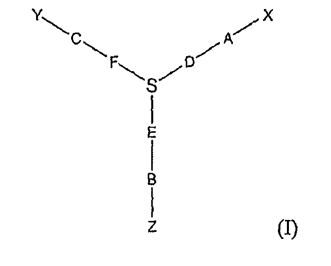 where X is a functional moiety comprising one or more building blocks; Z is an oligonucleotide attached at its 3' terminus to B; Y is an oligonucleotide which is attached at its 5' terminus to C; A is a functional group that forms a covalent bond with X; B is a functional group that forms a bond with the 3'-end of Z; C is a functional group that forms a bond with the 5'-end of Y; D, F and E are each, independently, a bifunctional linking group; and S an atom or a molecular scaffold. Such compounds include those which are synthesized using the methods of the invention.

The invention further relates to a compound library comprising compounds comprising a functional moiety comprising two or more building blocks which is operatively linked to an oligonucleotide which encodes the structure of the functional moiety. Such libraries can comprise from about 10² to about 10¹² or more distinct members, for example, 10², 10³, 10¹, 10⁵, 10⁶, 10⁷, 10¹, 10⁹, 10¹⁰, 1¹¹ 10¹² or more distinct members, i.e., distinct molecular structures.

In one embodiment, the compound library comprises compounds which are each independently of Formula I: 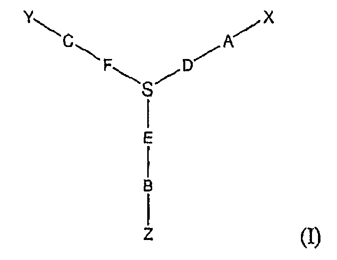 where X is a functional moiety comprising one or more building blocks; Z is an oligonucleotide attached at its 3' terminus to B; Y is an oligonucleotide which is attached at its 5' terminus to C; A is a functional group that forms a covalent bond with X; B is a functional group that forms a bond with the 3'-end of Z; C is a functional group that forms a bond with the 5'-end of Y; D, F and E are each, independently, bifunctional linking group; and S an atom or a molecular scaffold. Such libraries include those which are synthesized using the methods of the invention.

In another aspect, the invention provides a method for identifying a compound which binds to a biological target, said method comprising the steps of: (a)contacting the biological target with a compound library of the invention, where the compound library includes compounds which comprise a functional moiety comprising two or more building blocks which is operatively linked to an oligonucleotide which encodes the structure of the functional moiety. This step is conducted under conditions suitable for at least one member of the compound library to bind to the target; (2) removing library members that do not bind to the target; (3) amplifying the encoding oligonucleotides of the at least one member of the compound library which binds to the target; (4) sequencing the encoding oligonucleotides of step (3); and using the sequences determined in step (5) to determine the structure of the functional moieties of the members of the compound library which bind to the biological target.

The present invention provides several advantages in the identification of molecules having a desired property. For example, the methods of the invention allow the use of a range of chemical reactions for constructing the molecules in the presence of the oligonucleotide tag. The methods of the invention also provide a high-fidelity means of incorporating oligonucleotide tags into the chemical structures so produced. Further, they enable the synthesis of libraries having a large number of copies of each member, thereby allowing multiple rounds of selection against a biological target while leaving a sufficient number of molecules following the final round for amplification and sequence of the oligonucleotide tags.

### Brief description of the drawings

Figure 1 is a schematic representation of ligation of double stranded oligonucleotides, in which the initial oligonucleotide has an overhang which is complementary to the overhang of the incoming oligonucleotide. The initial strand is represented as either free, conjugated to an aminohexyl linker or conjugated to a phenylalanine residue via an aminohexyl linker.
Figure 2 is a schematic representation of oligonucleotide ligation using a splint strand. In this embodiment, the splint is a 12-mer oligonucleotide with sequences complementary to the single-stranded initial oligonucleotide and the single-stranded incoming oligonucleotide.
Figure 3 is a schematic representation of ligation of an initial oligonucleotide and an incoming oligonucleotide, when the initial oligonucleotide is double-stranded with covalently linked strands, and the incoming oligonucleotide is double-stranded.
Figure 4 is a schematic representation of oligonucleotide elongation using a polymerase. The initial strand is represented as either free, conjugated to an aminohexyl linker or conjugated to a phenylalanine residue via an aminohexyl linker.
Figure 5 is a schematic representation of the synthesis cycle of one embodiment of the invention.
Figure 6 is a schematic representation of a multiple round selection process using the libraries of the invention.
Figure 7 is a gel resulting from electrophoresis of the products of each of cycles 1 to 5 described in Example 1 and following ligation of the closing primer. Molecular weight standards are shown in lane 1, and the indicated quantities of a hyperladder, for DNA quantitation, are shown in lanes 9 to 12.
Figure 8 is a schematic depiction of the coupling of building blocks using azide-alkyne cycloaddition.
Figures 9 and 10 illustrate the coupling of building blocks via nucleophilic aromatic substitution on a chlorinated triazine.
Figure 11 shows representative chlorinated heteroaromatic structures suitable for use in the synthesis of functional moieties.

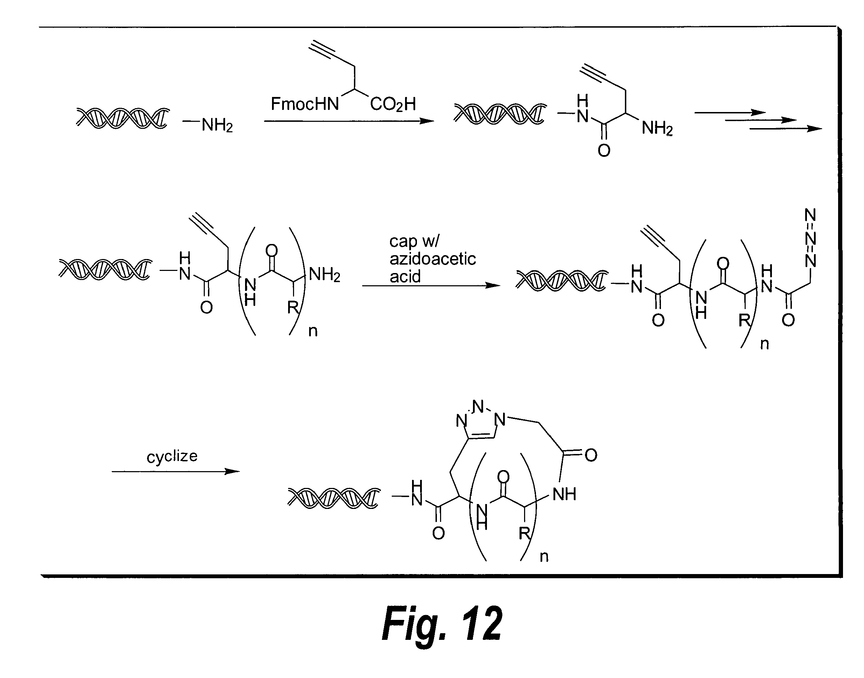

Figure 12 illustrates the cyclization of a linear peptide using the azide/alkyne cycloaddition reaction.
Figure 13a is a chromatogram of the library produced as described in Example 2 follwing Cycle 4.
Figure 13b is a mass spectrum of the library produced as described in Example 2 following Cycle 4.

### Detailed description of the invention

The present invention relates to methods of producing compounds and combinatorial compound libraries, the compounds and libraries produced via the methods of the invention, and methods of using the libraries to identify compounds having a desired property, such as a desired biological activity. The invention further relates to the compounds identified using these methods.

A variety of approaches have been taken to produce and screen combinatorial chemical libraries. Examples include methods in which the individual members of the library are physically separated from each other, such as when a single compound is synthesized in each of a multitude of reaction vessels. However, these libraries are typically screened one compound at a time, or at most, several compounds at a time and do not, therefore, result in the most efficient screening process. In other methods, compounds are synthesized on solid supports. Such solid supports include chips in which specific compounds occupy specific regions of the chip or membrane ("position addressable"). In other methods, compounds are synthesized on beads, with each bead containing a different chemical structure.

Two difficulties that arise in screening large libraries are (1) the number of distinct compounds that can be screened; and (2) the identification of compounds which are active in the screen. In one method, the compounds which are active in the screen are identified by narrowing the original library into ever smaller fractions and subfractions, in each case selecting the fraction or subfraction which contains active compounds and further subdividing until attaining an active subfraction which contains a set of compounds which is sufficiently small that all members of the subset can be individually synthesized and assessed for the desired activity. This is a tedious and time consuming activity.

Another method of deconvoluting the results of a combinatorial library screen is to utilize libraries in which the library members are tagged with an identifying label, that is, each label present in the library is associated with a discreet compound structure present in the library, such that identification of the label tells the structure of the tagged molecule. One approach to tagged libraries utilizes oligonucleotide tags, as described, for example, in US Patent Nos. 5,573,905; 5,708,153; 5,723,598, 6,060,596 published PCT applications WO 93/06121; WO 93/20242; WO 94/13623; WO 00/23458; WO 02/074929 and WO 02/103008, and by Brenner and Lerner (Proc. Natl. Acad. Sci. USA 89, 5381-5383 (1992); Nielsen and Janda (Methods: A Companion to Methods in Enzymology 6, 361-371 (1994); and Nielsen, Brenner and Janda (J. Am. Chem. Soc. 115, 9812-9813 (1993)), each of which is incorporated herein by reference in its entirety. Such tags can be amplified, using for example, polymerase chain reaction, to produce many copies of the tag and identify the tag by sequencing. The sequence of the tag then identifies the structure of the binding molecule, which can be synthesized in pure form and tested. The present invention provides an improvement in methods to produce DNA-encoded libraries, as well as the first examples of large (10⁵ members or greater) libraries of DNA-encoded molecules in which the functional moiety is synthesized using solution phase synthetic methods.

The present invention provides methods which enable facile synthesis of oligonucleotide-encoded combinatorial libraries, and permit an efficient, high-fidelity means of adding such an oligonucleotide tag to each member of a vast collection of molecules.

The methods of the invention include methods for synthesizing bifunctional molecules which comprise a first moiety ("functional moiety") which is made up of building blocks, and a second moiety operatively linked to the first moiety, comprising an oligonucleotide tag which identifies the structure of the first moiety, i.e., the oligonucleotide tag indicates which building blocks were used in the construction of the first moiety, as well as the order in which the building blocks were linked. Generally, the information provided by the oligonucleotide tag is sufficient to determine the building blocks used to construct the active moiety. In certain embodiments, the sequence of the oligonucleotide tag is sufficient to determine the arrangement of the building blocks in the functional moiety, for example, for peptidic moieties, the amino acid sequence.

The term "functional moiety" as used herein, refers to a chemical moiety comprising one or more building blocks. Preferably, the building blocks in the functional moiety are not nucleic acids. The functional moiety can be a linear or branched or cyclic polymer or oligomer or a small organic molecule.

The term "building block", as used herein, is a chemical structural unit which is linked to other chemical structural units or can be linked to other such units. When the functional moiety is polymeric or oligomeric, the building blocks are the monomeric units of the polymer or oligomer. Building blocks can also include a scaffold structure ("scaffold building block") to which is, or can be, attached one or more additional structures ("peripheral building blocks").

It is to be understood that the term "building block" is used herein to refer to a chemical structural unit as it exists in a functional moiety and also in the reactive form used for the synthesis of the functional moiety. Within the functional moiety, a building block will exist without any portion of the building block which is lost as a consequence of incorporating the building block into the functional moiety. For example, in cases in which the bond-forming reaction releases a small molecule (see below), the building block as it exists in the functional moiety is a "building block residue", that is, the remainder of the building block used in the synthesis following loss of the atoms that it contributes to the released molecule.

The building blocks can be any chemical compounds which are complementary, that is the building blocks must be able to react together to form a structure comprising two or more building blocks. Typically, all of the building blocks used will have at least two reactive groups, although it is possible that some of the building blocks (for example the last building block in an oligomeric functional moiety) used will have only one reactive group each. Reactive groups on two different building blocks should be complementary, i.e., capable of reacting together to form a covalent bond, optionally with the concomitant loss of a small molecule, such as water, HCl, HF, and so forth.

For the present purposes, two reactive groups are complementary if they are capable of reacting together to form a covalent bond. In a preferred embodiment, the bond forming reactions occur rapidly under ambient conditions without substantial formation of side products. Preferably, a given reactive group will react with a given complementary reactive group exactly once. In one embodiment, complementary reactive groups of two building blocks react, for example, via nucleophilic substitution, to form a covalent bond. In one embodiment, one member of a pair of complementary reactive groups is an electrophilic group and the other member of the pair is a nucleophilic group.

Complementary electrophilic and nucleophilic groups include any two groups which react via nucleophilic substitution under suitable conditions to form a covalent bond. A variety of suitable bond-forming reactions are known in the art. See, for example, March, Advanced Organic Chemistry, fourth edition, New York: John Wiley and Sons (1992), Chapters 10 to 16; Carey and Sundberg, Advanced Organic Chemistry, Part B, Plenum (1990), Chapters 1-11; and Collman et al., Principles and Applications of Organotransition Metal Chemistry, University Science Books, Mill Valley, Calif. (1987), Chapters 13 to 20; each of which is incorporated herein by reference in its entirety. Examples of suitable electrophilic groups include reactive carbonyl groups, such as acyl chloride groups, ester groups, including carbonyl pentafluorophenyl esters and succinimide esters, ketone groups and aldehyde groups; reactive sulfonyl groups, such as sulfonyl chloride groups, and reactive phosphonyl groups. Other electrophilic groups include terminal epoxide groups, isocyanate groups and alkyl halide groups. Suitable nucleophilic groups include primary and secondary amino groups and hydroxyl groups and carboxyl groups.

Suitable complementary reactive groups are set forth below. One of skill in the art can readily determine other reactive group pairs that can be used in the present method, and the examples provided herein are not intended to be limiting.

In a first embodiment, the complementary reactive groups include activated carboxyl groups, reactive sulfonyl groups or reactive phosphonyl groups, or a combination thereof, and primary or secondary amino groups. In this embodiment, the complementary reactive groups react under suitable conditions to form an amide, sulfonamide or phosphonamidate bond.

In a second embodiment, the complementary reactive groups include epoxide groups and primary or secondary amino groups. An epoxide-containing building block reacts with an amine-containing building block under suitable conditions to form a carbon-nitrogen bond, resulting in a β-amino alcohol.

In another embodiment, the complementary reactive groups include aziridine groups and primary or secondary amino groups. Under suitable conditions, an aziridine-containing building block reacts with an amine-containing building block to form a carbon-nitrogen bond, resulting in a 1,2-diamine. In a third embodiment, the complementary reactive groups include isocyanate groups and primary or secondary amino groups. An isocyanate-containing building block will react with an amino-containing building block under suitable conditions to form a carbon-nitrogen bond, resulting in a urea group.

In a fourth embodiment, the complementary reactive groups include isocyanate groups and hydroxyl groups.An isocyanate-containing building block will react with an hydroxyl-containing building block under suitable conditions to form a carbon-oxygen bond, resulting in a carbamate group.

In a fifth embodiment, the complementary reactive groups include amino groups and carbonyl-containing groups, such as aldehyde or ketone groups. Amines react with such groups via reductive animation to form a new carbon-nitrogen bond.

In a sixth embodiment, the complementary reactive groups include phosphorous ylide groups and aldehyde or ketone groups. A phosphorus-ylide-containing building block will react with an aldehyde or ketone-containing building block under suitable conditions to form a carbon-carbon double bond, resulting in an alkene.

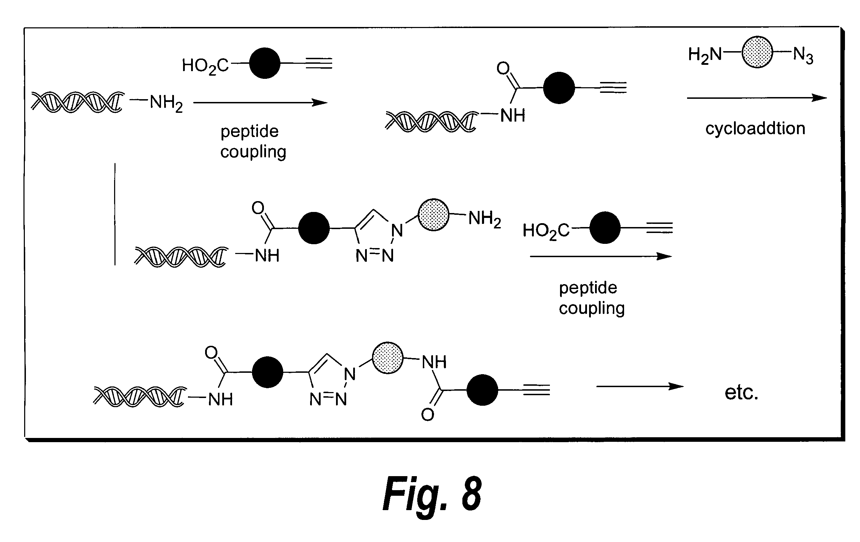

In a seventh embodiment, the complementary reactive groups react via cycloaddition to form a cyclic structure. One example of such complementary reactive groups are alkynes and organic azides, which react under suitable conditions to form a triazole ring structure. An example of the use of this reaction to link two building blocks is illustrated in Figure 8. Suitable conditions for such reactions are known in the art and include those disclosed in WO 03/101972, the entire contents of which are incorporated by reference herein.

In an eighth embodiment, the complementary reactive groups are an alkyl halide and a nucleophile, such as an amino group, a hydroxyl group or a carboxyl group. Such groups react under suitable conditions to form a carbon-nitrogen (alkyl halide plus amine) or carbon oxygen (alkyl halide plus hydroxyl or carboxyl group).

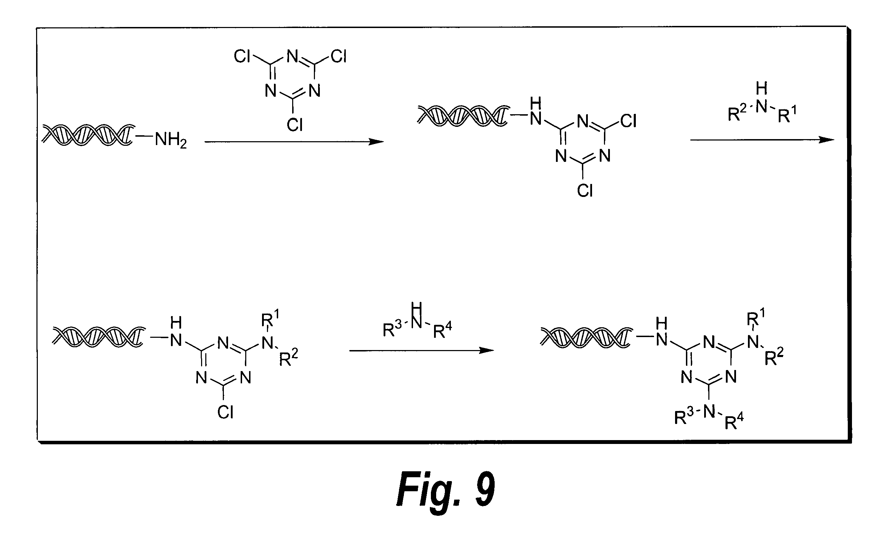

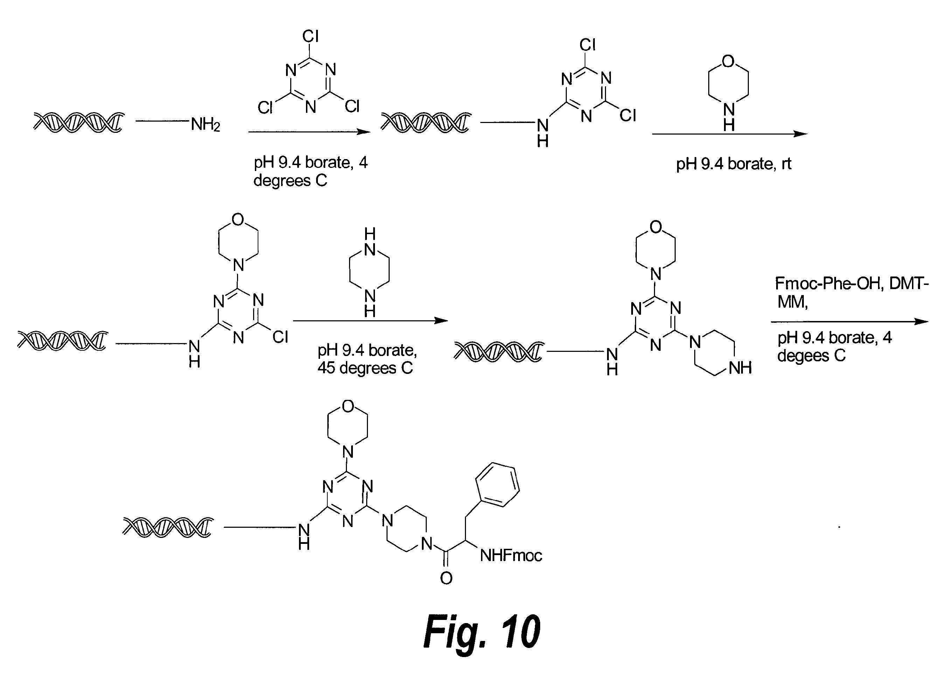

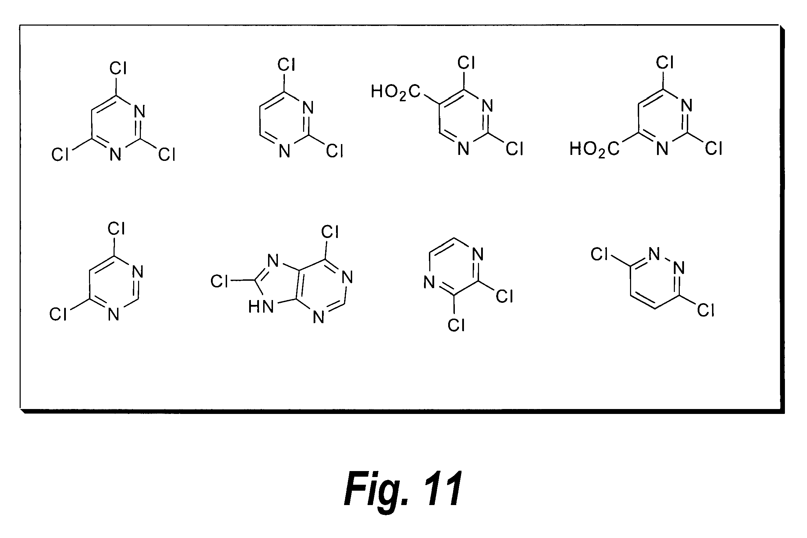

In a ninth embodiment, the complementary functional groups are a halogenated heteroaromatic group and a nucleophile, and the building blocks are linked under suitable conditions via aromatic nucleophilic substitution. Suitable halogenated heteroaromatic groups include chlorinated pyrimidines, triazines and purines, which react with nucleophiles, such as amines, under mild conditions in aqueous solution. Representative examples of the reaction of an oligonucleotide-tagged trichlorotriazine with amines are shown in Figures 9 and 10. Examples of suitable chlorinated heteroaromatic groups are shown in Figure 11.

Additional bond-forming reactions that can be used to join building blocks in the synthesis of the molecules and libraries of the invention include those shown below. The reactions shown below emphasize the reactive functional groups. Various substituents can be present in the reactants, including those labeled R₁, R₂, R₃ and R₄. The possible positions which can be substituted include, but are not limited, to those indicated by R₁, R₂, R₃ and R₄. These substituents can include any suitable chemical moieties, but are preferably limited to those which will not interfere with or significantly inhibit the indicated reaction, and, unless otherwise specified, can include hydrogen, alkyl, substituted alkyl, aryl, substituted aryl, heteroaryl, substituted heteroaryl, alkoxy, aryloxy, arylalkyl, substituted arylalkyl, amino, substituted amino and others as are known in the art. Suitable substituents on these groups include alkyl, aryl, heteroaryl, cyano, halogen, hydroxyl, nitro, amino, mercapto, carboxyl, and carboxamide. Where specified, suitable electron-withdrawing groups include nitro, carboxyl, haloalkyl, such as trifluoromethyl and others as are known in the art. Examples of suitable electron-donating groups include alkyl, alkoxy, hydroxyl, amino, halogen, acetamido and others as are known in the art. Addition of a primary amine to an alkene:

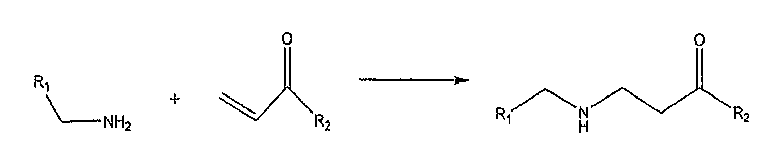

### Nucleophilic substitution:

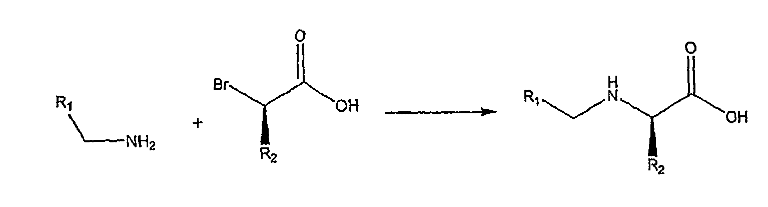

### Reductive alkylation of an amines:

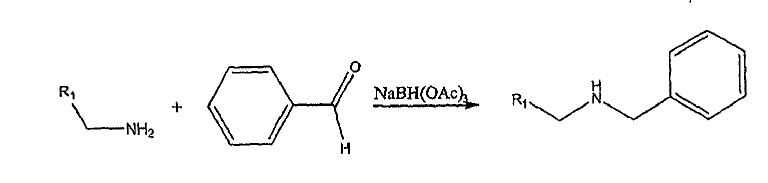

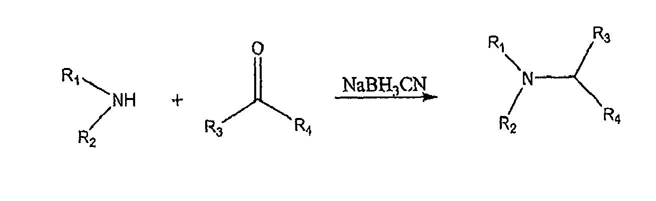

### Palladium catalyzed carbon-carbon bond forming reactions:

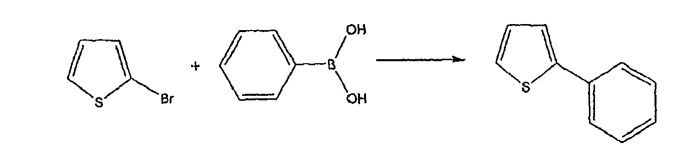

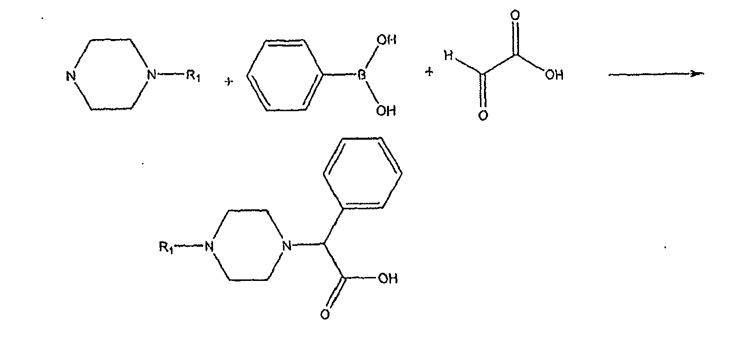

### Ugi condensation reactions:

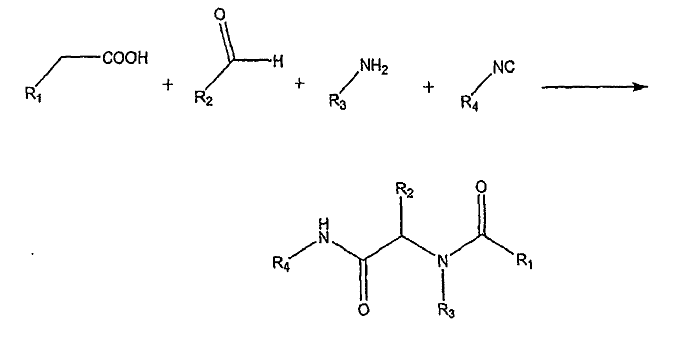

### Electrophilic aromatic substitution reactions:

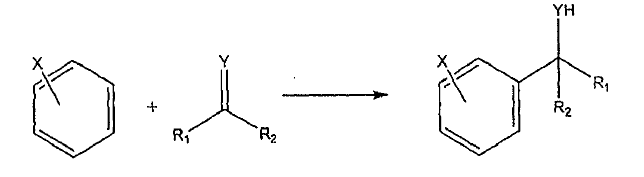

X is an electron-donating group.

### Imine/iminium/enamine forming reactions:

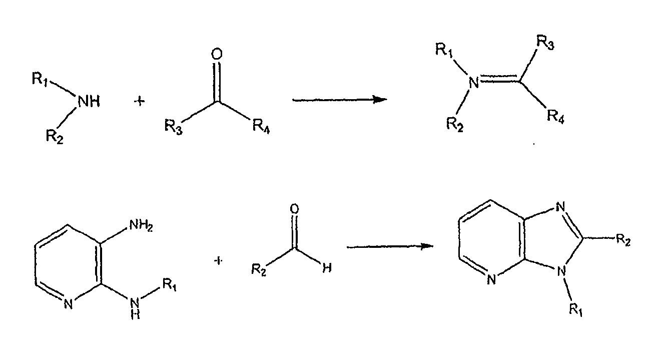

### Cycloaddition reactions:

Diels-Alder cycloaddition

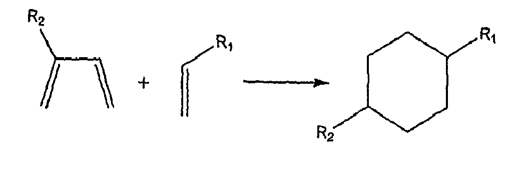

1,3-dipolar cycloaddition, X-Y-Z = C-N-O, C-N-S, N₃,

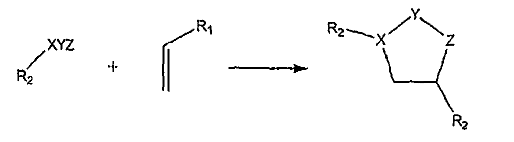

Nucleophilic aromatic substitution reactions: 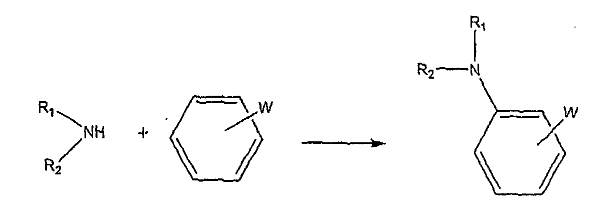 W is an election withdrawing group

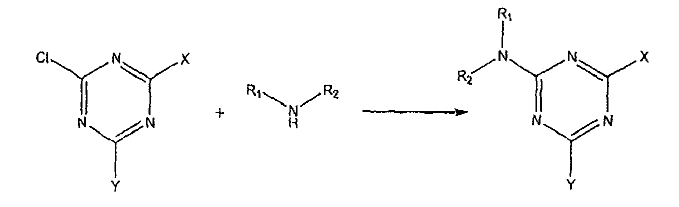

Examples of suitable substituents X and Y include substituted or unsubstituted amino, substituted or unsubstituted alkoxy, substituted or unsubstituted thioalkoxy, substituted or unsubstituted aryloxy and substituted and unsubstituted thioaryloxy.

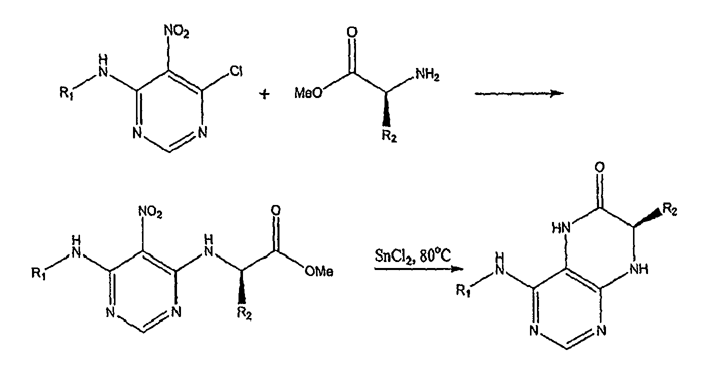

### Heck reaction:

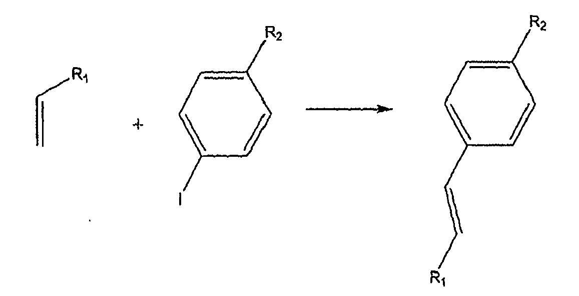

### Acetal formation:

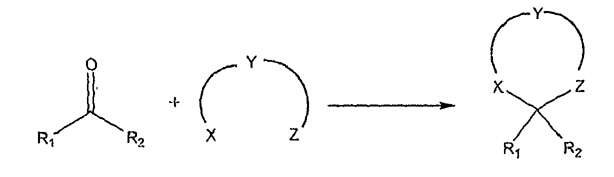

Examples of suitable substituents X and Y include substituted and unsubstituted amino, hydroxyl and sulhydryl; Y is a linker that connects X and Y and is suitable for forming the ring structure found in the product of the reaction

### Aldol reactions:

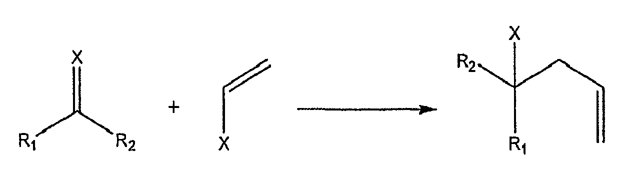

### Examples of suitable substituents X include O, S and NR₃.

Scaffold building blocks which can be used to form the molecules and libraries of the invention include those which have two or more functional groups which can participate in bond forming reactions with peripheral building block precursors, for example, using one or more of the bond forming reactions discussed above. Scaffold moieties may also be synthesized during construction of the libraries and molecules of the invention, for example, using building block precursors which can react in specific ways to form molecules comprising a central molecular moiety to which are appended peripheral functional groups. In one embodiment, a library of the invention comprises molecules comprising a constant scaffold moiety, but different peripheral moieties or different arrangements of peripheral moieties. In certain libraries, all library members comprise a constant scaffold moiety; other libraries can comprise molecules having two or more different scaffold moieties. Examples of scaffold moiety-forming reactions that can be used in the construction of the molecules and libraries of the invention are set forth in Table 8. The references cited in the table are incorporated herein by reference in their entirety. The groups R₁, R₂, R₃ and R₄ are limited only in that they should not interfere with, or significantly inhibit, the indicated reaction, and can include hydrogen, alkyl, substituted alkyl, heteroalkyl, substituted heteroalkyl, cycloalkyl, heterocycloalkyl, substituted cycloalkyl, substituted heterocycloalkyl, aryl, substituted aryl, arylalkyl, heteroarylalkyl, substituted arylalkyl, substituted heteroarylalkyl, heteroaryl, substituted heteroaryl, halogen, alkoxy, aryloxy, amino, substituted amino and others as are known in the art. Suitable substituents include, but are not limited to, alkyl, alkoxy, thioalkoxy, nitro, hydroxyl, sulfhydryl, aryloxy, aryl-S-, halogen, carboxy, amino, alkylamino, dialkylamino, arylamino, cyano, cyanate, nitrile, isocyanate, thiocyanate, carbamyl, and substituted carbamyl.

It is to be understood that the synthesis of a functional moiety can proceed via one particular type of coupling reaction, such as, but not limited to, one of the reactions discussed above, or via a combination of two or more coupling reactions, such as two or more of the coupling reactions discussed above. For example, in one embodiment, the building blocks are joined by a combination of amide bond formation (amino and carboxylic acid complementary groups) and reductive amination (amino and aldehyde or ketone complementary groups). Any coupling chemistry can be used, provided that it is compatible with the presence of an oligonucleotide. Double stranded (duplex) oligonucleotide tags, as used in certain embodiments of the present invention, are chemically more robust than single stranded tags, and, therefore, tolerate a broader range of reaction conditions and enable the use of bond-forming reactions that would not be possible with single-stranded tags.

A building block can include one or more functional groups in addition to the reactive group or groups employed to form the functional moiety. One or more of these additional functional groups can be protected to prevent undesired reactions of these functional groups. Suitable protecting groups are known in the art for a variety of functional groups (Greene and Wuts, Protective Groups in Organic Synthesis, second edition, New York: John Wiley and Sons (1991), incorporated herein by reference). Particularly useful protecting groups include t-butyl esters and ethers, acetals, trityl ethers and amines, acetyl esters, trimethylsilyl ethers, trichloroethyl ethers and esters and carbamates.

In one embodiment, each building block comprises two reactive groups, which can be the same or different. For example, each building block added in cycle s can comprise two reactive groups which are the same, but which are both complementary to the reactive groups of the building blocks added at steps s-1 and s + 1. In another embodiment, each building block comprises two reactive groups which are themselves complementary. For example, a library comprising polyamide molecules can be produced via reactions between building blocks comprising two primary amino groups and building blocks comprising two activated carboxyl groups. In the resulting compounds there is no N- or C-terminus, as alternate amide groups have opposite directionality. Alternatively, a polyamide library can be produced using building blocks that each comprise an amino group and an activated carboxyl group. In this embodiment, the building blocks added in step n of the cycle will have a free reactive group which is complementary to the available reactive group on the n-1 building block, while, preferably, the other reactive group on the nth building block is protected. For example, if the members of the library are synthesized from the C to N direction, the building blocks added will comprise an activated carboxyl group and a protected amino group.

The functional moieties can be polymeric or oligomeric moieties, such as peptides, peptidomimetics, peptide nucleic acids or peptoids, or they can be small non-polymeric molecules, for example, molecules having a structure comprising a central scaffold and structures arranged about the periphery of the scaffold. Linear polymeric or oligomeric libraries will result from the use of building blocks having two reactive groups, while branched polymeric or oligomeric libraries will result from the use of building blocks having three or more reactive groups, optionally in combination with building blocks having only two reactive groups. Such molecules can be represented by the general formula X₁X₂...Xₙ, where each X is a monomeric unit of a polymer comprising n monomeric units, where n is an integer greater than 1 In the case of oligomeric or polymeric compounds, the terminal building blocks need not comprise two functional groups. For example, in the case of a polyamide library, the C-terminal building block can comprise an amino group, but the presence of a carboxyl group is optional. Similarly, the building block at the N-terminus can comprise a carboxyl group, but need not contain an amino group.

Branched oligomeric or polymeric compounds can also be synthesized provided that at least one building block comprises three functional groups which are reactive with other building blocks. A library of the invention can comprise linear molecules, branched molecules or a combination thereof.

Libraries can also be constructed using, for example, a scaffold building block having two or more reactive groups, in combination with other building blocks having only one available reactive group, for example, where any additional reactive groups are either protected or not reactive with the other reactive groups present in the scaffold building block. In one embodiment, for example, the molecules synthesized can be represented by the general formula X(Y)ₙ, where X is a scaffold building block; each Y is a building block linked to X and n is an integer of at least two, and preferably an integer from 2 to about 6. In one preferred embodiment, the initial building block of cycle 1 is a scaffold building block. In molecules of the formula X(Y)ₙ, each Y can be the same or different, but in most members of a typical library, each Y will be different.

In one embodiment, the libraries of the invention comprise polyamide compounds. The polyamide compounds can be composed of building blocks derived from any amino acids, including the twenty naturally occurring α-amino acids, such as alanine (Ala; A), glycine (Gly; G), asparagine (Asn; N), aspartic acid (Asp; D), glutamic acid (Glu; E), histidine (His; H), leucine (Leu; L), lysine (Lys; K), phenylalanine (Phe; F), tyrosine (Tyr; Y), threonine (Thr; T), serine (Ser; S), arginine (Arg; R), valine (Val; V), glutamine (Gln; Q), isoleucine (Ile; I), cysteine (Cys; C), methionine (Met; M), proline (Pro; P) and tryptophan (Trp; W), where the three-letter and one-letter codes for each amino acid are given. In their naturally occurring form, each of the foregoing amino acids exists in the L-configuration, which is to be assumed herein unless otherwise noted. In the present method, however, the D-configuration forms of these amino acids can also be used. These D-amino acids are indicated herein by lower case three- or one-letter code, i.e., ala (a), gly (g), leu (I), gln (q), thr (t), ser (s), and so forth. The building blocks can also be derived from other α-amino acids, including, but not limited to, 3-arylalanines, such as naphthylalanine, phenyl-substituted phenylalanines, including 4-fluoro-, 4-chloro, 4-bromo and 4-methylphenylalanine; 3-heteroarylalanines, such as 3-pyridylalanine, 3-thienylalanine, 3-quinolylalanine, and 3-imidazolylalanine; ornithine; citrulline; homocitrulline; sarcosine; homoproline; homocysteine; substituted proline, such as hydroxyproline and fluoroproline; dehydroproline; norleucine; O-methyltyrosine; O-methylserine; O-methylthreonine and 3-cyclohexylalanine. Each of the preceding amino acids can be utilized in either the D- or L-configuration.

The building blocks can also be amino acids which are not α-amino acids, such as α-azaamino acids; β, γ, δ, ε,-amino acids, and N-substituted amino acids, such as N-substituted glycine, where the N-substituent can be, for example, a substituted or unsubstituted alkyl, aryl, heteroaryl, arylalkyl or heteroarylalkyl group. In one embodiment, the N-substituent is a side chain from a naturally-occurring or non-naturally occurring α-amino acid.

The building block can also be a peptidomimetic structure, such as a dipeptide, tripeptide, tetrapeptide or pentapeptide mimetic. Such peptidomimetic building blocks are preferably derived from amino acyl compounds, such that the chemistry of addition of these building blocks to the growing poly(aminoacyl) group is the same as, or similar to, the chemistry used for the other building blocks. The building blocks can also be molecules which are capable of forming bonds which are isosteric with a peptide bond, to form peptidomimetic functional moieties comprising a peptide backbone modification, such as ψ 1[CH₂S], ψ [CH₂NH], ψ[CSNH₂], ψ[NHCO], ψ[COCH₂], and ψ[(E) or (Z) CH=CH]. In the nomenclature used above, ψ indicates the absence of an amide bond. The structure that replaces the amide group is specified within the brackets.

In one embodiment, the invention provides a method of synthesizing a compound comprising or consisting of a functional moiety which is operatively linked to an encoding oligonucleotide. The method includes the steps of: (1) providing an initiator compound consisting of an initial functional moiety comprising n building blocks, where n is an integer of 1 or greater, wherein the initial functional moiety comprises at least one reactive group, and wherein the initial functional moiety is operatively linked to an initial oligonucleotide which encodes the n building blocks; (2) reacting the initiator compound with a building block comprising at least one complementary reactive group, wherein the at least one complementary reactive group is complementary to the reactive group of step (1), under suitable conditions for reaction of the reactive group and the complementary reactive group to form a covalent bond; (3) reacting the initial oligonucleotide with an incoming oligonucleotide in the presence of an enzyme which catalyzes ligation of the initial oligonucleotide and the incoming oligonucleotide, under conditions suitable for ligation of the incoming oligonucleotide and the initial oligonucleotide, thereby producing a molecule which comprises or consists of a functional moiety comprising n+1 building blocks which is operatively linked to an encoding oligonucleotide. If the functional moiety of step (3) comprises a reactive group, steps 1-3 can be repeated one or more times, thereby forming cycles 1 to i, where i is an integer of 2 or greater, with the product of step (3) of a cycle s-1, where s is an integer of i or less, becoming the initiator compound of step (1) of cycle s. In each cycle, one building block is added to the growing functional moiety and one oligonucleotide sequence, which encodes the new building block, is added to the growing encoding oligonucleotide.

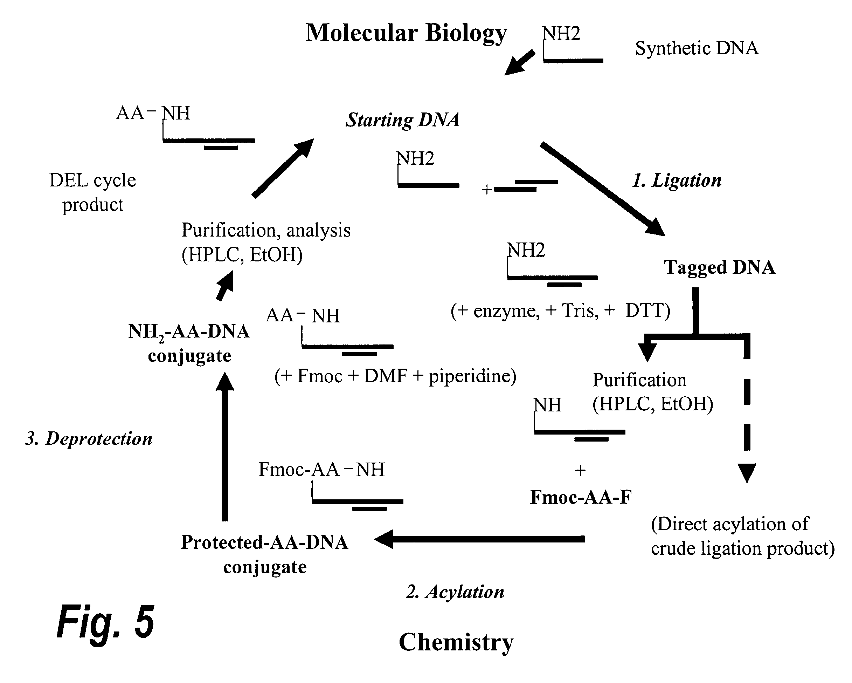

In one embodiment, the initial initiator compound(s) is generated by reacting a first building block with an oligonucleotide (e.g., an oligonucleotide which includes PCR primer sequences or an initial oligonucleotide) or with a linker to which such an oligonucleotide is attached. In the embodiment set forth in Figure 5, the linker comprises a reactive group for attachment of a first building block and is attached to an initial oligonucleotide. In this embodiment, reaction of a building block, or in each of multiple aliquots, one of a collection of building blocks, with the reactive group of the linker and addition of an oligonucleotide encoding the building block to the initial oligonucleotide produces the one or more initial initiator compounds.of the process set forth above.

In a preferred embodiment, each individual building block is associated with a distinct oligonucleotide, such that the sequence of nucleotides in the oligonucleotide added in a given cycle identifies the building block added in the same cycle.

The coupling of building blocks and ligation of oligonucleotides will generally occur at similar concentrations of starting materials and reagents. For example, concentrations of reactants on the order of micromolar to millimolar, for example from about 10 µM to about 10 mM, are preferred in order to have efficient coupling of building blocks.

In certain embodiments, the method further comprises, following step (2), the step of scavenging any unreacted initial functional moiety. Scavenging any unreacted initial functional moiety in a particular cycle prevents the initial functional moiety of the cycle from reacting with a building block added in a later cycle. Such reactions could lead to the generation of functional moieties missing one or more building blocks, potentially leading to a range of functional moiety structures which correspond to a particular oligonucleotide sequence. Such scavenging can be accomplished by reacting any remaining initial functional moiety with a compound which reacts with the reactive group of step (2). Preferably, the scavenger compound reacts rapidly with the reactive group of step (2) and includes no additional reactive groups that can react with building blocks added in later cycles. For example, in the synthesis of a compound where the reactive group of step (2) is an amino group, a suitable scavenger compound is an N-hydroxysuccinimide ester, such as acetic acid N-hydroxysuccinimide ester.

In another embodiment, the invention provides a method of producing a library of compounds, wherein each compound comprises a functional moiety comprising two or more building block residues which is operatively linked to an oligonucleotide. In a preferred embodiment, the oligonucleotide present in each molecule provides sufficient information to identify the building blocks within the molecule and, optionally, the order of addition of the building blocks. In this embodiment, the method of the invention comprises a method of synthesizing a library of compounds, wherein the compounds comprise a functional moiety comprising two or more building blocks which is operatively linked to an oligonucleotide which identifies the structure of the functional moiety. The method comprises the steps of (1) providing a solution comprising m initiator compounds, wherein m is an integer of 1 or greater, where the initiator compounds consist of a functional moiety comprising n building blocks, where n is an integer of 1 or greater, which is operatively linked to an initial oligonucleotide which identifies the n building blocks; (2) dividing the solution of step (1) into at least r fractions, wherein r is an integer of 2 or greater; (3) reacting each fraction with one of r building blocks, thereby producing r fractions comprising compounds consisting of a functional moiety comprising n+1 building blocks operatively linked to the initial oligonucleotide; (4) reacting each of the r fractions of step (3) with one of a set of r distinct incoming oligonucleotides under conditions suitable for enzymatic ligation of the incoming oligonucleotide to the initial oligonucleotide, thereby producing r fractions comprising molecules consisting of a functional moiety comprising n+1 building blocks operatively linked to an elongated oligonucleotide which encodes the n+1 building blocks. Optionally, the method can further include the step of (5) recombining the r fractions, produced in step (4), thereby producing a solution comprising molecules consisting of a functional moiety comprising n +1 building blocks, which is operatively linked to an elongated oligonucleotide which encodes the n + 1 building blocks. Steps (1) to (5) can be conducted one or more times to yield cycles 1 to i, where i is an integer of 2 or greater. In cycle s+1, where s is an integer of i-1 or less, the solution comprising m initiator compounds of step (1) is the solution of step (5) of cycle s. Likewise, the initiator compounds of step (1) of cycle s+1 are the products of step (4) in cycle s.

Preferably the solution of step (2) is divided into r fractions in each cycle of the library synthesis. In this embodiment, each fraction is reacted with a unique building block.

In the methods of the invention, the order of addition of the building block and the incoming oligonucleotide is not critical, and steps (2) and (3) of the synthesis of a molecule, and steps (3) and (4) in the library synthesis can be reversed, i.e., the incoming oligonucleotide can be ligated to the initial oligonucleotide before the new building block is added. In certain embodiments, it may be possible to conduct these two steps simultaneously.

In certain embodiments, the method further comprises, following step (2), the step of scavenging any unreacted initial functional moiety. Scavenging any unreacted initial functional moiety in a particular cycle prevents the initial functional moiety of a cycle from reacting with a building block added in a later cycle. Such reactions could lead to the generation of functional moieties missing one or more building blocks, potentially leading to a range of functional moiety structures which correspond to a particular oligonucleotide sequence. Such scavenging can be accomplished by reacting any remaining initial functional moiety with a compound which reacts with the reactive group of step (2). Preferably, the scavenger compound reacts rapidly with the reactive group of step (2) and includes no additional reactive groups that can react with building blocks added in later cycles. For example, in the synthesis of a compound where the reactive group of step (2) is an amino group, a suitable scavenger compound is an N-hydroxysuccinimide ester, such as acetic acid N-hydroxysuccinimide ester.

In one embodiment, the building blocks used in the library synthesis are selected from a set of candidate building blocks by evaluating the ability of the candidate building blocks to react with appropriate complementary functional groups under the conditions used for synthesis of the library. Building blocks which are shown to be suitably reactive under such conditions can then be selected for incorporation into the library. The products of a given cycle can, optionally, be purified. When the cycle is an intermediate cycle, i.e., any cycle prior to the final cycle, these products are intermediates and can be purified prior to initiation of the next cycle. If the cycle is the final cycle, the products of the cycle are the final products, and can be purified prior to any use of the compounds. This purification step can, for example, remove unreacted or excess reactants and the enzyme employed for oligonucleotide ligation. Any methods which are suitable for separating the products from other species present in solution can be used, including liquid chromatography, such as high performance liquid chromatography (HPLC) and precipitation with a suitable co-solvent, such as ethanol. Suitable methods for purification will depend upon the nature of the products and the solvent system used for synthesis.

The reactions are, preferably, conducted in aqueous solution, such as a buffered aqueous solution, but can also be conducted in mixed aqueous/organic media consistent with the solubility properties of the building blocks, the oligonucleotides, the intermediates and final products and the enzyme used to catalyze the oligonucleotide ligation.

It is to be understood that the theoretical number of compounds produced by a given cycle in the method described above is the product of the number of different initiator compounds, m, used in the cycle and the number of distinct building blocks added in the cycle, r. The actual number of distinct compounds produced in the cycle can be as high as the product of r and m (r x m), but could be lower, given differences in reactivity of certain building blocks with certain other building blocks. For example, the kinetics of addition of a particular building block to a particular initiator compound may be such that on the time scale of the synthetic cycle, little to none of the product of that reaction may be produced.

In certain embodiments, a common building block is added prior to cycle 1, following the last cycle or in between any two cycles. For example, when the functional moiety is a polyamide, a common N-terminal capping building block can be added after the final cycle. A common building block can also be introduced between any two cycles, for example, to add a functional group, such as an alkyne or azide group, which can be utilized to modify the functional moieties, for example by cyclization, following library synthesis.

The term "operatively linked", as used herein, means that two chemical structures are linked together in such a way as to remain linked through the various manipulations they are expected to undergo. Typically the functional moiety and the encoding oligonucleotide are linked covalently via an appropriate linking group. The linking group is a bivalent moiety with a site of attachment for the oligonucleotide and a site of attachment for the functional moiety. For example, when the functional moiety is a polyamide compound, the polyamide compound can be attached to the linking group at its N-terminus, its C-terminus or via a functional group on one of the side chains. The linking group is sufficient to separate the polyamide compound and the oligonucleotide by at least one atom, and preferably, by more than one atom, such as at least two, at least three, at least four, at least five or at least six atoms. Preferably, the linking group is sufficiently flexible to allow the polyamide compound to bind target molecules in a manner which is independent of the oligonucleotide.

In one embodiment, the linking group is attached to the N-teminus of the polyamide compound and the 5'-phosphate group of the oligonucleotide. For example, the linking group can be derived from a linking group precursor comprising an activated carboxyl group on one end and an activated ester on the other end. Reaction of the linking group precursor with the N-terminal nitrogen atom will form an amide bond connecting the linking group to the polyamide compound or N-terminal building block, while reaction of the linking group precursor with the 5'-hydroxy group of the oligonucleotide will result in attachment of the oligonucleotide to the linking group via an ester linkage. The linking group can comprise, for example, a polymethylene chain, such as a -(CH₂)ₙ- chain or a poly(ethylene glycol) chain, such as a -(CH₂CH₂O)ₙ chain, where in both cases n is an integer from 1 to about 20. Preferably, n is from 2 to about 12, more preferably from about 4 to about 10. In one embodiment, the linking group comprises a hexamethylene (-(CH₂)₆-) group.

When the building blocks are amino acid residues, the resulting functional moiety is a polyamide. The amino acids can be coupled using any suitable chemistry for the formation of amide bonds. Preferably, the coupling of the amino acid building blocks is conducted under conditions which are compatible with enzymatic ligation of oligonucleotides, for example, at neutral or near-neutral pH and in aqueous solution. In one embodiment, the polyamide compound is synthesized from the C-terminal to N-terminal direction. In this embodiment, the first, or C-terminal, building block is coupled at its carboxyl group to an oligonucleotide via a suitable linking group. The first building block is reacted with the second building block, which preferably has an activated carboxyl group and a protected amino group. Any activating/protecting group strategy which is suitable for solution phase amide bond formation can be used. For example, suitable activated carboxyl species include acyl fluorides (U.S. Patent No. 5,360,928, incorporated herein by reference in its entirety), symmetrical anhydrides and N-hydroxysuccinimide esters. The acyl groups can also be activated *in situ,* as is known in the art, by reaction with a suitable activating compound. Suitable activating compounds include dicyclohexylcarbodiimide (DCC), diisopropylcarbodiimide (DIC), 1-ethoxycarbonyl-2-ethoxy-1,2-dihydroquinoline (EEDQ), 1-ethyl-3-(3-dimethylaminopropyl)carbodiimide hydrochloride (etc), n-propane-phosphonic anhydride (PPA), N,N-bis (2-oxo-3-oxazolidinyl)imido-phosphoryl chloride (BOP-Cl), bromo-tris-pyrrolidinophosphonium hexafluorophosphate (PyBrop), diphenylphosphoryl azide (DPPA), Castro's reagent (BOP, PyBop), O-benzotriazolyl-N,N,N',N'-tetramethyluronium salts (HBTU), diethylphosphoryl cyanide (DEPCN), 2,5-diphenyl-2,3-dihydro-3-oxo-4-hydroxythiophene dioxide (Steglich's reagent; HOTDO), 1,1'-carbonyl-diimidazole (CDI), and 4-(4,6-dimethoxy-1,3,5-triazin-2-yl)-4-methylmorpholinium chloride (DMT-MM). The coupling reagents can be employed alone or in combination with additives such as N. N-dimethyl-4-aminopyridine (DMAP), N-hydroxy-benzotriazole (HOBt), N-hydroxybenzotriazine (HOOBt), N-hydroxysuccinimide (HOSu) N-hydroxyazabenzotriazole (HOAt), azabenzotriazolyl-tetramethyluronium salts (HATU, HAPyU) or 2-hydroxypyridine. In certain embodiments, synthesis of a library requires the use of two or more activation strategies, to enable the use of a structurally diverse set of building blocks. For each building block, one skilled in the art can determine the appropriate activation strategy.

The N-terminal protecting group can be any protecting group which is compatible with the conditions of the process, for example, protecting groups which are suitable for solution phase synthesis conditions. A preferred protecting group is the fluorenylmethoxycarbonyl ("Fmoc") group. Any potentially reactive functional groups on the side chain of the aminoacyl building block may also need to be suitably protected. Preferably the side chain protecting group is orthogonal to the N-terminal protecting group, that is, the side chain protecting group is removed under conditions which are different than those required for removal of the N-terminal protecting group. Suitable side chain protecting groups include the nitroveratryl group, which can be used to protect both side chain carboxyl groups and side chain amino groups. Another suitable side chain amine protecting group is the N-pent-4-enoyl group.

The building blocks can be modified following incorporation into the functional moiety, for example, by a suitable reaction involving a functional group on one or more of the building blocks. Building block modification can take place following addition of thc final building block or at any intermediate point in the synthesis of the functional moiety, for example, after any cycle of the synthetic process. When a library of bifunctional molecules of the invention is synthesized, building block modification can be carried out on the entire library or on a portion of the library, thereby increasing the degree of complexity of the library. Suitable building block modifying reactions include those reactions that can be performed under conditions compatible with the functional moiety and the encoding oligonucleotide. Examples of such reactions include acylation and sulfonation of amino groups or hydroxyl groups, alkylation of amino groups, esterification or thioesterification of carboxyl groups, amidation of carboxyl groups, epoxidation of alkenes, and other reactions as are known the art. When the functional moiety includes a building block having an alkyne or an azide functional group, the azide/alkyne cycloaddition reaction can be used to derivatize the building block. For example, a building block including an alkyne can be reacted with an organic azide, or a building block including an azide can be reacted with an alkyne, in either case forming a triazole. Building block modification reactions can take place after addition of the final building block or at an intermediate point in the synthetic process, and can be used to append a variety of chemical structures to the functional moiety, including carbohydrates, metal binding moieties and structures for targeting certain biomolecules or tissue types.

In another embodiment, the functional moiety comprises a linear series of building blocks and this linear series is cyclized using a suitable reaction. For example, if at least two building blocks in the linear array include sulfliydryl groups, the sulfhydryl groups can be oxidized to form a disulfide linkage, thereby cyclizing the linear array. For example, the functional moieties can be oligopeptides which include two or more L or D-cysteine and/or L or D-homocysteine moieties. The building blocks can also include other functional groups capable of reacting together to cyclize the linear array, such as carboxyl groups and amino or hydroxyl groups.

In a preferred embodiment, one of the building blocks in the linear array comprises an alkyne group and another building block in the linear array comprises an azide group. The azide and alkyne groups can be induced to react via cycloaddition, resulting in the formation of a macrocyclic structure. In the example illustrated in Figure 9, the functional moiety is a polypeptide comprising a propargylglycine building block at its C-terminus and an azidoacetyl group at its N-terminus. Reaction of the alkyne and the azide group under suitable conditions results in formation of a cyclic compound, which includes a triazole structure within the macrocycle. In the case of a library, in one embodiment, each member of the library comprises alkyne-and azide-containing building blocks and can be cyclized in this way. In a second embodiment, all members of the library comprise alkyne and azide-containing building blocks, but only a portion of the library is cyclized. In a third embodiment, only certain functional moieties include alkyne- and azide-containing building blocks, and only these molecules are cyclized. In the forgoing second and third embodiments, the library, following the cycloaddition reaction, will include both cyclic and linear functional moieties.

In some embodiments of the invention in which the same functional moiety, e.g., triazine, is added to each and all of the fractions of the library during a particular synthesis step, it may not be necessary to add an oligonucleotide tag encoding that function moiety.

Oligonucleotides may be ligated by chemical or enzymatic methods. In one embodiment, oligonucleotides are ligated by chemical means. Chemical ligation of DNA and RNA may be performed using reagents such as water soluble carbodiimide and cyanogen bromide as taught by, for example, Shabarova, et al. (1991) Nucleic Acids Research, 19,4247-4251), Federova, et al. (1996) Nucleosides and Nucleotides, 15, 1137-1147, and Carriero and Damha (2003) Journal of Organic Chemistry, 68, 8328-8338. In one embodiment, chemical ligation is performed using cyanogen bromide, 5 M in acetonitrile, in a 1:10 v/v ratio with 5' phosphorylated oligonucleotide in a pH 7.6 buffer (1 M MES + 20 mM MgCl2) at 0 degrees for 1 - 5 minutes. In another embodiment, the oligonucleotides are ligated using enzymatic methods. In either embodiment, the oligonucleotides may be double stranded, preferably with an overhang of about 5 to about 14 bases. The oligonucleotide may also be single stranded, in which case a splint with an overlap of about 6 bases with each of the oligonucleotides to be ligated is employed to position the reactive 5' and 3' moieties in proximity with each other.

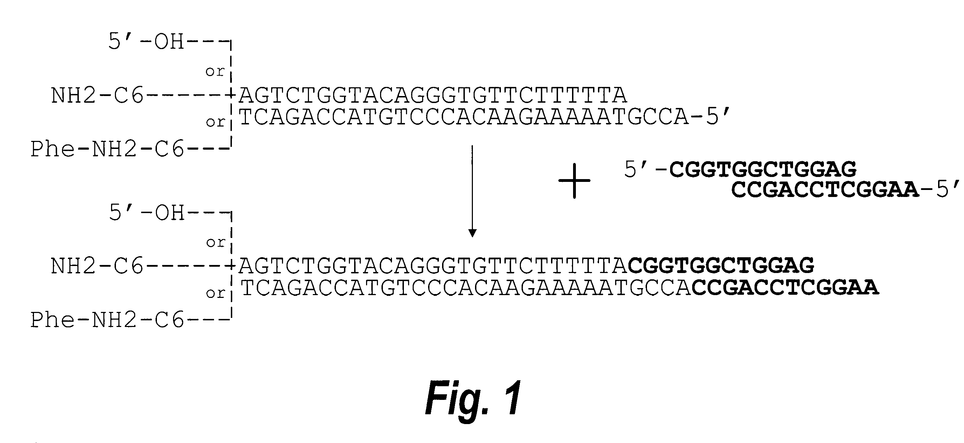

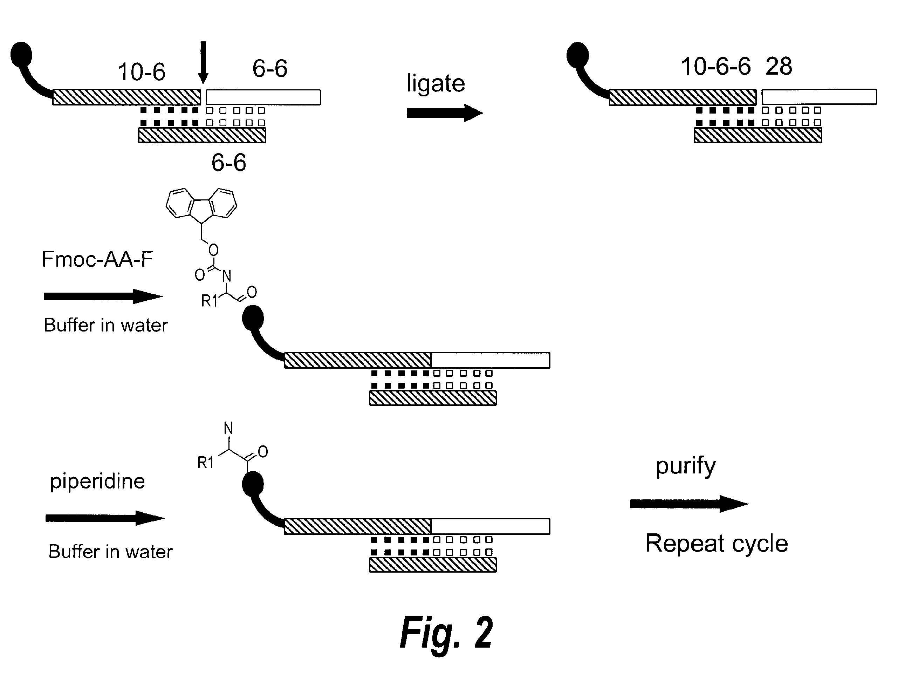

In one embodiment, the initial building block is operatively linked to an initial oligonucleotide. Prior to or following coupling of a second building block to the initial building block, a second oligonucleotide sequence which identifies the second building block is ligated to the initial oligonucleotide. Methods for ligating the initial oligonucleotide sequence and the incoming oligonucleotide sequence are set forth in Figures 1 and 2. In Figure 1, the initial oligonucleotide is double-stranded, and one strand includes an overhang sequence which is complementary to one end of the second oligonucleotide and brings the second oligonucleotide into contact with the initial oligonucleotide. Preferably the overhanging sequence of the initial oligonucleotide and the complementary sequence of the second oligonucleotide are both at least about 4 bases; more preferably both sequences are both the same length. The initial oligonucleotide and the second oligonucleotide can be ligated using a suitable enzyme. If the initial oligonucleotide is linked to the first building block at the 5' end of one of the strands (the "top strand"), then the strand which is complementary to the top strand (the "bottom strand") will include the overhang sequence at its 5' end, and the second oligonucleotide will include a complementary sequence at its 5' end. Following ligation of the second oligonucleotide, a strand can be added which is complementary to the sequence of the second oligonucleotide which is 3' to the overhang complementary sequence, and which includes additional overhang sequence.

In one embodiment, the oligonucleotide is elongated as set forth in Figure 2. The oligonucleotide bound to the growing functional moiety and the incoming oligonucleotide are positioned for ligation by the use of a "splint" sequence, which includes a region which is complementary to the 3' end of the initial oligonucleotide and a region which is complementary to the 5' end of the incoming oligonucleotide. The splint brings the 5' end of the oligonucleotide into proximity with the 3' end of the incoming oligo and ligation is accomplished using enzymatic ligation. In the example illustrated in Figure 2, the initial oligonucleotide consists of 16 nucleobases and the splint is complementary to the 6 bases at the 3' end. The incoming oligonucleotide consists of 12 nucleobases, and the splint is complementary to the 6 bases at the 5' terminus. The length of the splint and the lengths of the complementary regions are not critical. However, the complementary regions should be sufficiently long to enable stable dimer formation under the conditions of the ligation, but not so long as to yield an excessively large encoding nucleotide in the final molecules. It is preferred that the complementary regions are from about 4 bases to about 12 bases, more preferably from about 5 bases to about 10 bases, and most preferably from about 5 bases to about 8 bases in length.

The split-and-pool methods used for the methods for library synthesis set forth herein assure that each unique functional moiety is operatively linked to at least one unique oligonucleotide sequence which identifies the functional moiety. If 2 or more different oligonucleotide tags are used for at least one building bock in at least one of the synthetic cycles, each distinct functional moiety comprising that building block will be encoded by multiple oligonucleotides. For example, if 2 oligonucleotide tags are used for each building block during the synthesis of a 4 cycle library, there will be 16 DNA sequences (2⁴) that encode each unique functional moiety. There are several potential advantages for encoding each unique functional moiety with multiple sequences. First, selection of a different combination of tag sequences encoding the same functional moiety assures that those molecules were independently selected. Second, selection of a different combination of tag sequences encoding the same functional moiety eliminates the possibility that the selection was based on the sequence of the oligonucleotide. Third, technical artifact can be recognized if sequence analysis suggests that a particular functional moiety is highly enriched, but only one sequence combination out of many possibilities appears. Multiple tagging can be accomplished by having independent split reactions with the same building block but a different oligonucleotide tag. Alternatively, multiple tagging can be accomplished by mixing an appropriate ratio of each tag in a single tagging reaction with an individual building block.

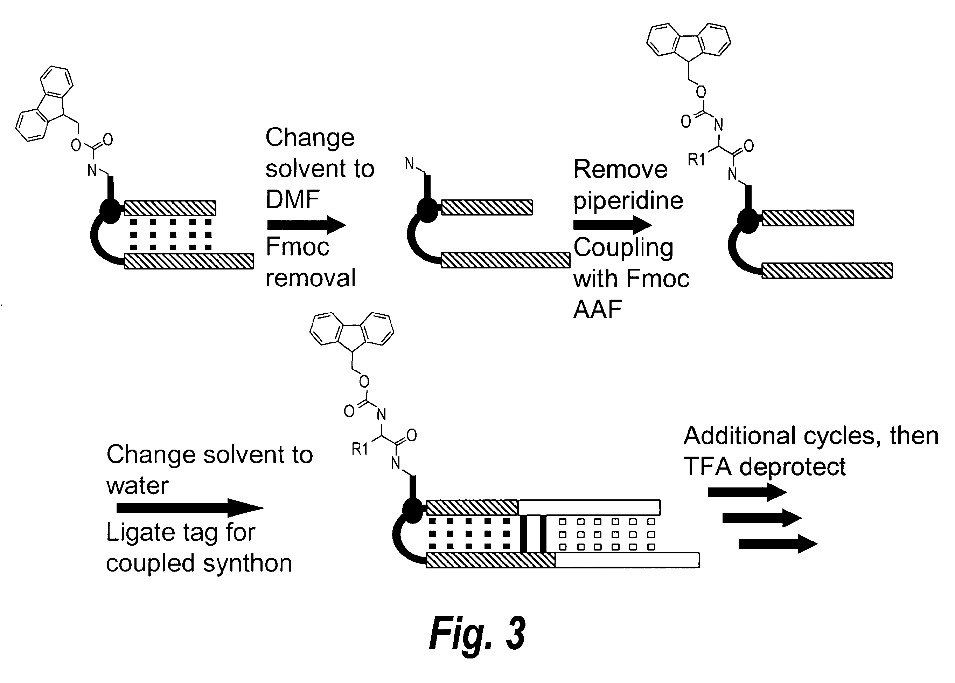

In one embodiment, the initial oligonucleotide is double-stranded and the two strands are covalently joined. One means of covalently joining the two strands is shown in Figure 3, in which a linking moiety, *e.g.*, a linker, is used to link the two strands and the functional moiety. The linking moiety can be any chemical structure which comprises a first functional group which is adapted to react with a building block, a second functional group which is adapted to react with the 3'-end of an oligonucleotide, and a third functional group which is adapted to react with the 5'-end of an oligonucleotide. Preferably, the second and third functional groups are oriented so as to position the two oligonucleotide strands in a relative orientation that permits hybridization of the two strands. For example, the linking moiety, *e.g.*, the linker, can have the general structure (I): 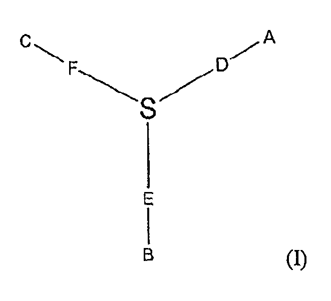 where A, is a functional group that can form a covalent bond with a building block, B is a functional group that can form a bond with the 5'-end of an oligonucleotide, and C is a functional group that can form a bond with the 3'-end of an oligonucleotide. D, F and E are chemical groups that link functional groups A, C and B to S, which is a core atom or scaffold. Preferably, D, E and F are each independently a chain of atoms, such as an alkylene chain or an oligo(ethylene glycol) chain, and D, E and F can be the same or different, and are preferably effective to allow hybridization of the two oligonucleotides and synthesis of the functional moiety. In one embodiment, the trivalent linking moiety is a linker having the structure 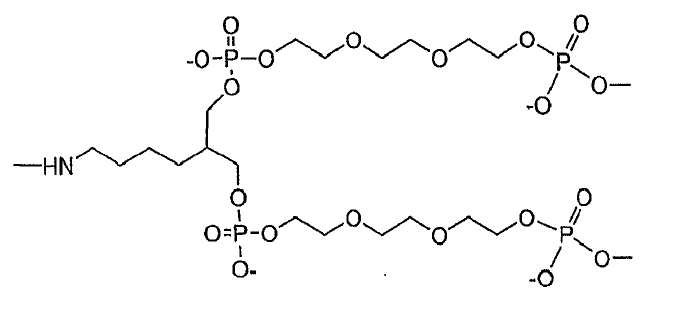 In this embodiment, the NH group is available for attachment to a building block, while the terminal phosphate groups are available for attachment to an oligonucleotide.

In embodiments in which the initial oligonucleotide is double-stranded, the incoming oligonucleotides are also double-stranded. As shown in Figure 3, the initial oligonucleotide can have one strand which is longer than the other, providing an overhang sequence. In this embodiment, the incoming oligonucleotide includes an overhang sequence which is complementary to the overhang sequence of the initial oligonucleotide. Hybridization of the two complementary overhang sequences brings the incoming oligonucleotide into position for ligation to the initial oligonucleotide. This ligation can be performed enzymatically using a DNA or RNA ligase. The overhang sequences of the incoming oligonucleotide and the initial oligonucleotide are preferably the same length and consist of two or more nucleotides, preferably from 2 to about 10 nucleotides, more preferably from 2 to about 6 nucleotides. In one preferred embodiment, the incoming oligonucleotide is a double-stranded oligonucleotide having an overhang sequence at each end. The overhang sequence at one end is complementary to the overhang sequence of the initial oligonucleotide, while, after ligation of the incoming oligonucleotide and the initial oligonucleotide, the overhang sequence at the other end becomes the overhang sequence of initial oligonucleotide of the next cycle. In one embodiment, the three overhang sequences are all 2 to 6 nucleotides in length, and the encoding sequence of the incoming oligonucleotide is from 3 to 10 nucleotides in length, preferably 3 to 6 nucleotides in length. In a particular embodiment, the overhang sequences are all 2 nucleotides in length and the encoding sequence is 5 nucleotides in length.

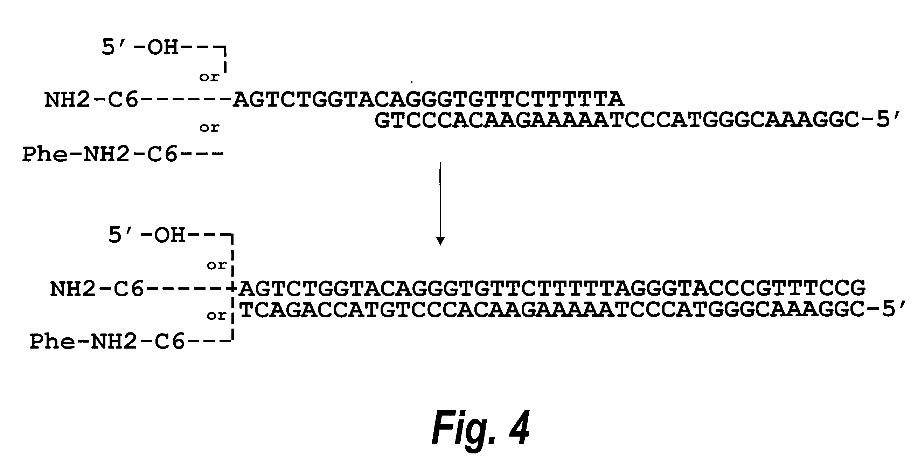

In the embodiment illustrated in Figure 4, the incoming strand has a region at its 3' end which is complementary to the 3' end of the initial oligonucleotide, leaving overhangs at the 5' ends of both strands. The 5' ends can be filled in using, for example, a DNA polymerase, such as vent polymerase, resulting in a double-stranded elongated oligonucleotide. The bottom strand of this oligonucleotide can be removed, and additional sequence added to the 3' end of the top strand using the same method.

The encoding oligonucleotide tag is formed as the result of the successive addition of oligonucleotides that identify each successive building block. In one embodiment of the methods of the invention, the successive oligonucleotide tags may be coupled by enzymatic ligation to produce an encoding oligonucleotide.

Enzyme-catalyzed ligation of oligonucleotides can be performed using any enzyme that has the ability to ligate nucleic acid fragments. Exemplary enzymes include ligases, polymerases, and topoisomerases. In specific embodiments of the invention, DNA ligase (EC 6.5.1.1), DNA polymerase (EC 2.7.7.7), RNA polymerase (EC 2.7.7.6) or topoisomerase (EC 5.99.1.2) are used to ligate the oligonucleotides. Enzymes contained in each EC class can be found, for example, as described in Bairoch (2000) Nucleic Acids Research 28:304-5.

In a preferred embodiment, the oligonucleotides used in the methods of the invention are oligodeoxynucleotides and the enzyme used to catalyze the oligonucleotide ligation is DNA ligase. In order for ligation to occur in the presence of the ligase, *i.e.*, for a phosphodiester bond to be formed between two oligonucleotides, one oligonucleotide must have a free 5' phosphate group and the other oligonucleotide must have a free 3' hydroxyl group. Exemplary DNA ligases that may be used in the methods of the invention include T4 DNA ligase, Taq DNA ligase, T₄ RNA ligase, DNA ligase *(E. coli)* (all available from, for example, New England Biolabs, MA).

One of skill in the art will understand that each enzyme used for ligation has optimal activity under specific conditions, *e.g.*, temperature, buffer concentration, pH and time. Each of these conditions can be adjusted, for example, according to the manufacturer's instructions, to obtain optimal ligation of the oligonucleotide tags.

The incoming oligonucleotide can be of any desirable length, but is preferably at least three nucleobases in length. More preferably, the incoming oligonucleotide is 4 or more nucleobases in length. In one embodiment, the incoming oligonucleotide is from 3 to about 12 nucleobases in length. It is preferred that the oligonucleotides of the molecules in the libraries of the invention have a common terminal sequence which can serve as a primer for PCR, as is known in the art. Such a common terminal sequence can be incorporated as the terminal end of the incoming oligonucleotide added in the final cycle of the library synthesis, or it can be added following library synthesis, for example, using the enzymatic ligation methods disclosed herein.

A preferred embodiment of the method of the invention is set forth in Figure 5. The process begins with a synthesized DNA sequence which is attached at its 5' end to a linker which terminates in an amino group. In step 1, this starting DNA sequence is ligated to an incoming DNA sequence in the presence of a splint DNA strand, DNA ligase and dithiothreitol in Tris buffer. This yields a tagged DNA sequence which can then be used directly in the next step or purified, for example, using HPLC or ethanol precipitation, before proceeding to the next step. In step 2 the tagged DNA is reacted with a protected activated amino acid, in this example, an Fmoc-protected amino acid fluoride, yielding a protected amino acid-DNA conjugate. In step 3, the protected amino acid-DNA conjugate is deprotected, for example, in the presence of piperidine, and the resulting deprotected conjugate is, optionally, purified, for example, by HPLC or ethanol precipitation. The deprotected conjugate is the product of the first synthesis cycle, and becomes the starting material for the second cycle, which adds a second amino acid residue to the free amino group of the deprotected conjugate.

In embodiments in which PCR is to be used to amplify and/or sequence the encoding oligonucleotides of selected molecules, the encoding oligonucleotides may include, for example, PCR primer sequences and/or sequencing primers (*e.g*., primers such as, for example, 3'-GACTACCGCGCTCCCTCCG-5' and 3'-GACTCGCCCGACCGTTCCG-5'). A PCR primer sequence can be included, for example, in the initial oligonucleotide prior to the first cycle of synthesis, and/or it can be included with the first incoming oligonucleotide, and/or it can be ligated to the encoding oligonucleotide following the final cycle of library synthesis, and/or it can be included in the incoming oligonucleotide of the final cycle. The PCR primer sequences added following the final cycle of library synthesis and/or in the incoming oligonucleotide of the final cycle are referred to herein as "capping sequences".

In one embodiment, the PCR primer sequence is designed into the encoding oligonucleotide tag. For example, a PCR primer sequence may be incorporated into the initial oligonucleotide tag and/or it may be incorporated into the final oligonucleotide tag. In one embodiment the same PCR primer sequence is incorporated into the initial and final oligonucleotide tag. In another embodiment, a first PCR primer sequence is incorporated into the initial oligonucleotide tag and a second PCR primer sequence is incorporated in the final oligonucleotide tag. Alternatively, the second PCR primer sequence may be incorporated into the capping sequence as described herein. In preferred embodiments, the PCR primer sequence is at least about 5, 7,10,13,15,17,20, 22, or 25 nucleotides in length.

PCR primer sequences suitable for use in the libraries of the invention are known in the art; suitable primers and methods are set forth, for example, in Innis, et al., eds., PCR Protocols: A Guide to Methods and Applications, San Diego: Academic Press (1990), the contents of which are incorporated herein by reference in their entirety. Other suitable primers for use in the construction of the libraries described herein are those primers described in PCT Publications WO 2004/069849 and WO 2005/003375, the entire contents of which are expressly incorporated herein by reference.

The term "polynucleotide" as used herein in reference to primers, probes and nucleic acid fragments or segments to be synthesized by primer extension is defined as a molecule comprised of two or more deoxyribonucleotides, preferably more than three.

The term "primer" as used herein refers to a polynucleotide whether purified from a nucleic acid restriction digest or produced synthetically, which is capable of acting as a point of initiation of nucleic acid synthesis when placed under conditions in which synthesis of a primer extension product which is complementary to a nucleic acid strand is induced, *i.e.,* in the presence of nucleotides and an agent for polymerization such as DNA polymerase, reverse transcriptase and the like, and at a suitable temperature and pH. The primer is preferably single stranded for maximum efficiency, but may alternatively be in double stranded form. If double stranded, the primer is first treated to separate it from its complementary strand before being used to prepare extension products. Preferably, the primer is a polydeoxyribonucleotide. The primer must be sufficiently long to prime the synthesis of extension products in the presence of the agents for polymerization. The exact lengths of the primers will depend on many factors, including temperature and the source of primer.

The primers used herein are selected to be "substantially" complementary to the different strands of each specific sequence to be amplified. This means that the primer must be sufficiently complementary so as to non-randomly hybridize with its respective template strand. Therefore, the primer sequence may or may not reflect the exact sequence of the template.

The polynucleotide primers can be prepared using any suitable method, such as, for example, the phosphotriester or phosphodiester methods described in Narang et al., (1979) Meth. Enzymol., 68:90; U.S. Pat. No. 4,356,270, U.S. Pat. No. 4,458,066, U.S. Pat. No. 4,416,988, U.S. Pat. No. 4,293,652; and Brown et al., (1979) Meth. Enzymol., 68:109. The contents of all the foregoing documents are incorporated herein by reference.

In cases in which the PCR primer sequences are included in an incoming oligonucleotide, these incoming oligonucleotides will preferably be significantly longer than the incoming oligonucleotides added in the other cycles, because they will include both an encoding sequence and a PCR primer sequence.

In one embodiment, the capping sequence is added after the addition of the final building block and final incoming oligonucleotide, and the synthesis of a library as set forth herein includes the step of ligating the capping sequence to the encoding oligonucleotide, such that the oligonucleotide portion of substantially all of the library members terminates in a sequence that includes a PCR primer sequence. Preferably, the capping sequence is added by ligation to the pooled fractions which are products of the final synthetic cycle. The capping sequence can be added using the enzymatic process used in the construction of the library.

In one embodiment, the same capping sequence is ligated to every member of the library. In another embodiment, a plurality of capping sequences are used. In this embodiment, oligonucleotide capping sequences containing variable bases are, for example, ligated onto library members following the final synthetic cycle. In one embodiment, following the final synthetic cycle, the fractions are pooled and then split into fractions again, with each fraction having a different capping sequence added. Alternatively, multiple capping sequences can be added to the pooled library following the final synthesis cycle. In both embodiments, the final library members will include molecules comprising specific functional moieties linked to identifying oligonucleotides including two or more different capping sequences.

In one embodiment, the capping primer comprises an oligonucleotide sequence containing variable, *i.e*., degenerate, nucleotides. Such degenerate bases within the capping primers permit the identification of library molecules of interest by determining whether a combination of building blocks is the consequence of PCR duplication (identical sequence) or independent occurrences of the molecule (different sequence). For example, such degenerate bases may reduce the potential number of false positives identified during the biological screening of the encoded library.

In one embodiment, a degenerate capping primer comprises or has the following sequence: 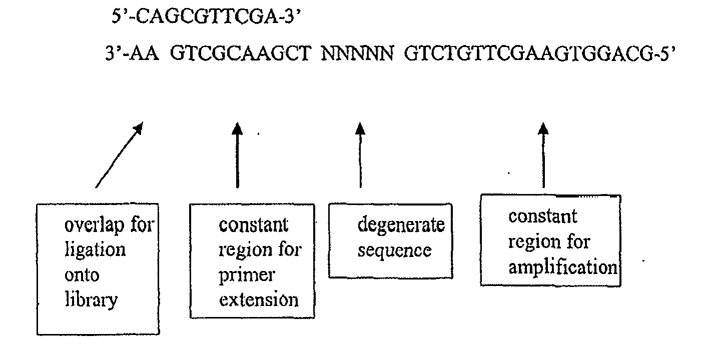
5'-CAGCGTTCGA-3'
3'-AA GTCGCAAGCT NNNNN GTCTGTTCGAAGTGGACG-5'
overlap for
ligation
onto
library
constant
region for
primer
extension
degenerate
sequence
constant
region for
amplification
 where N can be any of the 4 bases, permitting 1024 different sequences (4⁵). The primer has the following sequence after its ligation onto the library and primer-extension:
5'-CAGCGTTCGA N'N'N'N'N'CAGACAAGCTTCACCTGC-3'
3'-AA GTCGCAAGCT N N N N N GTCTGTTCGAAGTGGACG-5'

In another embodiment, the capping primer comprises or has the following sequence: 
3'-AA GTCGCAAGCTACG ABBBABBBABBBA GACTACCGCGCTCCCTCCG

overlap for
ligation
onto
library

constant
region for
primer
extension

degenerate
sequence

constant
region for
amplification
 where B can be any of C, G or T, permitting 19,683 different sequences (3⁹). The design of the degenerate region in this primer improves DNA sequence analysis, as the A bases that flank and punctuate the degenerate B bases prevent homopolymeric stretches of greater than 3 bases, and facilitate sequence alignment.

In one embodiment, the degenerate capping oligonucleotide is ligated to the members of the library using a suitable enzyme and the upper strand of the degenerate capping oligonucleotide is subsequently polymerized using a suitable enzyme, such as a DNA polymerase.

In another embodiment, the PCR priming sequence is a "universal adaptor" or "universal primer". As used herein, a "universal adaptor" or "universal primer" is an oligonucleotide that contains a unique PCR priming region, that is, for example, about 5, 7, 10, 13, 15, 17, 20, 22, or 25 nucleotides in length, and is located adjacent to a unique sequencing priming region that is, for example, about 5, 7, 10,13,15, 17, 20, 22, or 25 nucleotides in length, and is optionally followed by a unique discriminating key sequence (or sample identifier sequence) consisting of at least one of each of the four deoxyribonucleotides *(i.e.,* A, C, G, T).

As used herein, the term "discriminating key sequence' or "sample identifier sequence" refers to a sequence that may be used to uniquely tag a population of molecules from a sample. Multiple samples, each contaning a unique sample identifier sequence, can be mixed, sequenced and re-sorted after DNA sequencing for analysis of individual samples. The same discriminating sequence can be used for an entire library or, alternatively, different discriminating key sequences can be used to track different libraries. In one embodiment, the discriminating key sequence is on either the 5' PCR primer, the 3' PCR primer, or on both primers. If both PCR primers contain a sample identifier sequence, the number of different samples that can be pooled with unique sample identifier sequences is the product of the number of sample identifier sequences on each primer. Thus, 10 different 5' sample identifier sequence primers can be combined with 10 different 3' sample identifier sequence primers to yield 100 different sample identifier sequence combinations.

Non-limiting examples of 5' and 3' unique PCR primers containing discriminating key sequences include the following:
5' primers (variable positions bold and italicized):
   5' A - GCCTTGCCAGCCCGCTCAG***A***TGACTCCCAAATCGATGTG;
   5' C - GCCTTGCCAGCCCGCTCAG***C***TGACTCCCAAATCGATGTG;
   5' G - GCCTTGCCAGCCCGCTCAG***G***TGACTCCCAAATCGATGTG;
   5' T - GCCTTGCCAGCCCGCTCAG***T***TGACTCCCAAATCGATGTG;
   5' AA - GCCTTGCCAGCCCGCTCAG***AA***TGACTCCCAAATCGATGTG;
   5' AC - GCCTTGCCAGCCCGCTCAG***AC***TGACTCCCA.AATCGATGTG;
   5' AG - GCCTTGCCAGCCCGCTCAG***AG***TGACTCCCAAATCGATGTG;
   5' AT - GCCTTGCCAGCCCGCTCAG***AT***TGACTCCCAAATCGATGTG; and
   5' CA - GCCTTGCCAGCCCGCTCAG***CA***TGACTCCCAAATCGATGTG.
3' SID primers (variable positions bold and italicized):
   3' A - GCCTCCCTCGCGCCATCAG***A***GCAGGTGAAGCTTGTCTG;
   3' C - GCCTCCCTCGCGCCATCAG***C***GCAGGTGAAGCTTGTCTG;
   3' G - GCCTCCCTCGCGCCATCAG***G***GCAGGTGAAGCTTGTCTG;
   3' T - GCCTCCCTCGCGCCATCAG***T***GCAGGTGAAGCTTGTCTG;
   3' AA - GCCTCCCTCGCGCCATCAG***AA***GCAGGTGAAGCTTGTCTG;
   3' AC - GCCTCCCTCGCGCCATCAG***AC***GCAGGTGAAGCTTGTCTG;
   3' AG - GCCTCCCTCGCGCCATCAG***AG***GCAGGGTGAAGCTTGTCTG;
   3' AT - GCCTCCCTCGCGCCATCAG***AT***GCAGGTGAAGCTTGTCTG; and
   3' CA - GCCTCCCTCGCGCCATCAG***CA***GCAGGTGAAGCTTGTCTG

In one embodiment, the discriminating key sequence is about 4, 5, 6, 7, 8, 9, or 10 nucleotides in length. In another embodiment, the discriminating key sequence is a combination of about 1-4 nucleotides. In yet another embodiment, each universal adaptor is about forty-four nucleotides in length. In one embodiment the universal adaptors are ligated, using T4 DNA ligase, onto the end of the encoding oligonucleotide. Different universal adaptors may be designed specifically for each library preparation and will, therefore, provide a unique identifier for each library. The size and sequence of the universal adaptors may be modified as deemed necessary by one of skill in the art.

As indicated above, the nucleotide sequence of the oligonucleotide tag as part of the methods of this invention may be determined by the use of the polymerase chain reaction (PCR).

The oligonucleotide tag is comprised of polynucleotides that identify the building blocks that make up the functional moiety as described herein. The nucleic acid sequence of the oligonucleotide tag is determined by subjecting the oligonucleotide tag to a PCR reaction as follows. The appropriate sample is contacted with a PCR primer pair, each member of the pair having a preselected nucleotide sequence. The PCR primer pair is capable of initiating primer extension reactions by hybridizing to a PCR primer binding site on the encoding oligonucleotide tag. The PCR primer binding site is preferably designed into the encoding oligonucleotide tag. For example, a PCR primer binding site may be incorporated into the initial oligonucleotide tag and the second PCR primer binding site may be in the final oligonucleotide tag. Alternatively, the second PCR primer binding site may be incorporated into the capping sequence as described herein. In preferred embodiments, the PCR primer binding site is at least about 5,7,10,13,15,17,20,22, or 25 nucleotides in length.

The PCR reaction is performed by mixing the PCR primer pair, preferably a predetermined amount thereof, with the nucleic acids of the encoding oligonucleotide tag, preferably a predetermined amount thereof, in a PCR buffer to form a PCR reaction admixture. The admixture is thermocycled for a number of cycles, which is typically predetermined, sufficient for the formation of a PCR reaction product. A sufficient amount of product is one that can be isolated in a sufficient amount to allow for DNA sequence determination.

PCR is typically carried out by thermocycling i.e., repeatedly increasing and decreasing the temperature of a PCR reaction admixture within a temperature range whose lower limit is about 30 °C to about 55 °C and whose upper limit is about 90 °C to about 100 °C. The increasing and decreasing can be continuous, but is preferably phasic with time periods of relative temperature stability at each of temperatures favoring polynucleotide synthesis, denaturation and hybridization.

The PCR reaction is performed using any suitable method. Generally it occurs in a buffered aqueous solution, *i.e*., a PCR buffer, preferably at a pH of 7-9. Preferably, a molar excess of the primer is present. A large molar excess is preferred to improve the efficiency of the process.

The PCR buffer also contains the deoxyribonucleotide triphosphates (polynucleotide synthesis substrates) dATP, dCTP, dGTP, and dTTP and a polymerase, typically thermostable, all in adequate amounts for primer extension (polynucleotide synthesis) reaction. The resulting solution (PCR admixture) is heated to about 90° C-100° C for about 1 to 10 minutes, preferably from 1 to 4 minutes. After this heating period the solution is allowed to cool to 54° C, which is preferable for primer hybridization. The synthesis reaction may occur at a temperature ranging from room temperature up to a temperature above which the polymerase (inducing agent) no longer functions efficiently. Thus, for example, if DNA polymerase is used, the temperature is generally no greater than about 40° C. The thermocycling is repeated until the desired amount of PCR product is produced. An exemplary PCR buffer comprises the following reagents: 50 mM KCl; 10 mM Tris-HCl at pH 8.3; 1.5 mM MgCl.sub.2 ; 0.001% (wt/vol) gelatin, 200 µM dATP; 200 µM dTTP; 200 µM dCTP; 200 µM dGTP; and 2.5 units Thermus aquaticus (Taq) DNA polymerase I per 100 microliters of buffer.

Suitable enzymes for elongating the primer sequences include, for example, E. coli DNA polymerase I, Taq DNA polymerase, Klenow fragment of E. coli DNA polymerase I, T4 DNA polymerase, other available DNA polymerases, reverse transcriptase, and other enzymes, including heat-stable enzymes, which will facilitate combination of the nucleotides in the proper manner to form the primer extension products which are complementary to each nucleic acid strand. Generally, the synthesis will be initiated at the 3' end of each primer and proceed in the 5' direction along the template strand, until synthesis terminates, producing molecules of different lengths.

The newly synthesized DNA strand and its complementary strand form a double-stranded molecule which can be used in the succeeding steps of the analysis process.

PCR amplification methods are described in detail in U.S. Patent Nos. 4,683,192, 4,683,202, 4,800,159, and 4,965,188, and at least in PCR Technology: Principles and Applications for DNA Amplification, H. Erlich, ed., Stockton Press, New York (1989); and PCR Protocols: A Guide to Methods and Applications, Innis et al., eds., Academic Press, San Diego, Calif. (1990). The contents of all the foregoing documents are incorporated herein by reference.

Once the encoding oligonucleotide tag has been amplified, the sequence of the tag, and ultimately the composition of the selected molecule, can be determined using nucleic acid sequence analysis, a well known procedure for determining the sequence of nucleotide sequences. Nucleic acid sequence analysis is approached by a combination of (a) physiochemical techniques, based on the hybridization or denaturation of a probe strand plus its complementary target, and (b) enzymatic reactions with polymerases.

The invention further relates to the compounds which can be produced using the methods of the invention and collections of such compounds, either as isolated species or pooled to form a library of chemical structures. Compounds of the invention include compounds of the formula 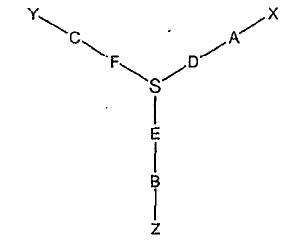 where X is a functional moiety comprising one or more building blocks, Z is an oligonucleotide attached at its 3' terminus to B and Y is an oligonucleotide which is attached to C at its 5' terminus. A is a functional group that forms a covalent bond with X, B is a functional group that forms a bond with the 3'-end of Z and C is a functional group that forms a bond with the 5'-end of Y. D, F and E are chemical groups that link functional groups A, C and B to S, which is a core atom or scaffold. Preferably, D, E and F are each independently a chain of atoms, such as an alkylene chain or an oligo(ethylene glycol) chain, and D, E and F can be the same or different, and are preferably effective to allow hybridization of the two oligonucleotides and synthesis of the functional moiety.

Preferably, Y and Z are substantially complementary and are oriented in the compound so as to enable Watson-Crick base pairing and duplex formation under suitable conditions. Y and Z are the same length or different lengths. Preferably, Y and Z are the same length, or one of Y and Z is from 1 to 10 bases longer than the other. In a preferred embodiment, Y and Z are each 10 or more bases in length and have complementary regions of ten or more base pairs. More preferably, Y and Z are substantially complementary throughout their length, i.e., they have no more than one mismatch per every ten base pairs. Most preferably, Y and Z are complementary throughout their length, *i.e*., except for any overhang region on Y or Z, the strands hybridize via Watson-Crick base pairing with no mismatches throughout their entire length.

S can be a single atom or a molecular scaffold. For example, S can be a carbon atom, a boron atom, a nitrogen atom or a phosphorus atom, or a polyatomic scaffold, such as a phosphate group or a cyclic group, such as a cycloalkyl, cycloalkenyl, heterocycloalkyl, heterocycloalkenyl, aryl or heteroaryl group. In one embodiment, the linker is a group of the structure 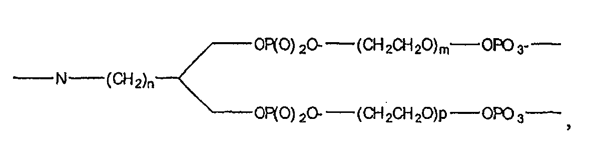 where each of n, m and p is, independently, an integer from 1 to about 20, preferably from 2 to eight, and more preferably from 3 to 6. In one particular embodiment, the linker has the structure shown below.

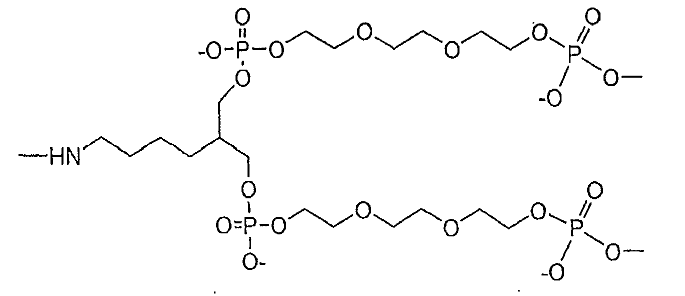

In one embodiment, the libraries of the invention include molecules consisting of a functional moiety composed of building blocks, where each functional moiety is operatively linked to an encoding oligonucleotide. The nucleotide sequence of the encoding oligonucleotide is indicative of the building blocks present in the functional moiety, and in some embodiments, the connectivity or arrangement of the building blocks. The invention provides the advantage that the methodology used to construct the functional moiety and that used to construct the oligonucleotide tag can be performed in the same reaction medium, preferably an aqueous medium, thus simplifying the method of preparing the library compared to methods in the prior art. In certain embodiments in which the oligonucleotide ligation steps and the building block addition steps can both be conducted in aqueous media, each reaction will have a different pH optimum. In these embodiments, the building block addition reaction can be conducted at a suitable pH and temperature in a suitable aqueous buffer. The buffer can then be exchanged for an aqueous buffer which provides a suitable pH for oligonucleotide ligation.

In another embodiment, the invention provides compounds, and libraries comprising such compounds, of Formula II

Z——L——Aₜ—X(Y)ₙ (II)

where X is a molecular scaffold, each Y is independently, a peripheral moiety, and n is an integer from 1 to 6. Each A is independently, a building block and n is an integer from 0 to about 5. L is a linking moiety and Z is a single- stranded or double-stranded oligonucleotide which identifies the structure -Aᵣ-X(Y)ₙ. The structure X(Y)ₙ can be, for example, one of the scaffold structures set forth in Table 8 (see below). In one embodiment, the invention provides compounds, and libraries comprising such compounds, of Formula III: 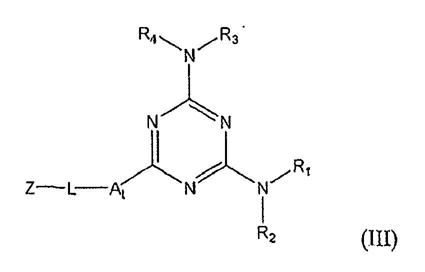 where t is an integer from 0 to about 5, preferably from 0 to 3, and each A is, independently, a building block. L is a linking moiety and Z is a single-stranded or double-stranded oligonucleotide which identifies each A and R₁, R₂, R₃ and R₄. R₁, R₂, R₃ and R₄ are each independently a substituent selected from hydrogen, alkyl, substituted alkyl, heteroalkyl, substituted heteroalkyl, cycloalkyl, heterocycloalkyl, substituted cycloalkyl, substituted heterocycloalkyl, aryl, substituted aryl, arylalkyl, heteroarylalkyl, substituted arylalkyl, substituted heteroarylalkyl, heteroaryl, substituted heteroaryl, alkoxy, aryloxy, amino, and substituted amino. In one embodiment, each A is an amino acid residue.

Libraries which include compounds of Formula II or Formula III can comprise at least about 100; 1000; 10,000; 100,000; 1,000,000 or 10,000,000 compounds of Formula II or Formula III. In one embodiment, the library is prepared via a method designed to produce a library comprising at least about 100; 1000; 10,000; 100,000; 1,000,000 or 10,000,000 compounds of Formula II or Formula III.

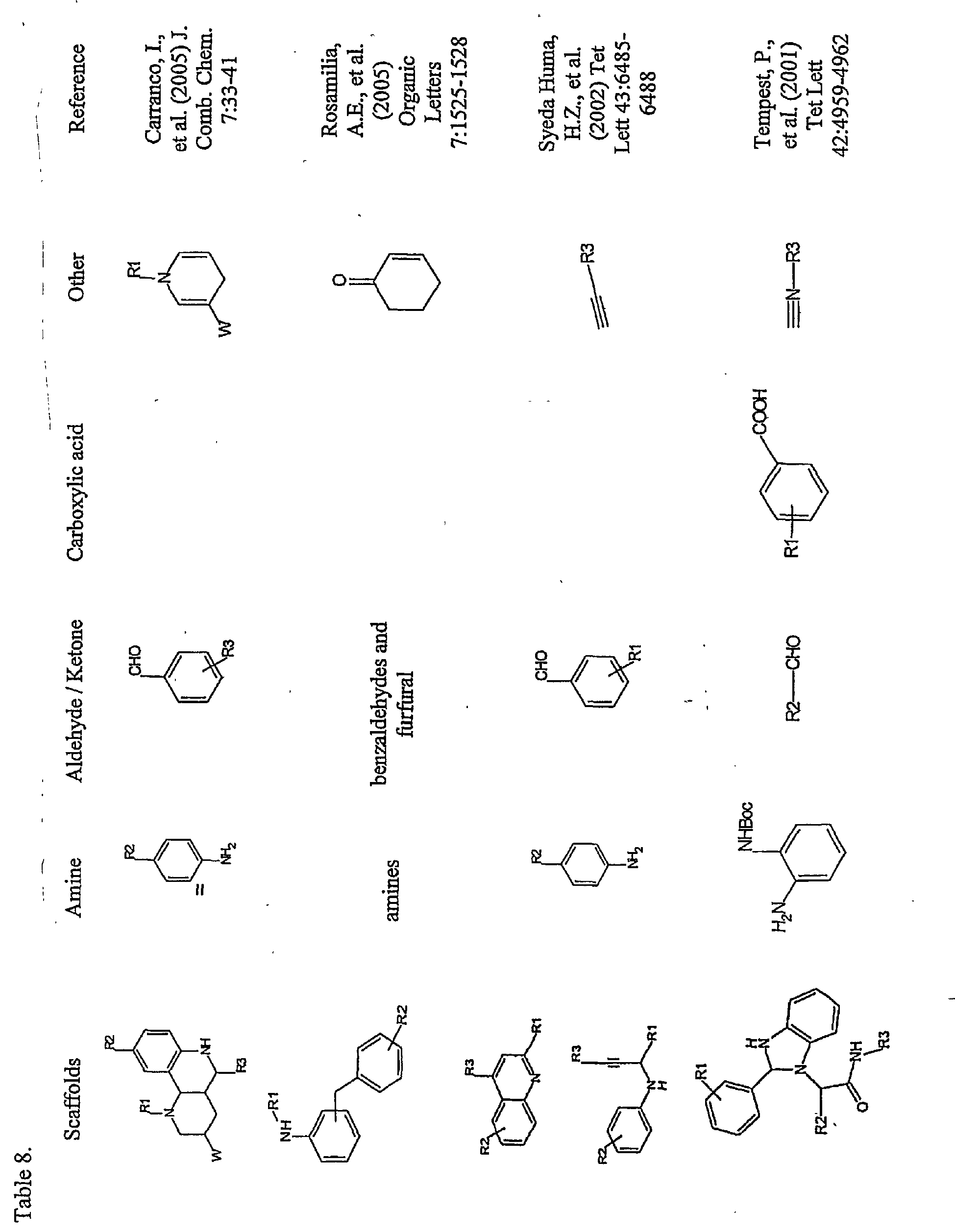

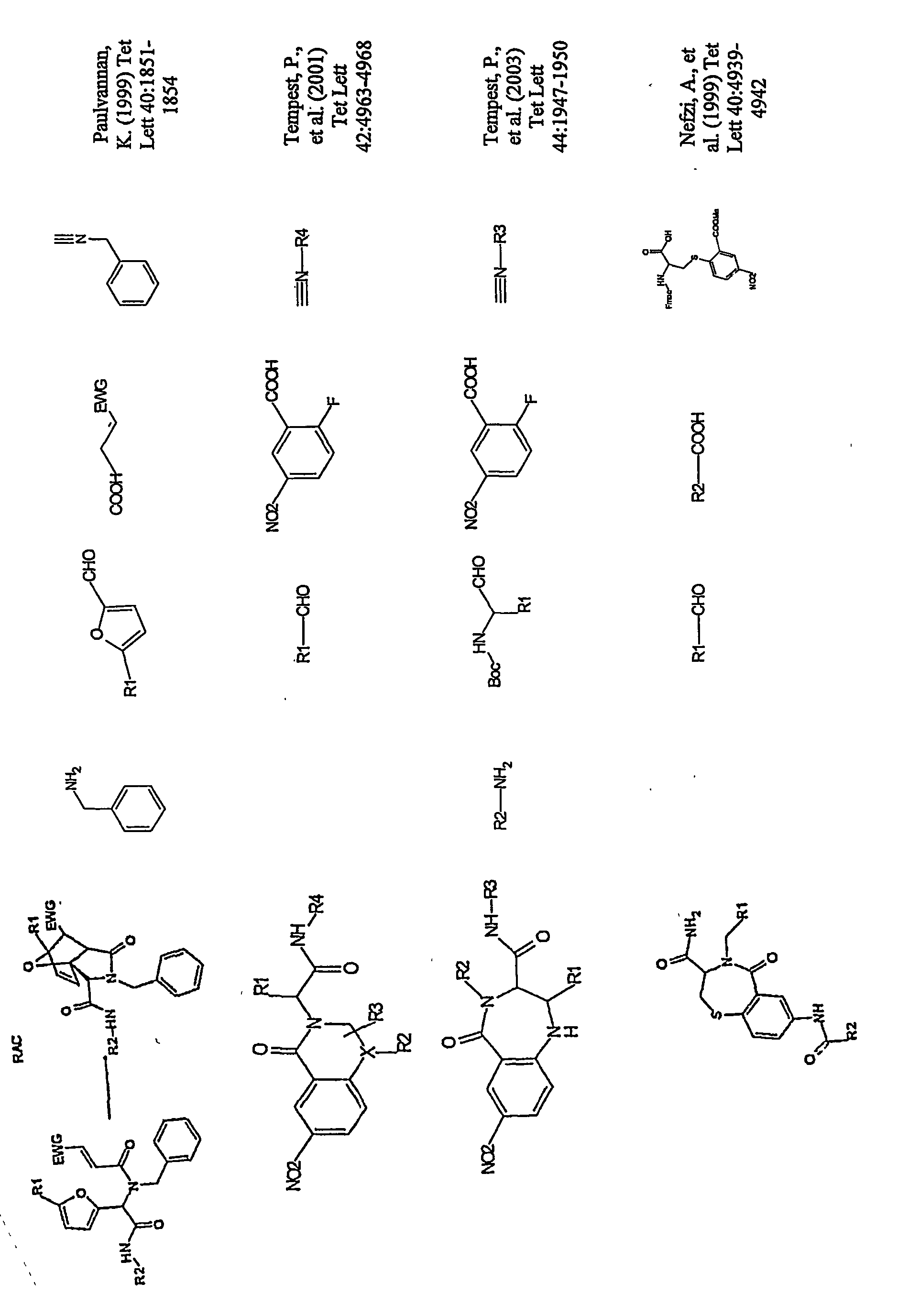

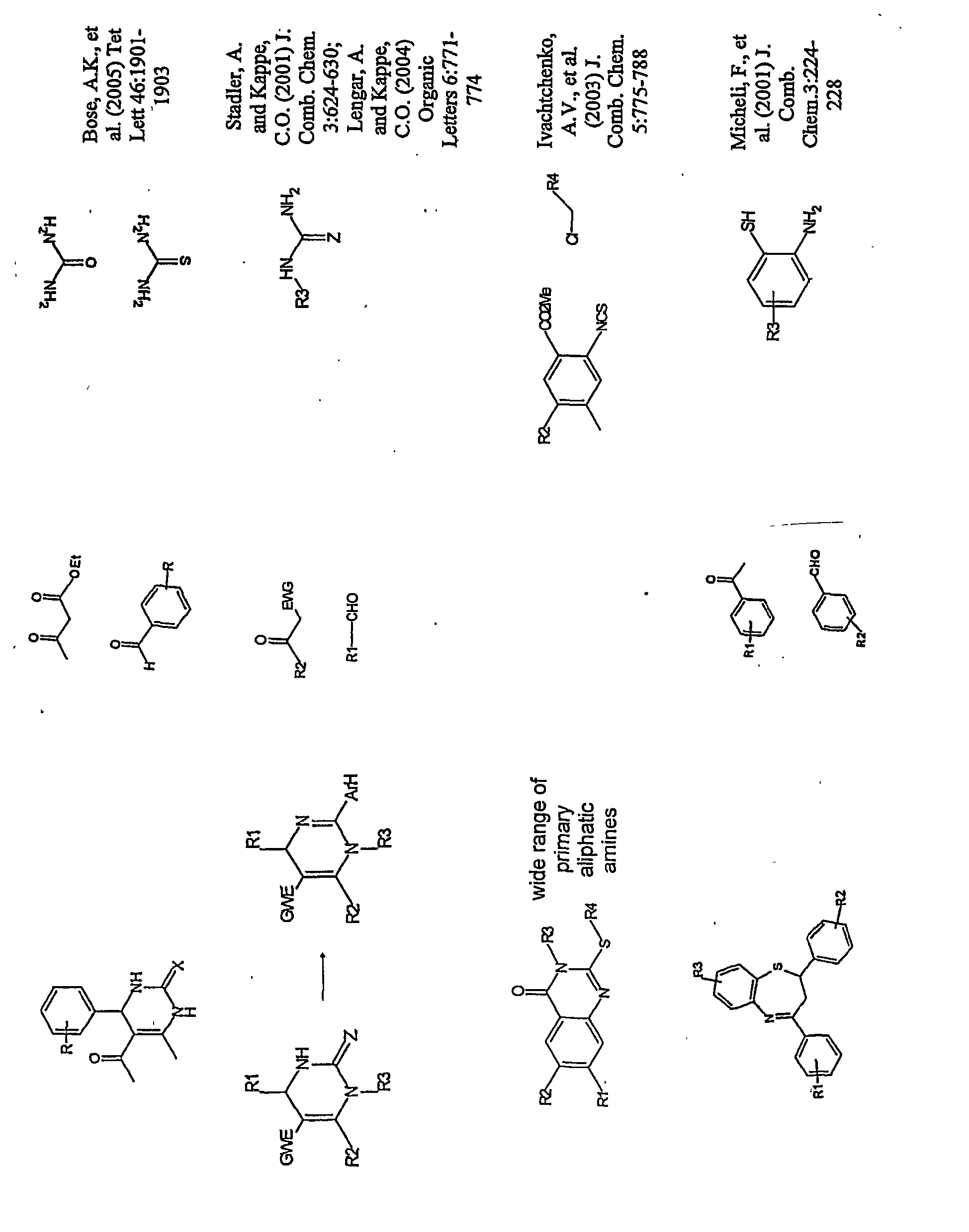

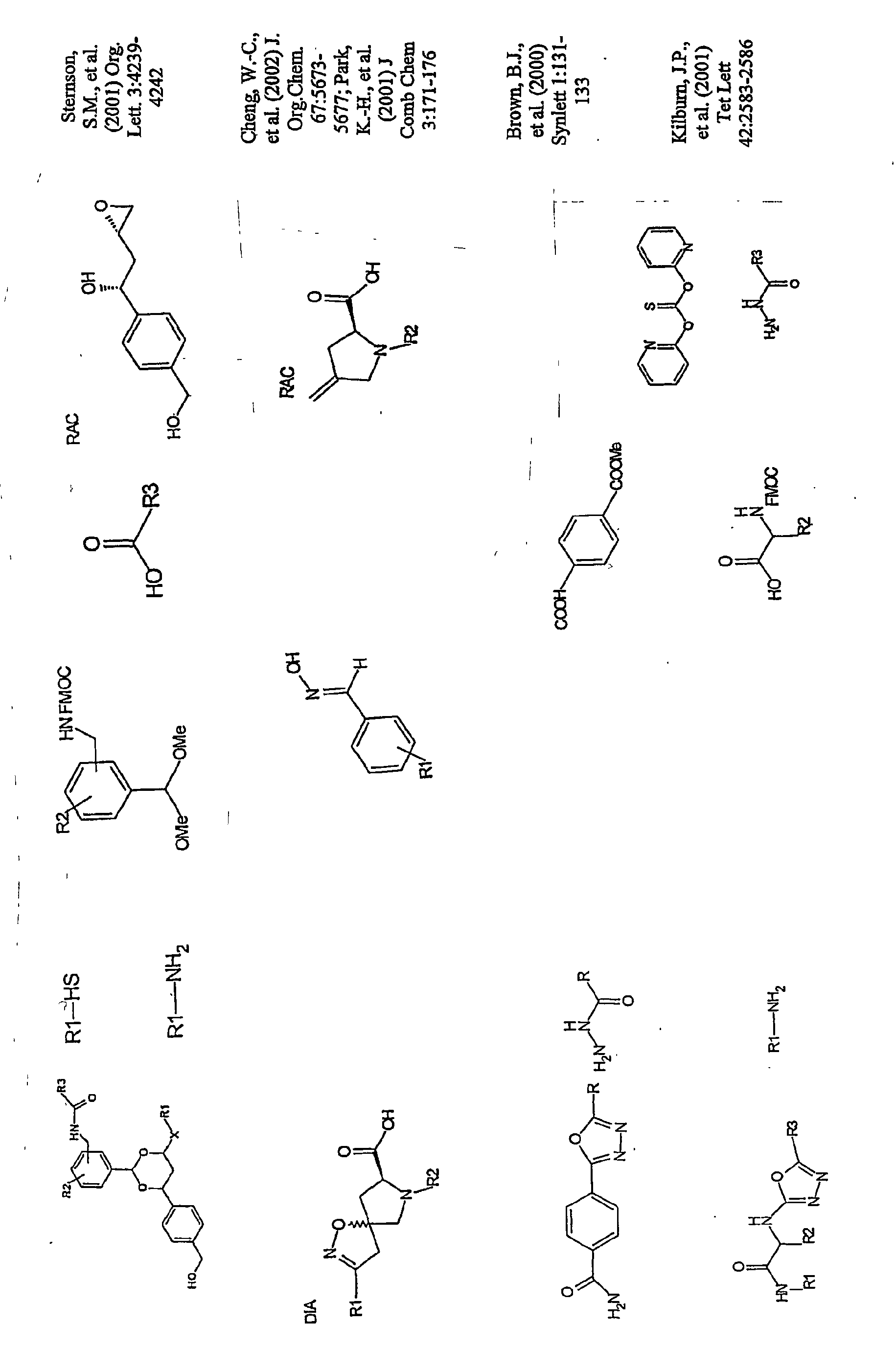

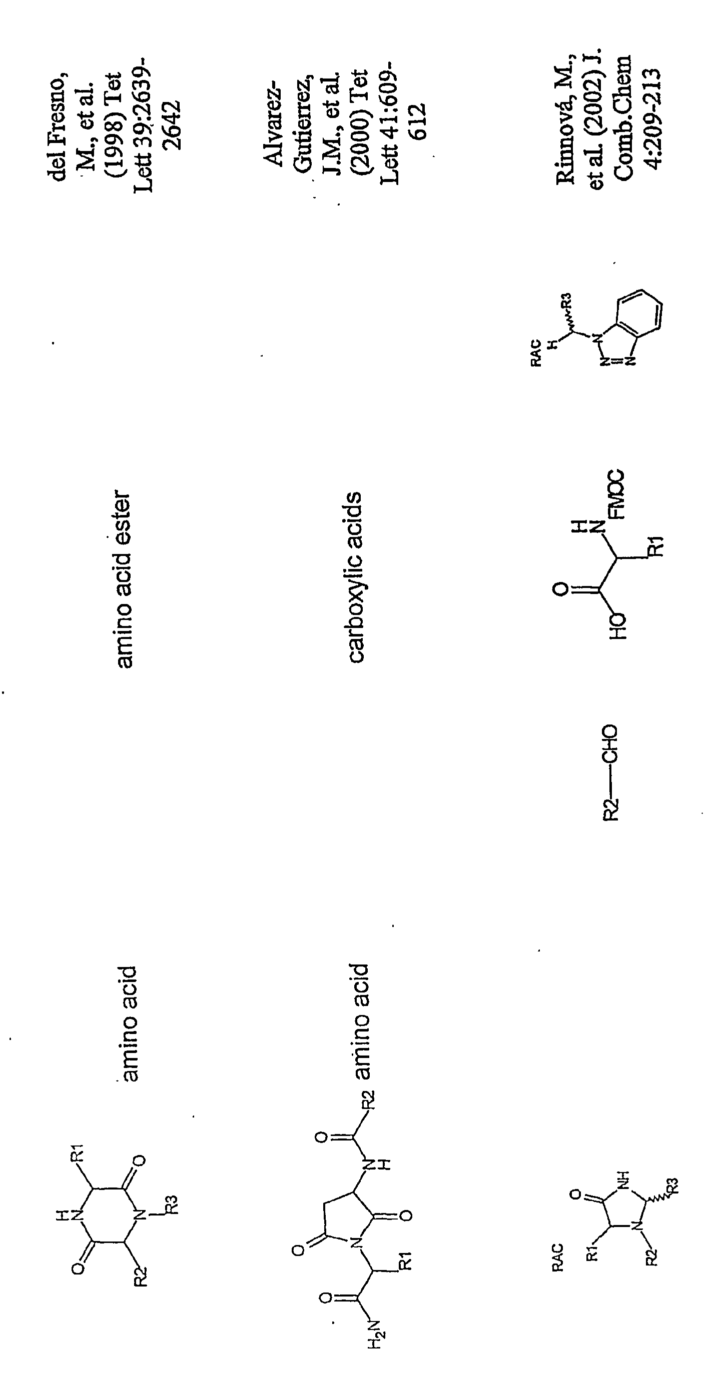

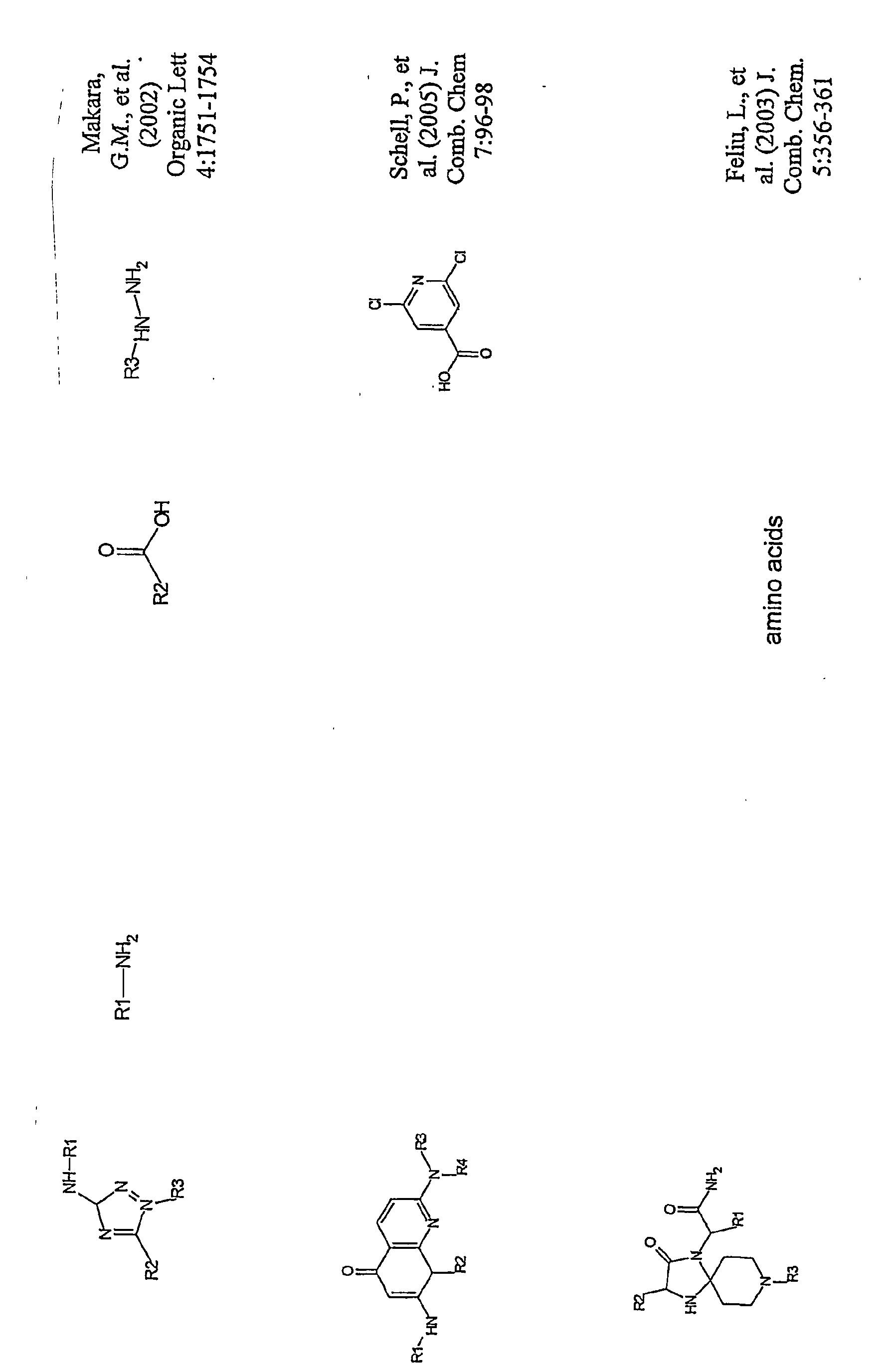

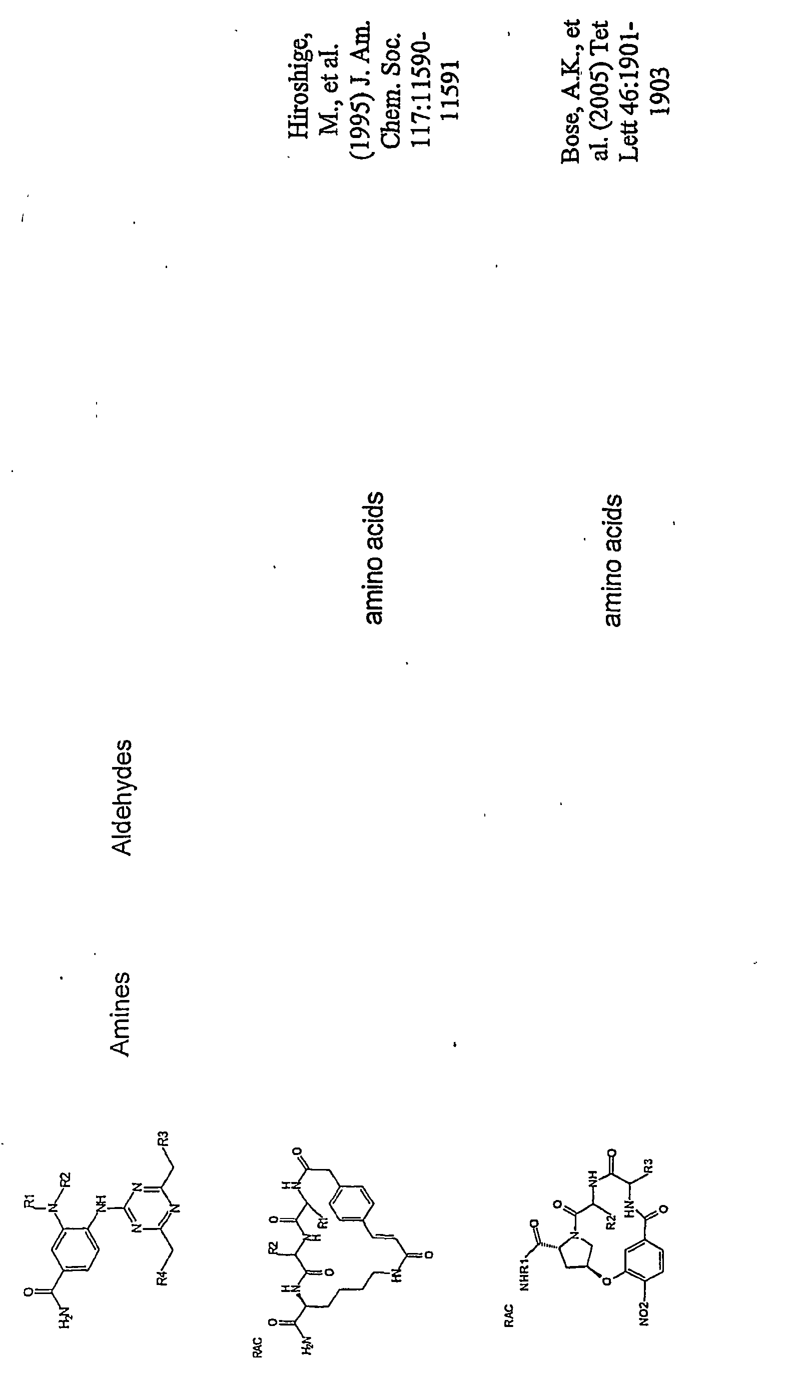

One advantage of the methods of the invention is that they can be used to prepare libraries comprising vast numbers of compounds. The ability to amplify encoding oligonucleotide sequences using known methods such as polymerase chain reaction ("PCR") means that selected molecules can be identified even if relatively few copies are recovered. This allows the practical use of very large libraries, which, as a consequence of their high degree of complexity, either comprise relatively few copies of any given library member, or require the use of very large volumes. For example, a library consisting of 10⁸ unique structures in which each structure has 1 x 10¹² copies (about 1 picomole), requires about 100 L of solution at 1 µM effective concentration. For the same library, if each member is represented by 1,000,000 copies, the volume required is 100 µL at 1 µM effective concentration.

In a preferred embodiment, the library comprises from about 10³ to about 10¹⁵ copies of each library member. Given differences in efficiency of synthesis among the library members, it is possible that different library members will have different numbers of copies in any given library. Therefore, although the number of copies of each member theoretically present in the library may be the same, the actual number of copies of any given library member is independent of the number of copies of any other member. More preferably, the compound libraries of the invention include at least about 10⁵, 10⁶ or 10⁷ copies of each library member, or of substantially all library members. By "substantially all" library members is meant at least about 85% of the members of the library, preferably at least about 90%, and more preferably at least about 95% of the members of the library.

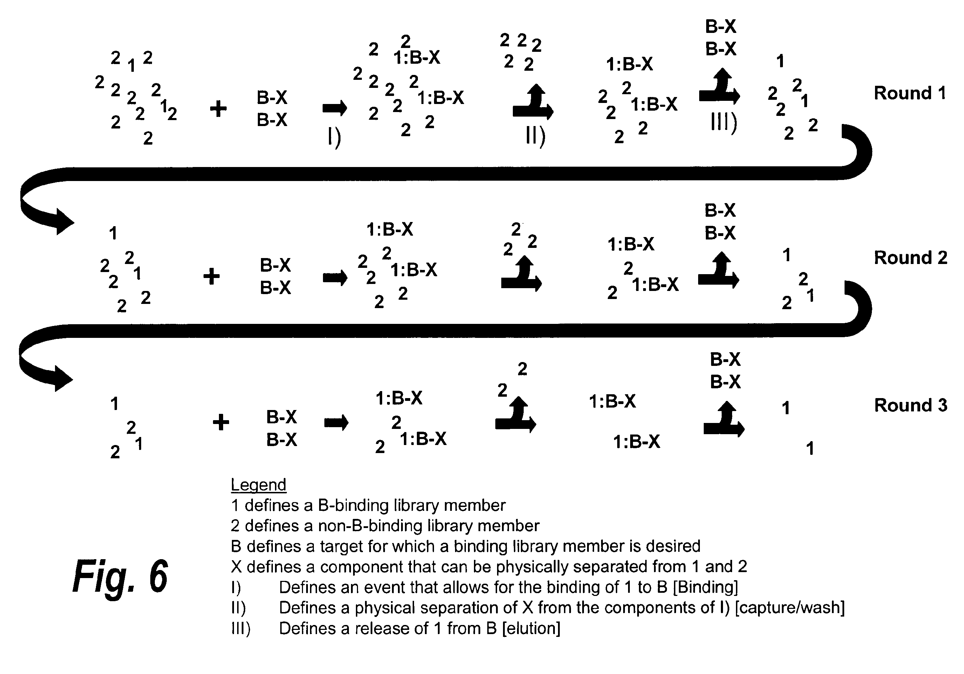

Preferably, the library includes a sufficient number of copies of each member that multiple rounds (i.e., two or more) of selection against a biological target can be performed, with sufficient quantities of binding molecules remaining following the final round of selection to enable amplification of the oligonucleotide tags of the remaining molecules and, therefore, identification of the functional moieties of the binding molecules. A schematic representation of such a selection process is illustrated in Figure 6, in which 1 and 2 represent library members, B is a target molecule and X is a moiety operatively linked to B that enables the removal ofB from the selection medium. In this example, compound 1 binds to B, while compound 2 does not bind to B. The selection process, as depicted in Round 1, comprises (I) contacting a library comprising compounds 1 and 2 with B-X under conditions suitable for binding of compound 1 to B; (II) removing unbound compound 2, (III) dissociating compound 1 from B and removing BX from the reaction medium. The result of Round 1 is a collection of molecules that is enriched in compound 1 relative to compound 2. Subsequent rounds employing steps I-III result in further enrichment of compound 1 relative to compound 2. Although three rounds of selection are shown in Figure 6, in practice any number of rounds may be employed, for example from one round to ten rounds, to achieve the desired enrichment of binding molecules relative to non-binding molecules.

In the embodiment shown in Figure 6, there is no amplification (synthesis of more copies) of the compounds remaining after any of the rounds of selection. Such amplification can lead to a mixture of compounds which is not consistent with the relative amounts of the compounds remaining after the selection. This inconsistency is due to the fact that certain compounds may be more readily synthesized that other compounds, and thus may be amplified in a manner which is not proportional to their presence following selection. For example, if compound 2 is more readily synthesized than compound 1, the amplification of the molecules remaining after Round 2 would result in a disproportionate amplification of compound 2 relative to compound 1, and a resulting mixture of compounds with a much lower (if any) enrichment of compound 1 relative to compound 2.

In one embodiment, the target is immobilized on a solid support by any known immobilization technique. The solid support can be, for example, a water-insoluble matrix contained within a chromatography column or a membrane. The encoded library can be applied to a water-insoluble matrix contained within a chromatography column. The column is then washed to remove non-specific binders. Target-bound compounds can then be dissociated by changing the pH, salt concentration, organic solvent concentration, or other methods, such as competition with a known ligand to the target.

In another embodiment, the target is free in solution and is incubated with the encoded library. Compounds which bind to the target (also referred to herein as "ligands") are selectively isolated by a size separation step such as gel filtration or ultrafiltration. In one embodiment, the mixture of encoded compounds and the target biomolecule are passed through a size exclusion chromatography column (gel filtration), which separates any ligand-target complexes from the unbound compounds. The ligand-target complexes are transferred to a reverse-phase chromatography column, which dissociates the ligands from the target. The dissociated ligands are then analyzed by PCR amplification and sequence analysis of the encoding oligonucleotides. This approach is particularly advantageous in situations where immobilization of the target may result in a loss of activity.

In some embodiments of the invention, the selection method may comprise amplifying the encoding oligonucleotide of at least one member of the library of compounds which binds to a target prior to sequencing.

In one embodiment, the library of compounds comprising encoding oligonucleotides is amplified prior to sequence analysis in order to minimize any potential skew in the population distribution of DNA molecules present in the selected library mix. For example, only a small amount of library is recovered after a selection step and is typically amplified using PCR prior to sequence analysis. PCR has the potential to produce a skew in the population distribution of DNA molecules present in the selected library mix. This is especially problematic when the number of input molecules is small and the input molecules are poor PCR templates. PCR products produced at early cycles are more efficient templates than covalent duplex library, and therefore the frequency of these molecules in the final amplified population may be much higher than in original input template.

Accordingly, in order to minimize this potential PCR skew, in one embodiment of the invention, a population of single-stranded oligonucleotides corresponding to the individual library members is produced by, for example, using one primer in a reaction, followed by PCR amplification using two primers. By doing so, there is a linear accumulation of single-stranded primer-extension product prior to exponential amplification using PCR, and the diversity and distribution of molecules in the accumulated primer-extension product more accurately reflect the diversity and distribution of molecules present in the original input template, since the exponential phase of amplification occurs only after much of the original molecular diversity present is represented in the population of molecules produced during the primer-extension reaction.

Once single ligands are identified by the above-described process, various levels of analysis can be applied to yield structure-activity relationship information and to guide further optimization of the affinity, specificity and bioactivity of the ligand. For ligands derived from the same scaffold, three-dimensional molecular modeling can be employed to identify significant structural features common to the ligands, thereby generating families of small-molecule ligands that presumably bind at a common site on the target biomolecule.

A variety of screening approaches can be used to obtain ligands that possess high affinity for one target but significantly weaker affinity for another closely related target. One screening strategy is to identify ligands for both biomolecules in parallel experiments and to subsequently eliminate common ligands by a cross-referencing comparison. In this method, ligands for each biomolecule can be separately identified as disclosed above. This method is compatible with both immobilized target biomolecules and target biomolecules free in solution.

For immobilized target biomolecules, another strategy is to add a preselection step that eliminates all ligands that bind to the non-target biomolecule from the library. For example, a first biomolecule can be contacted with an encoded library as described above. Compounds which do not bind to the first biomolecule are then separated from any first biomolecule-ligand complexes which form. The second biomolecule is then contacted with the compounds which did not bind to the first biomolecule. Compounds which bind to the second biomolecule can be identified as described above and have significantly greater affinity for the second biomolecule than to the first biomolecule.

A ligand for a biomolecule of unknown function which is identified by the method disclosed above can also be used to determine the biological function of the biomolecule. This is advantageous because although new gene sequences continue to be identified, the functions of the proteins encoded by these sequences and the validity of these proteins as targets for new drug discovery and development are difficult to determine and represent perhaps the most significant obstacle to applying genomic information to the treatment of disease. Target-specific ligands obtained through the process described in this invention can be effectively employed in whole cell biological assays or in appropriate animal models to understand both the function of the target protein and the validity of the target protein for therapeutic intervention. This approach can also confirm that the target is specifically amenable to small molecule drug discovery.

In one embodiment, one or more compounds within a library of the invention are identified as ligands for a particular biomolecule. These compounds can then be assessed in an in vitro assay for the ability to bind to the biomolecule. Preferably, the functional moieties of the binding compounds are synthesized without the oligonucleotide tag or linker moiety, and these functional moieties are assessed for the ability to bind to the biomolecule.

The effect of the binding of the functional moieties to the biomolecule on the function of the biomolecule can also be assessed using in vitro cell-free or cell-based assays. For a biomolecule having a known function, the assay can include a comparison of the activity of the biomolecule in the presence and absence of the ligand, for example, by direct measurement of the activity, such as enzymatic activity, or by an indirect measure, such as a cellular function that is influenced by the biomolecule. If the biomolecule is of unknown function, a cell which expresses the biomolecule can be contacted with the ligand and the effect of the ligand on the viability, function, phenotype, and/or gene expressionof the cell is assessed. The in vitro assay can be, for example, a cell death assay, a cell proliferation assay or a viral replication assay. For example, if the biomolecule is a protein expressed by a virus, a cell infected with the virus can be contacted with a ligand for the protein. The affect of the binding of the ligand to the protein on viral viability can then be assessed.

A ligand identified by the method of the invention can also be assessed in an in vivo model or in a human. For example, the ligand can be evaluated in an animal or organism which produces the biomolecule. Any resulting change in the health status (e.g., disease progression) of the animal or organism can be determined.

For a biomolecule, such as a protein or a nucleic acid molecule, of unknown function, the effect of a ligand which binds to the biomolecule on a cell or organism which produces the biomolecule can provide information regarding the biological function of the biomolecule. For example, the observation that a particular cellular process is inhibited in the presence of the ligand indicates that the process depends, at least in part, on the function of the biomolecule.

Ligands identified using the methods of the invention can also be used as affinity reagents for the biomolecule to which they bind. In one embodiment, such ligands are used to effect affinity purification of the biomolecule, for example, via chromatography of a solution comprising the biomolecule using a solid phase to which one or more such ligands are attached.

This invention is further illustrated by the following examples which should not be construed as limiting. The contents of all references, patents and published patent applications cited throughout this application, as well as the Figures and the Sequence Listing, are hereby incorporated in reference.

### Examples

### Example 1: Synthesis and Characterization of a library on the order of 10⁵ members

The synthesis of a library comprising on the order of 10⁵ distinct members was accomplished using the following reagents:

### Compound 1:

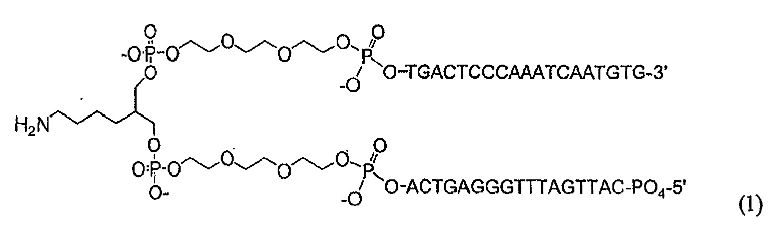

Single letter codes for deoxyribonucleotides:
A = adenosine
C = cytidine
G = guanosine
T = thymidine

### Building block precursors:

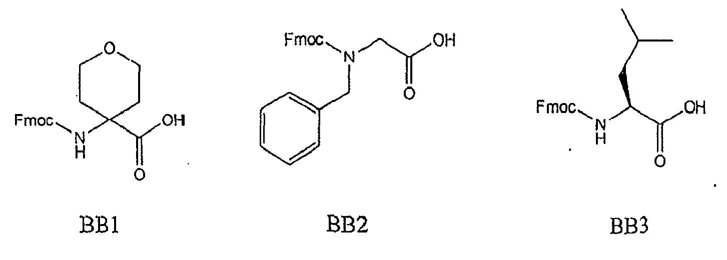

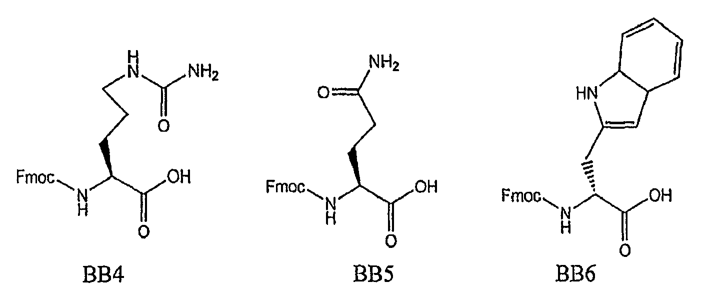

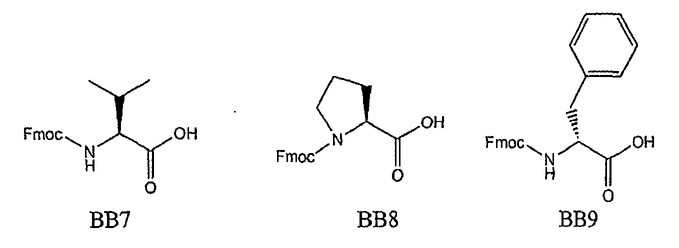

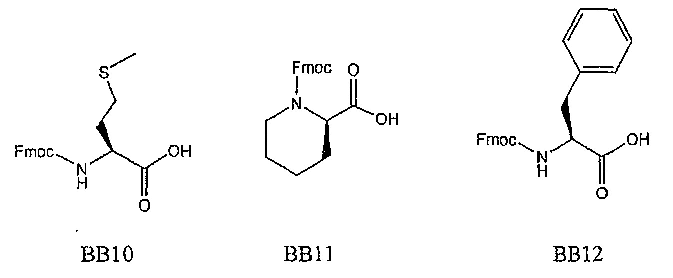

### Oligonucleotide tags:

| Sequence | Tag number |
|---|---|
| 5'-PO4-GCAACGAAG (SEQ ID NO:1) ACCGTTGCT-PO3-5'(SEQ ID NO:2) | 1.1 |
| 5′-PO3-GCGTACAAG (SEQ ID NO:3) ACCGCATGT-PO3-5′ (SEQ ID NO:4) | 1.2 |
| 5'-PO3-GCTCTGTAG (SEQ ID NO:5) ACCGAGACA-PO3-5' (SEQ ID NO:6) | 1.3 |
| 5'-PO3-GTGCCATAG (SEQ ID NO:7) ACCACGGTA-PO3-5' (SEQ ID NO:8) | 1.4 |
| 5′-PO3-GTTGACCAG (SEQ ID NO:9) ACCAACTGG-PO3-5′ (SEQ ID NO:10) | 1.5 |
| 5′-PO3-CGACTTGAC (SEQ ID NO:11) CAAGTCGCA-PO3-5′ (SEQ ID NO:12) | 1.6 |
| 5′-PO3-CGTAGTCAG (SEQ ID NO:13) ACGCATCAG-PO3-5′ (SEQ ID NO:14) | 1.7 |
| 5'-PO3-CCAGCATAG (SEQ ID NO:15) ACGGTCGTA-PO3-5' (SEQ ID NO:16) | 1.8 |
| 5′-PO3-CCTACAGAG (SEQ ID NO:17) ACGGATGTC-PO3-5′ (SEQ ID NO:18) | 1.9 |
| 5′-PO3-CTGAACGAG (SEQ ID NO:19) CGTTCAGCA-PO3-5′ (SEQ ID NO:20) | 1.10 |
| 5′-PO₃-CTCCAGTAG (SEQ ID NO:21) ACGAGGTCA-PO₃-5′ (SEQ ID NO:22) | 1.11 |
| 5′-PO₃-TAGGTCCAG (SEQ ID NO:23) ACATCCAGG-PO₃-5′ (SEQ ID NO:24) | 1.12 |
| 5′-PO3-GCGTGTTGT (SEQ ID NO:25) TCCGCACAA-PO3-5′ (SEQ ID NO:26) | 2.1 |
| 5′-PO3-GCTTGGAGT (SEQ ID NO:27) TCCGAACCT-PO3-5′ (SEQ ID NO:28) | 2.2 |
| 5′-PO3-GTCAAGCGT (SEQ ID NO:29) TCCAGTTCG-PO3-5′ (SEQ ID NO:30) | 2.3 |
| 5′-PO3-CAAGAGCGT (SEQ ID NO:31) TCGTTCTCG-PO3-5′ (SEQ ID NO:32) | 2.4 |
| 5'-PO3-CAGTTCGGT (SEQ ID NO:33) TCGTCAAGC-PO3-5' (SEQ ID NO:34) | 2.5 |
| 5′-PO₃-CGAAGGAGT (SEQ ID NO:35) TCGCTTCCT-PO₃-5′ (SEQ ID NO:36) | 2.6 |
| 5′-PO3-CGGTGTTGT (SEQ ID NO:37) TCGCCACAA-PO3-5′ (SEQ ID NO:38) | 2.7 |
| 5′-PO3-CGTTGCTGT (SEQ ID NO:39) TCGCAACGA-PO3-5′ (SEQ ID NO:40) | 2.8 |
| 5′-PO3-CCGATCTGT (SEQ ID NO:41) TCGGCTAGA-PO3-5′ (SEQ ID NO:42) | 2.9 |
| 5′-PO3-CCTTCTCGT (SEQ ID NO:43) TCGGAAGAG-PO3-5′ (SEQ ID NO:44) | 2.10 |
| 5'-PO3-TGAGTCCGT (SEQ ID NO:45) TCACTCAGG-PO3-5' (SEQ ID NO:46) | 2.11 |
| 5′-PO3-TGCTACGGT (SEQ ID NO:47) TCAGATTGC-PO3-5′ (SEQ ID NO:48) | 2.12 |
| 5′-PO3-GTGCGTTGA (SEQ ID NO:49) CACACGCAA-PO3-5′ (SEQ ID NO:50) | 3.1 |
| 5′-PO3-GTTGGCAGA (SEQ ID NO:51) CACAACCGT-PO3-5′ (SEQ ID NO:52) | 3.2 |
| 5′-PO3-CCTGTAGGA (SEQ ID NO:53) CAGGACATC-PO3-5′ (SEQ ID NO:54) | 3.3 |
| 5′-PO3-CTGCGTAGA (SEQ ID NO:55) CAGACGCAT-PO3-5′ (SEQ ID NO:56) | 3.4 |
| 5′-PO3-CTTACGCGA (SEQ ID NO:57) CAGAATGCG-PO3-5′ (SEQ ID NO:58) | 3.5 |
| 5'-PO3-TGGTCACGA (SEQ ID NO:59) CAACCAGTG-PO3-5' (SEQ ID NO:60) | 3.6 |
| 5′-PO₃-TCAGAGCGA (SEQ ID NO:61) CAAGTCTCG-PO₃-5′ (SEQ ID NO:62) | 3.7 |
| 5′-PO3-TTGCTCGGA (SEQ ID NO:63) CAAACGAGC-PO3-5′ (SEQ ID NO:64) | 3.8 |
| 5′-PO3-GCAGTTGGA (SEQ ID NO:65) CACGTCAAC-PO3-5′ (SEQ ID NO:66) | 3.9 |
| 5'-PO3-GCCTGAAGA (SEQ ID NO:67) CACGGACTT-PO3-5' (SEQ ID NO:68) | 3.10 |
| 5'-PO₃-GTAGCCAGA (SEQ ID NO:69) CACATCGGT-PO₃-5' (SEQ ID NO:70) | 3.11 |
| 5′-PO3-GTCGCTTGA (SEQ ID NO:71) CACAGCGAA-PO3-5′ (SEQ ID NO:72) | 3.12 |
| 5'-PO3-GCCTAAGTT (SEQ ID NO:73) CTCGGATTC-PO3-5' (SEQ ID NO:74) | 4.1 |
| 5'-PO3-GTAGTGCTT (SEQ ID NO:75) CTCATCACG-PO3-5' (SEQ ID NO:76) | 4.2 |
| 5′-PO3-GTCGAAGTT (SEQ ID NO:77) CTCAGCTTC-PO3-5′ (SEQ ID NO:78) | 4.3 |
| 5'-PO3-GTTTCGGTT (SEQ ID NO:79) CTCAAAGCC-PO3-5' (SEQ ID NO:80) | 4.4 |
| 5'-PO3-CAGCGTTTT (SEQ ID NO:81) CTGTCGCAA-PO3-5' (SEQ ID NO:82) | 4.5 |
| 5′-PO3-CATACGCTT (SEQ ID NO:83) CTGTATGCG-PO3-5′ (SEQ ID NO:84) | 4.6 |
| 5′-PO₃-CGATCTGTT (SEQ ID NO:85) CTGCTAGAC-PO₃-5′ (SEQ ID NO:86) | 4.7 |
| 5′-PO₃-CGCTTTGTT (SEQ ID NO:87) CTGCGAAAC-PO₃-5′ (SEQ ID NO:88) | 4.8 |
| 5'-PO3-CCACAGTTT (SEQ ID NO:89) CTGGTGTCA-PO3-5' (SEQ ID NO:90) | 4.9 |
| 5′-PO₃-CCTGAAGTT (SEQ ID NO:91) CTGGACTTC-PO₃-5′ (SEQ ID NO:92) | 4.10 |
| 5′-PO3-CTGACGATT (SEQ ID NO:93) CTGACTGCT-PO3-5′ (SEQ ID NO:94) | 4.11 |
| 5'-PO₃-CTCCACTTT (SEQ ID NO:95) CTGAGGTGA-PO₃-5' (SEQ ID NO:96) | 4.12 |
| 5′-PO₃-ACCAGAGCC (SEQ ID NO:97) AATGGTCTC-PO₃-5′ (SEQ ID NO:98) | 5.1 |
| 5′-PO3-ATCCGCACC (SEQ ID NO:99) AATAGGCGT-PO3-5′ (SEQ ID NO:100) | 5.2 |
| 5'-PO3-GACGACACC (SEQ ID NO:101) AACTGCTGT-PO3-5' (SEQ ID NO:102) | 5.3 |
| 5′-PO3-GGATGGACC (SEQ ID NO:103) AACCTACCT-PO3-5′ (SEQ ID NO:104) | 5.4 |
| 5'-PO3-GCAGAAGCC (SEQ ID NO:105) AACGTCTTC-PO3-5' (SEQ ID NO:106) | 5.5 |
| 5′-PO3-GCCATGTCC (SEQ ID NO:107) AACGGTACA-PO3-5′ (SEQ ID NO:108) | 5.6 |
| 5′-PO3-GTCTGCTCC (SEQ ID NO:109) AACAGACGA-PO3-5′ (SEQ ID NO:110) | 5.7 |
| 5′-PO3-CGACAGACC (SEQ ID NO:111) AAGCTGTCT-PO3-5′ (SEQ ID NO:112) | 5.8 |
| 5′-PO₃-CGCTACTCC (SEQ ID NO:113) AAGCGATGA-PO₃-5′ (SEQ ID NO:114) | 5.9 |
| 5′-PO3-CCACAGACC (SEQ ID NO:115) AAGGTGTCT-PO3-5′ (SEQ ID NO:116) | 5.10 |
| 5′-PO3-CCTCTCTCC (SEQ ID NO:117) AAGGAGAGA-PO3-5′ (SEQ ID NO:118) | 5.11 |
| 5′-PO3-CTCGTAGCC (SEQ ID NO:119) AAGAGCATC-PO3-5′ (SEQ ID NO:120) | 5.12 |

1X ligase buffer: 50 mM Tris, pH 7.5; 10 mM dithiothreitol; 10 mM MgCl₂; 2.5 mM ATP; 50 mM NaCl.
10X ligase buffer: 500 mM Tris, pH 7.5; 100 mM dithiothreitol; 100 mM MgCl₂; 25 mM ATP; 500 mM NaCl

### Cycle 1

To each of twelve PCR tubes was added 50 µL of a 1 mM solution of Compound 1 in water; 75 µL of a 0.80 mM solution of one of Tags 1.1-1.12; 15 µL 10X ligase buffer and 10 µL deionized water. The tubes were heated to 95 °C for 1 minute and then cooled to 16 °C over 10 minutes. To each tube was added 5,000 units T4 DNA ligase (2.5 µL of a 2,000,000 unit/mL solution (New England Biolabs, Cat. No. M0202)) in 50 µl 1X ligase buffer and the resulting solutions were incubated at 16°C for 16 hours.

Following ligation, samples were transferred to 1.5 ml Eppendorf tubes and treated with 20 µL 5 M aqueous NaCl and 500 µL cold (-20°C) ethanol, and held at - 20 °C for 1 hour. Following centrifugation, the supernatant was removed and the pellet was washed with 70% aqueous ethanol at -20 °C. Each of the pellets was then dissolved in 150 µL of 150 mM sodium borate buffer, pH 9.4.

Stock solutions comprising one each of building block precursors BB1 to BB12, N,N-diisopropylethanolamine and O-(7-azabenzotriazol-1-yl)-1,1,3,3-tetramethyluronium hexafluorophosphate, each at a concentration of 0.25 M, were prepared in DMF and stirred at room temperature for 20 minutes. The building block precursor solutions were added to each of the pellet solutions described above to provide a 10-fold excess of building block precursor relative to linker. The resulting solutions were stirred. An additional 10 equivalents of building block precursor was added to the reaction mixture after 20 minute, and another 10 equivalents after 40 minutes. The final concentration of DMF in the reaction mixture was 22%. The reaction solutions were then stirred overnight at 4°C. The reaction progress was monitored by RP-HPLC using 50mM aqueous tetraethylammonium acetate (pH=7.5) and acetonitrile, and a gradient of 2-46% acetonitrile over 14 min. Reaction was stopped when ~95% of starting material (linker) is acylated. Following acylation the reaction mixtures were pooled and lyophilized to dryness. The lyophilized material was then purified by HPLC, and the fractions corresponding to the library (acylated product) were pooled and lyophilized.

The library was dissolved in 2.5 ml of 0.01M sodium phosphate buffer (pH = 8.2) and 0.1ml of piperidine (4% v/v) was added to it. The addition of piperidine results in turbidity which does not dissolve on mixing. The reaction mixtures were stirred at room temperature for 50 minutes, and then the turbid solution was centrifuged (14,000 rpm), the supernatant was removed using a 200 µl pipette, and the pellet was resuspended in 0.1 ml of water. The aqueous wash was combined with the supernatant and the pellet was discarded. The deprotected library was precipitated from solution by addition of excess ice-cold ethanol so as to bring the final concentration of ethanol in the reaction to 70% v/v. Centrifugation of the aqueous ethanol mixture gave a white pellet comprising the library. The pellet was washed once with cold 70% aq. ethanol. After removal of solvent the pellet was dried in air (~5min.) to remove traces of ethanol and then used in cycle 2. The tags and corresponding building block precursors used in Round 1 are set forth in Table 1, below.

**Table 1**

| Building | Tag |
|---|---|
| Block | |
| Precursor | |
| BB1 | 1.11 |
| BB2 | 1.6 |
| BB3 | 1.2 |
| BB4 | 1.8 |
| BB5 | 1.1 |
| BB6 | 1.10 |
| BB7 | 1.12 |
| BB8 | 1.5 |
| BB9 | 1.4 |
| BB10 | 1.3 |
| BB11 | 1.7 |
| BB12 | 1.9 |

### Cycles 2-5

For each of these cycles, the combined solution resulting from the previous cycle was divided into 12 equal aliquots of 50 ul each and placed in PCR tubes. To each tube was added a solution comprising a different tag, and ligation, purification and acylation were performed as described for Cycle 1, except that for Cycles 3-5, the HPLC purification step described for Cycle 1 was omitted. The correspondence between tags and building block precursors for Cycles 2-5 is presented in Table 2.

The products of Cycle 5 were ligated with the closing primer shown below, using the method described above for ligation of tags.
5'-PO₃-GGCACATTGATTTGGGAGTCA
   GTGTAACTAAACCCTCAGT-PO₃-5'

**Table 2**

| Building | Cycle 2 | Cycle 3 | Cycle 4 | Cycle 5 |
|---|---|---|---|---|
| Block | Tag | Tag | Tag | Tag |
| Precursor | | | | |
| BB1 | 2.7 | 3.7 | 4.7 | 5.7 |
| BB2 | 2.8 | 3.8 | 4.8 | 5.8 |
| BB3 | 2.2 | 3.2 | 4.2 | 5.2 |
| BB4 | 2.10 | 3.10 | 4.10 | 5.10 |
| BB5 | 2.1 | 3.1 | 4.1 | 5.1 |
| BB6 | 2.12 | 3.12 | 4.12 | 5.12 |
| BB7 | 2.5 | 3.5 | 4.5 | 5.5 |
| BB8 | 2.6 | 3.6 | 4.6 | 5.6 |
| BB9 | 2.4 | 3.4 | 4.4 | 5.4 |
| BB10 | 2.3 | 3.3 | 4.3 | 5.3 |
| BB11 | 2.9 | 3.9 | 4.9 | 5.9 |
| BB12 | 2.11 | 3.11 | 4.11 | 5.11 |

### Results:

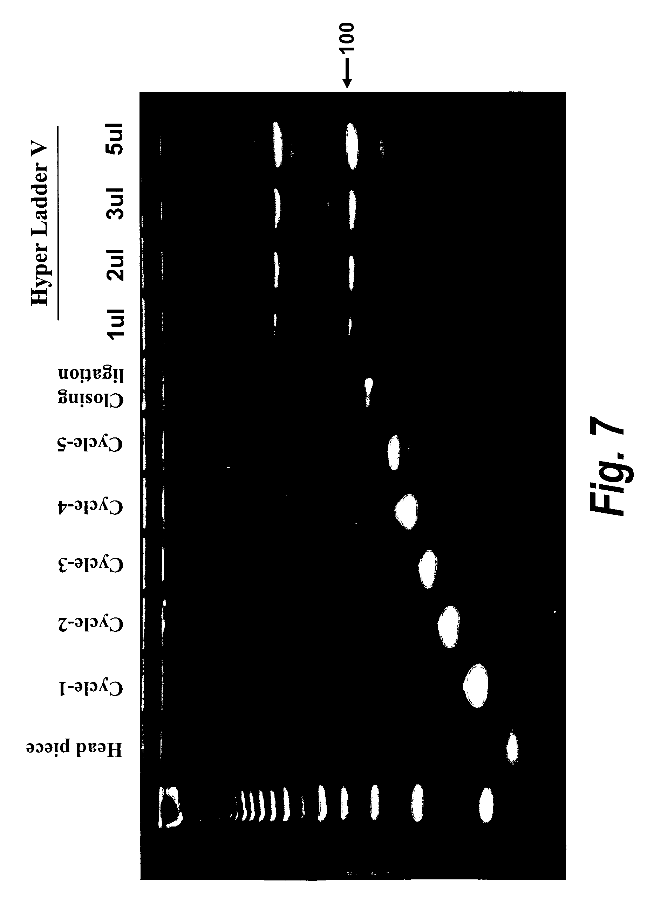

The synthetic procedure described above has the capability of producing a library comprising 12⁵ (about 249,000) different structures. The synthesis of the library was monitored via gel electrophoresis of the product of each cycle. The results of each of the five cycles and the final library following ligation of the closing primer are illustrated in Figure 7. The compound labeled "head piece" is Compound 1. The figure shows that each cycle results in the expected molecular weight increase and that the products of each cycle are substantially homogeneous with regard to molecular weight.

### Example 2: Synthesis and Characterization of a library on the order of 10⁸ members

The synthesis of a library comprising on the order of 10⁸ distinct members was accomplished using the following reagents:

### Compound 2:

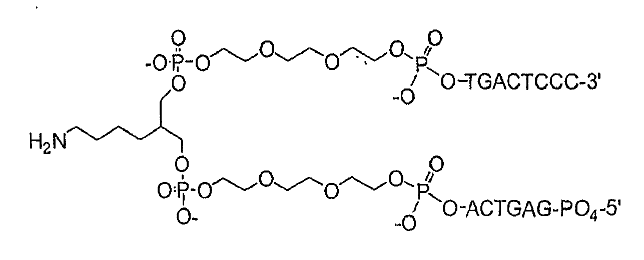

Single letter codes for deoxyribonucleotides:
A = adenosine
C = cytidine
G = guanosine
T = thymidine

### Building block precursors:

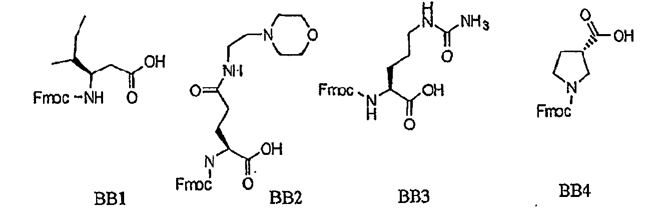

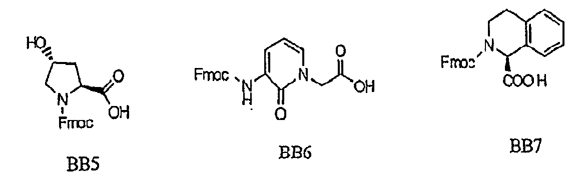

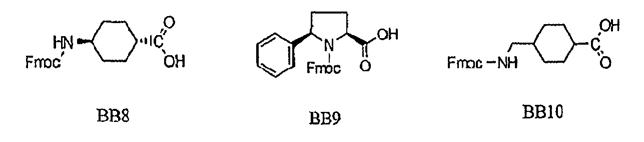

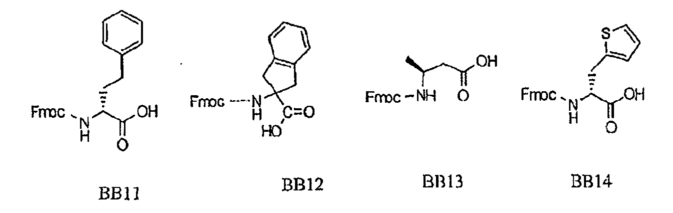

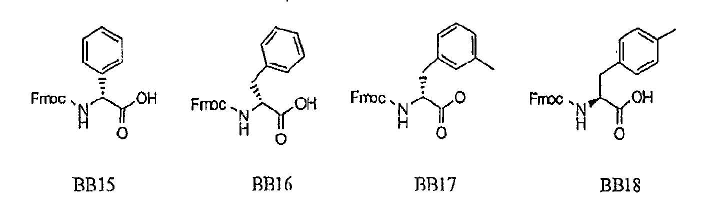

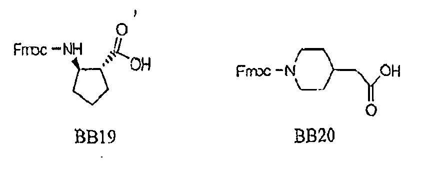

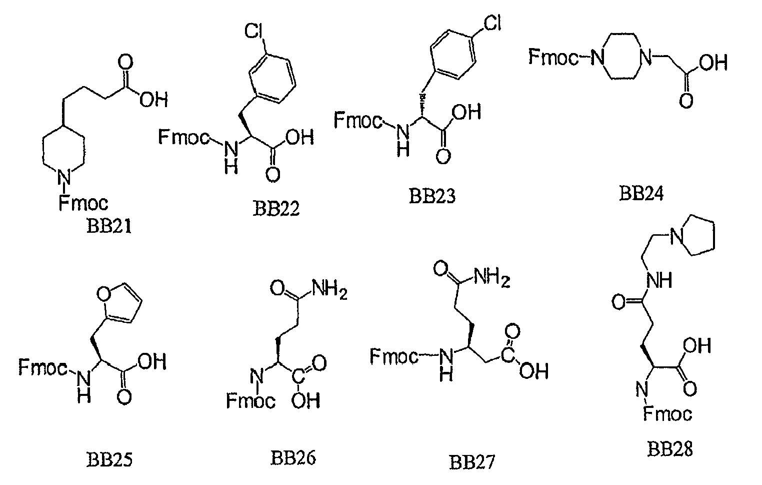

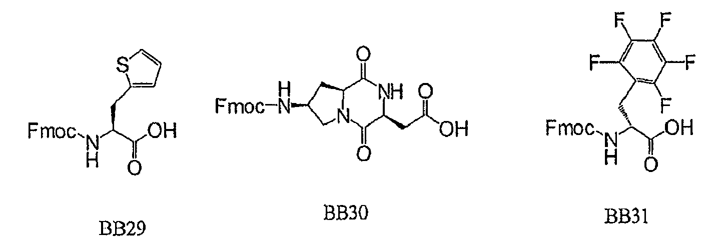

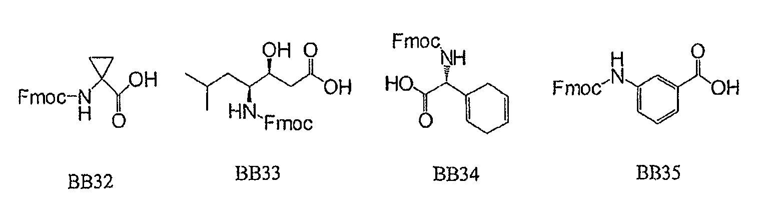

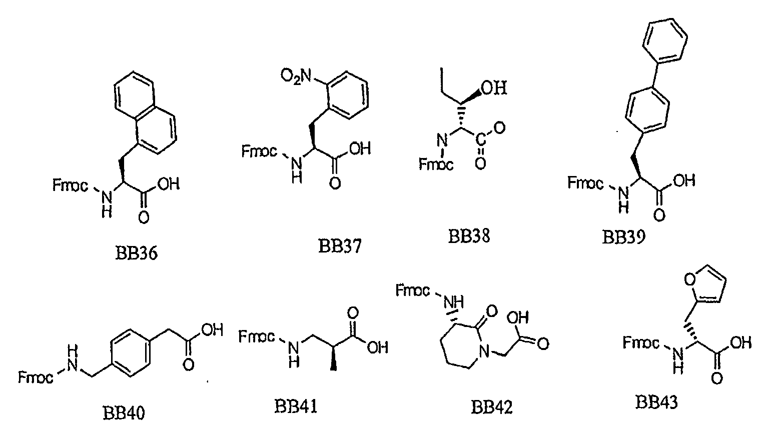

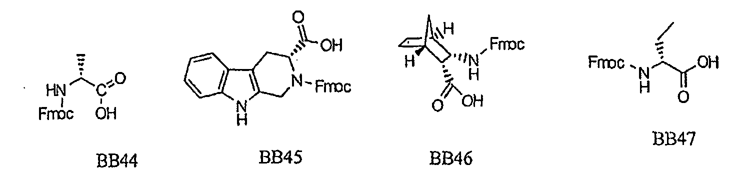

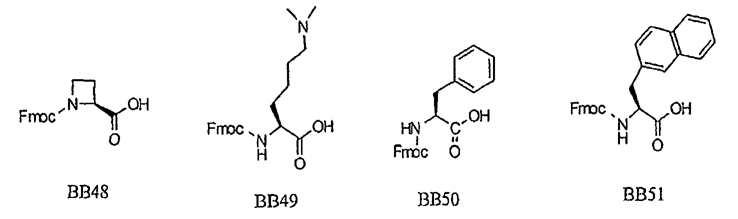

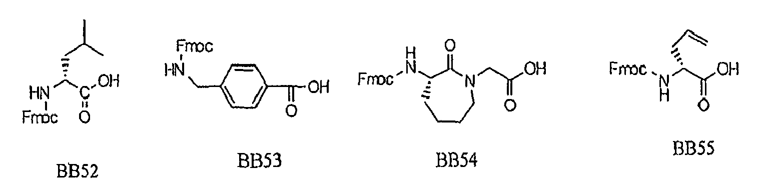

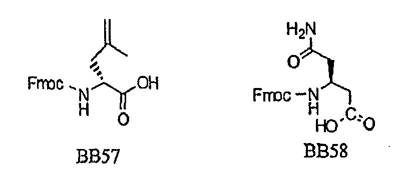

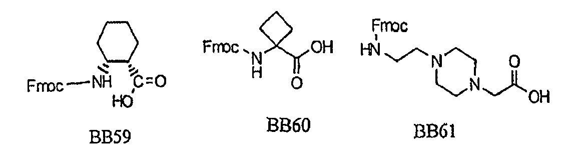

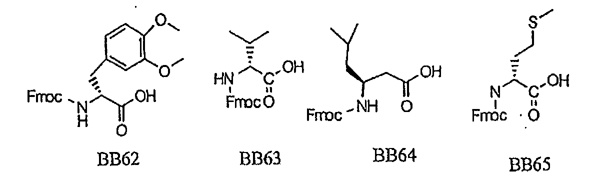

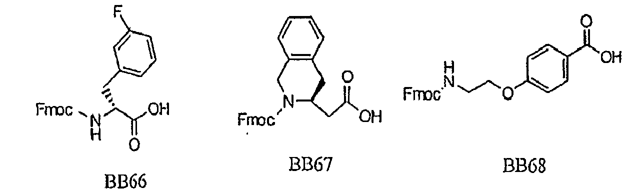

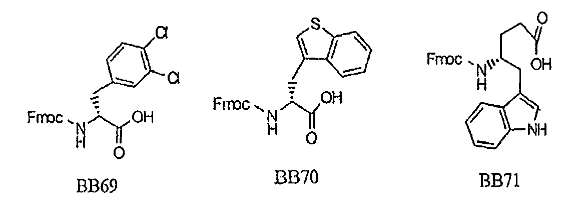

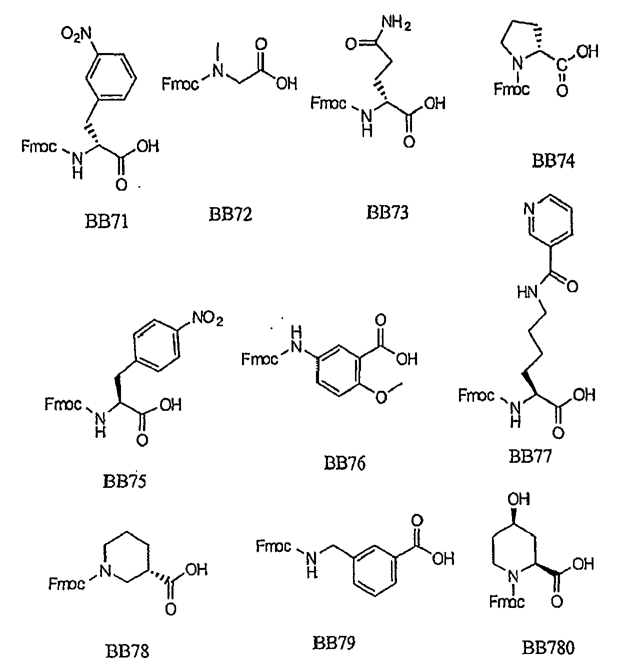

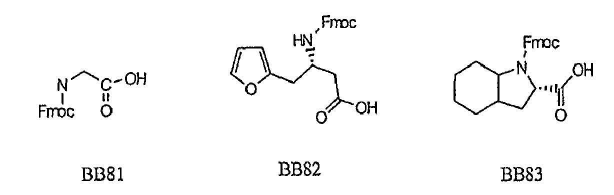

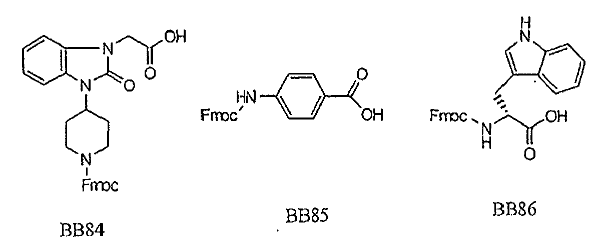

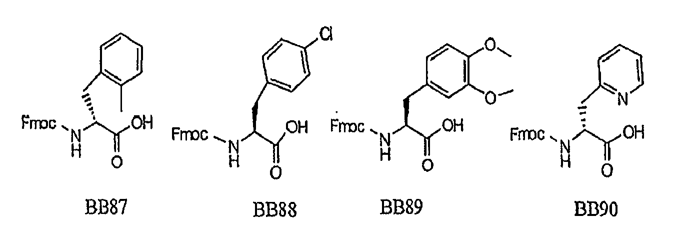

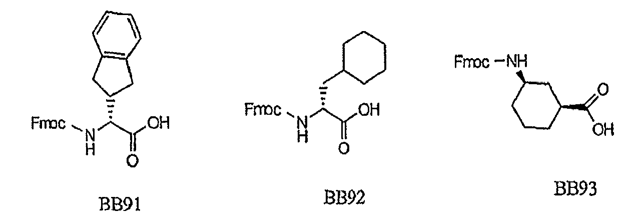

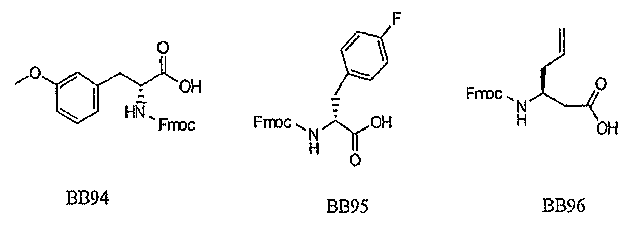

**Table 3: Oligonucleotide tags used in cycle 1:**

| Tag Number | Top Strand Sequence | Bottom Strand Sequence |
|---|---|---|
| 1.1 | 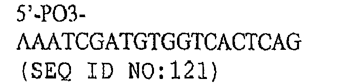 | 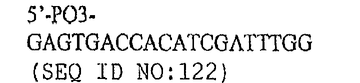 |
| 1.2 | 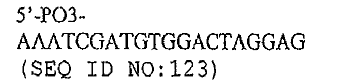 | 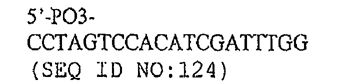 |
| 1.3 | 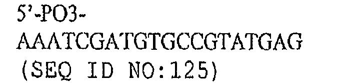 | 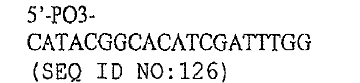 |
| 1.4 | 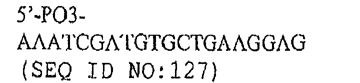 | 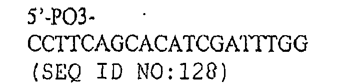 |
| 1.5 | 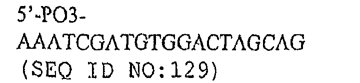 | 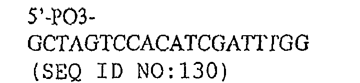 |
| 1.6 | 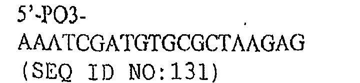 | 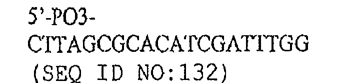 |
| 1.7 | 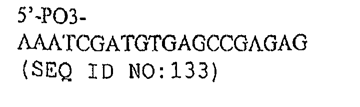 | 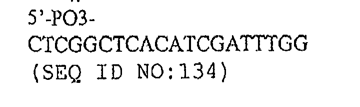 |
| 1.8 | 5'-PO3- AAATCGATGTGCCGTATCAG (SEQ ID NO:135) | 5'-PO3- GATACGGCACATCGATTTGG (SEQ ID NO:136) |
| 1.9 | 5'-PO3- AAATCGATGTGCTGAAGCAG (SEQ ID NO:137) | 5'-PO3- GCTTCAGCACATCGATTTGG (SEQ ID NO:138) |
| 1.10 | 5'-PO3- AAATCGATGTGTGCGAGTAG (SEQ ID NO:139) | 5'-PO3- ACTCGCACACATCGATTTGG (SEQ ID NO:140) |
| 1.11 | 5'-PO3- AAATCGATGTGTTTGGCGAG (SEQ ID NO:141) | 5'-PO3- CGCCAAACACATCGATTTGG (SEQ ID NO:142) |
| 1.12 | 5'-PO3- AAATCGATGTGCGCTAACAG (SEQ ID NO:143) | 5'-PO3- GTTAGCGCACATCGATTTGG (SEQ ID NO:144) |
| 1.13 | 5'-PO3- AAATCGATGTGAGCCGACAG (SEQ ID NO:145) | 5'-PO3- GTCGGCTCACATCGATTTGG (SEQ ID NO:146) |
| 1.14 | 5'-PO3- AAATCGATGTGAGCCGAAAG (SEQ ID NO:147) | 5'-PO3- TTCGGCTCACATCGATTTGG (SEQ ID NO:148) |
| 1.15 | 5'-PO3- AAATCGATGTGTCGGTAGAG (SEQ ID NO:149) | 5'-PO3- CTACCGACACATCGATTTGG (SEQ ID NO:150) |
| 1.16 | 5'-PO3- AAATCGATGTGGTTGCCGAG (SEQ ID NO:151) | 5'-PO3- CGGCAACCACATCGATTTGG (SEQ ID NO:152) |
| 1.17 | 5'-PO3- AAATCGATGTGAGTGCGTAG (SEQ ID NO:153) | 5'-PO3- ACGCACTCACATCGATTTGG (SEQ ID NO:154) |
| 1.18 | 5'-PO3- AAATCGATGTGGTTGCCAAG (SEQ ID NO:155) | 5'-PO3- TGGCAACCACATCGATTTGG (SEQ ID NO:156) |
| 1.19 | 5'-PO3- AAATCGATGTGTGCGAGGAG (SEQ ID NO:157) | 5'-PO3- CCTCGCACACATCGATTTGG (SEQ ID NO:158) |
| 1.20 | 5'-PO3- AAATCGATGTGGAACACGAG (SEQ ID NO:159) | 5'-PO3- CGTGTTCCACATCGATTTGG (SEQ ID NO:160) |
| 1.21 | 5'-PO3- AAATCGATGTGCTTGTCGAG (SEQ ID NO:161) | 5'-PO3- CGACAAGCACATCGATTTGG (SEQ ID NO:162) |
| 1.22 | 5'-PO3- AAATCGATGTGTTCCGGTAG (SEQ ID NO:163) | 5'-PO3- A0CCGGAACACATCGATTTGG (SEQ ID NO:164) |
| 1.23 | 5'-PO3- AAATCGATGTGTGCGAGCAG (SEQ ID NO:165) | 5'-PO3- GCTCGCACACATCGATTTGG (SEQ ID NO:166) |
| 1.24 | 5'-PO3- AAATCGATGTGGTCAGGTAG (SEQ ID NO:167) | 5'-PO3- ACCTGACCACATCGATTTGG (SEQ ID NO:168) |
| 1.25 | 5'-PO3- AAATCGATGTGGCCTGTTAG | 5'-PO3- AACAGGCCACATCGATTTGG |
| | (SEQ ID NO:169) | (SEQ ID NO:170) |
| 1.26 | 5'-PO3- AAATCGATGTGGAACACCAG . (SEQ ID NO:171) | 5'-PO3- GGTGTTCCACATCGATTTGG (SEQ ID NO:172) |
| 1.27 | 5'-PO3-AAATCGATGTGCTTGTCCAG (SEQ ID NO:173) | 5'-PO3- GGACAAGCACATCGATTTGG (SEQ ID NO:174) |
| 1.28 | 5'-PO3- AAATCGATGTGTGCGAGAAG (SEQ ID NO:175) | 5'-PO3- TCTCGCACACATCGATTTGG (SEQ ID NO:176) |
| 1.29 | 5'-PO3- AAATCGATGTGAGTGCGGAG (SEQ ID NO:177) | 5'-PO3- CCGCACTCACATCGATTTGG (SEQ ID NO:178) |
| 1.30 | 5'-PO3- AAATCGATGTGTTGTCCGAG (SEQ ID NO:179) | 5'-PO3- CGGACAACACATCGATTTGG (SEQ ID NO:180) |
| 1.31 | 5'-PO3- AAATCGATGTGTGGAACGAG (SEQ ID NO:181) | 5'-PO3- CGTTCCACACATCGATTTGG (SEQ ID NO:182) |
| 1.32 | 5'-PO3- AAATCGATGTGAGTGCGAAG (SEQ ID NO:183) | 5'-PO3- TCGCACTCACATCGATTTGG (SEQ ID NO:184) |
| 1.33 | 5'-PO3- AAATCGATGTGTGGAACCAG (SEQ ID NO:185) | 5'-PO3- GGTTCCACACATCGATTTGG (SEQ ID NO:186) |
| 1.34 | 5'-PO3- AAATCGATGTGTTAGGCGAG (SEQ ID NO:187) | 5'-PO3- CGCCTAACACATCGATTTGG (SEQ ID NO:188) |
| 1.35 | 5'-PO3- AAATCGATGTGGCCTGTGAG (SEQ ID NO:189) | 5'-PO3- CACAGGCCACATCGATTTGG (SEQ ID NO:190) |
| 1.36 | 5'-PO3-AAATCGATGTGCTCCTGTAG (SEQ ID NO:191) | 5'-PO3- ACAGGAGCACATCGATTTGG (SEQ ID NO:192) |
| 1.37 | 5'-PO3- AAATCGATGTGGTCAGGCAG (SEQ ID NO:193) | 5'-PO3- GCCTGACCACATCGATTTGG (SEQ ID NO:194) |
| 1.38 | 5'-PO3- AAATCGATGTGGTCAGGAAG (SEQ ID NO:195) | 5'-PO3- TCCTGACCACATCGATTTGG (SEQ ID NO:196) |
| 1.39 | 5'-PO3- AAATCGATGTGGTAGCCGAG (SEQ ID NO:197) | 5'-PO3- CGGCTACCACATCGATTTGG (SEQ ID NO:198) |
| 1.40 | 5'-PO3- AAATCGATGTGGCCTGTAAG (SEQ ID NO:199) | 5'-PO3- TACAGGCCACATCGATTTGG (SEQ ID NO:200) |
| 1.41 | 5'-PO3- AAATCGATGTGCTTTCGGAG (SEQ ID NO:201) | 5'-PO3- CCGAAAGCACATCGATTTGG (SEQ ID NO:202) |
| 1.42 | 5'-PO3- AAATCGATGTGCGTAAGGAG (SEQ ID NO:203) | 5'-PO3- CCTTACGCACATCGATTTGG (SEQ ID NO:204) |
| 1.43 | 5'-PO3- AAATCGATGTGAGAGCGTAG (SEQ ID NO:205) | 5'-PO3- ACGCTCTCACATCGATTTGG (SEQ ID NO:206) |
| 1.44 | 5'-PO3- AAATCGATGTGGACGGCAAG (SEQ ID NO:207) | 5'-PO3- TGCCGTCCACATCGATTTGG (SEQ ID NO:208) |
| 1.45 | 5'-PO3-AAATCGATGTGCTTTCGCAG (SEQ ID NO:209) | 5'-PO3- GCGAAAGCACATCGATTTGG (SEQ ID NO:210) |
| 1.46 | 5'-PO3- AAATCGATGTGCGTAAGCAG (SEQ ID NO:211) | 5'-PO3- GCTTACGCACATCGATTTGG (SEQ ID NO:212) |
| 1.47 | 5'-PO3- AAATCGATGTGGCTATGGAG (SEQ ID NO:213) | 5'-PO3- CCATAGCCACATCGATTTGG (SEQ ID NO:214) |
| 1.48 | 5'-PO3- AAATCGATGTGACTCTGGAG (SEQ ID NO:215) | 5'-PO3- CCAGAGTCACATCGATTTGG (SEQ ID NO:216) |
| 1.49 | 5'-PO3-AAATCGATGTGCTGGAAAG (SEQ ID NO:217) | 5'-PO3- TTCCAGCACATCGATTTGG (SEQ ID NO:218) |
| 1.50 | 5'-PO3- AAATCGATGTGCCGAAGTAG (SEQ ID NO:219) | 5'-PO3- ACTTCGGCACATCGATTTGG (SEQ ID NO:220) |
| 1.51 | 5'-PO3- AAATCGATGTGCTCCTGAAG (SEQ ID NO:221) | 5'-PO3- TCAGGAGCACATCGATTTGG (SEQ ID NO:222) |
| 1.52 | 5'-PO3- AAATCGATGTGTCCAGTCAG (SEO ID NO:223) | 5'-PO3- GACTGGACACATCGATTTGG (SEQ ID NO:224) |
| 1.53 | 5'-PO3- AAATCGATGTGAGAGCGGAG (SEQ ID NO:225) | 5'-PO3- CCGCTCTCACATCGATTTGG (SEQ ID NO:226) |
| 1.54 | 5'-PO3- AAATCGATGTGAGAGCGAAG (SEQ ID NO:227) | 5'-PO3- TCGCTCTCACATCGATTTGG (SEQ ID NO:228) |
| 1.55 | 5'-PO3- AAATCGATGTGCCGAAGGAG (SEQ ID NO:229) | 5'-PO3- CCTTCGGCACATCGATTTGG (SEQ ID NO:230) |
| 1.56 | 5'-PO3- AAATCGATGTGCCGAAGCAG (SEQ ID NO:231) | 5'-PO3- GCTTCGGCACATCGATTTGG (SEQ ID NO:232) |
| 1.57 | 5'-PO3- AAATCGATGTGTGTTCCGAG (SEQ ID NO:233) | 5'-PO3- CGGAACACACATCGATTTGG (SEQ ID NO:234) |
| 1.58 | 5'-PO3- AAATCGATGTGTCTGGCGAG (SEQ ID NO:235) | 5'-PO3- CGCCAGACACATCGATTTGG (SEQ ID NO:236) |
| 1.59 | 5'-PO3- AAATCGATGTGCTATCGGAG (SEQ ID NO:237) | 5'-PO3- CCGATAGCACATCGATTTGG (SEQ ID NO:238) |
| 1.60 | 5'-PO3- AAATCGATGTGCGAAAGGAG | 5'-PO3- CCTTTCGCACATCGATTTGG |
| | (SEQ ID NO:239) | (SEQ ID NO:240) |
| 1.61 | 5'-PO3- AAATCGATGTGCCGAAGAAG (SEQ ID NO:241) | 5'-PO3- TCTTCGGCACATCGATTTGG (SEQ ID NO:242) |
| 1.62 | 5'-PO3- AAATCGATGTGGTTGCAGAG (SEQ ID NO:243) | 5'-PO3- CTGCAACCACATCGATTTGG (SEQ ID NO:244) |
| 1.63 | 5'-PO3- AAATCGATGTGGATGGTGAG (SEQ ID NO:245) | 5'-PO3- CACCATCCACATCGATTTGG (SEQ ID NO:246) |
| 1.64 | 5'-PO3- AAATCGATGTGCTATCGCAG (SEQ ID NO:247) | 5'-PO3- GCGATAGCACATCGATTTGG (SEQ ID NO:248) |
| 1.65 | 5'-PO3- AAATCGATGTGCGAAAGCAG (SEQ ID NO:249) | 5'-PO3- GCTTTCGCACATCGATTTGG (SEQ ID NO:250) |
| 1.66 | 5'-PO3- AAATCGATGTGACACTGGAG (SEQ ID NO:251) | 5'-PO3- CCAGTGTCACATCGATTTGG (SEQ ID NO:252) |
| 1.67 | 5'-PO3- AAATCGATGTGTCTGGCAAG (SEQ ID NO:253) | 5'-PO3- TGCCAGACACATCGATTTGG (SEQ ID NO:254) |
| 1.68 | 5'-PO3- AAATCGATGTGGATGGTCAG (SEQ ID NO:255) | 5'-PO3- GACCATCCACATCGATTTGG (SEQ ID NO:256) |
| 1.69 | 5'-PO3- AAATCGATGTGGTTGCACAG (SEQ ID NO:257) | 5'-PO3- GTGCAACCACATCGATTTGG (SEQ ID NO:258) |
| 1.70 | 5'-PO3- AAATCGATGTGGGCATCGAG (SEQ ID NO:259) | 5'-PO3-CGATGCCCCATCCGA TTT GG (SEQ ID NO:260) |
| 1.71 | 5'-PO3- AAATCGATGTGTGCCTCCAG (SEQ ID NO:261) | 5'-PO3- GGAGGCACACATCGATTTGG (SEQ ID NO:262) |
| 1.72 | 5'-PO3- AAATCGATGTGTGCCTCAAG (SEQ ID NO:263) | 5'-PO3- TGAGGCACACATCGATTTGG (SEQ ID NO:264) |
| 1.73 | 5'-PO3- AAATCGATGTGGGCATCCAG (SEQ ID NO:265) | 5'-PO3- GGATGCCCACATCGATTTGG (SEQ ID NO:266) |
| 1.74 | 5'-PO3- AAATCGATGTGGGCATCAAG (SEQ ID NO:267) | 5'-PO3-TGATGCCCA CAT CGA TIT GG (SEQ ID NO:268) |
| 1.75 | 5'-PO3- AAATCGATGTGCCTGTCGAG (SEQ ID NO:269) | 5'-PO3-CGA CAG GCA CAT CGA TTT GG (SEQ ID NO:270) |
| 1.76 | 5'-PO3- AAATCGATGTGGACGGATAG (SEQ ID NO:271) | 5'-PO3-ATC CGT CCA CAT CGA TTT GG (SEQ ID NO:272) |
| 1.77 | 5'-PO3- AAATCGATGTGCCTGTCCAG (SEQ ID NO:273) | 5'-PO3-GGA CAG GCA CAT CGA TTT GG (SEQ ID NO:274) |
| 1.78 | 5’-PO3- AAATCGATGTGAAGCACGAG (SEQ ID NO:275) | 5'-PO3-CGT GCT TCA CAT CGA TTT GG (SEQ ID NO:276) |
| 1.79 | 5'-PO3- AAATCGATGTGCCTGTCAAG (SEQ ID NO:277) | 5'-PO3-TGA CAG GCA CAT CGA TTT GG (SEQ ID NO:278) |
| 1.80 | 5'-PO3- AAATCGATGTGAAGCACCAG (SEQ ID NO:279) | 5'-PO3-GGT GCT TCA CAT CGA TTT GG (SEQ ID NO:280) |
| 1.81 | 5'-PO3-AAATCGATGTGCCTTCGTAG (SEQ ID NO:281) | 5'-PO3-ACG AAG GCA CAT CGA TTT GG (SEQ ID NO:282) |
| 1.82 | 5'-PO3- AAATCGATGTGTCGTCCGAG (SEQ ID NO:283) | 5'-PO3-CGG ACG ACA CAT CGA TTT GG (SEQ ID NO:284) |
| 1.83 | 5'-PO3- AAATCGATGTGGAGTCTGAG (SEQ ID NO:285) | 5'-PO3-CAG ACT CCA CAT CGA TTT GG (SEQ ID NO:286) |
| 1.84 | 5'-PO3- AAATCGATGTGTGATCCGAG (SEQ ID NO:287) | 5'-PO3-CGG ATC ACA CAT CGA TTT GG (SEQ ID NO:288) |
| 1.85 | 5'-PO3- AAATCGATGTGTCAGGCGAG (SEQ ID NO:289) | 5'-PO3-CGC CTG ACA CAT CGA TTT GG (SEQ ID NO:290) |
| 1.86 | 5'-PO3- AAATCGATGTGTCGTCCAAG (SEQ ID NO:291) | 5'-PO3-TGG ACG ACA CAT CGA TTT GG (SEQ ID NO:292) |
| 1.87 | 5'-PO3- AAATCGATGTGGACGGAGAG (SEQ ID NO:293) | 5'-PO3-CTC CGT CCA CAT CGA TTT GG (SEQ ID NO:294) |
| 1.88 | 5'-PO3- AAATCGATGTGGTAGCAGAG (SEQ ID NO:295) | 5'-PO3-CTG CTA CCA CAT CGA TTT GG (SEQ ID NO:296) |
| 1.89 | 5'-PO3- AAATCGATGTGGCTGTGTAG (SEQ ID NO:297) | 5'-PO3- ACACAGCCACATCGATTTGG (SEQ ID NO:298) |
| 1.90 | 5'-PO3- AAATCGATGTGGACGGACAG (SEQ ID NO:299) | 5'-PO3-GTC CGT CCA CAT CGA TTT GG (SEQ ID NO:300) |
| 1.91 | 5'-PO3- AAATCGATGTGTCAGGCAAG (SEQ ID NO:301) | 5'-PO3-TGC CTG ACA CAT CGA TTT GG (SEQ ID NO:302) |
| 1.92 | 5'-PO3- AAATCGATGTGGCTCGAAAG (SEQ ID NO:303) | 5'-PO3- TTCGAGCCACATCGATTTGG (SEQ ID NO:304) |
| 1.93 | 5'-PO3- AAATCGATGTGCCTTCGGAG (SEQ ID NO:305) | 5'-PO3-CCG AAG GCA CAT CGA TTT GG (SEQ ID NO:306) |
| 1.94 | 5'-PO3- AAATCGATGTGGTAGCACAG (SEQ ID NO:307) | 5'-PO3-GTG CTA CCA CAT CGA TTT GG (SEQ ID NO:308) |
| 1.95 | 5'-PO3- AAATCGATGTGGAAGGTCAG | 5'-PO3-GAC CTT CCA CAT CGA TTT GG |
| | (SEQ ID NO:309) | (SEQ ID NO:310) |
| 1.96 | 5'-PO3- AAATCGATGTGGTGCTGTAG (SEQ ID NO:311) | 5'-PO3-ACA GCA CCA CAT CGA TTT GG (SEQ ID NO:312) |
| | | |

**Table 4: Oligonucleotide tags used in cycle 2:**

| Tag Number | Top strand sequence | Bottom strand sequence |
|---|---|---|
| 2.1 | 5'-PO3-GTT GCC TGT (SEQ ID NO:313) | 5'-PO3-AGG CAA CCT (SEQ ID NO:314) |
| 2.2 | 5'-P03-CAG GAC GGT (SEQ ID NO:315) | 5'-PO3-CGT CCT GCT (SEQ ID NO:316) |
| 2.3 | 5'-P03-AGA CGT GGT (SEQ ID NO:317) | 5'-PO3-CAC GTC TCT (SEQ ID NO:318) |
| 2.4 | 5'-PO3-CAG GAC CGT (SEQ ID NO:319) | 5'-P03-GGT CCT GCT (SEQ ID NO:320) |
| 2.5 | 5'-PO3-CAG GAC AGT (SEQ ID NO:321) | 5'-PO3-TGT CCT GCT (SEQ ID NO:322) |
| 2.6 | 5'-PO3-CAC TCT GGT (SEQ ID NO:323) | 5'-PO3-CAG AGT GCT (SEQ ID NO:324) |
| 2.7 | 5'-PO3-GAC GGC TGT (SEQ ID NO:325) | 5'-P03-AGC CGT CCT (SEQ ID NO:326) |
| 2.8 | 5'-PO3-CAC TCT CGT (SEQ ID NO:327) | 5'-PO3-GAG AGT GCT (SEQ ID NO:328) |
| 2.9 | 5'-PO3-GTA GCC TGT (SEQ ID NO:329) | 5'-PO3-AGG CTA CCT (SEQ ID NO:330) |
| 2.10 | 5'-PO3-GCC ACT TGT (SEQ ID NO:331) | 5'-PO3-AAG TGG CCT (SEQ ID NO:332) |
| 2.11 | 5'-PO3-CAT CGC TGT (SEQ ID NO:333) | 5'-P03-AGC GAT GCT (SEQ ID NO:334) |
| 2.12 | 5'-PO3-CAC TGG TGT (SEQ ID NO:335) | 5'-PO3-ACC AGT GCT (SEQ ID NO:336) |
| 2.13 | 5'-PO3-GCC ACT GGT (SEQ ID NO:337) | 5'-P03-CAG TGG CCT (SEQ ID NO:338) |
| 2.14 | 5'-PO3-TCT GGC TGT (SEQ ID NO:339) | 5'-PO3-AGC CAG ACT (SEQ ID NO:340) |
| 2.15 | 5'-PO3-GCC ACT CGT (SEQ ID NO:341) | 5'-PO3-GAG TGG CCT (SEQ ID NO:342) |
| 2.16 | 5'-P03-TGC CTC TGT (SEQ ID NO:343) | 5'-PO3-AGA GGC ACT (SEQ ID NO:344) |
| 2.17 | 5'-PO3-CAT CGC AGT (SEQ ID NO:345) | 5'-PO3-TGC GAT GCT (SEQ ID NO:346) |
| 2.18 | 5'-P03-CAG GAA GGT (SEQ ID NO:347) | 5'-PO3-CTT CCT GCT (SEQ ID NO:348) |
| 2.19 | 5'-PO3-GGC ATC TGT (SEQ ID NO:349) | 5'-PO3-AGA TGC CCT (SEQ ID NO:350) |
| 2.20 | 5'-PO3-CGG TGC TGT (SEQ ID NO:351) | 5'-P03-AGC ACC GCT (SEQ ID NO:352) |
| 2.21 | 5'-PO3-CAC TGG CGT (SEQ ID NO:353) | 5'-PO3-GCC AGT GCT (SEQ ID NO:354) |
| 2.22 | 5'-PO3-TCTCCTCGT (SEQ ID NO:355) | 5'-PO3-GAGGAGACT (SEQ ID NO:356) |
| 2.23 | 5'-PO3-CCT GTC TGT (SEQ ID NO:357) | 5'-P03-AGA CAG GCT (SEQ ID NO:358) |
| 2.24 | 5'-PO3-CAA CGC TGT (SEQ ID NO:359) | 5'-PO3-AGC GTT GCT (SEQ ID NO:360) |
| 2.25 | 5'-PO3-TGC CTC GGT (SEQ ID NO:361) | 5'-PO3-CGA GGC ACT (SEQ ID NO:362) |
| 2.26 | 5'-PO3-ACA CTG CGT (SEQ ID NO:363) | 5'-PO3-GCA GTG TCT (SEQ ID NO:364) |
| 2.27 | 5'-PO3-TCG TCC TGT (SEQ ID NO:365) | 5'-PO3-AGG ACG ACT (SEQ ID NO:366) |
| 2.28 | 5'-P03-GCT GCC AGT (SEQ ID NO:367) | 5'-P03-TGG CAG CCT (SEQ ID NO:368) |
| 2.29 | 5'-PO3-TCA GGC TGT (SEQ ID NO:369) | 5'-PO3-AGC CTG ACT (SEQ ID NO:370) |
| 2.30 | 5'-PO3-GCC AGG TGT (SEQ ID NO:371) | 5'-PO3-ACC TGG CCT (SEQ ID NO:372) |
| 2.31 | 5'-PO3-CGG ACC TGT (SEQ ID NO:373) | 5'-PO3-AGG TCC GCT (SEQ ID NO:374) |
| 2.32 | 5'-PO3-CAA CGC AGT (SEQ ID NO:375) | 5'-PO3-TGC GTT GCT (SEQ ID NO:376) |
| 2.33 | 5'-PO3-CAC ACG AGT (SEQ ID NO:377) | 5'-PO3-TCG TGT GCT (SEQ ID NO:378) |
| 2.34 | 5'-P03-ATG GCC TGT (SEQ ID NO:379) | 5'-PO3-AGG CCA TCT (SEQ ID NO:380) |
| 2.35 | 5'-PO3-CCA GTC TGT (SEQ ID NO:381) | 5'-PO3-AGA CTG GCT (SEQ ID NO:392) |
| 2.36 | 5'-PO3-GCC AGG AGT (SEQ ID NO:383) | 5'-PO3-TCC TGG CCT (SEQ ID NO:384) |
| 2.37 | 5'-P03-CGG ACC AGT (SEQ ID NO:385) | 5'-PO3-TGG TCC GCT (SEQ ID NO:386) |
| 2.38 | 5'-P03-CCT TCG CGT (SEQ ID NO:387) | 5'-PO3-GCG AAG GCT (SEQ ID NO:388) |
| 2.39 | 5'-PO3-GCA GCC AGT (SEQ ID NO:389) | 5'-PO3-TGG CTG CCT (SEQ ID NO:390) |
| 2.40 | 5'-PO3-CCA GTC GGT (SEQ ID NO:391) | 5'-P03-CGA CTG GCT (SEQ ID NO:392) |
| 2.41 | 5'-PO3-ACT GAG CGT (SEQ ID NO:393) | 5'-P03-GCT CAG TCT (SEQ ID NO:394) |
| 2.42 | 5'-PO3-CCA GTC CGT (SEQ ID NO:395) | 5'-PO3-GGA CTG GCT (SEQ ID NO:396) |
| 2.43 | 5'-PO3-CCA GTC AGT (SEQ ID NO:397) | 5'-PO3-TGA CTG GCT (SEQ ID NO:398) |
| 2.44 | 5'-P03-CAT CGA GGT (SEQ ID NO:399) | 5'-PO3-CTC GAT GCT (SEQ ID NO:400) |
| 2.45 | 5'-PO3-CC TCG TGT (SEQ ID NO:401) | 5'-PO3-ACG ATG GCT (SEQ ID NO:402) |
| 2.46 | 5'-PO3-GTG CTG CGT (SEQ ID NO:403) | 5'-PO3-GCA GCA CCT (SEQ ID NO:404) |
| 2.47 | 5'-P03-GAC TAC GGT (SEQ ID NO:405) | 5'-PO3-CGT AGT CCT (SEQ ID NO:406) |
| 2.48 | 5'-PO3-GTG CTG AGT (SEQ ID NO:407) | 5'-PO3-TCA GCA CCT (SEQ ID NO:408) |
| 2.49 | 5'-P03-GCTGCATGT (SEQ ID NO:409) | 5'-PO3-ATGCAGCCT (SEQ ID NO:410) |
| 2.50 | 5'-PO3-GAGTGGTGT (SEQ ID NO:411) | 5'-PO3-ACCACTCCT (SEQ ID NO:412) |
| 2.51 | 5'-PO3-GACTACCGT (SEQ ID NO:413) | 5'-PO3-GGTAGTCC (SEQ ID NO:414) |
| 2.52 | 5'-P03-CGGTGATGT (SEQ ID NO:415) | 5'-P03-ATCACCGCT (SEQ ID NO:416) |
| 2.53 | 5'-PO3-TGCGACTGT (SEQ ID NO:417) | 5'-PO3-AGTCGCACT (SEQ ID NO:418) |
| 2.54 | 5'-PO3-TCTGGAGGT (SEQ ID NO:419) | 5'-PO3-CTCCAGACT (SEQ ID NO:420) |
| 2.55 | 5'-P03-AGCACTGGT (SEQ ID NO:421) | 5'-PO3-CAGTGCTCT (SEQ ID NO:422) |
| 2.56 | 5'-PO3-TCGCTTGGT (SEQ ID NO:423) | 5'-PO3-CAAGCGACT (SEQ ID NO:424) |
| 2.57 | 5'-P03-AGCACTCGT (SEQ ID NO:425) | 5'-PO3-GAGTGCTCT (SEQ ID NO:426) |
| 2.58 | 5'-PO3-GCGATTGGT (SEQ ID NO:427) | 5'-PO3-CAATCGCCT (SEQ ID NO:428) |
| 2.59 | 5'-PO3-CCATCGCGT (SEQ ID NO:429) | 5'-PO3-GCGATGGCT (SEQ ID NO:430) |
| 2.60 | 5'-PO3-TCGCTTCGT (SEQ ID NO:431) | 5'-PO3-GAAGCGACT (SEQ ID NO:432) |
| 2.61 | 5'-PO3-AGTGCCTGT (SEQ ID NO:433) | 5'-PO3-AGGCACTCT (SEQ ID NO:434) |
| 2.62 | 5'-PO3-GGCATAGGT (SEQ ID NO:435) | 5'-PO3-CTATGCCCT (SEQ ID NO:436) |
| 2.63 | 5'-PO3-GCGATTCG (SEQ ID NO:437) | 5'-PO3-GAATCGCC (SEQ ID NO:438) |
| 2.64 | 5'-PO3-TGCGACGGT (SEQ ID NO:439) | 5'-PO3-CGTCGCACT (SEQ ID NO:440) |
| 2.65 | 5'-PO3-GAGTGGCGT (SEQ ID NO:441) | 5'-PO3-GCCACTCCT (SEQ ID NO:442) |
| 2.66 | 5'-PO3-CGGTGAGGT (SEQ ID NO:443) | 5'-PO3-CTCACCGCT (SEQ ID NO:444) |
| 2.67 | 5'-PO3-GCTGCAAGT (SEQ ID NO:445) | 5'-PO3-TTGCAGCCT (SEQ ID NO:446) |
| 2.68 | 5'-PO3-TTCCGCTGT (SEQ ID NO:447) | 5'-PO3-AGCGGAACT (SEQ ID NO:448) |
| 2.69 | 5'-PO3-GAGTGGAGT (SEQ ID NO:449) | 5'-PO3-TCCACTCCT (SEQ ID NO:450) |
| 2.70 | 5'-PO3-ACAGAGCGT (SEQ ID NO:451) | 5'-PO3-GCTCTGTCT (SEQ ID NO:452) |
| 2.71 | 5'-PO3-TGCGACCGT (SEQ ID NO:453) | 5'-PO3-GGTCGCACT (SEQ ID NO:454) |
| 2.72 | 5'-PO3-CCTGTAGGT (SEQ ID NO:455) | 5'-PO3-CTACAGGCT (SEQ ID NO:456) |
| 2.73 | 5'-PO3-TAGCCGTG (SEQ ID NO:457) | 5'-P03-ACGGCTACT (SEQ ID NO:458) |
| 2.74 | 5'-P03-TGCGACAGT (SEQ ID NO:459) | 5'-PO3-TGTCGCACT (SEQ ID NO:460) |
| 2.75 | 5'-PO3-GGTCTGTGT (SEQ ID NO:461) | 5'-P03-ACAGACCCT (SEQ ID NO:462) |
| 2.76 | 5'-PO3-CGGTGAAGT (SEQ ID NO:463) | 5'-PO3-TTCACCGCT (SEQ ID NO:464) |
| 2.77 | 5'-PO3-CAACGAGGT (SEQ ID NO:465) | 5'-PO3-CTCGTTGCT (SEQ ID NO:466) |
| 2.78 | 5'-PO3-GCAGCATGT (SEQ ID NO:467) | 5'-PO3-ATGCTGCCT (SEQ ID NO:468) |
| 2.79 | 5'-PO3-TCGTCAGGT (SEQ ID NO;469) | 5'-PO3-CTGACGAC (SEQ ID NO:470) |
| 2.80 | 5'-PO3-AGTGCCAGT (SEQ ID NO:471) | 5'-PO3-TGGCACTCT (SEQ ID NO:472) |
| 2.81 | 5'-PO3-TAGAGGCGT (SEQ TD NO:473) | 5'-PO3-GCCTCTACT (SEQ TD NO:474) |
| 2.82 | 5'-PO3-GTCAGCGGT (SEQ ID NO:475) | 5'-PO3-CGCTGACCT (SEQ ID NO:476) |
| 2.83 | 5'-PO3-TCAGGAGGT (SEQ ID NO:477) | 5'-PO3-CTCCTGACT (SEQ ID NO:478) |
| 2.84 | 5'-PO3-AGCAGGTGT (SEQ ID NO:479 | 5'-PO3-ACCTGCTCT (SEQ ID NO:480) |
| 2.85 | 5'-PO3-TTCCGCAGT (SEQ ID NO:481) | 5'-PO3-TGCGGAACT (SEQ ID NO:492) |
| 2.86 | 5'-PO3-GTCAGCCGT (SEQ ID NO:483) | 5'-PO3-GGCTGACCT (SEQ ID NO:484) |
| 2.87 | 5'-PO3-GGTCTGCGT (SEQ ID NO:485) | 5'-PO3-GCAGACCCT (SEQ ID NO:496) |
| 2.88 | 5'-PO3-TAGCCGAGT (SEQ ID NO:487) | 5'-PO3-TCGGCTACT (SEQ ID NO:138) |
| 2.89 | 5'-PO3-GTCAGCAGT (SEQ ID NO:489) | 5'-PO3-TGCTGACCT (SEQ ID NO:490) |
| 2.90 | 5'-PO3-GGTCTGAGT (SEQ ID NO:491) | 5'-PO3-TCAGACCCT (SEQ ID NO:492) |
| 2.91 | 5'-PO3-CGGACAGGT (SEQ ID NO:493) | 5'-PO3-CTGTCCGCT (SEQ ID NO:494) |
| 2.92 | 5'-PO3-TTAGCCGGT5'- PO3-3' (SEQ ID NO:495) | 5'-PO3-CGGCTAACT5'-PO3- 3' (SEQ ID NO:496) |
| 2.93 | 5'-PO3-GAGACGAGT (SEQ ID NO:497) | 5'-PO3-TCGTCTCCT (SEQ ID NO:498) |
| 2.94 | 5'PO3-CGTAACCGT (SEQ ID NO:499) | 5'-PO3-GGTTACGCT (SEQ ID NO**:**500) |
| 2.95 | 5'-PO3-TTGGCGTGT5'- PO3-3' (SEQ ID NO:501) | 5'-PO3-ACGCCAACT5'-PO3- 3' (SEQ ID NO:502) |
| 2.96 | 5'-PO3-ATGGCAGGT (SEQ ID NO:503) | 5'-PO3-CTGCCATCT (SEQ ID NO:504) |
| | | |

**Table 5. Oligonucleotide tags used in cycle 3**

| Tag number | Top strand sequence | Bottom strand sequence |
|---|---|---|
| 3.1 | 5'-PO3-CAG CTA CGA (SEQ ID NO:505) | 5'-PO3-GTA GCT GAC (SEQ ID NO:506) |
| 3.2 | 5'-P03-CTC CTG CGA (SEQ ID NO:507) | 5'-P03-GCA GGA GAC (SEQ ID NO:508) |
| 3.3 | 5'-P03-GCT GCC TGA (SEQ ID NO:509) | 5'-PO3-AGG CAG CAC (SEQ ID NO:510) |
| 3.4 | 5'-PO3-CAG GAA CGA (SEQ ID NO:511) | 5'-PO3-GTT CCT GAC (SEQ ID NO:512) |
| 3.5 | 5'-PO3-CAC ACG CGA (SEQ ID NO:513) | 5'-PO3-GCG TGT GAC (SEQ ID NO:514) |
| 3.6 | 5'-PO3-GC GCC TGA (SEQ ID NO:515) | 5'-PO3-AGG CTG CAC (SEQ ID NO:516) |
| 3.7 | 5'-PO3-CTG AAC GGA (SEQ ID NO:517) | 5'-PO3-CGT TCA GAC (SEQ ID NO:518) |
| 3.8 | 5'-PO3-CTG AAC CGA (SEQ ID NO:519) | 5'-PO3-GGT TCA GAC (SEQ ID NO:520) |
| 3.9 | 5'-P03-TCT GGA CGA (SEQ ID NO:521) | 5'-P03-GTC CAG AAC (SEQ ID NO:522) |
| 3.10 | 5'-PO3-TGC CTA CGA (SEQ ID NO:523) | 5'-PO3-GTA GGC AAC (SEQ ID NO:524) |
| 3.11 | 5'-P03-GGC ATA CGA (SEQ ID NO:525) | 5'-PO3-GTA TGC CAC (SEQ ID NO:526) |
| 3.12 | 5'-PO3-CGG TGA CGA (SEQ ID NO:527) | 5'-PO3-GTC ACC GAC (SEQ ID NO:528) |
| 3.13 | 5'-P03-CAA CGA CGA (SEQ ID NO:529) | 5'-PO3-GTC GTT GAC (SEQ ID NO:530) |
| 3.14 | 5'-PO3-CTC CTC TGA (SEQ ID NO:531) | 5'-PO3-AGA GGA GAC (SEQ ID NO:532) |
| 3.15 | 5'-PO3-TCA GGA CGA (SEQ ID NO:533) | 5'-PO3-GTC CTG AAC (SEQ ID NO:534) |
| 3.16 | 5'-PO3-AAA GGC GGA (SEQ ID NO:535) | 5'-P03-CGC CTT TAC (SEQ ID NO:536) |
| 3.17 | 5'-P03-CTC CTC GGA (SEQ ID NO:537) | 5'-PO3-CGA GGA GAC (SEQ ID NO:538) |
| 3.18 | 5'-PO3-CAG ATG CGA (SEQ ID NO:539) | 5'-PO3-GCA TCT GAC (SEQ ID NO:540) |
| 3.19 | 5'-PO3-GCA GCA AGA (SEQ ID NO:541) | 5'-PO3-TTG CTG CAC (SEQ ID NO:542) |
| 3.20 | 5'-PO3-GTG GAG TGA (SEQ ID NO:543) | 5'-PO3-ACT CCA CAC (SEQ ID NO:544) |
| 3.21 | 5'-PO3-CCA GTA GGA (SEQ ID NO:545) | 5'-PO3-CTA CTG GAC (SEQ ID NO:546) |
| 3.22 | 5'-PO3-ATG GCA CGA (SEQ ID NO:547) | 5'-PO3-GTG CCA TAC (SEQ ID NO:548) |
| 3.23 | 5'-PO3-GGA CTG TGA (SEQ ID NO:549) | 5'-PO3-ACA GTC CAC (SEQ ID NO:550) |
| 3.24 | 5'-P03-CCG AAC TGA (SEQ ID NO:551) | 5'-P03-AGT TCG GAC (SEQ ID NO:552) |
| 3.25 | 5'-P03-CTC CTC AGA (SEQ ID NO:553) | 5'-PO3-TGA GGA GAC (SEQ ID NO:554) |
| 3.26 | 5'-PO3-CAC TGC TGA (SEQ ID N0:555) | 5'-P03-AGC AGT GAC (SEQ ID NO:556) |
| 3.27 | 5'-PO3-AGC AGG CGA (SEQ ID NO:557) | 5'-P03-GCC TGC TAC (SEQ ID NO:558) |
| 3.28 | 5'-P03-AGC AGG AGA (SEQ ID NO:559) | 5'-P03-TCC TGC TAC (SEQ ID NO:560) |
| 3.29 | 5'-PO3-AGA GCC AGA (SEQ ID NO:561) | 5'-PO3-TGG CTC TAC (SEQ ID NO:562) |
| 3.30 | 5'-PO3-GTC GTT GGA (SEQ ID NO:563) | 5'-PO3-CAA CGA CAC (SEQ ID NO:564) |
| 3.31 | 5'-PO3-CCG AAC GGA (SEQ ID NO:565) | 5'-PO3-CGT TCG GAC (SEQ ID NO:566) |
| 3.32 | 5'-PO3-CAC TGC GGA (SEQ ID NO:567) | 5'-PO3-CGC AGT GAC (SEQ ID NO:568) |
| 3.33 | 5'-PO3-GTG GAG CGA (SEQ ID NO:569) | 5'-PO3-GC CCA CAC (SEQ ID NO:570) |
| 3.34 | 5'-P03-GTG GAG AGA (SEQ ID NO:571) | 5'-P03-TCT CCA CAC (SEQ ID NO:572) |
| 3.35 | 5'-PO3-GGA CTG CGA (SEQ ID NO:573) | 5'-PO3-GCA GTC CAC (SEQ ID NO:574) |
| 3.36 | 5'-PO3-CCG AAC CGA (SEQ ID NO:575) | 5'-PO3-GGT TCG GAC (SEQ ID NO:576) |
| 3.37 | 5'-PO3-CAC TGC CGA (SEQ ID NO:577) | 5'-PO3-GGC AGT GAC (SEQ ID NO:578) |
| 3.38 | 5'-P03-CGA AAC GGA (SEQ ID NO:579) | 5'-PO3-CGT TTC GAC (SEQ ID NO:580) |
| 3.39 | 5'-PO3-GGA CTG AGA (SEQ ID NO:581) | 5'-PO3-TCA GTC CAC (SEQ ID NO:582) |
| 3.40 | 5'-PO3-CCG AAC AGA (SEQ ID NO:583) | 5'-PO3-TGT TCG GAC (SEQ ID NO:584) |
| 3.41 | 5'-PO3-CGA AAC CGA (SEQ ID NO:585) | 5'-PO3-GGT TTC GAC (SEQ ID NO:586) |
| 3.42 | 5'-P03-CTG GCT TGA (SEQ ID NO:587) | 5'-PO3-AAG CCA GAC (SEQ ID N0:588) |
| 3.43 | 5'-PO3-CAC ACC TGA (SEQ ID NO:589) | 5'-PO3-AGG TGT GAC (SEQ ID NO:590) |
| 3.44 | 5'-PO3-AAC GAC CGA (SEQ ID NO:591) | 5'-P03-GGT CGT TAC (SEQ ID NO:592) |
| 3.45 | 5'-PO3-ATC CAG CGA (SEQ ID NO:593) | 5'-PO3-GCT GGA TAC (SEQ ID NO:594) |
| 3.46 | 5'-PO3-TGC GAA GGA (SEQ ID NO:595) | 5'-PO3-CTT CGC AAC (SEQ ID NO:596) |
| 3.47 | 5'-PO3-TGC GAA CGA (SEQ ID NO:597) | 5'-PO3-GTT CGC AAC (SEQ ID NO:598) |
| 3.48 | 5'-PO3-CTG GCT GGA (SEQ ID NO:599) | 5'-PO3-CAG CCA GAC (SEQ ID NO:600) |
| 3.49 | 5'-PO3-CAC ACC GGA (SEQ ID NO:601) | 5'-PO3-CGG TGT GAC (SEQ ID NO:602) |
| 3.50 | 5'-PO3-AGT GCA GGA (SEQ ID NO:603) | 5'-PO3-CTG CAC TAC (SEQ ID NO:604) |
| 3.51 | 5'-P03-GAC CGT TGA (SEQ ID NO:605) | 5'-PO3-AAC GGT CAC (SEQ ID NO:606) |
| 3.52 | 5'-PO3-GGT GAG TGA (SEQ ID NO:607) | 5'-PO3-ACT CAC CAC (SEQ ID NO:608) |
| 3.53 | 5'-PO3-CCT TCC TGA (SEQ ID NO:609) | 5'-PO3-AGG AAG GAC (SEQ ID NO:610) |
| 3.54 | 5'-P03-CTG GCT AGA (SEQ ID NO:611) | 5'-P03-TAG CCA GAC (SEQ ID NO:612) |
| 3.55 | 5'-PO3-CAC ACC AGA (SEQ ID NO:613) | 5'-PO3-TGG TGT GAC (SEQ ID NO:614) |
| 3.56 | 5'-PO3-AGC GGT AGA (SEQ ID NO:615) | 5'-PO3-TAC CGC TAC (SEQ ID NO:616) |
| 3.57 | 5'-PO3-GTC AGA GGA (SEQ ID NO:617) | 5'-PO3-CTC TGA CAC (SEQ ID NO:618) |
| 3.58 | 5'-PO3-TTC CGA CGA (SEQ ID NO:619) | 5'-PO3-GTC GGA AAC (SEQ ID NO:620) |
| 3.59 | 5'-P03-AGG CGT AGA (SEQ ID NO:621) | 5'-PO3-TAC GCC TAC (SEQ ID NO:622) |
| 3.60 | 5'-PO3-CTC GAC TGA (SEQ ID NO:623) | 5'-PO3-AGT CGA GAC (SEQ ID NO:624) |
| 3.61 | 5'-PO3-TAC GCT GGA (SEQ ID NO:625) | 5'-PO3-CAG CGT AAC (SEQ ID NO:626) |
| 3.62 | 5'-PO3-GTT CGG TGA (SEQ ID NO:627) | 5'-P03-ACC GAA CAC (SEQ ID NO:628) |
| 3.63 | 5'-PO3-GCC AGC AGA (SEQ ID NO:629) | 5'-PO3-TGC TGG CAC (SEQ ID NO:630) |
| 3.64 | 5'-PO3-GAC CGT AGA (SEQ ID NO:631) | 5'-PO3-TAC GGT CAC (SEQ ID NO:632) |
| 3.65 | 5'-PO3-GTG CTC TGA (SEQ ID NO:633) | 5'-PO3-AGA GCA CAC (SEQ ID NO:634) |
| 3.66 | 5'-PO3-GGT GAG CGA (SEQ ID NO:635) | 5'-PO3-GCT CAC CAC (SEQ ID NO:636) |
| 3.67 | 5'-PO3-GGT GAG AGA (SEQ ID NO:637) | 5'-PO3-TCT CAC CAC (SEQ ID NO:638) |
| 3.68 | 5'-PO3-CCT TCC AGA (SEQ ID NO:639) | 5'-PO3-TGG AAG GAC (SEQ ID NO:640) |
| 3.69 | 5'-PO3-CTC CTA CGA (SEQ ID NO:641) | 5'-PO3-GTA GGA GAC (SEQ ID NO:642) |
| 3.70 | 5'-PO3-CTC GAC GGA (SEQ ID NO:643) | 5'-PO3-CGT CGA GAC (SEQ ID NO:644) |
| 3.71 | 5'-P03-GCC GTT TGA (SEQ ID NO:645) | 5'-PO3-AAA CGG CAC (SEQ ID NO:646) |
| 3.72 | 5'-PO3-GCG GAG TGA (SEQ ID NO:647) | 5'-PO3-ACT CCG CAC (SEQ ID NO:648) |
| 3.73 | 5'-PO3-CGT GCT TGA (SEQ ID NO:649) | 5'-PO3-AAG CAC GAC (SEQ ID NO:650) |
| 3.74 | 5'-PO3-CTC GAC CGA (SEQ ID NO:651) | 5'-PO3-GGT CGA GAC (SEQ ID NO:652) |
| 3.75 | 5'-P03-AGA GCA GGA (SEQ ID NO:653) | 5'-PO3-CTG CTC TAC (SEQ ID NO:654) |
| 3.76 | 5'-PO3-GTG CTC GGA (SEQ ID NO:655) | 5'-PO3-CGA GCA CAC (SEQ ID NO:656) |
| 3.77 | 5'-PO3-CTC GAC AGA (SEQ ID NO:657) | 5'-PO3-TGT CGA GAC (SEQ ID NO:658) |
| 3.78 | 5'-P03-GGA GAG TGA (SEQ ID NO:659) | 5'-P03-ACT CTC CAC (SEQ ID NO:660) |
| 3.79 | 5'-P03-AGG CTG TGA (SEQ ID NO:661) | 5'-PO3-ACA GCC TAC (SEQ ID NO:662) |
| 3.80 | 5'-PO3-AGA GCA CGA (SEQ ID NO:663) | 5'-PO3-GTG CTC TAC (SEQ ID NO:664) |
| 3.81 | 5'-PO3-CCA TCC TGA (SEQ ID NO:665) | 5'-PO3-AGG ATG GAC (SEQ ID NO:666) |
| 3.82 | 5'-PO3-GTT CGG AGA (SEQ ID NO:667) | 5'-PO3-TCC GAA CAC (SEQ ID NO:668) |
| 3.83 | 5'-PO3-TGG TAG CGA (SEQ ID NO:669) | 5'-P03-GCT ACC AAC (SEQ ID NO:670) |
| 3.84 | 5'-P03-GTG CTC CGA (SEQ ID NO:671) | 5'-P03-GGA GCA CAC (SEQ ID NO:672) |
| 3.85 | 5'-P03-GTG CTC AGA (SEQ ID NO:673) | 5'-PO3-TGA GCA CAC (SEQ ID NO:674) |
| 3.86 | 5'-PO3-GCC GTT GGA (SEQ ID NO:675) | 5'-PO3-CAA CGG CAC (SEQ ID NO:676) |
| 3.87 | 5'-PO3-GAG TGC TGA (SEQ ID NO:677) | 5'-P03-AGC ACT CAC (SEQ ID NO:678) |
| 3.88 | 5'-PO3-GCT CCT TGA (SEQ ID NO:679) | 5'-PO3-AAG GAG CAC (SEQ ID NO:680) |
| 3.89 | 5'-PO3-CCG AAA GGA (SEQ ID NO:681) | 5'-PO3-CTT TCG GAC (SEQ ID NO:682) |
| 3.90 | 5'-PO3-CAC TGA GGA (SEQ ID NO:683) | 5'-PO3-CTC AGT GAC (SEQ ID NO:684) |
| 3.91 | 5'-PO3-CGT GCT GGA (SEQ ID NO:685) | 5'-PO3-CAG CAC GAC (SEQ ID NO:686) |
| 3.92 | 5'-PO3-CCG AAA CGA (SEQ ID NO:687) | 5'-PO3-GTT TCG GAC (SEQ ID NO:688) |
| 3.93 | 5'-PO3-GCG GAG AGA (SEQ ID NO:689) | 5'-PO3-TCT CCG CAC (SEQ ID NO:690) |
| 3.94 | 5'-PO3-GCC GTT AGA (SEQ ID NO:691) | 5'-PO3-TAA CGG CAC (SEQ ID NO:692) |
| 3.95 | 5'-PO3-TCT CGT GGA (SEQ ID NO:693) | 5'-PO3-CAC GAG AAC (SEQ ID NO:694) |
| 3.96 | 5'-PO3-CGT GCT AGA (SEQ ID NO:695) | 5'-PO3-TAG CAC GAC (SEQ ID NO:696) |
| | | |

**Table 6. Oligonucleotide tags used in cycle 4**

| Tag number | Top strand sequence | Bottom strand sequence |
|---|---|---|
| 4.1 | 5'-PO3-GCCTGTCTT (SEQ ID NO:697) | 5'-PO3-GAC AGG CTC (SEQ ID NO:698) |
| 4.2 | 5'-PO3-CTCCTGGTT (SEQ ID NO:699) | 5'-PO3-CCA GGA GTC (SEQ ID NO:700) |
| 4.3 | 5'-PO3-ACTCTGCTT (SEQ ID NO:701) | 5'-PO3-GCA GAG TTC (SEQ ID NO:702) |
| 4.4 | 5'-PO3-CATCGCCTT (SEQ ID NO:703) | 5'-PO3-GGC GAT GTC (SEQ ID NO:704) |
| 4.5 | 5'-PO3-GCCACTATT (SEQ ID NO:705) | 5'-P03-TAG TGG CTC (SEQ ID NO:706) |
| 4.6 | 5'-PO3-CACACGGTT (SEQ ID NO:707) | 5'-PO3-CCG TGT GTC (SEQ ID NO:708) |
| 4.7 | 5'-PO3-CAACGCCTT (SEQ ID NO:705) | 5'-P03-GGC GTT GTC (SEQ ID NO:710) |
| 4.8 | 5'-PO3-ACTGAGGTT (SEQ ID NO:711) | 5'-P03-CCT CAG TTC (SEQ ID NO:712) |
| 4.9 | 5'-PO3-GTGCTGGTT (SEQ ID NO:713) | 5'-PO3-CCA GCA CTC (SEQ ID NO:714) |
| 4.10 | 5'-PO3-CATCGACTT (SEQ ID NO:715) | 5'-P03-GTC GAT GTC (SEQ ID NO:716) |
| 4.11 | 5'-PO3-CCATCGGTT (SEQ ID NO:717) | 5'-PO3-CCG ATG GTC (SEQ ID NO:718) |
| 4.12 | 5'-PO3-GCTGCACTT (SEQ ID NO:719) | 5'-P03-GTG CAG CTC (SEQ ID NO:720) |
| 4.13 | 5'-PO3-ACAGAGGTT (SEQ ID NO:721) | 5'-P03-CCT CTG TTC (SEQ ID NO:722) |
| 4.14 | 5'-PO3-AGTGCCGTT (SEQ ID NO:723) | 5'-PO3-CGG CAC TTC (SEQ ID NO:724) |
| 4.15 | 5'-PO3-CGGACATTT (SEQ ID NO:725) | 5'-PO3-ATG TCC GTC (SEQ ID NO:726) |
| 4.16 | 5'-PO3-GGTCTGGTT (SEQ ID NO:727) | 5'-P03-CCA GAC CTC (SEQ ID NO:728) |
| 4.17 | 5'-PO3-GAGACGGTT (SEQ ID NO:729) | 5'-PO3-CCG TCT CTC (SEQ ID NO:730) |
| 4.18 | 5'-PO3-CTTTCCGTT (SEQ ID NO:731) | 5'-PO3-CGG AAA GTC (SEQ ID NO:732) |
| 4.19 | 5'-PO3-CAGATGGTT (SEQ ID NO:733) | 5'-PO3-CCA TCT GTC (SEQ ID NO:734) |
| 4.20 | 5'-P03-CGGACACTT (SEQ ID NO:735) | 5'-PO3-GTG TCC GTC (SEQ ID NO:736) |
| 4.21 | 5'-PO3-ACTCTCGTT (SEQ ID NO:737) | 5'-PO3-CGA GAG TTC (SEQ ID NO:738) |
| 4.22 | 5'-PO3-GCAGCACTT (SEQ ID NO:739) | 5'-PO3-GTG CTG CTC (SEQ ID NO:740) |
| 4.23 | 5'-PO3-ACTCTCCTT (SEQ ID NO:741) | 5'-PO3-GGA GAG TTC (SEQ ID NO:742) |
| 4.24 | 5'-PO3-ACCTTGGTT (SEQ ID NO:743) | 5'-PO3-CCA AGG TTC (SEQ ID NO:744) |
| 4.25 | 5'-PO3-AGAGCCGTT (SEQ ID NO:745) | 5'-PO3-CGG CTC TTC (SEQ ID NO:746) |
| 4.26 | 5'-PO3-ACCTTGCTT (SEQ ID NO:747) | 5'-P03-GCA AGG TTC (SEQ ID NO:748) |
| 4.27 | 5'-PO3-AAGTCCGTT (SEQ ID NO:749) | 5'-PO3-CGG ACT TTC (SEQ ID NO:750) |
| 4.28 | 5'-PO3-GGA CTG GTT (SEQ ID NO:751) | 5'-PO3-CCA GTC CTC (SEQ ID NO:752) |
| 4.29 | 5'-PO3-GTCGTTCTT (SEQ ID NO:753) | 5'-PO3-GAA CGA CTC (SEQ ID NO:754) |
| 4.30 | 5'-PO3-CAGCATC (SEQ ID NO:755) | 5'-PO3-GAT GCT GTC (SEQ ID NO:756) |
| 4.31 | 5'-PO3-CTATCCGTT (SEQ ID NO:757) | 5'-PO3-CGG ATA GTC (SEQ ID NO:758) |
| 4.32 | 5'-PO3-ACACTCGTT (SEQ ID NO:759) | 5'-P03-CGA GTG TTC (SEQ ID NO:760) |
| 4.33 | 5'-PO3-ATCCAGGTT (SEQ ID NO:761) | 5'-P03-CCT GGA TTC (SEQ ID NO:762) |
| 4.34 | 5'-PO3-GTTCCTGTT (SEQ ID NO:763) | 5'-PO3-CAG GAA CTC (SEQ ID NO:764) |
| 4.35 | 5'-P03-ACACTCCTT (SEQ ID NO:765) | 5'-PO3-GGA GTG TTC (SEQ ID NO:766) |
| 4.36 | 5'-PO3-GTTCCTCTT (SEQ ID NO:767) | 5'-PO3-GAG GAA CTC (SEQ ID NO:768) |
| 4.37 | 5'-PO3-CTGGCTCTT (SEQ ID NO:769) | 5'-PO3-GAG CCA GTC (SEQ ID NO:770) |
| 4.38 | 5'-PO3-ACGGCATTT (SEQ ID NO:771) | 5'-PO3-ATG CCG TTC (SEQ ID NO:772) |
| 4.39 | 5'-PO3-GGTGAGGTT (SEQ ID NO:773) | 5'-PO3-CCT CAC CTC (SEQ ID NO:774) |
| 4.40 | 5'-PO3-CCTTCCGTT (SEQ ID NO:775) | 5'-PO3-CGG AAG GTC (SEQ ID NO:776) |
| 4.41 | 5'-PO3-TACGCTCTT (SEQ ID NO:717) | 5'-PO3-GAG CGT ATC (SEQ ID NO:778) |
| 4.42 | 5'-PO3-ACGGCAGTT (SEQ ID NO:779) | 5'-P03-CTG CCG TTC (SEQ ID NO:780 |
| 4.43 | 5'-PO3-ACTGACGTT (SEQ ID NO:781) | 5'-PO3-CGT CAG TTC (SEQ ID NO:782) |
| 4.44 | 5'-PO3-ACGGCACTT (SEQ ID NO:783) | 5'-PO3-GTG CCG TTC (SEQ ID NO:784) |
| 4.45 | 5'-PO3-ACTGACCTT (SEQ ID NO:785) | 5'-PO3-GGT CAG TTC (SEQ ID NO:786) |
| 4.46 | 5'-PO3-TTTGCGGTT (SEQ ID NO:787) | 5'-PO3-CCG CAA ATC (SEQ ID NO:788) |
| 4.47 | 5'-PO3-TGGTAGGTT (SEQ ID NO:789) | 5'-PO3-CCT ACC ATC (SEQ ID NO:790) |
| 4.48 | 5'-PO3-GTTCGGCTT (SEQ ID NO:791) | 5'-PO3-GCC GAA CTC (SEQ ID NO:792) |
| 4.49 | 5'-PO3-GCC GTT CTT (SEQ ID NO:793) | 5'-PO3-GAA CGG CTC (SEQ ID NO:794) |
| 4.50 | 5'-PO3-GGAGAGGTT (SEQ ID NO:795) | 5'-PO3-CCT CTC CTC (SEQ ID NO:796) |
| 4.51 | 5'-PO3-CACTGACTT (SEQ ID NO:797) | 5'-PO3-GTC AGT GTC (SEQ ID NO:798) |
| 4.52 | 5'-PO3-CGTGCTCTT (SEQ ID NO:799) | 5'-PO3-GAG CAC GTC (SEQ ID NO:800) |
| 4.53 | 5'-PO3-AATCCGCTT (SEQ ID NO:801) | 5'-PO3-GCGGATTTC (SEQ ID NO:802) |
| 4.54 | 5'-PO3-AGGCTGGTT (SEQ ID NO:803) | 5'-PO3-CCA GCC TTC (SEQ ID NO:804) |
| 4.55 | 5'-PO3-GCTAGTGTT (SEQ ID NO:805) | 5'-PO3-CAC TAG CTC (SEQ ID NO:806) |
| 4.56 | 5'-P03-GGAGAGCTT (SEQ ID NO:807) | 5'-PO3-GCT CTC CTC (SEQ ID NO:808) |
| 4.57 | 5'-PO3-GGAGAGATT (SEQ ID NO:809) | 5'-PO3-TCT CTC CTC (SEQ ID NO:810) |
| 4.58 | 5'-PO3-AGGCTGCTT (SEQ ID NO:811) | 5'-PO3-GCA GCC TTC (SEQ ID NO:812) |
| 4.59 | 5'-PO3-GAGTGCGTT (SEQ ID NO:813) | 5'-PO3-CGC ACT CTC (SEQ ID NO:814) |
| 4.60 | 5'-PO3-CCATCCATT (SEQ ID NO:815) | 5'-PO3-TGG ATG GTC (SEQ ID NO:816) |
| 4,61 | 5'-PO3-GCTAGTCTT (SEQ ID NO:817) | 5'-PO3-GACTAGCTC (SEQ ID NO:818) |
| 4.62 | 5'-P03-AGGCTGATT (SEQ ID NO:819) | 5'-PO3-TCA GCC TTC (SEQ ID NO:820) |
| 4.63 | 5'-PO3-ACAGACGTT (SEQ-ID NO:821) | 5'-PO3-CGT CTG TTC (SEQ ID NO:822) |
| 4.64 | 5'-P03-GAGTGCCTT (SEQ ID NO:823) | 5'-PO3-GGC ACT CTC (SEQ ID NO:824) |
| 4.65 | 5'-P03-ACAGACCTT (SEQ ID NO:825) | 5'-PO3-GGT CTG TTC (SEQ ID NO:826) |
| 4.66 | 5'-PO3-CGAGCTTTT (SEQ ID NO:827) | 5'-PO3-AAG CTC GTC (SEQ ID NO:828) |
| 4.67 | 5'-PO3-TTAGCGGTT (SEQ ID NO:829) | 5'-PO3-CCG CTA ATC (SEQ ID NO:830) |
| 4.68 | 5'-PO3-CCTCTTGTT (SEQ ID NO:831) | 5'-PO3-CAA GAG GTC (SEQ ID NO:832) |
| 4.69 | 5'-PO3-GGTCTCTTT (SEQ ID NO:833) | 5'-P03-AGA GAC CTC (SEQ ID NO:834) |
| 4.70 | 5'-PO3-GCCAGATTT (SEQ ID NO:835) | 5'-PO3-ATC TGG CTC (SEQ ID NO:836) |
| 4.71 | 5'-PO3-GAGACCTTT (SEQ ID NO:837) | 5'-PO3-AGG TCT CTC (SEQ ID NO:838) |
| 4.72 | 5'-P03-CACACAGTT (SEQ ID NO:839) | 5'-PO3-CTG TGT GTC (SEQ ID NO:840) |
| 4.73 | 5'-P03-CCTCTTCTT (SEQ ID NO:841) | 5'-PO3-GAA GAG GTC (SEQ ID NO:842) |
| 4.74 | 5'-PO3-TAGAGCGTT (SEQ ID NO:843) | 5'-P03-CGC TCT ATC (SEQ ID NO:844) |
| 4.75 | 5'-P03-GCACCTTTT (SEQ ID NO:845) | 5'-PO3-AAG GTG CTC (SEQ ID NO:846) |
| 4.76 | 5'-P03-GGCTTGTTT (SEQ ID NO:847) | 5'-PO3-ACA AGC CTG (SEQ ID NO:848) |
| 4.77 | 5'-PO3-GACGCGATT (SEQ ID NO:849) | 5'-PO3-TCG CGT CTC (SEQ ID NO:850) |
| 4.78 | 5'-PO3-CGAGCTGTT (SEQ ID NO:851) | 5'-PO3-CAG CTC GTC (SEQ ID NO:852) |
| 4.79 | 5'-PO3-TAGAGCCTT (SEQ ID NO:853) | 5'-PO3-GGC TCT ATC (SEQ ID NO:854) |
| 4.80 | 5'-PO3-CATCCGTTT (SEQ ID NO:855) | 5'-PO3-ACG GAT GTC (SEQ ID NO:856) |
| 4.81 | 5'-PO3-GGTCTCGTT (SEQ ID NO:857) | 5'-PO3-CGA GAC CTC (SEQ ID NO:858) |
| 4.82 | 5'-PO3-GCCAGAGTT (SEQ ID NO:859) | 5'-PO3-CTC TGG CTC (SEQ ID NO:860) |
| 4.83 | 5'-P03-GAGACCGTT (SEQ ID NO:861) | 5'-PO3-CGG TCT CTC (SEQ ID NO:862) |
| 4.84 | 5'-P03-CGAGCTATT (SEQ ID NO:863) | 5'-PO3-TAG CTC GTC (SEQ ID NO:864) |
| 4.85 | 5'-PO3-GCAAGTGTT (SEQ ID NO:865) | 5'-PO3-CAC TTG CTC (SEQ ID NO:866) |
| 4.86 | 5'-PO3-GGTCTCCTT (SEQ ID NO:867) | 5'-PO3-GGA GAC CTC (SEQ ID NO:868) |
| 4.87 | 5'-PO3-GCCAGACTT (SEQ ID NO:869) | 5'-PO3-GTC TGG CTC (SEQ ID NO:870) |
| 4.88 | 5'-PO3-GGTCTCATT (SEQ ID NO:871) | 5'-P03-TGA GAC CTC (SEQ ID NO:872) |
| 4.89 | 5'-PO3-GAGACCATT (SEQ ID NO:873) | 5'-PO3-TGG TCT CTC (SEQ ID NO:874) |
| 4.90 | 5'-PO3-CCTTCAGTT (SEQ ID NO:875) | 5'-PO3-CTG AAG GTC (SEQ ID NO:876) |
| 4.91 | 5'-PO3-GCACCTGTT (SEQ ID NO:877) | 5'-PO3-CAG GTG CTC (SEQ ID NO:878) |
| 4.92 | 5'-PO3-AAAGGCGTT (SEQ ID NO:879) | 5'-PO3-GGC CTT TTC (SEQ ID NO:880) |
| 4.93 | 5'-PO3-CAGATCGTT (SEQ ID NO:881) | 5'-PO3-CGA TCT GTC (SEQ ID NO:882) |
| 4.94 | 5'-PO3-CATAGGCTT (SEQ ID NO:883) | 5'-P03-GCC TAT GTC (SEQ ID NO:884) |
| 4.95 | 5'-PO3-CCTTCACTT (SEQ ID NO:885) | 5'-PO3-GTG AAG GTC (SEQ ID NO:886) |
| 4.96 | 5'-PO3-GCACCTCTT (SEQ ID NO:887) | 5'-PO3-GAG GTG CTC (SEQ ID NO:888) |
| | | |

**Table 7: Correspondence between building blocks and oligonucleotide tags for Cycles 1-4.**

| Building block | Cycle 1 | Cycle 2 | Cycle 3 | Cycle 4 |
|---|---|---|---|---|
| BB1 | 1.1 | 2.1 | 3.1 | 4.1 |
| BB2 | 1.2 | 2.2 | 3.2 | 4.2 |
| BB3 | 1.3 | 2.3 | 3.3 | 4.3 |
| BB4 | 1.4 | 2.4 | 3.4 | 4.4 |
| BB5 | 1.5 | 2.5 | 3.5 | 4.5 |
| BB6 | 1.6 | 2.6 | 3.6 | 4.6 |
| BB7 | 1.7 | 2.7 | 3.7 | 4.7 |
| BB8 | 1.8 | 2.8 | 3.8 | 4.8 |
| BB9 | 1.9 | 2.9 | 3.9 | 4.9 |
| BB10 | 1.10 | 2.10 | 3.10 | 4.10 |
| BB11 | 1.11 | 2.11 | 3.11 | 4.11 |
| BB12 | 1.12 | 2.12 | 3.12 | 4.12 |
| BB13 | 1.13 | 2.13 | 3.13 | 4.13 |
| BB14 | 1.14 | 2.14 | 3.14 | 4.14 |
| BB15 | 1.15 | 2.15 | 3.15 | 4.15 |
| BB16 | 1.16 | 2.16 | 3.16 | 4.16 |
| BB17 | 1.17 | 2.17 | 3.17 | 4.17 |
| BB18 | 1.18 | 2.18 | 3.18 | 4.18 |
| BB19 | 1.19 | 2.19 | 3.19 | 4.19 |
| BB20 | 1.20 | 2.20 | 3.20 | 4.20 |
| BB21 | 1.21 | 2.21 | 3.21 | 4.21 |
| BB22 | 1.22 | 2.22 | 3.22 | 4.22 |
| BB23 | 1.23 | 2.23 | 3.23 | 4.23 |
| BB24 | 1.24 | 2.24 | 3.24 | 4.24 |
| BB25 | 1.25 | 2.25 | 3.25 | 4.25 |
| BB26 | 1.26 | 2.26 | 3.26 | 4.26 |
| BB27 | 1.27 | 2.27 | 3.27 | 4.27 |
| BB28 | 1.28 | 2.28 | 3.28 | 4.28 |
| BB29 | 1.29 | 2.29 | 3.29 | 4.29 |
| BB30 | 1.30 | 2.30 | 3.30 | 4.30 |
| BB31 | 1.31 | 2.31 | 3.31 | 4.31 |
| BB32 | 1.32 | 2.32 | 3.32 | 4.32 |
| BB33 | 1.33 | 2.33 | 3.33 | 4.33 |
| BB34 | 1.34 | 2.34 | 3.34 | 4.34 |
| BB35 | 1.35 | 2.35 | 3.35 | 4.35 |
| BB36 | 1.36 | 2.36 | 3.36 | 4.36 |
| BB37 | 1.37 | 2.37 | 3.37 | 4.37 |
| BB38 | 1.38 | 2.38 | 3.38 | 4.38 |
| BB39 | 1.39 | 2.39 | 3.39 | 4.39 |
| BB40 | 1.44 | 2.44 | 3.44 | 4.44 |
| BB41 | 1.41 | 2.41 | 3.41 | 4.41 |
| BB42 | 1.42 | 2.42 | 3.42 | 4.42 |
| BB43 | 1.43 | 2.43 | 3.43 | 4.43 |
| BB44 | 1.40 | 2.40 | 3.40 | 4.40 |
| BB45 | 1.45 | 2.45 | 3.45 | 4.45 |
| BB46 | 1.46 | 2.46 | 3.46 | 4.46 |
| BB47 | 1.47 | 2.47 | 3.47 | 4.47 |
| BB48 | 1.48 | 2.48 | 3.48 | 4.48 |
| BB49 | 1.49 | 2.49 | 3.49 | 4.49 |
| BB50 | 1.50 | 2.50 | 3.50 | 4.50 |
| BB51 | 1.51 | 2.51 | 3.51 | 4.51 |
| BB52 | 1.52 | 2.52 | 3.52 | 4.52 |
| BB53 | 1.53 | 2.53 | 3.53 | 4.53 |
| BB54 | 1.54 | 2.54 | 3.54 | 4.54 |
| BB55 | 1.55 | 2.55 | 3.55 | 4.55 |
| BB56 | 1.56 | 2.56 | 3.56 | 4.56 |
| BB57 | 1.57 | 2.57 | 3.57 | 4.57 |
| BB58 | 1.58 | 2.58 | 3.58 | 4.58 |
| BB59 | 1.59 | 2.59 | 3.59 | 4.59 |
| BB60 | 1.60 | 2.60 | 3.60 | 4.60 |
| BB61 | 1.61 | 2.61 | 3.61 | 4.61 |
| BB62 | 1.62 | 2.62 | 3.62 | 4.62 |
| BB63 | 1.63 | 2.63 | 3.63 | 4.63 |
| BB64 | 1.64 | 2.64 | 3.64 | 4.64 |
| BB65 | 1.65 | 2.65 | 3.65 | 4.65 |
| BB66 | 1.66 | 2.66 | 3.66 | 4.66 |
| BB67 | 1.67 | 2.67 | 3.67 | 4.67 |
| BB68 | 1.68 | 2.68 | 3.68 | 4.68 |
| BB69 | 1.69 | 2.69 | 3.69 | 4.69 |
| BB70 | 1.70 | 2.70 | 3.70 | 4.70 |
| BB71 | 1.71 | 2.71 | 3.71 | 4.71 |
| BB72 | 1.72 | 2.72 | 3.72 | 4.72 |
| BB73 | 1.73 | 2.73 | 3.73 | 4.73 |
| BB74 | 1.74 | 2.74 | 3.74 | 4.74 |
| BB75 | 1.75 | 2.75 | 3.75 | 4.75 |
| BB76 | 1.76 | 2.76 | 3.76 | 4.76 |
| BB77 | 1.77 | 2.77 | 3.77 | 4.77 |
| BB78 | 1.78 | 2.78 | 3.78 | 4.78 |
| BB79 | 1.79 | 2.79 | 3.79 | 4.79 |
| BB80 | 1.80 | 2.80 | 3.80 | 4.80 |
| BB81 | 1.81 | 2.81 | 3.81 | 4.81 |
| BB82 | 1.82 | 2.82 | 3.82 | 4.82 |
| BB83 | 1.96 | 2.96 | 3.96 | 4.96 |
| BB84 | 1.83 | 2.83 | 3.83 | 4.83 |
| BB85 | 1.84 | 2.84 | 3.84 | 4.84 |
| BB86 | 1.85 | 2.85 | 3.85 | 4.85 |
| BB87 | 1.86 | 2.86 | 3.86 | 4.86 |
| BB88 | 1.87 | 2.87 | 3.87 | 4.87 |
| BB89 | 1.88 | 2.88 | 3.88 | 4.88 |
| BB90 | 1.89 | 2.89 | 3.89 | 4.89 |
| BB91 | 1.90 | 2.90 | 3.90 | 4.90 |
| BB92 | 1.91 | 2.91 | 3.91 | 4.91 |
| BB93 | 1.92 | 2.92 | 3.92 | 4.92 |
| BB94 | 1.93 | 2.93 | 3.93 | 4.93 |
| BB95 | 1.94 | 2.94 | 3.94 | 4.94 |
| BB96 | 1.95 | 2.95 | 3.95 | 4.95 |

1X ligase buffer: 50 mM Tris, pH 7.5; 10 mM dithiothreitol; 10 mM MgCl₂; 2mM ATP; 50 mM NaCl.
10X ligase buffer: 500 mM Tris, pH 7.5; 100 mM dithiothreitol; 100 mM MgCl₂; 20 mM ATP; 500 mM NaCl

### Attachment of Water Soluble Spacer to Compound 2

To a solution of Compound 2 (60 mL, 1 mM) in sodium borate buffer (150 mM, pH 9.4) that was chilled to 4 °C was added 40 equivalents of N-Fmoc-15-amino-4,7,10,13-tetraoxaoctadecanoic acid (S-Ado) in N,N-dimethylformamide (DMF) (16 mL, 0.15 M) followed by 40 equivalents of 4-(4,6-dimethoxy[1.3.5]triazin-2-yl)-4-methylmorpholinium chloride hydrate (DMTMM) in water (9.6 mL, 0.25 M). The mixture was gently shaken for 2 hours at 4 °C before an additional 40 equivalents of S-Ado and DMTMM were added and shaken for a further 16 hours at 4 °C.

Following acylation, a 0.1X volume of 5 M aqueous NaCl and a 2.5X volume of cold (-20 °C) ethanol was added and the mixture was allowed to stand at -20 °C for at least one hour. The mixture was then centrifuge for 15 minutes at 14,000 rpm in a 4 °C centrifuge to give a white pellet which was washed with cold EtOH and then dried in a lyophilizer at room temperature for 30 minutes. The solid was dissolved in 40 mL of water and purified by Reverse Phase HPLC with a Waters Xterra RP₁₈ column. A binary mobile phase gradient profile was used to elute the product using a 50 mM aqueous triethylammonium acetate buffer at pH 7.5 and 99% acetontrile/1% water solution. The purified material was concentrated by lyophilization and the resulting residue was dissolved in 5 mL of water. A 0.1X volume of piperidine was added to the solution and the mixture was gently shaken for 45 minutes at room temperature. The product was then purified by ethanol precipitation as described above and isolated by centrifugation. The resulting pellet was washed twice with cold EtOH and dried by lyophilization to give purified Compound 3.

### Cycle 1

To each well in a 96 well plate was added 12.5 µL of a 4 mM solution of Compound 3 in water; 100 µL of a 1 mM solution of one of oligonucleotide tags 1.1 to 1.96, as shown in Table 3 (the molar ratio of Compound 3 to tags was 1:2). The plates were heated to 95°C for 1 minute and then cooled to 16°C over 10 minutes. To each well was added 10 µL of 10X ligase buffer, 30 units T4 DNA ligase (1 µL of a 30 unit/µL solution (FermentasLife Science, Cat. No. EL0013)), 76.5 µl of water and the resulting solutions were incubated at 16 °C for 16 hours.

After the ligation reaction, 20 µL of 5 M aqueous NaCl was added directly to each well, followed by 500 µL cold (-20 °C) ethanol, and held at -20 °C for 1 hour. The plates were centrifugated for 1 hour at 3200g in a Beckman Coulter Allegra 6R centrifuge using Beckman Microplus Carriers. The supernatant was carefully removed by inverting the plate and the pellet was washed with 70% aqueous cold ethanol at -20 °C. Each of the pellets was then dissolved in sodium borate buffer (50 µL, 150 mM, pH 9.4) to a concentration of 1 mM and chilled to 4 °C.

To each solution was added 40 equivalents of one of the 96 building block precursors in DMF (13 µL, 0.15 M) followed by 40 equivalents of DMT-MM in water (8 µL, 0.25M), and the solutions were gently shaken at 4°C. After 2 hours, an additional 40 equivalents of one of each building block precursor and DMTMM were added and the solutions were gently shaken for 16 hours at 4 °C. Following acylation, 10 equivalents of acetic acid-N-hydroxy-succinimide ester in DMF (2 µL, 0.25M) was added to each solution and gently shaken for 10 minutes.

Following acylation, the 96 reaction mixtures were pooled and 0.1 volume of 5M aqueous NaCl and 2.5 volumes of cold absolute ethanol were added and the solution was allowed to stand at -20 °C for at least one hour. The mixture was then centrifuged. Following centrifugation, as much supernatant as possible was removed with a micropipette, the pellet was washed with cold ethanol and centrifuged again. The supernatant was removed with a 200 µL, pipet. Cold 70% ethanol was added to the tube, and the resulting mixture was centrifuged for 5 min at 4°C.

The supernatant was removed and the remaining ethanol was removed by lyophilization at room temperature for 10 minutes. The pellet was then dissolved in 2 mL of water and purified by Reverse Phase HPLC with a Waters Xterra RP₁₈ column. A binary mobile phase gradient profile was used to elute the library using a 50 mM aqueous triethylammonium acetate buffer at pH 7.5 and 99% acetontrile/1% water solution. The fractions containing the library were collected, pooled, and lyophilized. The resulting residue was dissolved in 2.5 mL of water and 250 µL of piperidine was added. The solution was shaken gently for 45 minutes and then precipitated with ethanol as previously described. The resulting pellet was dried by lyophilization and then dissolved in sodium borate buffer (4.8 mL, 150 mM, pH 9.4) to a concentration of 1 mM.

The solution was chilled to 4 °C and 40 equivalents each of N-Fmoc-propargylglycine in DMF (1.2 mL, 0.15 M) and DMT-MM in water (7.7 mL, 0.25 M) were added. The mixture was gently shaken for 2 hours at 4 °C before an additional 40 equivalents of N-Fmoc-propargylglycine and DMT-MM were added and the solution was shaken for a further 16 hours. The mixture was later purified by EtOH precipitation and Reverse Phase HPLC as described above and the N-Fmoc group was removed by treatment with piperidine as previously described. Upon final purification by EtOH precipitation, the resulting pellet was dried by lyophilization and carried into the next cycle of synthesis

### Cycles 2-4

For each of these cycles, the dried pellet from the previous cycle was dissolved in water and the concentration of library was determined by spectrophotometry based on the extinction coefficient of the DNA component of the library, where the initial extinction coefficient of Compound 2 is 131,500 L/(mole.cm). The concentration of the library was adjusted with water such that the final concentration in the subsequent ligation reactions was 0.25 mM. The library was then divided into 96 equal aliquots in a 96 well plate. To each well was added a solution comprising a different tag (molar ratio of the library to tag was 1:2), and ligations were performed as described for Cycle 1. Oligonucleotide tags used in Cycles 2, 3 and 4 are set forth in Tables 4, 5 and 6, respectively. Correspondense between the tags and the building block precursors for each of Cycles 1 to 4 is provided in Table 7. The library was precipitated by the addition of ethanol as described above for Cycle 1, and dissolved in sodium borate buffer (150 mM, pH 9.4) to a concentration of 1 mM. Subsequent acylations and purifications were performed as described for Cycle 1, except HPLC purification was omitted during Cycle 3.

The products of Cycle 4 were ligated with the closing primer shown below, using the method described above for ligation of tags.
5'-PO₃-CAG AAG ACA GAC AAG CTT CAC CTG C
   (SEQ ID NO: 889)
5'-PO₃-GCA GGT GAA GCT TGT CTG TCT TCT GAA
   (SEQ ID NO:890)

### Results:

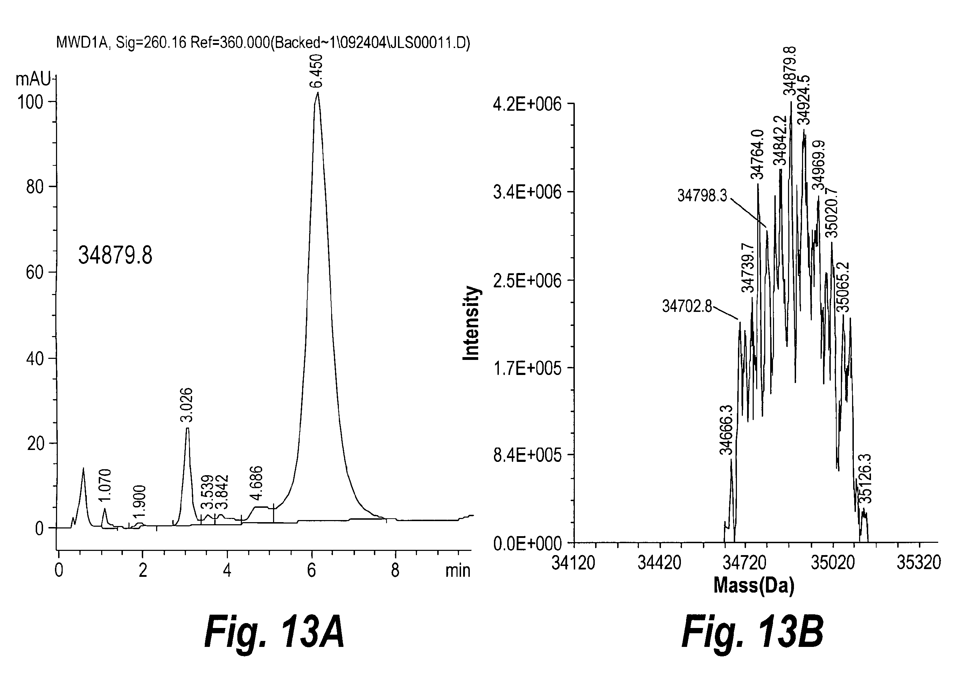

The synthetic procedure described above has the capability of producing a library comprising 96⁴ (about 10⁸) different structures. The synthesis of the library was monitored via gel electrophoresis and LC/MS of the product of each cycle. Upon completion, the library was analyzed using several techniques. Figure 13a is a chromatogram of the library following Cycle 4, but before ligation of the closing primer, Figure 13b is a mass spectrum of the library at the same synthetic stage. The average molecular weight was determined by negative ion LC/MS analysis. The ion signal was deconvoluted using ProMass software. This result is consistent with the predicted average mass of the library.

The DNA component of the library was analyzed by agarose gel electrophoresis, which showed that the majority of library material corresponds to ligated product of the correct size. DNA sequence analysis of molecular clones of PCR product derived from a sampling of the library shows that DNA ligation occurred with high fidelity and to near completion.

### Library cyclization

At the completion of Cycle 4, a portion of the library was capped at the N-terminus using azidoacetic acid under the usual acylation conditions. The product, after purification by EtOH precipitation, was dissolved in sodium phosphate buffer (150 mM, pH 8) to a concentration of 1 mM and 4 equivalents each of CuSO₄ in water (200 mM), ascorbic acid in water (200 mM), and a catalytic amount of the compound shown below as a solution in DMF (200 mM) were added. The reaction mixture was then gently shaken for 2 hours at room temperature.

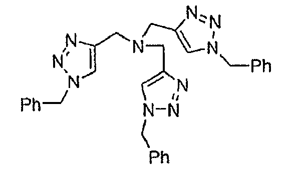

To assay the extent of cyclization, 5 µL aliquots from the library cyclization reaction were removed and treated with a fluorescently-labeled azide or alkyne (1µL of 100 mM DMF stocks) prepared as described in Example 4. After 16 hours, neither the alkyne or azide labels had been incorporated into the library by HPLC analysis at 500 nm. This result indicated that the library no longer contained azide or alkyne groups capable of cycloaddition and that the library must therefore have reacted with itself, either through cyclization or intermolecular reactions. The cyclized library was purified by Reverse Phase HPLC as previously described. Control experiments using uncyclized library showed complete incorporation of the fluorescent tags mentioned above.

### Example 4: Preparation of Fluorescent Tags for Cyclization Assay:

In separate tubes, propargyl glycine or 2-amino-3-phenylpropylazide (8 µmol each) was combined with FAM-OSu (Molecular Probes Inc.) (1.2 equiv.) in pH 9.4 borate buffer (250 µL). The reactions were allowed to proceed for 3 h at room temperature, and were then lyophilized overnight. Purification by HPLC afforded the desired fluorescent alkyne and azide in quantitative yield.

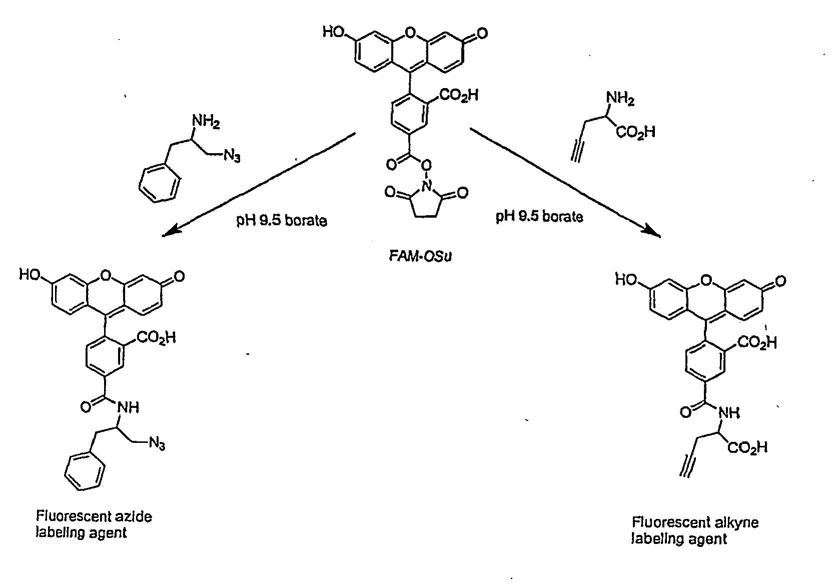

### Example 5: Cyclization of individual compounds using the azide/alkyne cycloaddition reaction

### Preparation of Azidoacetyl-Gly-Pro-Phe-Pra-NH₂:

Using 0.3 mmol of Rink-amide resin, the indicated sequence was synthesized using standard solid phase synthesis techniques with Fmoc-protected amino acids and HATU as activating agent (Pra = C-propargylglycine). Azidoacetic acid was used to cap the tetrapeptide. The peptide was cleaved from the resin with 20% TFA/DCM for 4 h. Purification by RP HPLC afforded product as a white solid (75 mg, 51 %). ¹H NMR (DMSO-d₆, 400 MHz): 8.4 - 7.8 (m, 3H), 7.4 - 7.1 (m, 7 H), 4.6 - 4.4 (m, 1H), 4.4 - 4.2 (m, 2H), 4.0 - 3.9 (m, 2H), 3.74 (dd, 1H, J = 6 Hz, 17 Hz), 3.5 - 3.3 (m, 2H), 3.07 (dt, 1H, J = 5 Hz, 14 Hz), 2.92 (dd, 1H, J = 5 Hz, 16 Hz), 2.86 (t, 1H, J = 2 Hz), 2.85 - 2.75 (m, 1H), 2.6 - 2.4 (m, 2H), 2.2 -1.6 (m, 4H). IR (mull) 2900, 2100, 1450, 1300 cm⁻¹. ESIMS 497.4 ([M+H], 100%), 993.4 ([2M+H], 50%). ESIMS with ion-source fragmentation: 519.3 ([M+Na], 100%), 491.3 (100%), 480.1 ([M-NH₂], 90%), 452.2 ([M-NH₂-CO], 20%), 424.2 (20%), 385.1 ([M-Pra], 50%), 357.1 ([M-Pra-CO], 40%), 238.0 ([M-Pra-Phe], 100%).

### Cyclization of Azidoacetyl-Gly-Pro-Phe-Pra-NH₂:

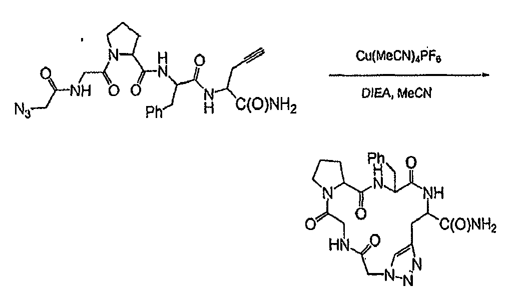

The azidoacetyl peptide (31 mg, 0.62 mmol) was dissolved in MeCN (30 mL). Diisopropylethylamine (DIEA, 1 mL) and Cu(MeCN)₄PF₆ (1 mg) were added. After stirring for 1.5 h, the solution was evaporated and the resulting residue was taken up in 20% MeCN/H₂O. After centrifugation to remove insoluble salts, the solution was subjected to preparative reverse phase HPLC. The desired cyclic peptide was isolated as a white solid (10 mg, 32%). ¹H NMR (DMSO-d₆, 400 MHz): 8.28 (t, 1H, J = 5 Hz), 7.77 (s, 1H), 7.2 - 6.9 (m, 9H), 4.98 (m, 2H), 4.48 (m, 1H), 4.28 (m, 1H), 4.1 - 3.9 (m, 2H), 3.63 (dd, 1H, J = 5 Hz, 16 Hz), 3.33 (m, 2H), 3.0 (m, 3H), 2.48 (dd, 1H, J = 11 Hz, 14 Hz), 1.75 (m, 1H0, 1.55 (m, 1H), 1.32 (m, 1H), 1.05 (m, 1H). IR (mull) 2900,1475,1400 cm⁻¹. ESIMS 497.2 ([M+H], 100%), 993.2 ([2M+H], 30%), 1015.2 ([2M+Na], 15%). ESIMS with ion-source fragmentation: 535.2 (70%), 519.3 ([M+Na], 100%), 497.2 ([M+H], 80%), 480.1 ([M-NH₂], 30%), 452.2 ([M-NH₂-CO], 40%), 208.1 (60%).

### Preparation of Azidoacetyl-Gly-Pro-Phe-Pra-Gly-OH:

Using 0.3 mmol of Glycine-Wang resin, the indicated sequence was synthesized using Fmoc-protected amino acids and HATU as the activating agent. Azidoacetic acid was used in the last coupling step to cap the pentapeptide. Cleavage of the peptide was achieved using 50% TFA/DCM for 2 h. Purification by RP HPLC afforded the peptide as a white solid (83 mg; 50%). ¹H NMR (DMSO-d₆, 400 MHz): 8.4 - 7.9 (m, 4H), 7.2 (m, 5H), 4.7 - 4.2 (m, 3H), 4.0 - 3.7 (m, 4H), 3.5 - 3.3 (m, 2H), 3.1 (m, 1H), 2.91 (dd, 1H, J = 4 Hz, 16 Hz), 2.84 (t, 1H, J = 2.5 Hz), 2.78 (m,1H), 2.6 - 2.4 (m, 2H), 2.2 - 1.6 (m, 4H). IR (mull) 2900, 2100, 1450, 1350 cm⁻¹. ESIMS 555.3 ([M+H], 100%). ESIMS with ion-source fragmentation: 577.1 ([M+Na], 90%), 555.3 ([M+H], 80%), 480.1 ([M-Gly], 100%), 385.1 ([M-Gly-Pra], 70%), 357.1 ([M-Gly-Pra-CO], 40%), 238.0 ([M-Gly-Pra-Phe], 80%).

### Cyclization of Azidoacetyl-Gly-Pro-Phe-Pra-Gly-OH:

The peptide (32 mg, 0.058 mmol) was dissolved in MeCN (60 mL). Diisopropylethylamine (1 mL) and Cu(MeCN)₄PF₆ (1 mg) were added and the solution was stirred for 2 h. The solvent was evaporated and the crude product was subjected to RP HPLC to remove dimers and trimers. The cyclic monomer was isolated as a colorless glass (6 mg, 20%). ESIMS 555.6 ([M+H], 100%), 1109.3 ([2M+H], 20%), 1131.2 ([2M+Na], 15%).
ESIMS with ion source fragmentation: 555.3 ([M+H], 100%), 480.4 ([M-Gly], 30%), 452.2 ([M-Gly-CO], 25%), 424.5 ([M-Gly-2CO], 10%, only possible in a cyclic structure).

### Conjugation of Linear Peptide to DNA:

Compound 2 (45 nmol) was dissolved in 45 µL sodium borate buffer (pH 9.4; 150 mM). At 4° C, linear peptide (18 µL of a 100 mM stock in DMF; 180 nmol; 40 equiv.) was added, followed by DMT-MM (3.6 µL of a 500 mM stock in water; 180 nmol; 40 equiv.). After agitating for 2 h, LCMS showed complete reaction, and product was isolated by ethanol precipitation. ESIMS 1823.0 ([M-3H]/3, 20%), 1367.2 ([M-4H]/4,20%), 1093.7 ([M-5H]/5, 40%), 911.4 ([M-6H]16, 100%).

### Conjugation of Cyclic Peptide to DNA:

Compound 2 (20 nmol) was dissolved in 20 µL sodium borate buffer (pH 9.4, 150 mM). At 4° C, linear peptide (8 µL of a 100 mM stock in DMF; 80 nmol; 40 equiv.) was added, followed by DMT-MM (1.6 µL of a 500 mM stock in water; 80 nmol; 40 equiv.). After agitating for 2 h, LCMS showed complete reaction, and product was isolated by ethanol precipitation. ESIMS 1823.0 ([M-3H]/3, 20%), 1367.2 ([M-4H]/4, 20%), 1093.7 ([M-5H]/5, 40%), 911.4 ([M-6H]/6, 100%).

### Cyclization of DNA-Linked Peptide:

Linear peptide-DNA conjugate (10 nmol) was dissolved in pH 8 sodium phosphate buffer (10 µL, 150mm). At room temperature, 4 equivalents each of CuSO₄, ascorbic acid, and the Sharpless ligand were all added (0.2 µL of 200 mM stocks). The reaction was allowed to proceed overnight. RP HPLC showed that no linear peptide-DNA was present, and that the product co-eluted with authentic cyclic peptide-DNA. No traces of dimers or other oligomers were observed.

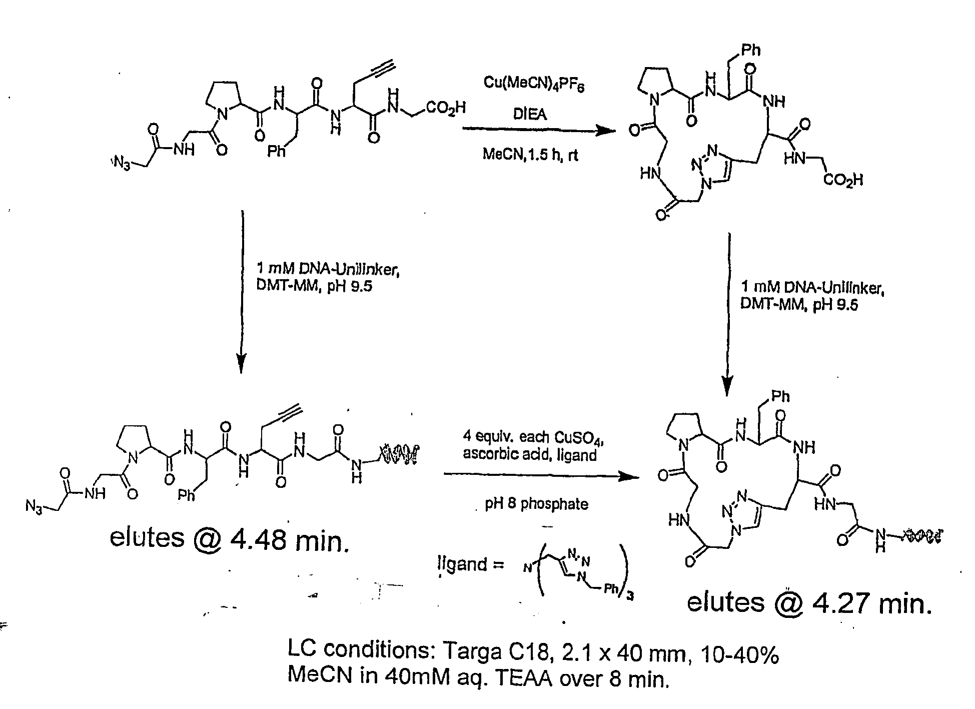

### Example 6: Application of Aromatic Nucleophile Substitution Reactions to Functional Moiety Synthesis

### General Procedure for Arylation of Compound 3 with Cyanuric Chloride:

Compound 2 is dissolved in pH 9.4 sodium borate buffer at a concentration of 1 mM. The solution is cooled to 4° C and 20 equivalents of cyanuric chloride is then added as a 500 mM solution in MeCN. After 2h, complete reaction is confirmed by LCMS and the resulting dichlorotriazine-DNA conjugate is isolated by ethanol precipitation.

### Procedure for Amine Substitution of Dichlorotriazine-DNA:

The dichlorotriazine-DNA conjugate is dissolved in pH 9.5 borate buffer at a concentration of 1 mM. At room temperature, 40 equivalents of an aliphatic amine is added as a DMF solution. The reaction is followed by LCMS and is usually complete after 2 h. The resulting alkylamino-monochlorotriazine-DNA conjugate is isolated by ethanol precipitation.

### Procedure for Amine Substitution of Monochlorotriazine-DNA:

The alkylamino-monochlorotriazine-DNA conjugate is dissolved in pH 9.5 borate buffer at a concentration of 1 mM. At 42° C, 40 equivalents of a second aliphatic amine is added as a DMF solution. The reaction is followed by LCMS and is usually complete after 2 h. The resulting diaminotriazine-DNA conjugate is isolated by ethanol precipitation.

### Example 7: Application of Reductive Animation Reactions to Functional Moiety Synthesis

### General Procedure for Reductive Amination of DNA-Linker Containing a Secondary Amine with an Aldehyde Building Block:

Compound 2 was coupled to an N-terminal proline residue. The resulting compound was dissolved in sodium phosphate buffer (50 µL, 150 mM, pH 5.5) at a concentration of 1 mM. To this solution was added 40 equivalents each of an aldehyde building block in DMF (8 µL, 0.25M) and sodium cyanoborohydride in DMF (8 µL, 0.25M) and the solution was heated at 80°C for 2 hours. Following alkylation, the solution was purified by ethanol precipitation.

### General Procedure for Reductive Aminations of DNA-Linker Containing an Aldehyde with Amine Building Blocks:

Compound 2 coupled to a building block comprising an aldehyde group was dissolved in sodium phosphate buffer (50 µL, 250 mM, pH 5.5) at a concentration of 1 mM. To this solution was added 40 equivalents each of an amine building block in DMF (8 µL, 0.25M and sodium cyanoborohydride in DMF (8 µL, 0.25M) and the solution was heated at 80 °C for 2 hours. Following alkylation, the solution was purified by ethanol precipitation.

### Example 8: Application of Peptoid Building Reactions to Functional Moiety Synthesis

### General Procedure for Peptoid Synthesis on DNA-Linker:

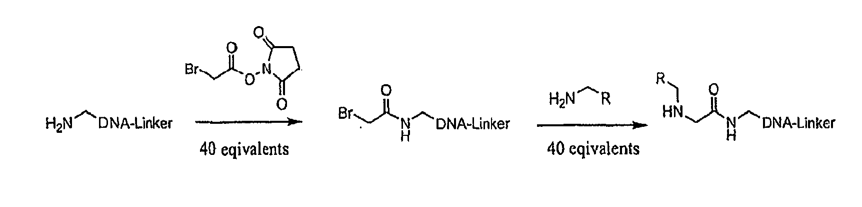

Compound 2 was dissolved in sodium borate buffer (50 µL, 150 mM, pH 9.4) at a concentration of 1 mM and chilled to 4 °C. To this solution was added 40 equivalents of N-hydroxysuccinimidyl bromoacetate in DMF (13 µL, 0.15 M) and the solution was gently shaken at 4 °C for 2 hours. Following acylation, the DNA-Linker was purified by ethanol precipitation and redissolved in sodium borate buffer (50 µL, 150 mM, pH 9.4) at a concentration of 1mM and chilled to 4°C. To this solution was added 40 eqivalents of an amine building block in DMF (13 µL, 0.15 M) and the solution was gently shaken at 4 °C for 16 hours. Following alkylation, the DNA-linker was purified by ethanol precipitation and redissolved in sodium borate buffer (50 µL, 150 mM, pH 9.4) at a concentration of 1 mM and chilled to 4°C. Peptoid synthesis is continued by the stepwise addition of N-hydroxysuccinimidyl bromoacetate followed by the addition of an amine building block.

### Example 9: Application of the Azide-Alkyne Cycloaddition Reaction to Functional Moiety Synthesis

### General procedure

An alleyne-containing DNA conjugate is dissolved in pH 8.0 phosphate buffer at a concentration of ca. 1mM. To this mixture is added 10 equivalents of an organic azide and 5 equivalents each of copper (II) sulfate, ascorbic acid, and the ligand (tris-((1-benzyltriazol-4-yl)methyl)amine all at room temperature. The reaction is followed by LCMS, and is usually complete after 1- 2 h. The resulting triazole-DNA conjugate can be isolated by ethanol precipitation.

### Example 10 Identification of a ligand to Abl kinase from within an encoded library

The ability to enrich molecules of interest in a DNA-encoded library above undesirable library members is paramount to identifying single compounds with defined properties against therapeutic targets of interest. To demonstrate this enrichment ability a known binding molecule (described by Shah et al., Science 305, 399-401 (2004), incorporated herein by reference) to rhAb1 kinase (GenBank U07563) was synthesized. This compound was attached to a double stranded DNA oligonucleotide via the linker described in the preceding examples using standard chemistry methods to produce a molecule similar (functional moiety linked to an oligonucleotide) to those produced via the methods described in Examples 1 and 2. A library generally produced as described in Example 2 and the DNA-linked Abl kinase binder were designed with unique DNA sequences that allowed qPCR analysis of both species. The DNA-linked Abl kinase binder was mixed with the library at a ratio of 1:1000. This mixture was equilibrated with to rhAble kinase, and the enzyme was captured on a solid phase, washed to remove non-binding library members and binding molecules were eluted. The ratio of library molecules to the DNA-linked Abl kinase inhibitor in the eluate was 1:1, indicating a greater than 500-fold enrichment of the DNA-linked Abl-kinase binder in a 1000-fold excess of library molecules.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. A method of synthesizing a molecule comprising a functional moiety which is operatively linked to an encoding oligonucleotide, said method comprising the steps of:
(a) providing an initiator compound consisting of an initial functional moiety comprising n building blocks, where n is an integer of 1 or greater, wherein the initial functional moiety comprises at least one reactive group, and is operatively linked to an initial oligonucleotide;
(b) reacting the initiator compound with a building block comprising at least one complementary reactive group, wherein the at least one complementary reactive group is complementary to the reactive group of step (a), under conditions suitable for reaction of the complementary reactive group to form a covalent bond;
(c) reacting the initial oligonucleotide with an incoming oligonucleotide which identifies the building block of step (b) in the presence of an enzyme which catalyzes ligation of the initial oligonucleotide and the incoming oligonucleotide, under conditions suitable for ligation of the incoming oligonucleotide and the initial oligonucleotide to form an encoding oligonucleotide;
thereby producing a molecule which comprises a functional moiety comprising n+1 building blocks which is operatively linked to an encoding oligonucleotide.

2. A method of synthesizing a library of compounds, wherein the compounds comprise a functional moiety comprising two or more building blocks which is operatively linked to an initial oligonucleotide which identifies the structure of the functional moiety, said method comprising the steps of
(a) providing a solution comprising m initiator compounds, wherein m is an integer of 1 or greater, where the initiator compounds consist of a functional moiety comprising n building blocks, where n is an integer of 1 or greater, which is operatively linked to an initial oligonucleotide which identifies the n building blocks;
(b) dividing the solution of step (a) into r reaction vessels, wherein r is an integer of 2 or greater, thereby producing r aliquots of the solution;
(c) reacting the initiator compounds in each reaction vessel with one of r building blocks, thereby producing r aliquots comprising compounds consisting of a functional moiety comprising n+1 building blocks operatively linked to the initial oligonucleotide; and
(d) reacting the initial oligonucleotide in each aliquot with one of a set of r distinct incoming oligonucleotides in the presence of an enzyme which catalyzes the ligation of the incoming oligonucleotide and the initial oligonucleotide, under conditions suitable for enzymatic ligation of the incoming oligonucleotide and the initial oligonucleotide;
thereby producing r aliquots comprising molecules consisting of a functional moiety comprising n+1 building blocks operatively linked to an elongated oligonucleotide which encodes the n+1 building blocks.

3. The method of Claim 1 wherein the functional moiety of step (c) comprises a reactive group, and steps (a) to (c) are repeated one or more times, thereby forming cycles 1 to i, where i is an integer of 2 or greater, wherein the product of step (c) of a cycles, where s is an integer of i-1 or less, is the initiator compound of cycle s + 1.

4. The method of Claim 1 wherein step (c) precedes step (b) or step (b) precedes step (c).

5. The method of Claim 1 wherein at least one of the building blocks is an amino acid or an activated amino acid.

6. The method of Claim 1, wherein the enzyme is selected from the group consisting of a DNA ligase, an RNA ligase, a DNA polymerase, an RNA polymerase and a topoisomerase.

7. The method of Claim 1 wherein the initial oligonucleotide is double-stranded or single stranded.

8. The method of Claim 7 wherein the initial oligonucleotide comprises a PCR primer sequence.

9. The method of claim 7 wherein the initial oligonucleotide is single-stranded and the incoming oligonucleotide is single-stranded; or the initial oligonucleotide is double-stranded and the incoming oligonucleotide is double-stranded, optionally wherein the initial functional moiety and the initial oligonucleotide are linked by a linking moiety, optionally wherein the initial oligonucleotide is double-stranded and the linking moiety is covalently coupled to the initial functional moiety and to both strands of the initial oligonucleotide.

10. The method of Claim 1 wherein the incoming oligonucleotide is from 3 to 10 nucleotides in length.

11. The method of claim 3, wherein the incoming oligonucleotide of cycle i comprises a PCR closing primer.

12. The method of claim 3, further comprising in cycle i, the step of
(d) ligating an oligonucleotide comprising a closing PCR primer sequence to the encoding oligonucleotide, optionally wherein the oligonucleotide comprising a closing PCR primer sequence is ligated to the encoding oligonucleotide in the presence of an enzyme which catalyzes said ligation.

13. The method of Claim 3, further comprising after cycle i, the step of
(e) cyclizing the functional moiety, optionally wherein the functional moiety comprises an alkynyl group and an azido group, and the compound is subjected to conditions suitable for cycloaddition of the alkynyl group and the azido group to form a triazole group, thereby cyclizing the functional moiety.

14. The method of Claim 2, further comprising the step of
(e) combining two or more of the r aliquots, thereby producing a solution comprising molecules consisting of a functional moiety comprising n + 1 building blocks, which is operatively linked to an elongated oligonucleotide which encodes the n+1 building blocks.

15. The method of claim 14 wherein r aliquots are combined.

16. The method of Claim 14 wherein the steps (a) to (e) are conducted one or more times to yield cycles 1 to i, where i is an integer of 2 or greater, wherein in cycle s+1, where s is an integer of i-1 or less, the solution comprising m initiator compounds of step (a) is the solution of step (e) of cycle s, optionally wherein in at least one of cycles 1 to i step (d) precedes step (c).

17. The method of of Claim 14 wherein at least one of building blocks is an amino acid.

18. The method of Claim 2, wherein the enzyme is DNA ligase, RNA ligase, DNA polymerase, RNA polymerase or topoisomerase.

19. The method of claim 14 wherein the initial oligonucleotide is a double-stranded oligonucleotide, optionally wherein the incoming oligonucleotide is a double-stranded oligonucleotide.

20. The method of Claim 14 wherein the initiator compounds comprise a linker moiety comprising a first functional group adapted to bond with a building block, a second functional group adapted to bond to the 5'end of an oligonucleotide, and a third functional group adapted to bond to the 3'-end of an oligonucleotide.

21. The method of Claim 20 wherein the linker moiety is of the structure 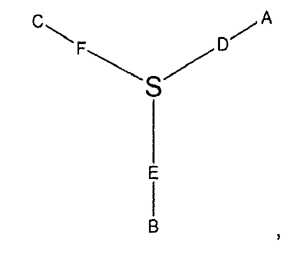 wherein
A is a functional group adapted to bond to a building block;
B is a functional group adapted to bond to the 5'-end of an oligonucleotide;
C is a functional group adapted to bond to the 3'-end of an oligonucleotide; S is an atom or a scaffold;
D is a chemical structure that connects A to S;
E is a chemical structure that connects B to S; and
F is a chemical structure that connects C to S, optionally
(i) wherein:
A is an amino group;
B is a phosphate group; and
C is a phosphate group, or
(ii) wherein D, E and F are each, independently, an alkylene group or an oligo(ethylene glycol) group, or
(iii) wherein S is a carbon atom, a nitrogen atom, a phosphorus atom, a boron atom, a phosphate group, a cyclic group or a polycyclic group, optionally wherein the linker moiety is of the structure 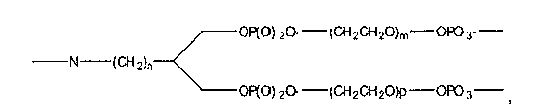 wherein each of n, m and p is, independently, an integer from 1 to about 20, optionally
(A) wherein each of n, m and p is independently an integer from 2 to eight, optionally wherein each of n, m and p is independently an integer from 3 to 6, or
(B) wherein the linker moiety has the structure

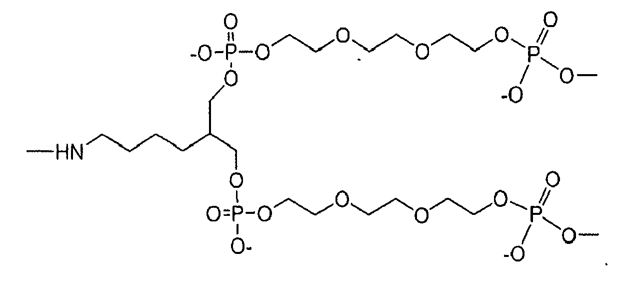

22. The method of claim 2, wherein each of said initiator compounds comprises a reactive group and wherein each of said r building blocks comprises a complementary reactive group which is complementary to said reactive group.

23. The method of claim 14, further comprising following cycle i, the step of:
(f) cyclizing one or more of the functional moieties, optionally wherein a functional moiety of step (f) comprises an azido group and an alkynyl group, optionally wherein the functional moiety is maintained under conditions suitable for cycloaddition of the azido group and the alkynyl group to form a triazole group, thereby forming a cyclic functional moiety, optionally wherein the cycloaddition reaction is conducted in the presence of a copper catalyst, optionally wherein at least one of the one or more functional moieties of step (f) comprises at least two sulfhydryl groups, and said functional moiety is maintained under conditions suitable for reaction of the two sulfhydryl groups to form a disulfide group, thereby cyclicizing the functional moiety.

24. The method of Claim 2 wherein the initial oligonucleotide comprises a PCR primer sequence.

25. The method of claim 14, wherein the incoming oligonucleotide of cycle i comprises a PCR closing primer.

26. The method of claim 14, further comprising following cycle i, the step of
(d) ligating an oligonucleotide comprising a closing PCR primer sequence to the encoding oligonucleotide, optionally wherein the oligonucleotide comprising a closing PCR primer sequence is ligated to the encoding oligonucleotide in the presence of an enzyme which catalyzes said ligation.

27. The method of Claim 1 or 22 wherein the reactive group and the complementary reactive group are selected from (i) the group consisting of an amino group; a carboxyl group; a sulfonyl group; a phosphonyl group; an epoxide group; an aziridine group; and an isocyanate group, or (ii) the group consisting of a hydroxyl group; a carboxyl group; a sulfonyl group; a phosphonyl group; an epoxide group, an aziridine group; and an isocyanate group, or (iii) the group consisting of an amino group and an aldehyde or ketone group, optionally wherein the reaction between the reactive group and the complementary reactive group is conducted under reducing conditions, or (iv) the group consisting of a phosphorous ylide group and an aldehyde or ketone group.

28. The method of Claim 1 or 22 wherein the reactive group and the complementary reactive group react via cycloaddition to form a cyclic structure, optionally wherein the reactive group and the complementary reactive group are selected from (i) the group consisting of an alkyne and an azide, or (ii) the group consisting of a halogenated heteroaromatic group and a nucleophile, optional wherein the halogenated heteroaromatic group is selected from the group consisting of chlorinated pyrimidines, chlorinated triazines and chlorinated purines, or wherein the nucleophile is an amino group.

29. A compound of the formula 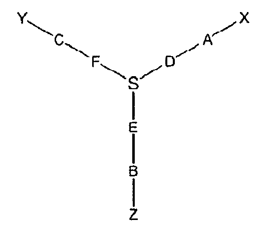 wherein:
X is a functional moiety comprising one or more building blocks;
Z is an oligonucleotide attached at its 3' terminus to B;
Y is an oligonucleotide which is attached at its 5' terminus to C;
A is a functional group that forms a covalent bond with X;
B is a functional group that forms a bond with the 3'-end of Z;
C is a functional group that forms a bond with the 5'-end of Y;
D, F and E are each, independently, a bifunctional linking group; and
S an atom or a molecular scaffold.

30. A compound library comprising at least about 10² distinct compounds, said compounds comprising a functional moiety comprising two or more building blocks which is operatively linked to an oligonucleotide which identifies the structure of the functional moiety.

31. The compound library of Claim 30, said library comprising at least about 10⁵ copies of each of the distinct compounds, or at least about 10⁶ copies of each of the distinct compounds.

32. The compound library of Claim 30 comprising at least about 10⁴ distinct compounds, or at least about 10⁶ distinct compounds, or at least about 10⁸ distinct compounds, or at least about 10¹⁰ distinct compounds, or at least about 10¹² distinct compounds.

33. The compound library of claim 30 wherein said library comprises a multiplicity of compounds which are independently of Formula I: 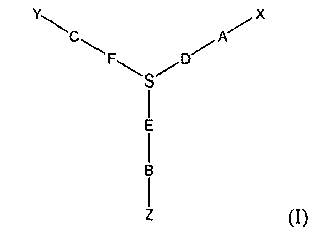 wherein:
X is a functional moiety comprising one or more building blocks;
Z is an oligonucleotide attached at its 3' terminus to B;
Y is an oligonucleotide which is attached at its 5' terminus to C;
A is a functional group that forms a covalent bond with X;
B is a functional group that forms a bond with the 3'-end of Z,;
C is a functional group that forms a bond with the 5'-end of Y;
D, F and E are each, independently, a bifunctional linking group; and
S an atom or a molecular scaffold.

34. The compound library of Claim 33 wherein A, B, C, D, E, F and S each have the same identify for each compound of Formula I.

35. The compound library of Claim 33, said library consisting essentially of a multiplicity of compounds of Formula I.

36. The compound of claim 29 or the compound library of claim 33 wherein D, E and F are each independently an alkylene chain or an oligo(ethylene glycol) chain.

37. The compound of Claim 29 or the compound library of claim 33, wherein (i) Y and Z arc substantially complementary and are oriented in the compound so as to enable Watson-Crick base pairing and duplex formation under suitable conditions, or (ii) Y and Z are the same length or different lengths, optionally wherein Y and Z are the same length, or (iii) Y and Z are each 10 or more bases in length and have complementary regions of ten or more base pairs.

38. The compound of Claim 29 or the compound library of claim 33, wherein S is a carbon atom, a boron atom, a nitrogen atom, a phosphorus atom, or a polyatomic scaffold, optionally wherein S is a phosphate group or a cyclic group, optionally wherein S is a cycloalkyl, cycloalkenyl, heterocycloalkyl, heterocycloalkenyl, aryl or heteroaryl group.

39. The compound of claim 29 or the compound library of claim 33 wherein the linker moiety is of the structure 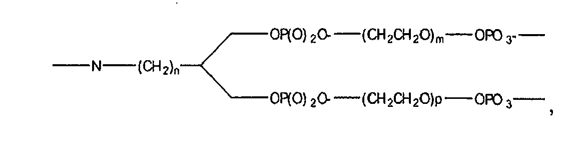 wherein each of n, m and p is, independently, an integer from 1 to about 20, optionally wherein each of n, m and p is independently an integer from 2 to eight, optionally wherein each of n, m and p is independently an integer from 3 to 6.

40. The compound of Claim 29 or the compound library of claim 33 wherein the linker moiety has the structure

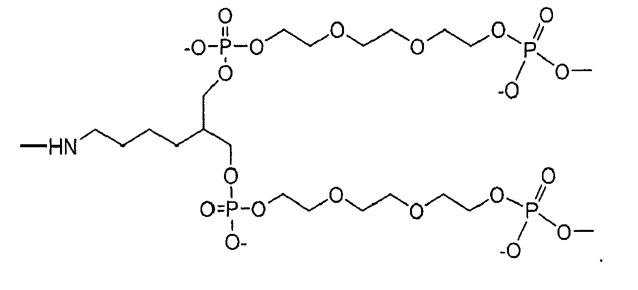

41. The compound of Claim 29 or the compound library of claim 33 wherein X and Y comprise a PCR primer sequence.

42. A compound prepared by the method of Claim 1.

43. A compound library prepared by the method of Claim 2.

44. A method for identifying one or more compounds which bind to a biological target, said method comprising the steps of:
(a) contacting the biological target with a compound library prepared by the method of Claim 2 under conditions suitable for at least one member of the compound library to bind to the target;
(b) removing library members that do not bind to the target;
(c) amplifying the encoding oligonucleotides of the at least one member of the compound library which binds to the target;
(d) sequencing the encoding oligonucleotides of step (c); and
(e) using the sequences determined in step (d) to determine the structure of the functional moieties of the members of the compound library which bind to the biological target;
thereby identifying one or more compounds which bind to the biological target.

45. A method for identifying a compound which binds to a biological target, said method comprising the steps of
(a) contacting the biological target with a compound library comprising at least about 10² distinct compounds, said compounds comprising a functional moiety comprising two or more building blocks which is operatively linked to an oligonucleotide which identifies the structure of the functional moiety under conditions suitable for at least one member of the compound library to bind to the target;
(b) removing library members that do not bind to the target;
(c) amplifying the encoding oligonucleotides of the at least one member of the compound library which binds to the target;
(d) sequencing the encoding oligonucleotides of step (c); and
(e) using the sequences determined in step (d) to determine the structure of the functional moieties of the members of the compound library which bind to the biological target;
thereby identifying one or more compounds which bind to the biological target.

46. The method of Claim 45 wherein the library comprises at least about 10⁵ copies of each of the distinct compounds, or at least about 10⁶ copies of each of the distinct compounds, or at least about 10⁴ distinct compounds, or at least about 10⁶ distinct compounds, or at least about 10⁸ distinct compounds, or at least about 10¹⁰ distinct compounds, or at least about 10¹² distinct compounds.

47. The method of claim 45 wherein the compound library comprises a multiplicity of compounds which are independently of Formula I: 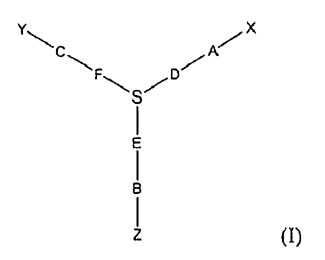 wherein:
X is a functional moiety comprising one or more building blocks;
Z is an oligonucleotide attached at its 3' terminus to B;
Y is an oligonucleotide which is attached at its 5' terminus to C;
A is a functional group that forms a covalent bond with X;
B is a functional group that forms a bond with the 3'-end of Z;
C is a functional group that forms a bond with the 5'-end of Y;
D, F and E are each, independently, a bifunctional linking group; and
S an atom or a molecular scaffold.

48. The method of Claim 47 wherein A, B, C, D, E, F and S each have the same identity for each compound of Formula I.

49. The method of Claim 47 wherein the compound library consists essentially of a multiplicity of compounds of Formula I.

50. The method of claim 47 wherein D, E and F are each independently an alkylene chain or an oligo(ethylene glycol) chain.

51. The method of Claim 47, wherein (i) Y and Z are substantially complementary and are oriented in the compound so as to enable Watson-Crick base pairing and duplex formation under suitable conditions, or (ii) Y and Z are the same length or different lengths, optionally wherein Y and Z are the same length, or (iii) Y and Z are each 10 or more bases in length and have complementary regions of ten or more base pairs.

52. The method of Claim 47, wherein S is a carbon atom, a boron atom, a nitrogen atom, a phosphorus atom, or a polyatomic scaffold, or wherein S is a phosphate group or a cyclic group, optionally wherein S is a cycloalkyl, cycloalkenyl, heterocycloalkyl, heterocycloalkenyl, aryl or heteroaryl group.

53. The method of claim 47 wherein the linker moiety is of the structure 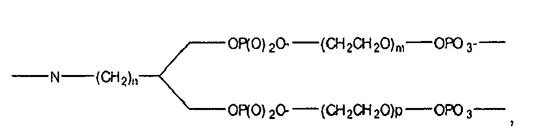 wherein each of n, m and p is, independently, an integer from 1 to about 20, optionally wherein each of n, m and p is independently an integer from 2 to eight, optionally wherein each of n, m and p is independently an integer from 3 to 6.

54. The method of Claim 53 wherein the linker moiety has the structure

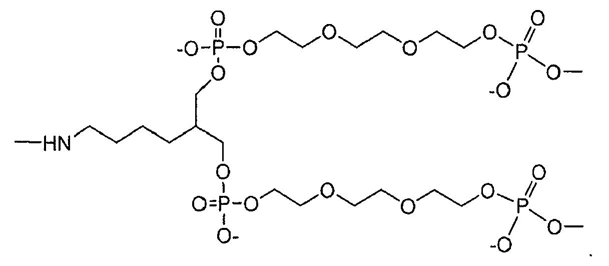

55. The method of claim 47 wherein X and Z comprise a PCR primer sequence.
